# EUROPÄISCHE PATENTANMELDUNG

(11) **EP 2 108 648 A1**
(43) Veröffentlichungstag der Anmeldung: **14.10.2009**
(21) Anmeldenummer: 09160781.2
(22) Anmeldetag: 23.03.2005
(51) Int. Cl.: C07D 401/04, C07D 401/14, A61K 31/551, A61P 25/06, C07D 401/12

(54) **Ausgewählte CGRP-Antagonisten, Verfahren zu deren Herstellung sowie deren Verwendung als Arzneimittel**

(62) Teilanmeldung aus: 05741783.4
(71) Anmelder: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: Die Erfindernennung liegt noch nicht vor
(74) Vertreter: Hammann, Heinz

(57) **Zusammenfassung**

Gegenstand der vorliegenden Erfindung sind die CGRP-Antagonisten der allgemeinen Formel 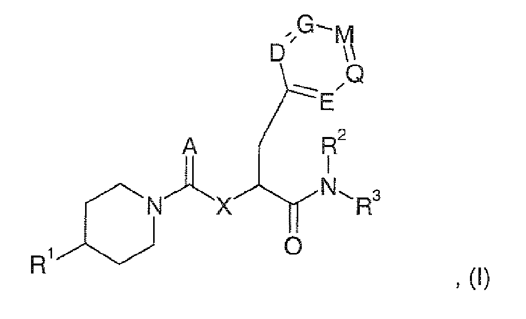 in der A, X, D, E, G, M, Q und R¹ bis R³ wie in Anspruch 1 definiert sind,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren, diese Verbindungen enthaltende Arzneimittel, deren Verwendung und Verfahren zu ihrer Herstellung.

## Beschreibung

Gegenstand der vorliegenden Erfindung sind die CGRP-Antagonisten der allgemeinen Formel 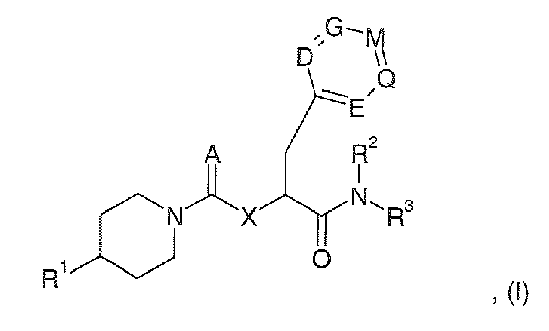 in denen A, D, E, G, M, Q, X, R¹, R² und R³ wie in Anspruch 1 definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

In der obigen allgemeinen Formel (I) bedeuten in einer ersten Ausführungsform
A ein Sauerstoff- oder Schwefelatom,
X ein Sauerstoff- oder Schwefelatom,
(a) D, E unabhängig voneinander jeweils eine Methingruppe oder das Stickstoffatom und
   G eine durch die Gruppe R^{a} substituierte Methingruppe,
   M eine durch die Gruppe R^{b} substituierte Methingruppe,
   Q eine durch die Gruppe R^{c} substituierte Methingruppe,
   wobei eine oder zwei der Gruppen G, M und Q jeweils auch ein Stickstoffatom bedeuten können,
   oder
(b) D und E jeweils eine Methingruppe, wobei eine der Gruppen D und E auch ein Stickstoffatom bedeuten kann, und
   G, M und Q jeweils ein Stickstoffatom,
wobei R^{a}, R^{b} und R^{c} unabhängig voneinander jeweils ein Wasserstoff- oder Halogenatom, eine C₁₋₆-Alkyl-, C₂₋₆-Alkenyl-, C₂₋₆-Alkinyl-, Cyclo-C₃₋₇-alkyl-, Cyclo-C₃₋₇-alkenyl-, Cyano-, Hydroxy-, Hydroxy-C₁₋₆-alkyl-, Hydroxy-C₃₋₈-alkenyl-, Hydroxy-C₃₋₈-alkinyl-, C₁₋₆-Alkoxy-, C₁₋₆-Alkoxy-C₁₋₆-alkyl, C₁₋₆-Alkoxy-C₃₋₆-alkenyl-, C₁₋₆-Alkoxy-C₃₋₆-alkinyl-, C₃₋₆-Alkenoxy-C₁₋₆-alkyl-, C₃₋₆-Alkenoxy-C₃₋₆-alkenyl-, C₃₋₆-Alkenoxy-C₃₋₆-alklnyl-, C₃₋₆-Alkinoxy-C₁₋₆-alkyl-, C₃₋₆-Alkinoxy-C₃₋₆-alkenyl-, C₃₋₆-Alkinoxy-C₃₋₆-alkinyl-, Thiohydroxy-, C₁₋₆-Alkylthio-, C₃₋₆-Alkenylthio-, C₃₋₆-Alkinylthio-, Amino-, C₁₋₆-Alkyl-amino-, C₃₋₆-Alkenyl-amino-, C₃₋₆-Alkinyl-amino-, Di-(C₁₋₆-alkyl)-amino-, Di-(C₃₋₆-alkenyl)-amino-, Di-(C₃₋₆-alkinyl)-amino-, Amino-C₁₋₆-alkyl-, C₁₋₃-Alkyl-amino-C₁₋₆-alkyl-, Di(C₁₋₃-Alkyl)-amino-C₁₋₆-alkyl-, Amino-C₃₋₆-alkenyl-, C₁₋₃-Alkyl-amino-C₃₋₆-alkenyl-, Di-(C₁₋₃-alkyl)-amino-C₃₋₆-alkenyl-, Amino-C₃₋₆-alkinyl-, C₁₋₃-Alkyl-amino-C₃₋₆-alkinyl-, Di-(C₁₋₃-alkyl)-amino-C₃₋₆-alkinyl-, Hydroxycarbonyl-, Phenylcarbonyl-, Pyridylcarbonyl-, C₁₋₆-Alkyl-carbonyl-, C₂₋₆-Alkenyl-carbonyl-, C₂₋₆-Alkinyl-carbonyl-, Formyl-, C₁₋₆-Alkoxy-carbonyl-, C₃₋₆-Alkenoxy-carbonyl-, C₃₋₆-Alklnoxy-carbonyl-, Aminocarbonyl-, C₁₋₆-Alkyl-aminocarbonyl-, C₃₋₆-Alkenyl-aminocarbonyl-, C₃₋₆-Alkinyl-aminocarbonyl-, Di-(C₁₋₆-alkyl)-aminocarbonyl-, Di-(C₃₋₆-alkenyl)-aminocarbonyl-, Di-(C₃₋₆-alkinyl)-aminocarbonyl-, Formylamino-, C₁₋₆-Alkyl-carbonylamino-, C₂₋₆-Alkenyl-carbonylamino-, C₂₋₆-Alkinyl-carbonylamino-, Formyl-C₁₋₆-alkyl-amino-, Formyl-C₃₋₆-alkenyl-amino-, Formyl-C₃₋₆-alkinyl-amino-, C₁₋₆-Alkyl-carbonyl-C₁₋₆-alkyl-amino-, C₂₋₆-Alkenyl-carbonyl-C₁₋₆-alkyl-amino-, C₂₋₆-Alkinyl-carbonyl-C₁₋₆-alkyl-amino-, C₁₋₆-Alkyl-carbonyl-C₃₋₆-alkenyl-amino-, C₂₋₆-Alkenyl-carbonyl-C₃₋₆-alkenyl-amino-, C₂₋₆-Alkinyl-carbonyl-C₃₋₆-alkenyl-amino-, C₁₋₆-Alkyl-carbonyl-C₃₋₆-alkinyl-amino-, C₂₋₆-Alkonyl-carbonyl-C₃₋₆-alkinyl-amino-, C₂₋₆-Alkinyl-carbonyl-C₃₋₆-alkinyl-amino-, C₁₋₆-Alkyl-sulfonyl-, C₂₋₆-Alkenyl-sulfonyl-, C₂₋₆-Alkinyl-sulfonyl-, C₁₋₆-Alkyl-sulfinyl-, C₂₋₆-Alkenyl-sulfinyl-, C₂₋₆-Alkinyl-sulfinyl-, C₁₋₆-Alkyl-sulfonylamino-, C₂₋₆-Alkenyl-sulfonylamino-, C₂₋₆-Alkinyl-sulfonylamino-, C₁₋₆-Alkyl-sulfonyl-C₁₋₆-alkylamino-, C₁₋₆-Alkyl-sulfonyl-C₃₋₆-alkenylamino-, C₁₋₆-Alkyl-sulfonyl-C₃₋₆-alkinylamino-, C₂₋₆-Alkenyl-sulfonyl-C₁₋₆-alkylamino-, C₂₋₆-Alkenyl-sulfonyl-C₃₋₆-alkenylamino-, C₂₋₆-Alkenyl-sulfonyl-C₃₋₆-alkinylamino-, C₂₋₆-Alkinyl-sulfonyl-C₁₋₆-alkylamino-, C₂₋₆-Alkinyl-sulfonyl-C₃₋₆-alkenylamino-, C₂₋₆-Alkinyl-sulfonyl-C₃₋₆-alkinylamino-, Aminosulfonyl-, C₁₋₆-Alkylaminosulfonyl-, Di-(C₁₋₆-alkyl)-aminosulfonyl-, C₃₋₆-Alkenylaminosulfonyl-, Di-(C₃₋₆-alkenyl)-aminosulfonyl-, C₃₋₆-Alkinylaminosulfonyl- oder Di-(C₃₋₆-alkinyl)-aminosulfonylgruppe bedeuten
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₆-Alkylgruppe darstellen,
(ii) R^{c} kein Wasserstoffatom bedeutet, wenn R^{a} sowie R^{b} jeweils eine C₁₋₆-Alkylgruppe darstellen,
(iii) R^{a} nicht die Bedeutungen eines Wasserstoff-, Fluor-, Chlor-, Brom- oder Idatoms oder einer Difluor- oder Trifluormethylgruppe annimmt, wenn R^{c} eine C₁₋₆-Alkyl-, C₂₋₆-Alkenyl- oder C₂₋₆-Alkinylgruppe und R^{b} ein Chlor- oder Bromatom, eine Amin-, Methylamino- oder Hydroxylgruppe darstellen,
(iv) R^{c} nicht die Bedeutungen eines Wasserstoff-, Fluor-, Chlor-, Brom- oder Iodatoms oder einer Difluor- oder Trifluormethylgruppe annimmt, wenn R^{a} eine C₁₋₆-Alkyl-, C₂₋₆-Alkenyl- oder C₂₋₆-Alkinylgruppe und R^{b} ein Chlor- oder Bromatom, eine Amino-, Methylamino- oder Hydroxygruppe darstellen,
R¹ einen gesättigten, einfach oder zweifach ungesättigten 5- bis 7-gliedrigen Aza-, Diaza-, Triaza-, Oxaza-, Thiaza-, Thiadiaza- oder S,S-Dioxido-thiadiaza-Heterocyclus,
wobei die vorstehend erwähnten Heterocyclen über ein Kohlenstoff- oder Stickstoffatom mit dem Piperidinring in Formel I verknüpft sind oder
über zwei Kohlenstoffatome, über ein Kohlenstoff- und ein Stickstoffatom, über ein Kohlenstoff- und ein Sauerstoffatom oder über ein Kohlenstoff- und ein Schwefelatom mit dem Piperidinring in Formel spirocyclisch verknüpft sind,
eine oder zwei Carbonyl- oder Thiocarbonylgruppen benachbart zu einem Stickstoffatom enthalten,
an einem der Stickstoffatome durch eine C₁₋₆-Alkyl-, C₃₋₆-Alkenyl- oder C₃₋₆-Alkenylgruppe substituiert sein können,
an einem oder an zwei Kohlenstoffatomen durch eine C₁₋₆-Alkyl-, C₂₋₆-Alkenyl-oder C₂₋₆-Alkinylgruppe, durch eine Phenyl-, Phenylethyl-, Naphthyl-, Biphenylyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-(C₁₋₃-Alkyl)-pyrazolyl-, Imidazolyl- oder 1-(C₁₋₃-Alkyl)-imidazolylgruppe substituiert sein können, wobei die Substituenten gleich oder verschieden sein können, und
eine olefinische Doppelbindung eines der vorstehend erwähnten ungesättigten Heterocyclen mit einem Phenyl-, Naphthyl-, Pyridin-, Diazin-, 1,3-Oxazol-, Thienyl-, Furan-, Thiazol-, Pyrrol-, *N*-C₁₋₃-Alkyl-pyrrol- oder Chinolin-Ring, mit einem am Stickstoffatom gegebenenfalls durch eine C₁₋₆-Alkyl-, C₃₋₆-Alkenyl-oder C₃₋₆-Alkinylgruppe substituierten 1*H*-Chinolin-2-on-Ring oder mit einem Imidazol- oder *N*-C₁₋₃-Alkyl-imidazol-Ring kondensiert sein kann oder auch zwei olefinische Doppelbindungen eines der vorstehend erwähnten ungesättigten Heterocyclen jeweils mit einem Phenyl- oder Pyridinring kondensiert sein können,
wobei die in R¹ enthaltenen Phenyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-C₁₋₃-Alkyl-pyrazolyl-, Imidazolyl- oder 1-C₁₋₃-Alkyl-imidazolylgruppen sowie benzo-, thieno-, pyrido- und diazinokondensierten Heterocyclen im Kohlenstoffgerüst zusätzlich durch Halogenatome, C₁₋₆-Alkyl-, C₂₋₆-Alkonyl-, C₂₋₆-Alkinyl-, Cyclo-C₃₋₇-alkyl-, Cyclo-C₃₋₇-alkenyl-, Cyano-, Hydroxy-, Hydroxy-C₁₋₆-alkyl-, Hydroxy-C₃₋₆-alkenyl-, Hydroxy-C₃₋₆-alkinyl-, C₁₋₆-Alkoxy-, C₁₋₆-Alkoxy-C₁₋₆-alkyl-, C₁₋₆-Alkoxy-C₃₋₆-alkenyl-, C₁₋₆-Alkoxy-C₃₋₆-alkinyl-, C₃₋₆-Alkenoxy-C₁₋₆-alkyl-, C₃₋₆-Alkenoxy-C₃₋₆-alkenyl-, C₃₋₆-Alkenoxy-C₃₋₆-alkinyl-, C₃₋₆-Alkinoxy-C₁₋₆-alkyl-, C₃₋₆-Alkinoxy-C₃₋₆-alkenyl-, C₃₋₆-Alkinoxy-C₃₋₆-alkinyl-, Thiohydroxy-, C₁₋₆-Alkylthio-, C₃₋₆-Alkenylthio-, C₃₋₆-Alkinylthio-, Amino-, C₁₋₆-Alkyl-amino-, C₃₋₆-Alkenyl-amino-, C₃₋₆-Alkinyl-amino-, Di-(C₁₋₆-alkyl)-amino-, Di-(C₃₋₆-alkenyl)-amino-, Di-(C₃₋₆-alkinyl)-amino-, Amino-C₁₋₆-alkyl-, C₁₋₃-Alkyl-amino-C₁₋₆-alkyl-, Di-(C₁₋₃-alkyl)-amino-C₁₋₆-alkyl-, Amino-C₃₋₆-alkenyl-, C₁₋₃-Alkyl-amino-C₃₋₆-alkenyl-, Di-(C₁₋₃-alkyl)-amino-C₃₋₆-alkenyl-, Amino-C₃₋₆-alkinyl-, C₁₋₃-Alkyl-amino-C₃₋₆-alkinyl-, Di-(C₁₋₃-alkyl)-amino-C₃₋₆-alkinyl-, Hydroxycarbonyl-, Phenylcarbonyl-, Pyridylcarbonyl-, C₁₋₆-Alkyl-carbonyl-, C₂₋₆-Alkenyl-carbonyl-, C₂₋₆-Alkinyl-carbonyl-, Formyl-, C₁₋₆-Alkoxy-carbonyl-, C₃₋₆-Alkenoxy-carbonyl-, C₃₋₆-Alkinoxy-carbonyl-, Aminocarbonyl-, C₁₋₆-Alkyl-aminocarbonyl-, C₃₋₆-Alkenyl-aminocarbonyl-, C₃₋₆-Alkinyl-aminocarbonyl-, Di-(C₁₋₆-alkyl)-aminocarbonyl-, Di-(C₃₋₆-alkenyl)-aminocarbonyl-, Di-(C₃₋₆-alkinyl)-aminocarbonyl-, Formylamino-, C₁₋₆-Alkyl-carbonylamino-, C₂₋₆-Alkenyl-carbonylamino-, C₂₋₆-Alkinyl-carbonylamino-, Formyl-C₁₋₆-alkyl-amino-, Formyl-C₃₋₆-alkenyl-amino-, Formyl-C₃₋₆-alkinyl-amino-, C₁₋₆-Alkyl-carbonyl-C₁₋₆-alkyl-amino-, C₂₋₆-Alkenyl-carbonyl-C₁₋₆-alkyl-amino-, C₂₋₆-Alkinyl-carbonyl-C₁₋₆-alkyl-amino-, C₁₋₆-Alkyl-carbonyl-C₃₋₆-alkenyl-amino-, C₂₋₆-Alkenyl-carbonyl-C₃₋₆-alkenyl-amino-, C₂₋₆-Alkinyl-carbonyl-C₃₋₆-alkenyl-amino-, C₁₋₆-Alkyl-carbonyl-C₃₋₆-alkinyl-amino-, C₂₋₆-Alkenyl-carbonyl-C₃₋₆-alkinyl-amino-, C₂₋₆-Alkinyl-carbonyl-C₃₋₆-alkinyl-amino-, C₁₋₆-Alkylsulfonyl-, C₂₋₆-Alkonyl-sulfonyl-, C₂₋₆-Alkinyl-sulfonyl-, C₁₋₆-Alkyl-sulfinyl-, C₂₋₆-Alkenyl-sulfinyl-, C₂₋₆-Alkinyl-sulfinyl-, C₁₋₆-Alkyl-sulfonylamino-, C₂₋₆-Alkenyl-sulfonylamino-, C₂₋₆-Alkinyl-sulfonylamino-, C₁₋₆-Alkyl-sulfonyl-C₁₋₆-alkylamino-, C₁₋₆-Alkyl-sulfonyl-C₃₋₆-alkenylamino-, C₁₋₆-Alkyl-sulfonyl-C₃₋₆-alkinylamino-, C₂₋₆-Alkenyl-sulfonyl-C₁₋₆-alkylamino-, C₂₋₆-Alkenyl-sulfonyl-C₃₋₆-alkenylamino-, C₂₋₆-Alkenyl-sulfonyl-C₃₋₆-alkinylamino-, C₂₋₆-Alkinyl-sulfonyl-C₁₋₆-alkylamino-, C₂₋₆-Alkinyl-sulfonyl-C₃₋₆-alkenylamino-, C₂₋₆-Alkinyl-sulfonyl-C₃₋₆-alkinylamino-, Aminosulfonyl-, C₁₋₆-Alkylaminosulfonyl-, Di-(C₁₋₆-alkyl)-aminosulfonyl-, C₃₋₆-Alkenylaminosulfonyl-, Di-(C₃₋₆-alkenyl)-aminosulfonyl-, C₃₋₆-Alkinylaminosulfonyl-, Di-(C₃₋₆-alkinyl)-aminosulfonylgruppen mono-, di- oder trisubstituiert sein können, wobei die Substituenten gleich oder verschieden sein können und
wobei die Doppel- und Dreifachbindungen der in den für R^{a}, R^{b}, R^{c} und R¹ vorstehend definierten Gruppen enthaltenen C₃₋₆-Alkenyl- oder C₃₋₆-Alkinylgruppen von in diesen Gruppen gegebenenfalls ebenfalls enthaltenen Heteroatomen isoliert sind,
R² das Wasserstoffatom,
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Diazinyl-, Hydroxy-, Amino-, C₁₋₆-Alkylamino-, Di-(C₁₋₆-alkyl)-amino-, C₃₋₆-Alkenylamino-, Di-(C₃₋₆-alkenyl)amino-, C₃₋₆-Alkinylamino-, Di-(C₃₋₆-alkinyl)amino-, Hydroxycarbonyl-, C₁₋₆-Alkoxycarbonyl-, Aminocarbonyl-, Aminocarbonylamino-, C₁₋₆-Alkylcarbonylamino-, C₂₋₆-Alkenyl-carbonylamino-, C₂₋₆-Alkinylcarbonylamino-, 4-Morpholinyl-, [Bis-(2-hydroxyethyl)amino-, 4-(C₁₋₆-Alkyl)-1-piperazinyl- oder 4-(ω-Hydroxy-C₂₋₇-alkyl)-1-piperazinylgruppe substituiert sein kann,
eine Phenyl- oder Pyridinylgruppe,
wobei die in den für R² vorstehend definierten Gruppen genannten oder als Substituenten enthaltenen Phenyl-, Pyridinyl- und Diazinylgruppen zusätzlich im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₃-Alkylamino-, Di-(C₁₋₃-alkyl)-amino-, Amino-C₁₋₃₋alkyl-, C₁₋₃-Alkyl-amino-C₁₋₃-alkyl-, Di-(C₁₋₃-alkyl)-amino-C₁₋₃-alkyl-, C₁₋₃-Alkylcarbonylamino-, C₁₋₃-Alkylcarbonylamino-C₁₋₃-alkyl-, Aminocarbonyl-, C₁₋₃-Alkyl-aminocarbonyl-, Di-(C₁₋₃-alkyl)-aminocarbonyl-, Cyano-, Aminosulfonyl-, C₁₋₃-Alkyl-aminosulfonyl-, Di-(C₁₋₃-alkyl)-aminosulfonyl-, C₁₋₃-Alkyl-thio-, C₁₋₃-Alkyl-sulfinyl-, oder C₁₋₃-Alkyl-sulfonyl- mono-, di- oder trisubstituiert und die Substituenten gleich oder verschieden sein können,
R³ das Wasserstoffatom oder eine durch eine Phenyl- oder Pyridinylgruppe substituierte C₁₋₃-Alkylgruppe,
wobei die C₁₋₃-Alkylgruppe mit einer in R² enthaltenen Alkylgruppe oder einem in R² enthaltenen Phenyl- oder Pyridylring unter Einschluss des Stickstoffatoms, an welches R² und R³ gebunden sind, unter Ausbildung eines 4- 7-gliedrigen Ringes verbunden sein kann,
oder
R² und R³ zusammen mit dem eingeschlossenen Stickstoffatom einen Rest der allgemeinen Formel 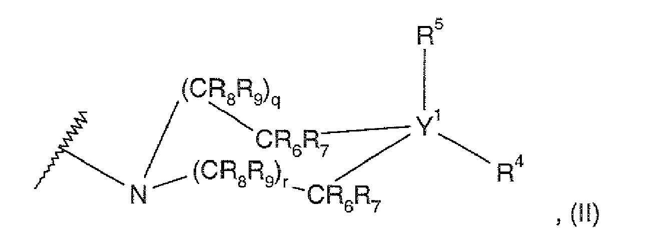 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl- oder Cyclo-C₃₋₇-alkenylgruppe, eine Amino-C₂₋₇-alkyl-, C₁₋₄-Alkyl-amino-C₂₋₇-alkyl-, Di-(C₁₋₄-alkyl-amino)-C₂₋₇-alkyl-, Aminoiminomethyl-, Aminocarbonylamino-, C₁₋₄-Alkyl-aminocarbonylamino-, Di-(C₁₋₄-alkyl)-aminocarbonylamino-, C₁₋₄-Alkyl-aminocarbonyl-C₁₋₄-alkyl-amino-, Di-(C₁₋₄-alkyl)-aminocarbonyl-C₁₋₄-alkyl-amino-, Phenylaminocarbonylamino-, Aminocarbonyl-, C₁₋₄-Alkyl-aminocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl-, Di-(C₁₋₄-alkyl)-aminocarbonyl-C₁₋₃-alkyl-, Aminocarbonylamino-C₁₋₃-alkyl-, C₁₋₆-Alkoxycarbonyl-, C₃₋₆-Alkenoxycarbonyl-, C₃₋₆-Alkinoxycarbonyl-, C₁₋₆-Alkoxycarbonyl-C₁₋₃-alkyl-, C₁₋₆-Alkenoxycarbonyl-C₁₋₃-alkyl-, C₁₋₆-Alkinoxycarbonyl-C₁₋₃-alkyl- oder Hydroxycarbonyl-C₁₋₃-alkylgruppe,
eine Phenyl-, Pyridinyl-, Diazinyl-, 1-Naphthyl-, 2-Naphthyl-, Pyridinylcarbonyl-oder Phenylcarbonylgruppe, die jeweils im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkyl-amino-C₁₋₃-alkyl-, Di-(C₁₋₄-alkyl)-amino-C₁₋₃-alkyl-, C₁₋₄-Alkylcarbonylamino-, C₁₋₄-Alkylcarbonylamino-C₁₋₃-alkyl-, Aminocarbonyl-, C₁₋₄-Alkyl-aminocarbonyl-, Di-(C₁₋₄-alkyl)-aminocarbonyl-, Cyano-, Aminosulfonyl-, C₁₋₄-Alkyl-aminosulfonyl-, Di-(C₁₋₄-alkyl)-aminosulfonyl-, C₁₋₄-Alkyl-thio-, C₁₋₄-Alkyl-sulfinyl-, oder C₁₋₄-Alkylsulfonyl- mono-, di- oder trisubstituiert und die Substituenten gleich oder verschieden sein können,
ein Heterocyclus ausgewählt aus einer 4- bis 10-gliedrigen Azacycloalkylgruppe, einer 6- bis 10-gliedrige Oxaza-, Thiaza-, *S,S*-Dioxothiaza- und Diazacycloalkylgruppe sowie einer 6- bis 10-gliedrigen Azabicycloalkylgruppe, eine 1-Alkyl-4-piperidinylcarbonyl- oder 4-Alkyl-1-piperazinylcarbonylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methingruppe durch ein Fluoratom sowie eine nicht direkt mit einem Stickstoff-, Sauerstoff- oder Schwefelatom verknüpfte Methylengruppe durch ein oder zwei Fluoratome substituiert sein kann,
die vorstehend genannten mono- und bicyclischen Heterocyclen sowie die 1-(C₁₋₆-Alkyl)-4-piperidinylcarbonyl- und 4-(C₁₋₆-Alkyl)-1-piperazinylcarbonylgruppe im Ring ein- bis vierfach durch Hydroxy-, C₁₋₅-Alkyl- oder Hydroxy-C₁₋₃-alkylgruppen, oder, gegebenenfalls zusätzlich, einfach durch eine Cyclo-C₃₋₇-alkyl-, Hydroxy-C₃₋₇-cycloalkyl-, Cyclo-C₃₋₇-alkenyl-, Cyclo-C₃₋₇-alkyl-C₁₋₃-alkyl-, Phonyl-C₁₋₃-alkyl-, Pyridyl-C₁₋₃-alkyl-, C₁₋₆-Alkylcarbonyl-, C₁₋₆-Alkylcarbonyl-C₁₋₃-alkyl-, Hydroxy-, C₁₋₆-Alkoxy-, Amino-, C₁₋₄-Alkylamino-, Di-(C₁₋₄-alkyl)amino-, Phenylcarbonyl-, Pyridinylcarbonyl-, C₁₋₆-Alkoxycarbonyl-, Hydroxycarbonyl-carbonyl-, C₁₋₆-Alkoxycarbonyl-carbonyl-, Hydroxycarbonyl-C₁₋₃-alkyl-, C₁₋₆-Alkoxycarbonyl-C₁₋₃-alkyl, Hydroxycarbonyl-C₁₋₃-alkylcarbonyl-, C₁₋₆-Alkoxycarbonyl-C₁₋₃-alkylcarbonyl-, Aminocarbonyl-, C₁₋₄-Alkylaminocarbonyl-, Di-(C₁₋₄-alkyl)aminocarbonyl-, Aminosulfonyl-, C₁₋₄-Alkylaminosulfonyl-, Di-(C₁₋₄-alkyl)aminosulfonyl-, C₁₋₃-Alkylsulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)aminocarbonyl-C₁₋₃-alkyl-, Hydroxyaminocarbonyl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppe, durch eine im Ring gegebenenfalls C₁₋₃-alkylsubstituierte Cyclo-C₃₋₇-alkylcarbonyl-, Azacyclo-C₄₋₇-alkylcarbonyl-, Diazacyclo-C₅₋₇-alkylcarbonyl- oder Oxazacyclo-C₅₋₇-alkylcarbonylgruppe substituiert sein können, wobei die Substituenten gleich oder verschieden und an ein Ring-Kohlenstoff- oder Ring-Stickstoffatom gebunden sein können,
wobei die in den für R⁴ vorstehend definierten Resten enthaltenen Phenyl- und Pyridinyl-Reste ihrerseits durch Halogenatom, durch C₁₋₃-Alkyl-, C₂₋₃-Alkenyl-, C₂₋₃-Alkinyl-, C₁₋₃-Alkoxy-, Hydroxy-, Amin-, C₁₋₄-Alkylamino-, Di-(C₁₋₄-alkyl)-amino-, Amino-C₁₋₃-alkyl-, C₁₋₄-Alkyl-amino-C₁₋₃-alkyl-, Di-(C₁₋₄-alkyl)-amino-C₁₋₃-alkyl-, C₁₋₄-Alkylcarbonyl-amino-, C₁₋₄-Alkylcarbonylamino-C₁₋₃-alkyl-, Aminocarbonyl-, C₁₋₃-Alkyl-aminocarbonyl-, Di-C₁₋₄-alkyl-aminocarbonyl-, Cyano-, Aminosulfonyl-, C₁₋₄-Alkyl-aminosulfonyl-, Dii-(C₁₋₄-alkyl)-aminosulfonyl-, C₁₋₃-Alkylthio-, C₁₋₃-Alkyl-sulfinyl-, oder C₁₋₃-Alkyl-sulfonyl- mono-, di- oder trisubstituiert sein können, wobei die Substituenten gleich oder verschieden sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom oder eine Hydroxygruppe,
einen C₁₋₄-Alkylrest, wobei ein unverzweigter Alkylrest in ω-Stellung durch eine Phenyl-, Pyridinyl-, Diazinyl-, Amin-, C₁₋₄-Alkylamino-, Di-(C₁₋₄-alkyl)-amino-, 4 C₁₋₄-Alkyl-1-piperazinyl- oder 4-Morpholinylgruppe substituiert sein kann,
eine C₁₋₆-Alkoxycarbonyl-, Cyano- oder Aminocarbonylgruppe oder auch, wenn Y¹ ein Stickstoffatom darstellt, ein freies Elektronenpaar,
oder, wenn Y¹ das Kohlenstoffatom darstellt, auch das Fluoratom, oder
R⁴ zusammen mit R⁵ und Y¹ einen 4- bis 7-gliedrigen cycloaliphatischen Ring, in dem eine Methylengruppe durch eine Gruppe -NH-, -N(C₁₋₄-Alkyl)-, -N(C₃₋₄-Alkenyl)-, -N(C₃₋₄-Alkinyl)-, -N(Cyclo-C₃₋₇-alkyl)-, -N(C₃₋₇-Cycloalkyl-C₁₋₃-alkyl)-, -N(Hydroxycarbonyl-C₁₋₃-alkyl)- oder -N(C₁₋₆-Alkoxycarbonyl-C₁₋₃-alkyl)- ersetzt sein kann,
wobei ein an ein Stickstoffatom in einer der für R⁴ vorstehend definierten Gruppen gebundenes Wasserstoffatom durch einen Schutzrest ersetzt sein kann,
R⁶ und R⁷, die gleich oder verschieden sein können, jeweils ein Wasserstoffatom, eine C₁₋₄-Alkylgruppe oder auch, wenn Y¹ ein Kohlenstoffatom darstellt, das Fluoratom, eine Amino-, C₁₋₄-Alkylamino- oder Di-(C₁₋₄-alkyl)-aminogruppe, wobei die beiden C₁₋₄-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratome und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Hafogen, durch Cyano- oder Hydroxygruppen mono-, di- oder trisubstituiert und die Substituenten gleich oder verschieden sein können und
unter den in den vor- und nachstehenden Definitionen genannten Schutzresten die aus der Peptidchemie geläufigen Schutzgruppen zur verstehen sind, insbesondere
eine im Phenylkern gegebenenfalls durch ein Halogenatom, durch eine Nitro- oder Phenylgruppe, durch eine oder zwei Methoxygruppen substituierte Phenylalkoxycarbonylgruppe mit 1 bis 3 Kohlenstoffatomen im Alkoxyteil,
beispielsweise die Benzyloxycarbonyl-, 2-Nitrobenzyloxycarbonyl-, 4-Nitrobenzyloxycarbonyl-, 4-Methoxy-benzyloxycarbonyl-, 2-Chlor-benzyloxycarbonyl-, 3-Chlor-benzyloxycarbonyl-, 4-Chlor-benzyloxycarbonyl-, 4-Biphenylyl-α,α-dimethyl-benzyloxycarbonyl- oder 3,5-Dimethoxy-α,α-dimethyl-benzyloxycarbonylgruppe,
eine Alkoxycarbonylgruppe mit insgesamt 1 bis 5 Kohlenstoffatomen im Alkylteil,
beispielsweise die Methoxycarbonyl-, Ethoxycarbonyl-, *n*-Propoxycarbonyl-, Isopropoxycarbonyl-, *n*-Butoxycarbonyl-, 1-Methylpropoxycarbonyl-, 2-Methylpropoxy-carbonyl- oder *tert*.Butyloxycarbonylgruppe,
die Allyloxycarbonyl-, 2,2,2-Trichlor-(1,1-dimethylethoxy)carbonyl- oder 9-Fluorenylmethoxycarbonylgruppe oder
die Formyl-, Acetyl- oder Trifluoracetylgruppe.

In den vor- und nachstehenden Definitionen ist unter einer in ω-Stellung substituierten Gruppe eine terminal substituierte Gruppe,
unter einem Halogenatom ein Fluor-, Chlor-, Brom- oder Iodatom und
unter einer von einem Heteroatom isolierten Doppel- oder Dreifachbindung eine Doppel- oder Dreifachbindung, die über mindestens ein gesättigtes Kohlenstoffatom mit einem Heteroatom verknüpft ist, zu verstehen.

Eine zweite Ausführungsform der vorliegende Erfindung besteht in den Verbindungen der obigen allgemeinen Formel (I), in denen
A, X, D, E, G, M, Q und R¹ wie vorstehend unter der ersten Ausführungsform erwähnt definiert sind und
R² das Wasserstoffatom,
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Diazinyl-, Hydroxy-, Amino-, C₁₋₆-Alkylamino-, Di-(C₁₋₆-alkyl)-amino-, C₃₋₆-Alkenylamino-, Di-(C₃₋₆-alkenyl)amino-, C₃₋₆-Alkinylamino-, Di-(C₃₋₆-alkinyl)amino-, Hydroxycarbonyl-, C₁₋₆-Alkoxycarbonyl-, Aminocarbonyl-, Aminocarbonylamino-, C₁₋₆-Aikylcarbonylamino-, C₂₋₆-Alkenyl-carbonylamino-, C₂₋₆-Alkinylcarbonylamino-, 4-Morpholinyl-, [Bis-(2-hydroxyethyl)]amino-, 4-(C₁₋₆-Alkyl)-1-piperazinyl- oder 4-(ω-Hydroxy-C₂₋₇-alkyl)-1-piperazinylgruppe substituiert sein kann,
eine Phenyl- oder Pyridinylgruppe,
wobei die in den für R² vorstehend definierten Gruppen genannten oder als Substituenten enthaltenen Phenyl-, Pyridinyl- und Diazinylgruppen zusätzlich im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy", Amino-, C₁₋₃-Alkylamino-, Di-(C₁₋₃-alkyl)-amino-, Amino-C₁₋₃-alkyl-, C₁₋₃-Alkyl-amino-C₁₋₃-alkyl-, Di-(C₁₋₃-alkyl)-amino-C₁₋₃-alkyl-, C₁₋₃-Alkylcarbonylamino-, C₁₋₃-Alkylcarbonylamlno-C₁₋₃-alkyl-, Aminocarbonyl-, C₁₋₃-Alkyl-aminocarbonyl-, Di-(C₁₋₃-alkyl)-aminocarbonyl-, Cyano-, Aminosulfonyl-, C₁₋₃-Alkyl-aminosulfonyl-, Di-(C₁₋₃-alkyl)-aminosulfonyl-, C₁₋₃-Alkyl-thio-, C₁₋₃-Alkyl-sulfinyl-, oder C₁₋₃-Alkyl-sulfonyl- mono-, di- oder trisubstituiert und die Substituenten gleich oder verschieden sein können,
R³ das Wasserstoffatom oder eine durch eine Phenyl- oder Pyridinylgruppe substituierte C₁₋₃-Alkylgruppe,
wobei die C₁₋₃-Alkylgruppe mit einer in R² enthaltenen Alkylgruppe oder einem in R² enthaltenen Phenyl- oder Pyridylring unter Einschluss des Stickstoffatoms, an welches R² und R³ gebunden sind, unter Ausbildung eines 4- 7-gliedrigen Ringes verbunden sein kann,
oder
R² und R³ zusammen mit dem eingeschossene Stickstoffatom einen Rest der allgemeinen Formel 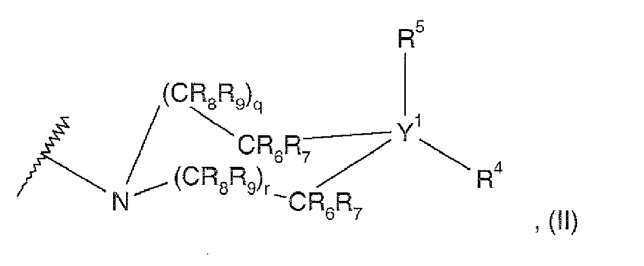 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, oder
q und r, wenn Y¹ das Stickstoffatom darstellt, die Zahlen 1 oder 2,
R⁴ das Wasserstoffatom, eine Amin-, C₁₋₄-Alkyl-amino-, Di-(C₁₋₄-alkyl)-alkylamin-, C₁₋₆-Alkyl-, eine Cyclo-C₃₋₇-alkyl-, Cyclo-C₃₋₇-alkenyl-, Amino-C₂₋₇-alkyl-, C₁₋₄-Alkyl-amino-C₂₋₇-alkyl-, Di-(C₁₋₄-alkyl-amino)-C₂₋₇-alkyl-, Aminoiminomethyl-, Aminocarbonylamino-, C₁₋₄-Alkyl-aminocarbonylamino-, Di-(C₁₋₄-alkyl)-aminocarbonylamino-, C₁₋₄-Alkyl-aminocarbonyl-C₁₋₄-alkyl-amino-, Di-(C₁₋₄-alkyl)-aminocarbonyl-C₁₋₄-alkyl-amino-, Phenylaminocarbonylamino-, Aminocarbonyl-, C₁₋₄-Alkyl-aminocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl-, Di-(C₁₋₄-alkyl)-aminocarbonyl-C₁₋₃-alkyl-, Aminocarbonylamino-C₁₋₃-alkyl-, C₁₋₆-Alkoxycarbonyl-, C₃₋₆-Alkenoxycarbonyl-, C₃₋₆-Alkinoxycarbonyl-, C₁₋₆-Alkoxycarbonyl-C₁₋₃-alkyl-, C₁₋₅-Alkenoxycarbonyl-C₁₋₃-alkyl-, C₁₋₆-Alkinoxycarbonyl-C₁₋₃-alkyl- oder Hydroxycarbonyl-C₁₋₃-alkylgruppe,
eine Phenyl-, Pyridinyl-, Diazinyl-, 1-Naphthyl-, 2-Naphthyl-, Pyridinylcarbonyl-oder Phenylcarbonylgruppe, die jeweils im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkyl-amino-C₁₋₃-alkyl-, Di-(C₁₋₄-alkyl)-amino-C₁₋₃-alkyl-, C₁₋₄-Alkylcarbonylamino-, C₁₋₄-Alkylcarbonylamino-C₁₋₃-alkyl-, Aminocarbonyl-, C₁₋₄-Alkyl-aminocarbonyl-, Di-(C₁₋₄-alkyl)-aminocarbonyl-, Cyano-, Aminosulfionyl-, C₁₋₄-Alkyl-aminosulfonyl-, Di-(C₁₋₄-alkyl)-aminosulfonyl-, C₁₋₄₋Alkyl-thio-, C₁₋₄-Alkyl-sulfinyl-, oder C₁₋₄-Alkylsulfonyl- mono-, di- oder trisubstituiert und die Substituenten gleich oder verschieden sein können,
ein Heterocyclus ausgewählt aus einer 4- bis 10-gliedrigen Azacycloalkylgruppe, einer 6- bis 10-gliedrige Oxaza-, Thiaza- und Diazacycloalkylgruppe sowie einer 6- bis 10-gliedrigen Azabicycloalkylgruppe,
eine 1-Alkyl-4-piperidinylcarbonyl- oder 4-Alkyl-1-piperazinylcarbonylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methingruppe durch ein Fluoratom sowie eine nicht direkt mit einem Stickstoff-, Sauerstoff- oder Schwefelatom verknüpfte Methylengruppe durch ein oder zwei Fluoratome substituiert sein kann,
die vorstehend genannten mono- und bicyclischen Heterocyclen sowie die 1-(C₁₋₆-Alkyl)-4-piperidinylcarbonyl- und 4-(C₁₋₆-Alkyl)-1-piperazinylcarbonylgruppe im Ring ein- bis vierfach durch C₁₋₆-Alkylgruppen, oder, gegebenenfalls zusätzlich, einfach durch eine Cyclo-C₃₋₇-alkyl-, Cyclo-C₃₋₇-alkenyl-, Cyclo-C₃₋₇-alkyl-C₁₋₃-alkyl-, Phenyl-C₁₋₃-alkyl-, Pyridyl-C₁₋₃-alkyl-, C₁₋₆-Alkylcarbonyl-, Hydroxy-, C₁₋₆-Alkoxy-, Amino-, C₁₋₄-Alkylamino-, Di-(C₁₋₄-alkyl)amino-, Phenylcarbonyl-, Pyridinylcarbonyl-, Hydroxycarbonyl-, Hydroxycarbonyl-C₁₋₃-alkyl-, C₁₋₆-Alkoxycarbonyl-, C₁₋₆-Alkoxycarbonyl-C₁₋₃-alkyl, Aminocarbonyl-, C₁₋₄-Alkylaminocarbonyl-, Di-(C₁₋₄-alkyl)aminocarbonyl-, C₁₋₃-Alkylsulfonylgruppe, durch eine im Ring gegebenenfalls C₁₋₃-alkylsubstituierte Cyclo-C₃₋₇-alkylcarbonyl-, Azacyclo-C₄₋₇-alkylcarbonyl-, Diazacyclo-C₅₋₇-alkylcarbonyl- oder Oxazacyclo-C₅₋₇-alkylcarbonylgruppe substituiert sein können, wobei die Substituenten gleich oder verschieden und an ein Ring-Kohlenstoff- oder Ring-Stickstoffatom gebunden sein können,
wobei die in den für R⁴ vorstehend definierten Resten enthaltenen Phenyl- und Pyridinyl-Reste ihrerseits durch Halogenatome, durch C₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkyl-amino-C₁₋₃-alkyl-, Di-(C₁₋₄-alkyl)-amino-C₁₋₃-alkyl-, C₁₋₄-Alkylcarbonyl-amino-, C₁₋₄-Alkylcarbonylamino-C₁₋₃-alkyl-, Aminocarbonyl-, C₁₋₃-Alkyl-aminocarbonyl-, Di-C₁₋₄-alkyl-aminocarbonyl-, Cyano-, Aminosulfonyl-, C₁₋₄-Alkyl-aminosulfonyl-, Di-(C₁₋₄-alkyl)-aminosulfonyl-, C₁₋₃-Alkylthio-, C₁₋₃-Alkyl-sulfinyl-, oder C₁₋₃-Alkyl-sulfonyl- mono-, di- oder trisubstituiert sein können, wobei die Substituenten gleich oder verschieden sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom,
einen C₁₋₄-Alkylrest, wobei ein unverzweigter Alkylrest in ω-Stellung durch eine Phenyl-, Pyridinyl-, Diazinyl-, Amino-, C₁₋₄-Alkylamino-, Di-(C₁₋₄-alkyl)-amino-, 4 C₁₋₄-Alkyl-1-piperazinyl- oder 4-Morpholinylgruppe substituiert sein kann,
eine C₁₋₆-Alkoxycarbonyl-, Cyano- oder Aminocarbonylgruppe oder auch, wenn Y¹ ein Stickstoffatom darstellt, ein freies Elektronenpaar,
oder, wenn Y¹ das Kohlenstoffatom darstellt, auch das Fluoratom, oder
R⁴ zusammen mit R⁵ und Y¹ einen 4- bis 7-gliedrigen cycloaliphatischen Ring, in dem eine Methylengruppe durch eine Gruppe -NH-, -N(C₁₋₄-Alkyl)-, -N(C₃₋₄-Alkenyl)-, -N(C₃₋₄-Alkinyl)-, -N(Cyclo-C₃₋₇-alkyl)- oder -N(C₃₋₇-Cycloalkyl-C₁₋₃-alkyl)- ersetzt sein kann,
wobei ein an ein Stickstoffatom in einer der für R⁴ vorstehend definierten Gruppen gebundenes Wasserstoffatom durch einen Schutzrest ersetzt sein kann,
R⁶ und R⁷, die gleich oder verschieden sein können, jeweils ein Wasserstoffatom, eine C₁₋₄-Alkylgruppe oder auch, wenn Y¹ ein Kohlenstoffatom darstellt, das Fluoratom, eine C₁₋₄-Alkylamino- oder Di-(C₁₋₄-alkyl)-aminogruppe,
wobei die beiden C₁₋₄-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine dritte Ausführungsform der vorliegenden Erfindung besteht in den Verbindungen der obigen allgemeinen Formel (I), in denen
A, X, D, E, G, M, Q, R² und R³ wie vorstehend unter der ersten oder zweiten Ausführungsform erwähnt definiert sind und
R¹ einen einfach oder zweifach ungesättigten 5- bis 7-gliedrigen Aza-, Diaza-, Triaza-oder Thiaza-Heterocyclus bedeutet,
wobei die vorstehend erwähnten Heterocyclen über ein Kohlenstoff- oder Stickstoffatom verknüpft sind oder
über ein Kohlenstoff- und ein Stickstoffatom, über ein Kohlenstoff- und ein Sauerstoffatom oder über ein Kohlenstoff- und ein Schwefelatom spirocyclisch verknüpft sind,
eine oder zwei Carbonylgruppen benachbart zu einem Stickstoffatom enthalten,
an einem Kohlenstoffatom durch eine Phenyl-, Pyridinyl-, Diazinyl-, Thienyl-, Pyrrolyl-, 1,3-Thiazolyl-, Isoxazolyl-, Pyrazolyl- oder 1-(C₁₋₄-Alkyl)-pyrazolylgruppe substituiert sein können und
wobei eine olefinische Doppelbindung eines der vorstehend erwähnten ungesättigten Heterocyclen mit einem Phenyl-, Naphthyl-, Pyridin-, Diazin-, Thienyl- oder Chinolin-Ring oder mit einem gegebenenfalls am Stickstoffatom durch eine Methylgruppe substituierten 1*H*-Chinolin-2-on-Ring kondensiert sein kann,
wobei die in R¹ enthaltenen Phenyl-, Pyridinyl-, Diazinyl-, Thienyl-, Pyrrolyl-, 1,3-Thiazolyl-, Isoxazolyl-, Pyrazolyl- oder 1-(C₁₋₄-Alkyl)-pyrazolylgruppen sowie die benzo-, pyrido- und diazinokondensierten Heterocyclen im Kohlenstoffgerüst zusätzlich durch Halogen, durch C₁₋₆-Alkyl-, Cyclo-C₃₋₇-alkyl-, Cyclo-C₃₋₇-alkenyl-, C₁₋₆₋Alkoxy-, Hydroxycarbonyl-, C₁₋₆-Alkoxycarbonyl-, Cyano-, Hydroxy-, Amino-, C₁₋₄-Alkyiamino-, Di-C₁₋₄-alkylamino-, C₁₋₄-Alkylcarbonylamino- oder C₁₋₄-Alkylcarbonylgruppen mono-, di- oder trisubstituiert sein können, wobei die Substituenten gleich oder verschieden sein können,
wobei, soweit nichts anderes erwähnt wurde, alle in den vorstehend unter R¹ definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können, und
alle in den vorstehend unter R¹ definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine vierte Ausführungsform der vorliegenden Erfindung besteht in den Verbindungen der obigen allgemeinen Formel (I), in denen
A, X, D, E, G, M, Q, R² und R³ wie voranstehend unter der ersten oder zweiten Ausführungsform erwähnt definiert sind und
R¹ einen einfach ungesättigten 5- bis 7-gliedrigen Diaza- oder Triaza-Heterocyclus bedeutet,
wobei die vorstehend erwähnten Heterocyclen über ein Stickstoffatom verknüpft sind oder
über ein Kohlenstoff- und ein Stickstoffatom oder über ein Kohlenstoff- und ein Sauerstoffatom spirocyclisch verknüpft sind,
eine Carbonylgruppe benachbart zu einem Stickstoffatom enthalten,
zusätzlich an einem Kohlenstoffatom durch eine Phenylgruppe substituiert sein können und
wobei eine olefinische Doppelbindung eines der vorstehend erwähnten ungesättigten Heterocyclen mit einem Phenyl-, Thienyl- oder Chinolin-Ring kondensiert sein kann,
wobei die in R¹ enthaltenen Phenylgruppen sowie benzokondensierten Heterocyclen im Kohlenstoffgerüst zusätzlich durch Halogen, durch Methyl-, Methoxy-, Difluormethyl-, Trifluormethyl-, Hydroxy-, Amino-, C₁₋₄-Alkylamino-, Di-(C₁₋₄-alkyl)-amino-, Acetylamino-, Acetyl-, Hydroxycarbonyl-, C₁₋₃₋Aikoxycarbonyl-, Cyano- Difluormethoxy- oder Trifluormethoxygruppen mono-, di- oder trisubstituiert sein können, wobei die Substituenten gleich oder verschieden sein können, vorzugsweise jedoch unsubstituiert oder durch ein Halogenatom, durch eine Methyl- oder Methoxygruppe monosubstituiert sind,
wobei, soweit nichts anderes erwähnt wurde, alle in den vorstehend unter R¹ definierten Resten erwähnten oder enthaltenen Alkylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine fünfte Ausführungsform der vorliegenden Erfindung besteht in den Verbindungen der obigen allgemeinen Formel (I), in denen
A, X, D, E, G, M, Q, R² und R³ wie vorstehend unter der ersten oder zweiten Ausführungsform erwähnt definiert sind und

R¹ eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-c]chinolin-2-on-3-yl-, 1,3-Dihydro-naphth[1,2-d]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H*-thieno-[3,2-*d*]pyrimidin-2-on-3-yl- oder 3,4-Dihydro-1*H*-thieno[3,4-*d*]pyrimidin-2-on-3-yl-gruppe bedeutet,
wobei die vorstehend unter R¹ erwähnten Heterocyclen im Kohlenstoffgerüst zusätzlich durch eine Methoxygruppe monosubstituiert sein können,
alle in den vorstehend unter R¹ definierten Resten erwähnten oder enthaltenen aromatischen und heteroaromatischen Reste zusätzlich durch Halogenatome, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine sechste Ausführungsform der vorliegenden Erfindung besteht in den Verbindungen der obigen allgemeinen Formel (I), in denen
A, X, R¹, R² und R³ wie vorstehend unter der ersten, zweiten, dritten, vierten oder fünften Ausführungsform erwähnt definiert sind und
(a) D, E unabhängig voneinander jeweils eine Methingruppe oder das Stickstoffatom, und
   G eine durch die Gruppe R^{a} substituierte Methingruppe,
   M eine durch die Gruppe R^{b} substituierte Methingruppe,
   Q eine durch die Gruppe R^{c} substituierte Methingruppe,
   wobei eine oder zwei der Gruppen G, M und Q jeweils auch ein Stickstoffatom bedeuten kann,
   oder
(b) D und E jeweils eine Methingruppe, wobei eine der Gruppen D und E auch ein Stickstoffatom bedeuten kann, und
   G, M und Q jeweils ein Stickstoffatom bedeuten,
wobei R³, R^{b} und R^{c} unabhängig voneinander jeweils ein Wasserstoff- oder - Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₄-alkyl-, Hydroxy-C₃₋₄-alkenyl-, Hydroxy-C₃₋₄-alkinyl-, C₁₋₄-Alkoxy-, C₁₋₄-Alkoxy-C₁₋₄-alkyl-, C₁₋₄-Alkoxy-C₃₋₄-alkenyl-, C₁₋₄-Alkoxy-C₃₋₄-alkinyl-, Thiohydroxy-, C₁₋₄-Alkylthio-, Amin-, C₁₋₄-Alkyl-amino-, C₃₋₄-Alkenyl-amino-, C₃₋₄-Alkinyl-amino-, Di-(C₁₋₄-alkyl)-amino-, Di-(C₃₋₄-alkenyl)-amino-, Di-(C₃₋₄-alkinyl)-amino-, Amino-C₁₋₄-alkyl-, C₁₋₃-Alkyl-amino-C₁₋₄-alkyl-, Di-(C₁₋₃-alkyl)-amino-C₁₋₄-alkyl-, Amino-C₃₋₄-alkenyl-, C₁₋₃-Alkyl-amino-C₃₋₄-alkenyl-, Di-(C₁₋₃-alkyl)-amino-C₃₋₄-alkenyl-, Amino-C₃₋₄-alkinyl-, C₁₋₃-Alkyl-amino-C₃₋₄-alkinyl-, Di-(C₁₋₃-alkyl)-amino-C₃₋₄-alkinyl-, Hydroxycarbonyl-, Phenylcarbonyl-, Pyridylcarbonyl-, C₁₋₄-Alkyl-carbonyl-, Formyl-, C₁₋₄-Alkoxy-carbonyl-, C₃₋₄-Alkenoxy-carbonyl-, C₃₋₄-Alkinoxy-carbonyl-, Aminocarbonyl-, C₁₋₄-Alkyl-aminocarbonyl-, C₃₋₄-Alkenyl-aminocarbonyl-, C₃₋₄-Alkinyl-aminocarbonyl-, Di-(C₁₋₄-alkyl)-aminocarbonyl-, Di-(C₃₋₄-alkenyl)-aminocarbonyl-, Di-C₃₋₄-(alkinyl)-aminocarbonyl-, Formylamino-, C₁₋₄-Alkyl-carbonylamino-, Formyl-C₁₋₄-alkyl-amino-, Formyl-C₃₋₄-alkenyl-amino-, Formyl-C₃₋₄-alkinyl-amino-, C₁₋₄-Alkyl-carbonyl-C₁₋₄-alkyl-amino-, C₁₋₄-Alkyl-carbonyl-C₃₋₄-alkenyl-amino-, C₁₋₄-Alkyl-carbonyl-C₃₋₄-alkinyl-amino-, C₁₋₄-Alkyl-sulfonyl-, C₂₋₄-Alkenyl-sulfonyl-, C₂₋₄-Alkinyl-sulfonyl-, C₁₋₄-Alkyl-sulfinyl-, C₂₋₄-Alkenyl-sulfinyl-, C₂₋₄-Alkinyl-sulfinyl-, C₁₋₄-Alkyl-sulfionylamino-, C₁₋₄-Alkyl-sulfonyl-C₁₋₄-alkylamino-, C₁₋₄-Alkyl-sulfonyl-C₃₋₄-alkenylamino-, C₁₋₄-Alkyl-sulfonyl-C₃₋₄-alkinylamino-, Aminosulfonyl-, C₁₋₄-Alkylaminosulfonyl-, Di-(C₁₋₄-alkyl)-aminosulfonyl-, C₃₋₄-Alkenylamino-sulfonyl-, Di-(C₃₋₄-alkenyl)-aminosulfonyl-, C₃₋₄-Alkinylaminosulfonyl- oder Di-(C₃₋₄-alkinyl)-aminosulfonylgruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₄-Alkylgruppe darstellen,
(ii) R^{c} kein Wasserstoffatom bedeutet, wenn R^{a} sowie R^{b} jeweils eine C₁₋₄-Alkylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
wobei, soweit nichts anderes erwähnt wurde, alle in den vorstehend für R^{a}, R^{b} und R^{c} definierten Resten erwähnten oder enthaltenen Alkyl-, Alkenyl- und Alkenylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können,
die Doppel- und Dreifachbindungen der in den für R^{a}, R^{b} und R^{c} vorstehend definierten Gruppen enthaltenen C₃₋₄-Alkenyl- oder C₃₋₄-Alkinylgruppen von in diesen Gruppen gegebenenfalls ebenfalls enthaltenen Heteroatomen isoliert sind,
und alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine siebte Ausführungsform der vorliegende Erfindung besteht in den Verbindungen der obigen allgemeinen Formel (I), in denen
A, X, R¹, R² und R³ wie vorstehend unter der ersten, zweiten, dritten, vierten oder fünften Ausführungsform erwähnt definiert sind und
(a) D, E unabhängig voneinander jeweils eine Methingruppe oder das Stickstoffatom und
   G eine durch die Gruppe R^{a} substituierte Methingruppe,
   M eine durch die Gruppe R^{b} substituierte Methingruppe,
   Q eine durch die Gruppe R^{c} substituierte Methingruppe,
   wobei eine oder zwei der Gruppen G, M und Q jeweils auch ein Stickstoffatom bedeuten kann,
   oder
(b) D und E jeweils eine Methingruppe, wobei eine der Gruppen D und E auch ein Stickstoffatom bedeuten kann, und
   G, M und Q jeweils ein Stickstoffatom bedeuten,
wobei R^{a}, R^{b} und R^{c} unabhängig voneinander jeweils ein Wasserstoff- oder - Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₂-alkyl-, Hydroxy-C₃-alkenyl-, Hydroxy-C₃-alkinyl-, C₁₋₄-Alkoxy-, C₁₋₄-Alkoxy-C₁₋₂-alkyl-, Amino-, C₁₋₄-Alkyl-amino-, C₃₋₄-Alkonyl-amino-, C₃₋₄-Alkinyl-amino-, Di-(C₁₋₄-alkyl)-amino-, Di-(C₃₋₄-alkenyl)-amino-, Di-(C₃₋₄-alkinyl)-amino-, Amino-C₁₋₂-alkyl-, C₁₋₃-Alkyl-amino-C₁₋₂-alkyl-, Di-(C₁₋₃-alkyl)-amino-C₁₋₂-alkyl-, Amino-C₃-alkenyl-, C₁₋₃-Alkyl-amino-C₃-alkenyl-, Di-(C₁₋₃-alkyl)-amino-C₃-alkenyl-, Amino-C₃-alkinyl-, C₁₋₃-Alkyl-amino-C₃-alkinyl-, Di-(C₁₋₃-alkyl)-amino-C₃-alkinyl-, Hydroxycarbonyl-, C₁₋₄-Alkyl-carbonyl-, Formyl-, C₁₋₄-Alkoxy-carbonyl-, Aminocarbonyl-, C₁₋₄-Alkyl-aminocarbonyl-, Di-(C₁₋₄-alkyl)-aminocarbonyl-, Formylamino-, C₁₋₄-Alkylcarbonylamino-, Formyl-C₁₋₄-alkyl-amino-, C₁₋₄-Alkyl-carbonyl-C₁₋₄-alkyl-amino-, C₁₋₄-Alkyl-sulfonyl-, C₁₋₄-Alkyl-sulfinyl-, C₁₋₄-Alkyl-sulfonylamino-, C₁₋₄-Alkyl-sulfonyl-C₁₋₄-alkylamino-, Aminosulfonyl-, C₁₋₄-Alkylaminosulfonyl- oder Di-(C₁₋₄-alkyl)-aminosulfonylgruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₄-Alkylgruppe darstellen,
(ii) R^{c} kein Wasserstoffatom bedeutet, wenn R^{a} sowie R^{b} jeweils eine C₁₋₄-Alkylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder Iodatoms oder einer Difluor- oder Trifluormethylgruppe annimmt, wenn R^{a} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
wobei, soweit nichts anderes erwähnt wurde, alle in den vorstehend für R^{a}, R^{b} und R^{c} definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können, und
die Doppel- und Dreifachbindungen der in den für R^{a}, R^{b} und R^{c} vorstehend definierten Gruppen enthaltenen C₃₋₄-Alkonyl- oder C₃₋₄-Alkinylgruppen von in diesen Gruppen gegebenenfalls ebenfalls enthaltenen Heteroatomen isoliert sind,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine achte Ausführungsform der vorliegenden Erfindung besteht in den Verbindungen der obigen allgemeinen Formel (I), in denen
A, X, R¹, R² und R³ wie vorstehend unter der ersten, zweiten, dritten, vierten oder fünften Ausführungsform erwähnt definiert sind und
(a) D, E unabhängig voneinander jeweils eine Methingruppe oder das Stickstoffatom und
   G eine durch die Gruppe R^{a} substituierte Methingruppe,
   M eine durch die Gruppe R^{b} substituierte Methingruppe,
   Q eine durch die Gruppe R^{c} substituierte Methingruppe,
   wobei eine oder zwei der Gruppen G, M und Q jeweils auch ein Stickstoffatom bedeuten kann,
   oder
(b) D und E jeweils eine Methingruppe, wobei eine der Gruppen D und E auch ein Stickstoffatom bedeuten kann, und
   G, M und Q jeweils ein Stickstoffatom bedeuten,
wobei R^{a}, R^{b} und R^{c} unabhängig voneinander jeweils ein Wasserstoff- oder - Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₂-alkyl-, C₁₋₄-Alkoxy-, Amino-, C₁₋₄-Alkyl-amino-, Di-(C₁₋₄-alkyl)-amino-, Amino-C₁₋₂-alkyl-, C₁₋₃-Alkyl-amino-C₁₋₂-alkyl-, Di-(C₁₋₃-alkyl)-amino-C₁₋₂-alkyl-, Hydroxycarbonyl-, C₁₋₄-Alkyl-carbonyl-, Formyl-, C₁₋₄-Alkoxy-carbonyl-, Aminocarbonyl-, C₁₋₄-Alkyl-aminocarbonyl-, Di-(C₁₋₄-alkyl)-aminocarbonyl-, Formylamino-, C₁₋₄-Alkyl-carbonylamino-, Formyl-C₁₋₄-alkyl-amino-oder C₁₋₄-Alkyl-carbonyl-C₁₋₄-alkyl-aminogruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₄-Alkylgruppe darstellen,
(ii) R^{c} kein Wasserstoffatom bedeutet, wenn R^{a} sowie R^{b} jeweils eine C₁₋₄-Alkylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
wobei, soweit nichts anderes erwähnt wurde, alle in den vorstehend für R^{a}, R^{b} und R^{c} definierten Resten erwähnten oder enthaltenen Alkyl-, Alkenyl- und Alkenylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine neunte Ausführungsform der vorliegenden Erfindung besteht in den Verbindungen der obigen allgemeinen Formel (I), in denen
A, X, R¹, R² und R³ wie vorstehend unter der ersten, zweiten, dritten, vierten oder fünften Ausführungsform erwähnt definiert sind und
(a) D, E unabhängig voneinander jeweils eine Methingruppe oder das Stickstoffatom und
   G eine durch die Gruppe R^{a} substituierte Methingruppe,
   M eine durch die Gruppe R^{b} substituierte Methingruppe,
   Q eine durch die Gruppe R^{c} substituierte Methingruppe,
   wobei eine oder zwei der Gruppen G, M und Q jeweils auch ein Stickstoffatom bedeuten kann,
   oder
(b) D und E jeweils eine Methingruppe, wobei eine der Gruppen D und E auch ein Stickstoffatom bedeuten kann, und
   G, M und Q jeweils ein Stickstoffatom bedeuten,
wobei R^{a}, R^{b} und R^{c} unabhängig voneinander jeweils ein Wasserstoff- oder - Halogenatom, eine Methyl-, Difluormethyl-, Trifluormethyl-, Ethyl-, Vinyl-, Ethinyl-, Cyano-, Hydroxy-, Methoxy-, Difluormethoxy-, Trifluormethoxy-, Amino-, Methylamino- oder Dimethylaminogruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine Methyl-oder Ethylgruppe darstellen,
(ii) R^{c} kein Wasserstoffatom bedeutet, wenn R^{a} sowie R^{b} jeweils eine Methyl-_ oder Ethylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine Methyl-, Ethyl-, Vinyl- oder Ethinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder Iodatoms oder einer Difluor- oder Trifluormethylgruppe annimmt, wenn R^{a} eine Methyl-, Ethyl-, Vinyl- oder Ethinylgruppe und R^{b} ein Chlor- oder Bromatom, eine Amino-, Methylamino- oder Hydroxygruppe darstellen,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine zehnte Ausführungsform der vorliegenden Erfindung besteht in den Verbindungen der obigen allgemeinen Formel (I), in denen
A, X, D, E, G, M, Q und R¹ wie vorstehend unter der ersten, zweiten, dritten, vierten, fünften, sechsten, siebten, achten oder neunten Ausführungsform erwähnt definiert sind und
R² das Wasserstoffatom oder
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Hydroxy-, Amino-, C₁₋₆-Alkylamino-, Di-(C₁₋₆-alkyl)-amino-, Hydroxycarbonyl-, C₁₋₆-Alkoxycarbonyl-, Aminocarbonyl-, Aminocarbonylamino-, C₁₋₆-Alkylamino-, 4-Morpholinylgruppe substituiert sein kann,
wobei die in den für R² vorstehend definierten Gruppen genannten oder als Substituenten enthaltenen Phenyl- und Pyridinylreste zusätzlich im Kohlenstoffgerüst durch Halogen, durch C₁₋₃-Alkyl-, C₁₋₃-Alkoxy-, Hydroxy-, Amino-, C₁₋₃-Alkylamino-, Di-(C₁₋₃-alkyl)-amino-, Amino-C₁₋₃-alkyl-, C₁₋₃-Alkyl-amino-C₁₋₃-alkyl-, Di-(C₁₋₃-alkyl)-amino-C₁₋₃-alkyl-, C₁₋₃-Alkylcarbonylamino-, C₁₋₃-Alkylcarbonyl-C₁₋₃-alkylamino-, Aminocarbonyl-, C₁₋₃-Alkylaminocarbonyl-oder Di-(C₁₋₃-alkyl)-aminocarbonylgruppen mono-, di- oder trisubstituiert und die Substituenten gleich oder verschieden sein können,
R³ das Wasserstoffatom oder eine C₁₋₃-Alkylgruppe darstellen oder
R² und R³ zusammen mit dem eingeschlossenen Stickstoffatom einen Rest der allgemeinen Formel 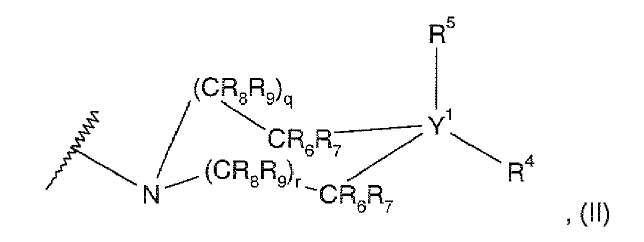 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl- oder Cyclo-C₃₋₇-alkenylgruppe, eine Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₄-Alkyl-aminocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl-, Di-(C₁₋₄-alkyl)-aminocarbonyl-C₁₋₃-alkyl-, Aminocarbonylamino-C₁₋₃-alkyl-, C₁₋₆-Alkoxycarbonyl-, _{C1-6}-Alkoxycarbonyl-C₁₋₃-alkyl- oder Hydroxycarbonyl-C₁₋₃-alkylgruppe,
eine Phenyl-, Pyridinyl- oder Diazinylgruppe, die jeweils durch ein Halogen, durch eine C₁₋₃-Alkyl-, C₁₋₃-Alkoxy-, Hydroxy-, Amino-, C₁₋₄-Alkyl-amino-, Di-(C₁₋₄-alkyl)-amino- Amino-C₁₋₃-alkyl-, C₁₋₄-Alkyl-amino-C₁₋₃-alkyl-, Di-(C₁₋₄-alkyl)-amino-C₁₋₃-alkylgruppe substituiert sein können,
ein Heterocyclus ausgewählt aus einer 4- bis 7-gliedrigen Azacycloalkylgruppe, einer 6- bis 7-gliedrigen Oxaza-, *S,S*-Dioxothiaza- und Diazacycloalkylgruppe und einer 7- bis 9-gliedrigen Azabicycloalkylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methylengruppe durch ein oder zwei Fluoratome substituiert sein kann und
die vorstehend genannten mono- und bicyclischen Heterocyclen ein- oder zweifach durch Hydroxy-, C₁₋₃-Alkyl- oder Hydroxy-C₁₋₃-alkylgruppen oder einfach durch eine Benzyl-, Cyclo-C₃₋₆-alkyl-, Hydroxycyclo-C₃₋₆-alkyl-, Cyclo-C₃₋₆-alkyl-C₁₋₃-alkyl, C₁₋₄-Alkylcarbonyl-, C₁₋₄-Alkylcarbonyl-C₁₋₃-alkyl-, Hydroxy-, C₁₋₄-Alkoxy-, Amino-, D₁₋₄-Alkylamino-, Di-(C₁₋₄-alkyl)-amino-, C₁₋₃-Alkoxycarbonyl-, Hydroxycarbonyl-carbonyl-, C₁₋₃-Alkoxycarbonyl-carbonyl-, Hydroxycarbonyl-C₁₋₃-alkyl-, C₁₋₃-Alkoxycarbonyl-C₁₋₃-alkyl-, Hydroxycarbonyl-C₁₋₃-alkylcarbonyl-, C₁₋₃-Alkoxycarbonyl-C₁₋₃-alkylcarbonyl-, Aminosulfonyl-, C₁₋₄-Alkylaminosulfonyl-, Di-(C₁₋₄-alkyl)-aminosulfonyl-, C₁₋₃-Alkylsulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)-aminocarbonyl-C₁₋₃-alkyl-, Hydroxyaminocarbonyl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppen substituiert sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom, einen C₁₋₃-Alkylrest oder, wenn Y¹ ein Stickstoffatom darstellt, auch ein freies Elektronenpaar,
R⁶ und R⁷, die gleich oder verschieden sein können, jeweils ein Wasserstoffatom, eine C₁₋₃-Alkylgruppe oder auch, wenn Y¹ ein Kohlenstoffatom darstellt, eine Amino-, C₁₋₃-Alkylamino- oder Di-(C₁₋₃-alkyl)-aminogruppe, wobei die beiden C₁₋₃-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
bedeuten,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine elfte Ausführungsform der vorliegenden Erfindung besteht in den Verbindungen der obigen allgemeinen Formel (I), in denen
A, X, D, E, G, M, Q und R¹ wie vorstehend unter der ersten, zweiten, dritten, vierten, fünften, sechsten, siebten, achten oder neunten Ausführungsform erwähnt definiert sind und
R² das Wasserstoffatom oder
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Hydroxy-, Amino-, C₁₋₆-Alkylamino-, Di-(C₁₋₆-alkyl)-amino-, Hydroxycarbonyl-, C₁₋₆-Alkoxycarbonyl-, Aminocarbonyl-, Aminocarbonylamino-, C₁₋₆-Alkylamino-, 4-Morpholinylgruppe substituiert sein kann,
wobei die in den für R² vorstehend definierten Gruppen genannten oder als Substituenten enthaltenden Phenyl- und Pyridinylreste zusätzlich im Kohlenstoffgerüst durch Halogen, durch C₁₋₃-Alkyl-, C₁₋₃-Alkoxy-, Fiydroxy-, Amino-, C₁₋₃-Alkylamino-, Di-(C₁₋₃-alkyl)-amino-, Amino-C₁₋₃-alkyl-, C₁₋₃-Alkyl-amino-C₁₋₃-alkyl-, Di-(C₁₋₃-alkyl)-amino-C₁₋₃-alkyl-, C₁₋₃-Alkylcarbonylamino-, C₁₋₃-Alkylcarbonyl-C₁₋₃-alkylamino-, Aminocarbonyl-, C₁₋₃-Alkylaminocarbonyl-oder Di-(C₁₋₃-alkyl)-aminocarbonylgruppen mono-, di- oder trisubstituiert und die Substituenten gleich oder verschieden sein können,
R³ das Wasserstoffatom oder eine C₁₋₃-Alkylgruppe darstellen oder
R² und R³ zusammen mit dem eingeschlossenen Stickstoffatom einen Rest der allgemeinen Formel 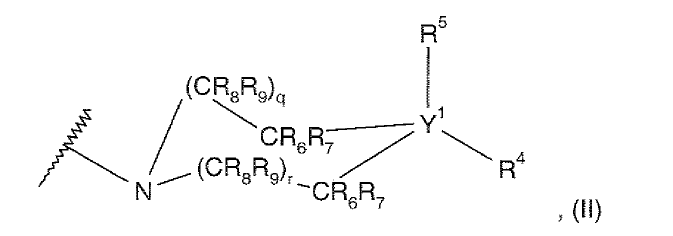 in der
Y¹ das Kohlenstoffatom oder, wenn R⁵ ein freies Elektrodenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, Cyclo-C₃₋₇-alkyl-, Cyclo-C₃₋₇-alkonyl-, Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₄-Alkyl-aminocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl-, Di-(C₁₋₄-alkyl)-aminocarbonyl-C₁₋₃-alkyl-, Aminocarbonylamino-C₁₋₃-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl-, Pyridinyl- oder Diazinylgruppe, die jeweils durch ein Halogen, durch eine C₁₋₃-Alkyl-, C₁₋₃-Alkoxy-, Hydroxy-, Amino-, C₁₋₄-Alkyl-amino-, Di-(C₁₋₄-alkyl)-amino- Amino-C₁₋₃-alkyl-, C₁₋₄-Alkyl-amino-C₁₋₃-alkyl-, Di-(C₁₋₄-alkyl)-amino-C₁₋₃-alkylgruppe substituiert sein können,
ein Heterocyclus ausgewählt aus einer 4- bis 7-gliedrigen Azacycloalkylgruppe, einer 6- bis 7-gliedrigen Oxaza- und Diazacycloalkylgruppe und einer 7- bis 9-gliedrigen Azabicycloalkylgreppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclische Heterocyclen eine nicht direkt mit einem Stickstoff-, Sauerstoff- oder Schwefelatom verknüpfte Methylengruppe durch ein oder zwei Fluoratome substituiert sein kann und
die vorstehend genannten mono- und bicyclischen Heterocyclen ein- oder mehrfach, beispielsweise ein- bis dreifach, durch C₁₋₃-Alkylgruppen oder einfach durch eine Benzyl-, Cyclo-C₃₋₆-alkyl-, Cyclo-C₃₋₆-alkyl-C₁₋₃-alkyl, C₁₋₄-Alkylcarbonyl-, Hydroxy-, C₁₋₄-Alkoxy-, Amino-, C₁₋₄-Alkylamino-, Di-(C₁₋₄-alkyl)-amino-, Hydroxycarbonyl-, C₁₋₃-Alkoxycarbonyl-, Hydroxycarbonyl-carbonyl-, C₁₋₃-Alkoxycarbonyl-carbonyl-, Hydroxycarbonyl-C₁₋₃-alkyl-, C₁₋₃-Alkoxycarbonyl-C₁₋₃-alkyl- oder C₁₋₃-Alkylsulfonylgruppen substituiert sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom, einen C₁₋₃-Alkylrest oder, wenn Y¹ ein Stickstoffatom darstellt, auch ein freies Elektronenpaar,
R⁶ und R⁷, die gleich oder verschieden sein können, jeweils ein Wasserstoffatom, eine C₁₋₃-Alkylgruppe oder auch, wenn Y¹ ein Kohlenstoffatom darstellt, eine C₁₋₃-Alkylamino- oder Di-(C₁₋₃-alkyl)-aminogruppe, wobei die beiden C₁₋₃-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
bedeuten,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine zwölfte Ausführungsform der vorliegenden Erfindung besteht in den Verbindungen der obigen allgemeinen Formel (I), in denen
A, X, D, E, G, M, Q und R¹ wie vorstehend unter der ersten, zweiten, dritten, vierten, fünften, sechsten, siebten, achten oder neunten Ausführungsform erwähnt definiert sind und
R² das Wasserstoffatom oder
eine Phenylmethylgruppe oder eine C₂₋₇-Alkylgruppe, die in ω-Stellung durch eine Phenyl-, Amino-, C₁₋₆-Alkylamino-, Di-(C₁₋₆-alkyl)-aminogruppe substituiert sein kann,
wobei die vorstehend erwähnte Phenyl- und Phenylmethylgruppe zusätzlich an einem aromatischen Kohlenstoffatom durch Halogen, durch C₁₋₃-Alkyl-, C₁₋₃-Alkoxy-, Amino-C₁₋₃-alkyl-, C₁₋₃-Alkylamino-C₁₋₃-alkyl- oder Di-(C₁₋₃-alkyl)-amino-C₁₋₃-alkylgruppen mono- oder disubstituiert und die Substituenten gleich oder verschieden sein können,
R³ das Wasserstoffatom oder eine C₁₋₃-Alkylgruppe darstellen oder
R² und R³ zusammen mit dem eingeschlossenen Stickstoffatom einen Rest der allgemeinen Formel 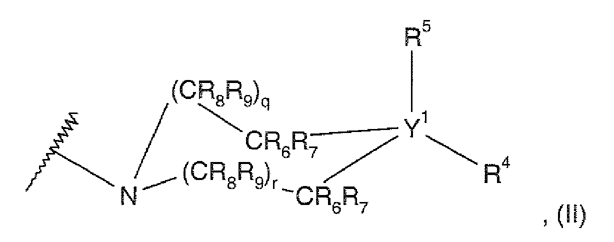 in der
Y¹ das Kohlenstoffatom oder, wenn R⁵ ein freies Elektrodenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Amino-, C₁₋₄-Alkylamino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl- oder Cyclo-C₃₋₇-alkenylgruppe, eine Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl- oder Pyridylgruppe, die jeweils durch ein Halogen, durch eine C₁₋₃-Alkyl-, C₁₋₃-Alkoxy-, Amino-, C₁₋₄-Alkyl-amino-, Di-(C₁₋₄-alkyl)-aminogruppe substituiert sein kann,
ein Heterocyclus ausgewählt aus einer 6- bis 7-gliedrige Azacycloalkylgruppe, einer 6- bis 7-gliedrige *S*,*S*-Dioxothiaza- und Diazacycloalkylgruppe und einer 7-bis 9-gliedrige Azabicycloalkylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methylengruppe durch ein oder zwei Fluoratome substituiert sein kann und
die vorstehend genannten mono- und bicyclischen Heterocyclen ein- oder zweifach durch eine Hydroxy-, C₁₋₃-Alkyl- oder Hydroxy-C₁₋₃-alkylgruppe, durch eine Benzyl-, Cyclo-C₃₋₆-alkyl-, hydroxy-C₃₋₆-cycloalkyl-, Cyclo-C₃₋₆-alkyl-C₁₋₃-alkyl, C₁₋₃-Alkylcarbonyl-C₁₋₃-alkyl-, Amino-, C₁₋₄-Alkylamino-oder Di-(C₁₋₄-alkyl)-amino-, Hydroxycarbonyl-carbonyl-, C₁₋₆-Alkoxycarbonyl-carbonyl-, Hydroxycarbonyl-C₁₋₃-alkylcarbonyl-, C₁₋₃-Alkoxycarbonyl-C₁₋₃-alkylcarbonyl-, Aminosulfonyl-, C₁₋₄-Alkylaminosulfonyl-, Di-(C₁₋₄-alkyl)-aminosulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)-aminocarbonyl-C₁₋₃-alkyl-, Hydroxyaminocarbonyl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppen substituiert sein können,
oder auch, wenn Y¹ das Kohlenstoffatom darstellt die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom oder, wenn Y¹ ein Stickstoffatom darstellt, auch ein freies Elektronenpaar,
R⁶ und R⁷, die gleich oder verschieden sein können, jeweils ein Wasserstoffatom, eine C₁₋₃-Alkylgruppe oder auch, wenn Y¹ ein Kohlenstoffatom darstellt, eine C₁₋₃-Alkylamino- oder Di-(C₁₋₃-alkyl)-aminogruppe, wobei die beiden C₁₋₃-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
bedeuten, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine dreizehnte Ausführungsform der vorliegenden Erfindung besteht in den Verbindungen der obigen allgemeinen Formel (I), in denen
A, X, D, E, G, M, Q und R¹ wie vorstehend unter der ersten, zweiten, dritten, vierten, fünften, sechsten, siebten, achten oder neunten Ausführungsform erwähnt definiert sind und
R² eine Phonylmethylgruppe oder eine C₂₋₇-Alkylgruppe, die in ω-Stellung durch eine Phenyl-, Amino-, C₁₋₆-Alkylamino-, Di-(C₁₋₆-alkyl)-aminogruppe substituiert sein kann,
wobei die vorstehend genannte Phenyl- und Phenylmethylgruppe an einem aromatischen Kohlenstoffatom durch eine Amino-C₁₋₃-alkyl-, C₁₋₃-Alkylamino-C₁₋₃-alkyl- oder Di-(C₁₋₃-alkyl)-amino-C₁₋₃-alkylgruppe substituiert sein kann,
R³ das Wasserstoffatom oder eine C₁₋₃-Alkylgruppe oder
R² und R³ zusammen mit dem eingeschlossenen Stickstoffatom einen Rest der allgemeinen Formen 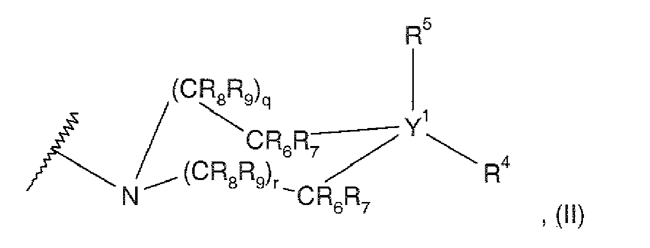 in der
R⁶ und R⁷ jeweils ein Wasserstoffatom oder eine Dimethylaminogruppe,
R⁸ und R⁹ jeweils das Wasserstoffatom und
(a) Y¹ das Kohlenstoffatom,
   q und r die Zahlen 0 oder 1,
   R⁴ das Wasserstoffatom,
   eine Phenyl-, Pyridinyl- oder Pyrimidinylgruppe, die jeweils durch ein Halogen, durch eine Amino-, Methylamino-, Dimethylamino-, Methyl-oder Methoxygruppe substituiert sein kann, eine Hydroxy-, 2-Diethylamino-ethyl-, Amino-, Methylamino-, Dimethylamino-, Diethylamino-, Pyrrolidin-1-yl-, 3-Hydroxy-pyrrolidin-1-yl-, 2-Hydroxycarbonyl-pyrrolidin-1-yl-, 2-Methoxycarbonyl-pyrrolidin-1-yl-, Piperidin-1-yl-, 4,4-Dimethylpiperidin-1-yl-, 4-Amino-4-methyl-piperidin-1-yl-, 2-Hydroxycarbonyl-piperidin-1-yl-, 2-Methoxycarbonyl-piperidin-1-yl-, 4-Hydroxymethyl-piperidin-1-yl-, 4-(1-Hydroxycyclopropyl)-piperidin-1-yl-, 4-Amino-piperidin-1-yl-, 4-Methylamino-piperidin-1-yl-, 4-Dimethylamino-piperidin-1-yl-, 4-Hydroxy-4-methyl-piperidin-1-yl-, 4-Hydroxy-4-ethyl-piperidin-1-yl-, 4-Hydroxy-4-trifluormethyl-piperidin-1-yl-, 4-Hydroxy-4-hydroxymethyl-piperidin-1-yl-, 3-Amino-piperidin-1-yl-, 3-Methylamino-piporidin-1-yl-, 3-Dimethylamino-piperidin-1-yl-, 3-Hydroxy-piperidin-1-yl-, 4-Hydroxypiperidin-1-yl-, 4-Hydroxycarbonylmethyl-piperidin-1-yl-, 4-Ethoxycarbonylmethyl-piperidin-1-yl-, Perhydro-azepin-1-yl-, Perhydro-1,4-diazepin-1-yl-, 4-Methyl-perhydro-1,4-diazepin-1-yl-, 1-Methyl-piperidin-4-yl-, Piperidin-4-yl-, 1-Ethylpiperidin-4-yl-, 1-(2-Hydroxyethyl)-piperidin-4-yl-, 1-Cyclopropyl-piperidin-4-yl-, 1-Cyclopropylmethyl-piperidin-4-yl-, 1-Methylsulfonyl-piperidin-4-yl-, 1-Ethylsulfonyl-piperidin-4-yl-, 1-Isopropylsulfonyl-piperidin-4-yl-, 1-Cyclopropylsulfonyl-piperidin-4-yl-, 4-Hydroxy-1-methylsulfonyl-piperidin-4-yl-, 1-Aminosulfonyl-piperidin-4-yl-, 1-(Methylaminosulfonyl)-piperidin-4-yl-, 1-(Dimethylaminosulfonyl)-piperidin-4-yl-, 1-Hydroxycarbonylmethyl-piperidin-4-yl-, 1-Ethoxycarbonylmethyl-piperidin-4-yl-, 1-(2-Hydroxycarbonylethyl)-piperidin-4-yl-, 1-(2-Ethoxycarbonylethyl)-piperidin-4-yl-, 1-(3-Hydroxycarbonyl-propionyl)-piperidin-4-yl-, 1-(3-Ethoxycarbonyl-propionyl)-piperidin-4-yl-, 1-(Hydroxycarbamoyl-methyl)-piperidin-4-yl-, 1-(Hydroxy-methylcarbamoyl-methyl)-piperidin-4-yl-, 1-(Methoxycarbamoyl-methyl)-piperidin-4-yl-, 1-Oxalyl-piperidin-4-yl-, 1-Ethoxyoxalyl-piperidin-4-yl-, Piperazin-1-yl-, 4-Mothyl-piperazin-1-yl-, 4-Cyclopropylmethyl-piperazin-1-yl-, 4-Ethyl-piperazin-1-yl-, 4-(2-Hydroxyethyl)-piperazin-1-yl-, 4-Cyclopropyl-piperazin-1-yl-, 4-Methylsulfonyl-piperazin-1-yl-, 4-Aminosulfonyl-piperazin-1-yl-, 4-(Methylaminosulfonyl)-piperazin-1-yl-, 4-(Dimethylaminosulfonyl)-piperazin-1-yl-, 4-Hydroxycarbonylmethyl-piperazin-1-yl-, 4-Ethoxycarbonylmethyl-piperazin-1-yl-, 4-(2-Hydroxycarbonylethyl)-piperazin-1-yl-, 4-(2-Ethoxycarbonylethyl)-piperazin-1-yl-, 4-(3-Hydroxycarbonyl-propionyl)-piperazin-1-yl-, 4-(3-Ethoxycarbonyl-propionyl)-piperazin-1-yl-, 4-(Hydroxycarbamoyl)-methyl-piperazin-1-yl-, 4-(Hydroxy-methyl-carbamoyl)-methyl-piporazin-1-yl-, 4-(Methoxycarbamoyl)-methyl-piperazin-1-yl-, 1,2-Dimethyl-piperazin-1-yl-, 3-Mothyl-piporazin-1-yl-, 3,4,5-Trimethyl-piperazin-1-yl-, 3,5-Dimethyl-piperazin-1-yl-, 3,3,4-Trimethyl-piperazin-1-yl-, 3,3-Dimethyl-piperazin-1-yl-, 3,3,4,5,5-Pentamethyl-piperazin-1-yl-, 3,3,5,5-Tetramethyl-piperazin-1-yl-, 3,3,3-Trifluor-2-oxo-propyl)-piperazin-1-yl-, Morpholin-4-yl-, 1,1-Dioxo-1λ⁶-thiomorpholin-4-yl-, Tetrahydropyran-4-yl-, 4,4-Difluor-piperidin-1-yl-, 8-Methyl-8-aza-bicyclo[3.2.1]oct-3-yl-, 8-Aza-bicyclo[3.2.1]oct-3-yl-, Azetidin-1-yl-, 1-(Methoxycarbonylmethyl)-piporidin-4-yl-, 1-(Ethoxycarbonylmethyl)-piperidin-4-yl-, 4-(Ethoxycarbonylmethyl)-piperazin-1-yl-, 1-Hydroxycarbonylmethyl-piperidin-4-yl- oder 4-Hydroxycarbonylmethyl-piperazin-1-yl-gruppe, und
   R⁵ ein Wasserstoffatom, oder
(b) Y¹ ein Stickstoffatom,
   q und r die Zahlen 1 oder 2,
   R⁴ das Wasserstoffatom,
   eine Phenyl-, Pyridinyl- oder Pyrimidinylgruppe, die jeweils durch ein Halogen, durch eine Amino-, Methylamino-, Dimethylamino-, Methyl-oder Methoxygruppe substituiert sein kann, eine Methyl-, Ethyl-, Isopropyl-, Cyclopropyl-, Cyclopentyl-, Cyclohexyl-, Cycloheptyl-, 4-Hydroxycarbonylmethyl-cylohexyl-, 4-Ethoxycarbonylmethyl-cyclohexyl-, Cyclopropylmethyl-, 2-Diethylamino-propyl-, 1-Chinuclidin-3-yl-, Tetrahydropyran-4-yl-, 1-Piperidin-4-yl-, 1-Methyl-piperidin-4-yl-, 1-Ethyl-piperidin-4-yl-, 1-(2-Hydroxyethyl)-piperidin-4-yl-, 1-Methylsulfonyl-piperidin-4-yl-, 1-Aminosulfonyl-piperidin-4-yl-, 1-(Methylaminosulfonyl)-piperidin-4-yl-, 1-(Dimethylaminosulfonyl)-piperidin-4-yl-, 1-Hydroxycarbonylmethyl-piperidin-4-yl-, 1-Ethoxycarbonylmethyl-piperidin-4-yl-, 1-(2-Hydroxycarbonylethyl)-piperidin-4-yl-, 1-(2-Ethoxycarbonylethyl)-piperidin-4-yl-, 1-(3-Hydroxycarbonyl-propionyl)-piperidin-4-yl-, 1-(3-Ethoxycarbonyl-propionyl)-piperidin-4-yl-, 1-(Hydroxycarbamoyl-methyl)-piperidin-4-yl-, 1-(Hydroxy-methyl-carbamoyl-methyl)-piperidin-4-yl-, 1-(Methoxycarbamoyl-methyl)-piperidin-4-yl-, 1-Cyclopropyl-piperidin-4-yl-, 1-Cyclopropylmethyl-piperidin-4-yl-, 1-Hydroxycarbonylmethyl-piperidin-4-yl- oder 1-Ethoxycarbonylmethyl-piperidin-4-yl-gruppe und
   R⁵ ein freies Elektronenpaar darstellen,
   bedeuten, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine vierzehnte Ausführungsform der vorliegenden Erfindung besteht in den Verbindungen der obigen allgemeinen Formel (I), in denen
A, X, D, E, G, M, Q und R¹ wie vorstehend unter der ersten, zweiten, dritten, vierten, fünften, sechsten, siebten, achten oder neunten Ausführungsform erwähnt definiert sind und
R² eine Phenylmethylgruppe oder eine C₂₋₇-Alkylgruppe, die in ω-Stellung durch eine Phenyl-, Amin-, C₁₋₆-Alkylamino-, Di-(C₁₋₆-alkyl)-aminogruppe substituiert sein kann,
wobei die vorstehend genannte Phenyl- und Phenylmethylgruppe an einem aromatischen Kohlenstoffatom durch eine Amino-C₁₋₃-alkyl-, C₁₋₃-Alkylamino-C₁₋₃-alkyl- oder Di-(C₁₋₃-alkyl)-amino-C₁₋₃-alkylgruppe substituiert sein kann,
R³ das Wasserstoffatom oder eine C₁₋₃-Alkylgruppe oder
R² und R³ zusammen mit dem eingeschlossenen Stickstoffatom einen Rest der allgemeinen Formel 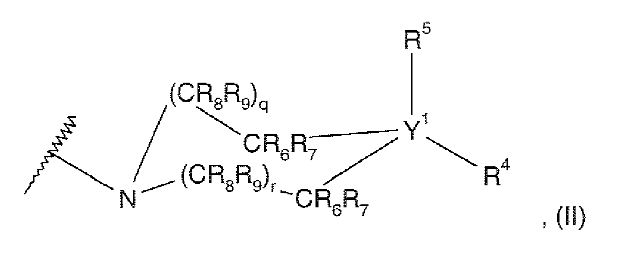 in der
R⁶ und R⁷ jeweils ein Wasserstoffatom oder eine Dimethylaminogruppe,
R⁸ und R⁹ jeweils das Wasserstoffatom und
(a) Y¹ das Kohlenstoffatom,
   q und r die Zahlen 0 oder 1,
   R⁴ das Wasserstoffatom,
   eine Phenyl-, Pyridinyl- oder Pyrimidinylgruppe, die jeweils durch ein Halogen, durch eine Amino-, Methylamino-, Dimethylamino-, Methyl-oder Methoxygruppe substituiert sein kann,
   eine Hydroxy-, 2-Diethylamino-ethyl-, Amino-, Methylamino-, Dimethylamino-, Diethylamino-, Pyrrolidin-1-yl-, Piperidin-1-yl-, 4-Amino-piperidin-1-yl-, 4-Methylamino-piperidin-1-yl-, 4-Dimethylamino-piperidin-1-yl-, 3-Amino-piperidin-1-yl-, 3-Methylamino-piperidin-1-yl-, 3-Dimethylamino-piperidin-1-yl-, Perhydro-azepin-1-yl-, Perhydro-1,4-diazepin-1-yl-, 4-Methyl-perhydro-1,4-diazepin-1-yl-, 1-Methyl-piperidin-4-yl-, Piperidin-4-yl-, 1-Ethylpiperidin-4-yl-, 1-Cyclopropyl-piperidin-4-yl-, 1-Cyclopropylmethyl-piperidin-4-yl-, Piperazin-1-yl-, 4-Methyl-piperazin-1-yl-, 4-Cyclopropylmethyl-piperazin-1-yl-, 4-Ethyl-piperazin-1-yl-, 4-Cyclopropyl-piperazin-1-yl-, 1,2-Dimethyl-piperazin-1-yl-, 3-Methylpiperazin-1-yl-, 3,4,5-Trimethyl-piperazin-1-yl-, 3,5-Dimethyl-piperazin-1-yl-, 3,3,4-Trimethyl-piperazin-1-yl-, 3,3-Dimethyl-piperazin-1-yl-, 3,3,4,5,5-Pentamethyl-piperazin-1-yl-, 3,3,5,5-Tetramethyl-piperazin-1-yl-, Morpholin-4-yl-, 4,4-Difluor-piperidin-1-yl-, 8-Methyl-8-aza-bicyclo[3.2.1]oct-3-yl-, 8-Aza-bicyclo[3.2.1]oct-3-yl-, Azetidin-1-yl-, 1-(Methoxycarbonylmethyl)-piperidin-4-yl-, 1-(Ethoxycarbonylmethyl)-piperidin-4-yl-, 4-(Ethoxycarbonylmethyl)-piperazin-1-yl-, 1-Hydroxycarbonylmethyl-piperidin-4-yl- oder 4-Hydroxycarbonylmethyl-piperazin-1-ylgruppe, und
   R⁵ ein Wasserstoffatom, oder
(b) Y¹ ein Stickstoffatom,
   q und r die Zahlen 1 oder 2,
   R⁴ das Wasserstoffatom,
   eine Phenyl-, Pyridinyl- oder Pyrimidinylgruppe, die jeweils durch ein Halogen, durch eine Amino-, Methylamino-, Dimethylamino-, Methyl-oder Methoxygruppe substituiert sein kann,
   eine Methyl-, Ethyl-, Isopropyl-, Cyclopropyl-, Cyclopropylmethyl-, 2-Diethylamino-propyl-, 1-Chinuclidin-3-yl-, 1-Piperidin-4-yl-, 1-Methylpiperidin-4-yl-, 1-Ethyl-piperidin-4-yl-, 1-Cyclopropyl-piperidin-4-yl-, 1-Cyclopropylmethyl-piperidin-4-yl-, 1-Hydroxycarbonylmethyl-piperidin-4-yl- oder 1-Ethoxycarbonylmethyl-piperidin-4-ylgruppe und
   R⁵ ein freies Elektronenpaar darstellen,
   bedeuten, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine fünfzehnte Ausführungsform der vorliegenden Erfindung besteht in den Verbindungen der obigen allgemeinen Formel (I), in denen
D, E, G, M, Q, R¹, R² und R³ wie vorstehend unter der ersten, zweiten, dritten, vierten, fünften, sechsten, siebten, achten, neunten, zehnten, elften, zwölften oder dreizehnten Ausführungsform erwähnt definiert sind und
A und X jeweils ein Sauerstoffatom darstellen,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine sechzehnte Ausführungsform der vorliegenden Erfindung besteht in den Verbindungen der obigen allgemeinen Formel (I), in denen
A und X jeweils ein Sauerstoffatom darstellen,
R¹ eine 1,3,4,5-Tetrahydro-1,3-benzdiazepin-2-on-3-yl-, 3,4-Dihydro-1*H*-chinazolin-2-on-3-yl-, 5-Phonyl-2,4-dihydro-1,2,4-triazol-3-on-2-yl-, 1,3-Dihydro-imidazo[4,5-*c*]-chinolin-2-on-3-yl-, 1,3-Dihydro-naphth[1,2-*d*]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H*-thieno-[3,2-*d*]pyrimidin-2-on-3-yl- oder 3,4-Dihydro-1*H*-thieno[3,4-*d*]pyrimidin-2-on-3-ylgrup-pe bedeutet,
und R² und R³ wie vorstehend unter der ersten oder zweiten Ausführungsform erwähnt definiert sind,
wobei die vorstehend unter R¹ erwähnten Heterocyclen im Kohlenstoffgerüst zusätzlich durch eine Methoxygruppe monosubstituiert sein können,
und wobei alle in den unter R¹ definierten Resten erwähnten oder enthaltenen aromatischen und heteroaromatischen Reste und Molekülteile zusätzlich durch Halogenatome, durch Cyano- oder Hydroxygruppen mono-, di- oder trisubstituiert und die Substituenten gleich oder verschieden sein können,
und wobei bei dieser und allen voranstehend genannten Ausführungsformen jeweils den Verbindungen, in denen
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe und
R^{a}, R^{b} und R^{c} unabhängig voneinander jeweils ein Wasserstoff- oder Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₂-alkyl-, Hydroxy-C₃-alkenyl-, Hydroxy-C₃-alkinyl-, C₁₋₄-Alkoxy-, C₁₋₄-Alkoxy-C₁₋₂-alkyl-, Amino-, C₁₋₄-Alkyl-amino-, C₃₋₄-Alkenyl-amino-, C₃₋₄-Alkinyl-amino-, Di-(C₁₋₄-alkyl)-amino-, Di-(C₃₋₄-alkenyl)-amino-, Di-(C₃₋₄-alkinyl)-amino-, Amino-C₁₋₂-alkyl-, C₁₋₃-Alkyl-amino-C₁₋₂-alkyl-, Di-(C₁₋₃-alkyl)-amino-C₁₋₂-alkyl-, Amino-C₃-alkenyl-, C₁₋₃-Alkyl-amino-C₃-alkenyl-, Di-(C₁₋₃-alkyl)-amino-C₃-alkenyl-, Amino-C₃-alkinyl-, C₁₋₃-Alkyl-amino-C₃-alkinyl-, Di-(C₁₋₃-alkyl)-amino-C₃-alkinyl-, Hydroxycarbonyl-, C₁₋₄-Alkyl-carbonyl-, Formyl-, C₁₋₄-Alkoxy-carbonyl-, Aminocarbonyl-, C₁₋₄-Alkyl-aminocarbonyl-, Di-(C₁₋₄-alkyl)-aminocarbonyl-, Formylamino-, C₁₋₄-Alkyl-carbonylamino-, Formyl-C₁₋₄-alkyl-amino-, C₁₋₄-Alkylcarbonyl-C₁₋₄-alkyl-amino-, C₁₋₄-Alkyl-sulfonyl-, C₁₋₄-Alkyl-sulfinyl-, C₁₋₄-Alkyl-sulfonylamino-, C₁₋₄-Alkyl-sulfonyl-C₁₋₄-alkylamino-, Aminosulfonyl-, C₁₋₄-Alkylaminosulfonyl- oder Di-(C₁₋₄-alkyl)-aminosulfonylgruppe darstellen,
wobei in den Definitionen der Reste R^{a}, R^{b} und R^{c} erwähnte oder enthaltene Alkyl-, Alkenyl- und Alkinylgruppen geradkettig oder verzweigt sein können, jede in diesen Resten enthaltene Methingruppe mit einem Fluoratom, jede Methylengruppe mit bis zu 2 Fluoratomen und jede Methylgruppe mit bis zu 3 Fluoratomen substituiert sein kann und
die Doppel- und Dreifachbindungen der in den für R^{a}, R^{b} und R^{c} vorstehend definierten Gruppen enthaltenen C₃₋₄-Alkenyl- oder C₃₋₄-Alkinylgruppen von in diesen Gruppen gegebenenfalls ebenfalls enthaltenen Heteroatomen isoliert sind,
eine herausragende Bedeutung zukommt,
den Verbindungen, in denen
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe und
R^{a}, R^{b} und R^{c} unabhängig voneinander jeweils ein Wasserstoff- oder Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₂-alkyl-, C₁₋₄-Alkoxy-, Amin-, C₁₋₄-Alkyl-amino-, Di-(C₁₋₄-alkyl)-amino-, Amino-C₁₋₂-alkyl-, C₁₋₃-Alkyl-amino-C₁₋₂-alkyl-, Di-(C₁₋₃-alkyl)-amino-C₁₋₂-alkyl-, Hydroxycarbonyl-, C₁₋₄-Alkyl-carbonyl-, Formyl-, C₁₋₄-Alkoxycarbonyl-, Aminocarbonyl-, C₁₋₄-Alkyl-aminocarbonyl-, Di-(C₁₋₄-alkyl)-aminocarbonyl-, Formylamino-, C₁₋₄-Alkyl-carbonylamino-, Formyl-C₁₋₄-alkyl-amino- oder C₁₋₄-Alkylcarbonyl-C₁₋₄-alkyl-aminogruppe darstellen,
wobei in den Definitionen der Reste R^{a}, R^{b} und R^{c} erwähnte oder enthaltene Alkyl-, Alkenyl- und Alkinylgruppen geradkettig oder verzweigt sein können und jede in diesen Resten enthaltene Methingruppe mit einem Fluoratom, jede Methylengruppe mit bis zu 2 Fluoratomen und jede Methylgruppe mit bis zu 3 Fluoratomen substituiert sein kann,
eine besonders herausragende Bedeutung zukommt, und
den Verbindungen, in denen
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe und
R^{a}, R^{b} und R^{c} unabhängig voneinander jeweils ein Wasserstoff- oder Halogenatom, eine Methyl-, Difluormethyl-, Trifluormethyl-, Ethyl-, Vinyl-, Ethinyl-, Cyano-, Hydroxy-, Methoxy-, Difluormethoxy-, Trifluormethoxy-, Amino-, Methylamin- oder Dimethylaminogruppe darstellen,
eine ganz besonders herausragende Bedeutung zukommt,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine siebzehnte Ausführungsform der vorliegenden Erfindung besteht in den Verbindungen der obigen allgemeinen Formel (I), in denen
A und X jeweils ein Sauerstoffatom bedeuten,
R¹ wie vorstehend unter der fünften Ausführungsform definiert ist,
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe und
R^{a}, R^{b} und R^{c} unabhängig voneinander jeweils ein Wasserstoff- oder Halogenatom, eine Methyl-, Difluormethyl-, Trifluormethyl-, Ethyl-, Vinyl-, Ethinyl-, Cyano-, Hydroxy-, Methoxy-, Difluormethoxy-, Trifluormethoxy-, Amino-, Methylamino- oder Dimethylaminogruppe darstellen,
und wobei bei dieser und allen voranstehend genannten Ausführungsformen jeweils den Verbindungen, in denen
R² und R³ wie vorstehend unter der zehnten oder elften Ausführungsform definiert sind, eine herausragende Bedeutung zukommt,
den Verbindungen, in denen R² und R³ wie vorstehend unter der zwölften Ausführungsform definiert sind, eine besonders herausragende Bedeutung zukommt, und den Verbindungen, in denen R² und R³ wie vorstehend unter der dreizehnten Ausführungsform definiert sind, eine ganz besonders herausragende Bedeutung zukommt,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine achzehnte Ausführungsform der vorliegenden Erfindung besteht in den Verbindungen der obigen allgemeinen Formel (I), in denen
A und X jeweils ein Sauerstoffatom,
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe,
R^{a}, R^{b} und R^{c} unabhängig voneinander jeweils ein Wasserstoff- oder Halogenatom, eine C₁₋₃-Alkyl-, Trifluormethyl-, Cyano-, Hydroxy-, Methoxy-, Trifluormethoxy-, Amino-, Methylamino- oder Dimethylaminogruppe,
R¹ einen einfach ungesättigten, über ein Stickstoffatom mit dem Piperidinring in Formel (I) verknüpften 5- bis 7-gliedrigen Diaza-Heterocyclus,
wobei der vorstehend erwähnte Heterocyclus eine Carbonylgruppe benachbart zu einem Stickstoffatom enthält und die Carbonylgruppe vorzugsweise mit zwei Stickstoffatomen verknüpft ist sowie
die olefinische Doppelbindung des Heterocyclus mit einem Phenyl- oder Thienyl-Ring kondensiert ist und der Phenyl- und Thienyl-Ring durch Halogenatome, durch Methyl-, Methoxy-, Difluormethyl-, Trifluormethyl-, Hydroxy-, Amino-, C₁₋₃-Alkylamino-, Di-(C₁₋₃-alkyl)-amino-, Acetylamino-, Acetyl-, Cyano-Difluormethoxy- oder Trifluormethoxygruppen mono-, di- oder trisubstituiert sein kann, wobei die Substituenten gleich oder verschieden sein können, vorzugsweise jedoch unsubstituiert oder durch ein Halogenatom, durch eine Methyl- oder Methoxygruppe monosubstituiert ist,
und für R¹ beispielsweise eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-*c*]chinolin-2-on-3-yl-, 1,3-Dihydro-naphth[1,2-*d*]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H*-thieno[3,2-*d*]pyrimidin-2-on-3-yl- oder 3,4-Dihydro-1*H*-thieno[3,4-*d*]pyrimidin-2-on-3-ylgruppe genannt seien, die an einem ungesättigten Kohlenstoffatom des aromatischen oder heteroaromatischen Teils durch Halogenatome, durch Cyano- oder Hydroxygruppen mono-, di- oder trisubstituiert und die Substituenten gleich oder verschieden sein können, vorzugsweise jedoch unsubstituiert sind,
R² und R³ zusammen mit dem eingeschlossenen Stickstoffatom einen Rest der allgemeinen Formel 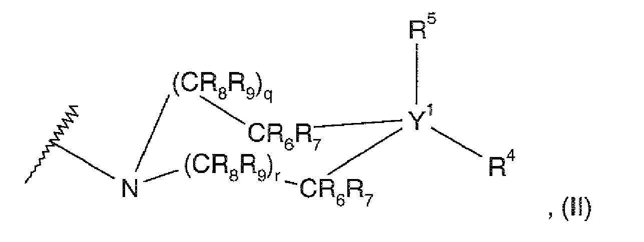 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, wobei die Summe aus q und r 1, 2 oder 3 beträgt,
q und r, wenn Y¹ das Stickstoffatom darstellt, die Zahlen 1 oder 2, wobei die Summe aus q und r 2 oder 3 beträgt,
R⁴ das Wasserstoffatom, eine Amino-, C₁₋₄-Alkylamino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl-, Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl- oder Pyridylgruppe, die jeweils durch ein Halogenatom, durch eine C₁₋₃-Alkyl-, C₁₋₃-Alkoxy-, Amino-, C₁₋₄-Alkyl-amino-, oder Di-(C₁₋₄-alkyl)-aminogruppe substituiert sein kann,
ein Heterocyclus ausgewählt aus einer 5- bis 7-gliedrigen Azacycloalkyl- oder *S*,*S*-Dioxothiazagruppe und einer 6- bis 7-gliedrigen Diazacycloalkylgruppe,
wobei die vorstehend genannten Heterocyclen über ein Stickstoff- oder ein Kohlenstoffatom an Y¹ in Formel (II) gebunden sind und
durch eine oder zwei Hydroxy-, C₁₋₃-Alkyl- oder Hydroxy-C₁₋₃-alkylgruppen oder durch eine Cylco-C₃₋₆-alkyl-, Hydroxy-C₃₋₆-cycloalkyl-, Cyclo-C₃₋₆-alkyl-C₁₋₃-alkyl, C₁₋₃-Alkylcarbonyl-C₁₋₃-alkyl-, Amin-, C₁₋₄-Alkylamino-, Di-(C₁₋₄-alkyl)-amino-, Hydroxycarbonyl-carbonyl-, C₁₋₃-Alkoxycarbonyl-carbonyl-, Hydroxycarbonyl-C₁₋₃-alkylcarbonyl-, C₁₋₃-Akoxycarbonyl-C₁₋₃-alkylcarbonyl-, Aminosulfonyl-, C₁₋₄-Alkylaminosulfonyl-, Di-(C₁₋₄-alkyl)-aminosulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)-aminocarbonyl-C₁₋₃-alkyl-, Hydroxyaminocarbonyl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppen substituiert sein können,
R⁵ ein Wasserstoffatom oder, wenn Y¹ ein Stickstoffatom darstellt, auch ein freies Elektronenpaar und
R⁶, R⁷, R⁸ und R⁹, die gleich oder verschieden sein können, jeweils ein Wasserstoffatom oder eine C₁₋₃-Alkylgruppe darstellen,
bedeuten, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine neunzehnte Ausführungsform der vorliegenden Erfindung besteht in den Verbindungen der obigen allgemeinen Formel (I), in denen
A und X jeweils ein Sauerstoffatom,
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe,
R^{a}, R^{b} und R^{c} unabhängig voneinander jeweils ein Wasserstoff- oder Halogenatom, eine C₁₋₃-Alkyl-, Trifluormethyl-, Cyano-, Hydroxy-, Methoxy-, Trifluormethoxy-, Amino-, Methylamino- oder Dimethylaminogruppe,
R¹ einen einfach ungesättigten, über ein Stickstoffatom mit dem Piperidinring in Formel (I) verknüpften 5- bis 7-gliedrigen Diaza-Heterocyclus,
wobei der vorstehend erwähnte Heterocyclus eine Carbonylgruppe benachbart zu einem Stickstoffatom enthält und die Carbonylgruppe vorzugsweise mit zwei Stickstoffatomen verknüpft ist sowie
die olefinische Doppelbindung des Heterocyclus mit einem Phenyl- oder Thienyl-Ring kondensiert ist und der Phenyl- und Thienyl-Ring durch Halogenatome, durch Methyl-, Methoxy-, Difluormethyl-, Trifluormethyl-, Hydroxy-, Amino-, C₁₋₃-Alkylamino-, Di-(C₁₋₃-alkyl)-amino-, Acetylamino-, Acetyl-, Cyano-Difluormethoxy- oder Trifluormethoxygruppen mono-, di- oder trisubstituiert sein kann, wobei die Substituenten gleich oder verschieden sein können, vorzugsweise jedoch unsubstituiert oder durch ein Halogenatom, durch eine Methyl- oder Methoxygruppe monosubstituiert ist,
und für R¹ beispielsweise eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-*c*]chinolin-2-on-3-yl-, 1,3-Dihydro-naphth[1,2-*d*]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H*-thieno[3,2-*d*]pyrimidin-2-on-3-yl- oder 3,4-Dihydro-1*H*-thieno[3,4-*d*]pyrimidin-2-on-3-ylgruppe genannt seien, die an einem ungesättigten Kohlenstoffatom des aromatischen oder heteroaromatischen Teils durch Halogenatome, durch Cyano- oder Hydroxygruppen mono-, di- oder trisubstituiert und die Substituenten gleich oder verschieden sein können, vorzugsweise jedoch unsubstituiert sind,
R² und R³ zusammen mit dem eingeschlossenen Stickstoffatom einen Rest der allgemeinen Formel 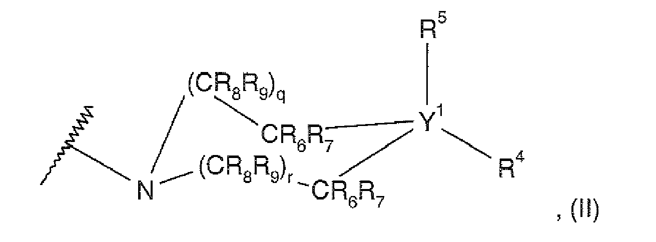 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, wobei die Summe aus q und r 1, 2 oder 3 beträgt,
q und r, wenn Y¹ das Stickstoffatom darstellt, die Zahlen 1 oder 2, wobei die Summe aus q und r 2 oder 3 beträgt,
R⁴ das Wasserstoffatom, eine Amino-, C₁₋₄-Alkyl-amino-, Di-(C₁₋₄-alkyl)-alkylamino-, C₁₋₆-Alkyl-, Cyclo-C₃₋₇-alkyl-, Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl- oder Pyridylgruppe, die jeweils durch ein Halogenatom, durch eine C₁₋₃-Alkyl-, C₁₋₃-Alkoxy-, Amino-, C₁₋₄-Alkyl-amino-, oder Di-(C₁₋₄-alkyl)-aminogruppe substituiert sein kann,
ein Heterocyclus ausgewählt aus einer 5- bis 7-gliedrigen Azacycloalkylgruppe und einer 6- bis 7-gliedrigen Diazacycloalkylgruppe,
wobei die vorstehend genannten Heterocyclen über ein Stickstoff- oder ein Kohlenstoffatom an Y¹ in Formel (II) gebunden sind und
durch eine C₁₋₃-Alkyl-, Cyclo-C₃₋₆-alkyl-, Cyclo-C₃₋₆-alkyl-C₁₋₃-alkyl, Amino-, C₁₋₄-Alkylamino- oder Di-(C₁₋₄-alkyl)-aminogruppen substituiert sein können,
R⁵ ein Wasserstoffatom oder, wenn Y¹ ein Stickstoffatom darstellt, auch ein freies Elektronenpaar und
R⁶, R⁷, R⁸ und R⁹, die gleich oder verschieden sein können, jeweils ein Wasserstoffatom oder eine C₁₋₃-Alkylgruppe darstellen,
bedeuten, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Als ganz besonders bevorzugte Verbindungen der obigen allgemeinen Formel (I) seien beispielsweise folgende Verbindungen genannt:

| | Struktur | Name |
|---|---|---|
| (1) | 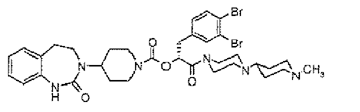 | 4-(2-Oxo-1,2,4,5-tetrahydro-1,3-benzdiazepin-3-yl)-piperidin-1-carbonsäure-(R)-1-(3,4-dibrom-benzyl)-2-[4-(1-methyl-piperidin-4-yl)-piperazin-1-yl]-2-oxo-ethylester |
| (2) | 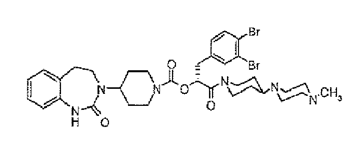 | 4-(2-Oxo-1,2,4,5-tetrahydro-1,3-benzdiazepin-3-yl)-piperidin-1-carbonsäure -(R)-1-(3,4-dibrom-benzyl)-2-[4-(4-methyl-piperazin-1-yl)-piperidin-1-yl]-2-oxo-ethylester |
| (3) | 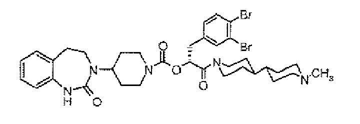 | 4-(2-Oxo-1,2,4,5-tetrahydro-1,3-benzdiazepin-3-yl)-piperidin-1-carbonsäure-(R)-1-(3,4-dibrom-benzyl)-2-(1'-methyl-4,4'-bipiperidinyl-1-yl)-2-oxo-ethylester |
| (4) | 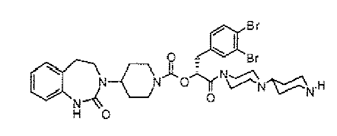 | 4-(2-Oxo-1,2,4,5-tetrahydro-1,3-benzdiazepin-3-yl)-piperidin-1-carbonsäure-(R)-1-(3,4-dibrom-benzyl)-2-oxo-2-(4-piperidin-4-yl-piperazin-1-yl)-ethylester |
| (5) | 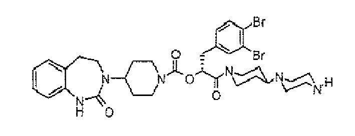 | 4-(2-Oxo-1,2,4,5-tetrahydro-1,3-benzdiazepin-3-yl)-piperidin-1-carbonsäure-(R)-1-(3,4-dibrom-benzyl)-2-oxo-2-(4-piperazin-1-yl-piperidin-1-yl)-ethylester |
| (6) | 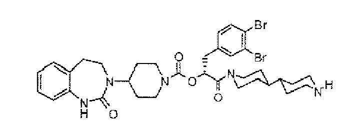 | 4-(2-Oxo-1,2,4,5-tetrahydro-1,3-benzdiazepin-3-yl)-piperidin-1-carbonsäure-(R)-2-4,4'-bipiperidinyl-1-yl-1-(3,4-dibrom-benzyl)-2-oxo-ethylester |
| (7) | 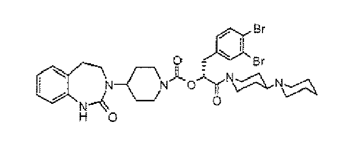 | 4-(2-Oxo-1,2,4,5-tetrahydro-1,3-benzdiazepin-3-yl)-piperidin-1-carbonsäure-(R)-2-1,4'-bipiperidinyl-1'-yl-1-(3,4-dibrom-benzyl)-2-oxo-ethylester |
| (8) | 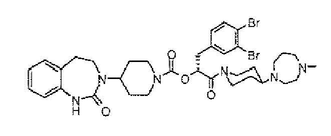 | 4-(2-Oxo-1,2,4,5-tetrahydro-1,3-benzdiazepin-3-yl)-piperidin-1-carbonsäure-(R)-1-(3,4-dibrom-benzyl)-2-[4-(4-methyl-perhydro-1,4-diazepin-1-yl)-piperidin-1-yl]-2-oxo-ethylester |
| (9) | 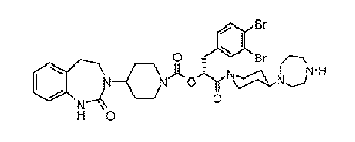 | 4-(2-Oxo-1,2,4,5-tetrahydro-1,3-benzdiazepin-3-yl)-piperidin-1-carbonsäure-(R)-1-(3,4-dibrom-benzyl)-2-oxo-2-(4-perhydro-1,4-diazepin-1-yl-piperidin-1-yl)-ethylester |
| (10) | 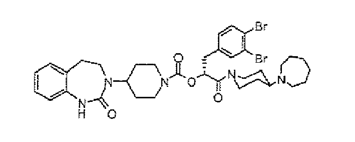 | 4-(2-Oxo-1,2,4,5-tetrahydro-1,3-benzdiazepin-3-yl)-piperidin-1-carbonsäure-(R)-1-(3,4-dibrom-benzyl)-2-oxo-2-(4-perhydro-azepin-1-yl-piperidin-1-yl)-ethylester |
| (11) | 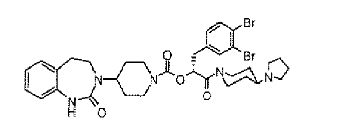 | 4-(2-Oxo-1,2,4,5-tetrahydro-1,3-benzdiazepin-3-yl)-piperidin-1-carbonsäure-(R)-1-(3,4-dibrom-benzyl)-2-oxo-2-(4-pyrrolidin-1-yl-piperidin-1-yl)-ethylester |
| (12) | 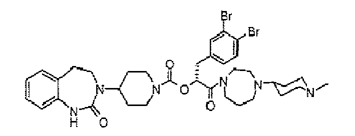 | 4-(2-Oxo-1,2,4,5-tetrahydro-1,3-benzdiazepin-3-yl)-piperidin-1-carbonsäure-(R)-1-(3,4-dibrom-benzyl)-2-[4-(1-methyl-piperidin-4-yl)-perhydro-1,4-diazepin-1-yl]-2-oxo-ethylester |
| (13) | 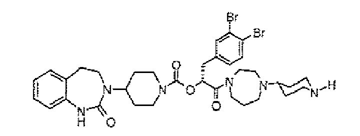 | 4-(2-Oxo-1,2,4,5-tetrahydro-1,3-benzdiazepin-3-yl)-piperidin-1-carbonsäure-(R)-1-(3,4-dibrom-benzyl)-2-oxo-2-(4-piperidin-4-yl-perhydro-1,4-diazepin-1-yl)-ethylester |
| (14) | 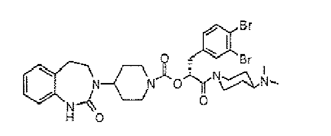 | 4-(2-Oxo-1,2,4,5-tetrahydro-1,3-benzdiazepin-3-yl)-piperidin-1-carbonsäure-(R)-1-(3,4-dibrom-benzyl)-2-(4-dimethylamino-piperidin-1-yl)-2-oxo-ethylester |
| (15) | 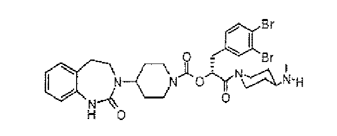 | 4-(2-Oxo-1,2,4,5-tetrahydro-1,3-benzdiazepin-3-yl)-piperidin-1-carbonsäure-(R)-1-(3,4-dibrom-benzyl)-2-(4-methylamino-piperidin-1-yl)-2-oxo-ethylester |
| (16) | 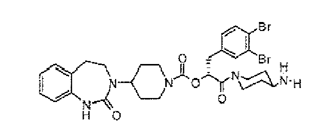 | 4-(2-Oxo-1,2,4,5-tetrahydro-1,3-benzdiazepin-3-yl)-piperidin-1-carbonsäure-(R)-1-(3,4-dibrom-benzyl)-2-(4-amino-piperidin-1-yl)-2-oxo-ethylester |
| (17) | 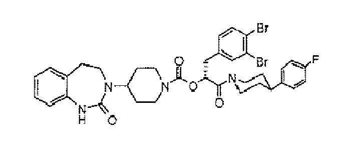 | 4-(2-Oxo-1,2,4,5-tetrahydro-1,3-benzdiazepin-3-yl)-piperidin-1-carbonsäure-(R)-1-(3,4-dibrom-benzyl)-2-[4-(4-fluor-phenyl)-piperidin-1-yl]-2-oxo-ethylester |
| (18) | 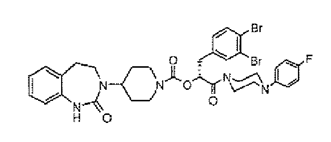 | 4-(2-Oxo-1,2,4,5-tetrahydro-1,3-benzdiazepin-3-yl)-piperidin-1-carbonsäure-(R)-1-(3,4-dibrom-benzyl)-2-[4-(4-fluor-phenyl)-piperazin-1-yl]-2-oxo-ethylester |
| (19) | 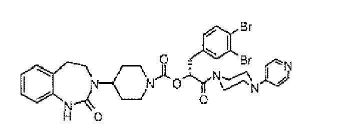 | 4-(2-Oxo-1,2,4,5-tetrahydro-1,3-benzdiazepin-3-yl)-piperidin-1-carbonsäure-(R)-1-(3,4-dibrom-benzyl)-2-oxo-2-(4-pyridin-4-yl-piperazin-1-yl)-ethylester |
| (20) | 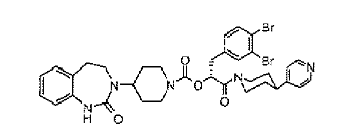 | 4-(2-Oxo-1,2,4,5-tetrahydro-1,3-benzdiazepin-3-yl)-piperidin-1-carbonsäure-(R)-1-(3,4-dibrom-benzyl)-2-oxo-2-(3,4,5,6-tetrahydro-2*H*-4,4'-bipyridinyl-1-yl)-ethylester |
| (21) | 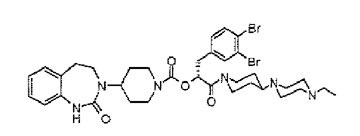 | 4-(2-Oxo-1,2,4,5-tetrahydro-1,3-benzdiazepin-3-yl)-piperidin-1-carbonsäure-(R)-1-(3,4-dibrom-benzyl)-2-[4-(4-ethyl-piperazin-1-yl)-piperidin-1-yl]-2-oxo-ethylester |
| (22) | 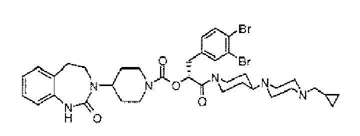 | 4-(2-Oxo-1,2,4,5-tetrahydro-1,3-benzdiazepin-3-yl)-piperidin-1-carbonsäure-(R)-2-[4-(4-cyclopropylmethyl-piperazin-1-yl)-piperidin-1-yl]-1-(3,4-dibrom-benzyl)-2-oxo-ethylester |
| (23) | 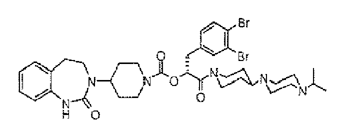 | 4-(2-Oxo-1,2,4,5-tetrahydro-1,3-benzdiazepin-3-yl)-piperidin-1-carbonsäure-(R)-1-(3,4-dibrom-benzyl)-2-[4-(4-isopropyl-piperazin-1-yl)-piperidin-1-yl]-2-oxo-ethylester |
| (24) | 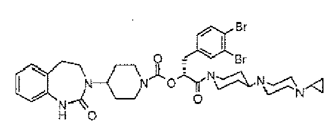 | 4-(2-Oxo-1,2,4,5-tetrahydro-1,3-benzdiazepin-3-yl)-piperidin-1-carbonsäure-(R)-2-[4-(4-cyclopropyl-piperazin-1-yl)-piperidin-1-yl]-1-(3,4-dibrom-benzyl)-2-oxo-ethylester |
| (25) | 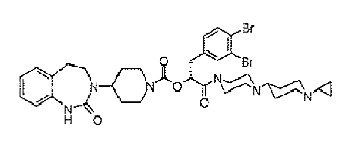 | 4-(2-Oxo-1,2,4,5-tetrahydro-1,3-benzdiazepin-3-yl)-piperidin-1-carbonsäure-(R)-2-[4-(1-cyclopropyl-piperidin-4-yl)-piperazin-1-yl]-1-(3,4-dibrom-benzyl)-2-oxo-ethylester |
| (26) | 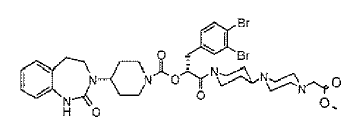 | 4-(2-Oxo-1,2,4,5-tetrahydro-1,3-benzdiazepin-3-yl)-piperidin-1-carbonsäure-(R)-1-(3,4-dibrom-benzyl)-2-[4-(4-methoxycarbonylmethyl-piperazin-1-yl)-piperidin-1-yl]-2-oxo-ethylester |
| (27) | 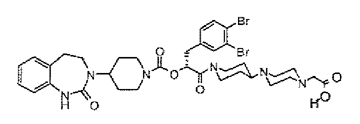 | 4-(2-Oxo-1,2,4,5-tetrahydro-1,3-benzdiazepin-3-yl)-piperidin-1-carbonsäure-(R)-2-[4-(4-carboxymethyl-piperazin-1-yl)-piperidin-1-yl]-1-(3,4-dibrom-benzyl)-2-oxo-ethylester |
| (28) | 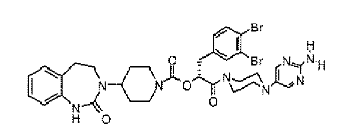 | 4-(2-Oxo-1,2,4,5-tetrahydro-1,3-benzdiazepin-3-yl)- piperidin-1-carbonsäure-(R)-2-[4-(2-amino-pyrimidin-5-yl)-piperazin-1-yl]-1-(3,4-dibrom-benzyl)-2-oxo-ethyl ester |
| (29) | 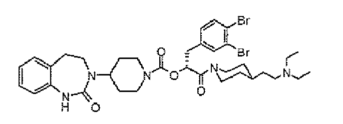 | 4-(2-Oxo-1,2,4,5-tetrahydro-1,3-benzdiazepin-3-yl)-piperidin-1-carbonsäure-(R)-1-(3,4-dibrom-benzyl)-2-[4-(2-diethylamino-ethyl)-piperidin-1-yl]-2-oxo-ethylester |
| (30) | 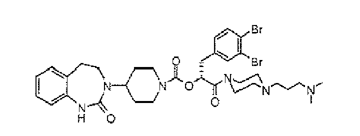 | 4-(2-Oxo-1,2,4,5-tetrahydro-1,3-benzdiazepin-3-yl)-piperidin-1-carbonsäure-(R)-1-(3,4-dibrom-benzyl)-2-[4-(3-dimethylamino-propyl)-piperazin-1-yl]-2-oxo-ethylester |
| (31) | 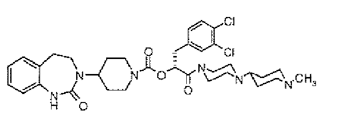 | 4-(2-Oxo-1,2,4,5-tetrahydro-1,3-benzdiazepin-3-yl)-piperidin-1-carbonsäure-(R)-1-(3,4-dichlor-benzyl)-2-[4-(1-methyl-piperidin-4-yl)-piperazin-1-yl]-2-oxo-ethylester |
| (32) | 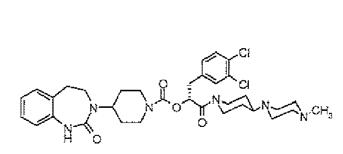 | 4-(2-Oxo-1,2,4,5-tetrahydro-1,3-benzdiazepin-3-yl)-piperidin-1-carbonsäure -(R)-1-(3,4-dichlor-benzyl)-2-[4-(4-methyl-piperazin-1-yl)-piperidin-1-yl]-2-oxo-ethylester |
| (33) | 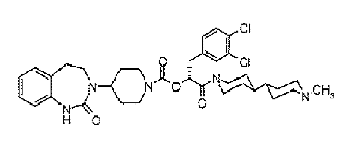 | 4-(2-Oxo-1,2,4,5-tetrahydro-1,3-benzdiazepin-3-yl)-piperidin-1-carbonsäure-(R)-1-(3,4-dichlor-benzyl)-2-(1'-methyl-4,4'-bipiperidinyl-1-yl)-2-oxo-ethylester |
| (34) | 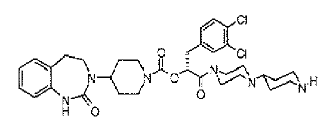 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-piperidin-4-yl-piperazin-1-yl)-ethylester |
| (35) | 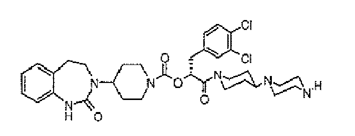 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-piperazin-1-yl-piperidin-1-yl)-ethylester |
| (36) | 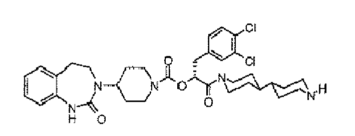 | 4-(2-Oxo-1,2,4,5-tetrahydro-1,3-benzdiazepin-3-yl)-piperidin-1-carbonsäure-(R)-2-4,4'-bipiperidinyl-1-yl-1-(3,4-dichlor-benzyl)-2-oxo-ethylester |
| (37) | 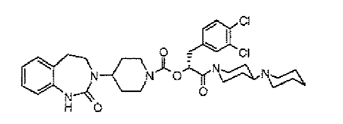 | 4-(2-Oxo-1,2,4,5-tetrahydro-1,3-benzdiazepin-3-yl)-piperidin-1-carbonsäure-(R)-2-1,4'-bipiperidinyl-1'-yl-1-(3,4-dichlor-benzyl)-2-oxo-ethylester |
| (38) | 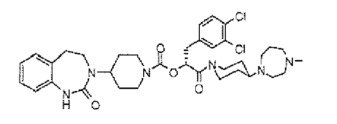 | 4-(2-Oxo-1,2,4,5-tetrahydro-1,3-benzdiazepin-3-yl)-piperidin-1-carbonsäure-(R)-1-(3,4-dichlor-benzyl)-2-[4-(4-methyl-perhydro-1,4-diazepin-1-yl)-piperidin-1-yl]-2-oxo-ethylester |
| (39) | 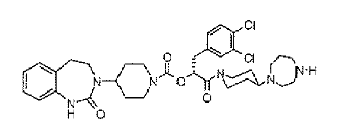 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-perhydro-1,4-diazepin-1-yl-piperidin-1-yl)-ethylester |
| (40) | 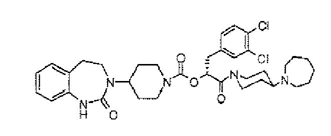 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-perhydro-azepin-1-yl-piperidin-1-yl)-ethylester |
| (41) | 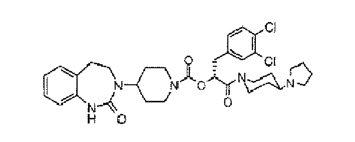 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-pyrrolidin-1-yl-piperidin-1-yl)-ethylester |
| (42) | 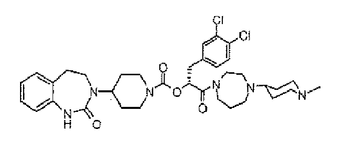 | 4-(2-Oxo-1,2,4,5-tetrahydro-1,3-benzdiazepin-3-yl)-piperidin-1-carbonsäure-(R)-1-(3,4-dichlor-benzyl)-2-[4-(1-methyl-piperidin-4-yl)-perhydro-1,4-diazepin-1-yl]-2-oxo-ethylester |
| (43) | 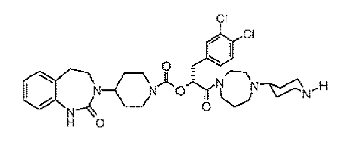 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-piperidin-4-yl-perhydro-1,4-diazepin-1-yl)-ethylester |
| (44) | 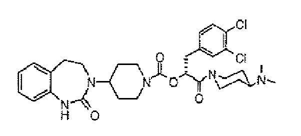 | 4-(2-Oxo-1,2,4,5-tetrahydro-1,3-benzdiazepin-3-yl)-piperidin-1-carbonsäure-(R)-1-(3,4-dichlor-benzyl)-2-(4-dimethylamino-piperidin-1-yl)-2-oxo-ethylester |
| (45) | 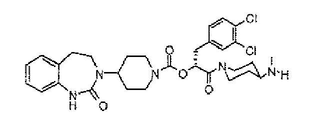 | 4-(2-Oxo-1,2,4,5-tetrahydro-1,3-benzdiazepin-3-yl)-piperidin-1-carbonsäure-(R)-1-(3,4-dichlor-benzyl)-2-(4-methylamino-piperidin-1-yl)-2-oxo-ethylester |
| (46) | 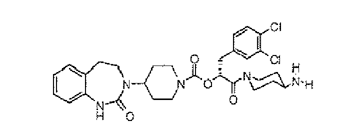 | 4-(2-Oxo-1,2,4,5-tetrahydro-1,3-benzdiazepin-3-yl)-piperidin-1-carbonsäure-(R)-1-(3,4-dichlor-benzyl)-2-(4-amino-piperidin-1-yl)-2-oxo-ethylester |
| (47) | 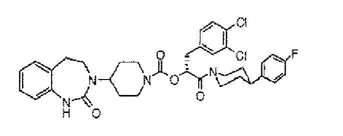 | 4-(2-Oxo-1,2,4,5-tetrahydro-1,3-benzdiazepin-3-yl)-piperidin-1-carbonsäure-(R)-1-(3,4-dichlor-benzyl)-2-[4-(4-fluor-phenyl)-piperidin-1-yl]-2-oxo-ethylester |
| (48) | 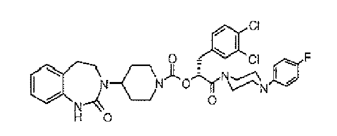 | 4-(2-Oxo-1,2,4,5-tetrahydro-1,3-benzdiazepin-3-yl)-piperidin-1-carbonsäure-(R)-1-(3,4-dichlor-benzyl)-2-[4-(4-f luor-phenyl)-piperazin-1-yl]-2-oxo-ethylester |
| (49) | 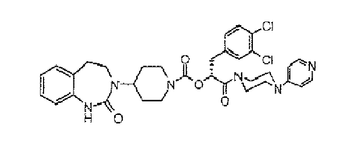 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-pyridin-4-yl-piperazin-1-yl)-ethylester |
| (50) | 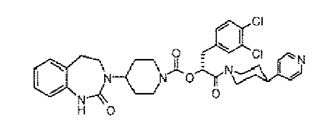 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(3,4,5,6-tetrahydro-2*H*-4,4'-bipyridinyl-1-yl)-ethylester |
| (51) | 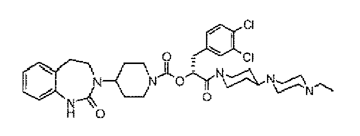 | 4-(2-Oxo-1,2,4,5-tetrahydro-1,3-benzdiazepin-3-yl)-piperidin-1-carbonsäure-(R)-1-(3,4-dichlor-benzyl)-2-[4-(4-ethyl-piperazin-1-yl)-piperidin-1-yl]-2-oxo-ethylester |
| (52) | 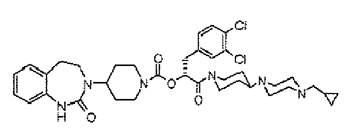 | 4-(2-Oxo-1,2,4,5-tetrahydro-1,3-benzdiazepin-3-yl)-piperidin-1-carbonsäure-(R)-2-[4-(4-cyclopropylmethyl-piperazin-1-yl)-piperidin-1-yl]-1-(3,4-dichlor-benzyl)-2-oxo-ethylester |
| (53) | 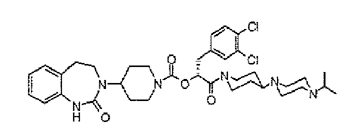 | 4-(2-Oxo-1,2,4,5-tetrahydro-1,3-benzdiazepin-3-yl)-piperidin-1-carbonsäure-(R)-1-(3,4-dichlor-benzyl)-2-[4-(4-isopropyl-piperazin-1-yl)-piperidin-1-yl]-2-oxo-ethylester |
| (54) | 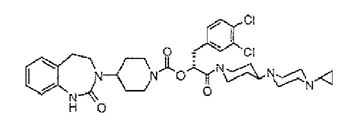 | 4-(2-Oxo-1,2,4,5-tetrahydro-1,3-benzdiazepin-3-yl)-piperidin-1-carbonsäure-(R)-2-[4-(4-cyclopropyl-piperazin-1-yl)-piperidin-1-yl]-1-(3,4-dichlor-benzyl)-2-oxo-ethylester |
| (55) | 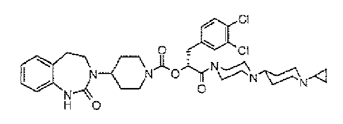 | 4-(2-Oxo-1,2,4,5-tetrahydro-1,3-benzdiazepin-3-yl)-piperidin-1-carbonsäure-(R)-2-[4-(1-cyclopropyl-piperidin-4-yl)-piperazin-1-yl]-1-(3,4-dichlor-benzyl)-2-oxo-ethylester |
| (56) | 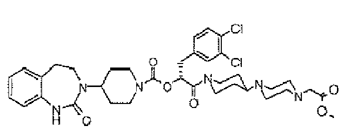 | 4-(2-Oxo-1,2,4,5-tetrahydro-1,3-benzdiazepin-3-yl)-piperidin-1-carbonsäure-(R)-1-(3,4-dichlor-benzyl)-2-[4-(4-methoxycarbonylmethyl-piperazin-1-yl)-piperidin-1-yl]-2-oxo-ethylester |
| (57) | 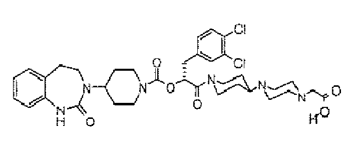 | 4-(2-Oxo-1,2,4,5-tetrahydro-1,3-benzdiazepin-3-yl)-piperidin-1-carbonsäure-(R)-2-[4-(4-carboxymethyl-piperazin-1-yl)-piperidin-1-yl]-1-(3,4-dichlor-benzyl)-2-oxo-ethylester |
| (58) | 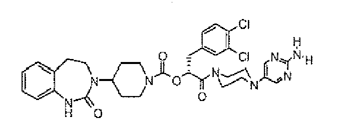 | 4-(2-Oxo-1,2,4,5-tetrahydro-1,3-benzdiazepin-3-yl)- piperidin-1-carbonsäure-(R)-2-[4-(2-amino-pyrimidin-5-yl)-piperazin-1-yl]-1-(3,4-dichlor-benzyl)-2-oxo-ethyl ester |
| (59) | 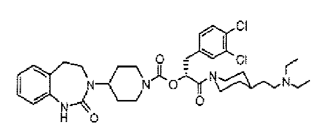 | 4-(2-Oxo-1,2,4,5-tetrahydro-1,3-benzdiazepin-3-yl)-piperidin-1-carbonsäure-(R)-1-(3,4-dichlor-benzyl)-2-[4-(2-diethylamino-ethyl)-piperidin-1-yl]-2-oxo-ethylester |
| (60) | 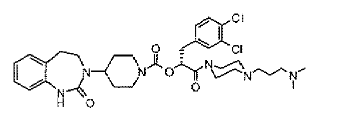 | 4-(2-Oxo-1,2,4,5-tetrahydro-1,3-benzdiazepin-3-yl)-piperidin-1-carbonsäure-(R)-1-(3,4-dichlor-benzyl)-2-[4-(3-dimethylamino-propyl)-piperazin-1-yl]-2-oxo-ethylester |
| (61) | 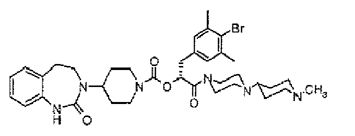 | 4-(2-Oxo-1,2,4,5-tetrahydro-1,3-benzdiazepin-3-yl)-piperidin-1-carbonsäure (R)-1-(4-brom-3,5-dimethyl-benzyl)-2-[4-(1-methyl-piperidin-4-yl)-piperazin-1-yl]-2-oxo-ethyl ester |
| (62) | 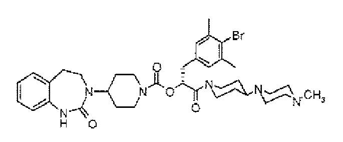 | 4-(2-Oxo-1,2,4,5-tetrahydro-1,3-benzdiazepin-3-yl)-piperidin-1-carbonsäure (R)-1-(4-brom-3,5-dimethyl-benzyl)-2-[4-(4-methyl-piperazin-1-yl)-piperidin-1-yl]-2-oxo-ethyl ester |
| (63) | 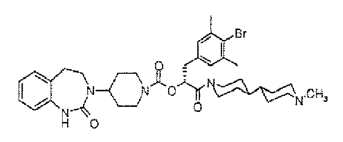 | 4-(2-Oxo-1,2,4,5-tetrahydro-1,3-benzdiazepin-3-yl)-piperidin-1-carbonsäure (R)-1-(4-brom-3,5-dimethyl-benzyl)-2-(1'-methyl-4,4'-bipiperidinyl-1-yl)-2-oxo-ethyl ester |
| (64) | 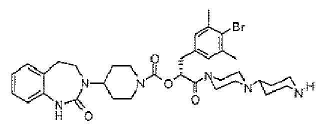 | 4-(2-Oxo-1,2,4,5-tetrahydro-1,3-benzdiazepin-3-yl)-piperidin-1-carbonsäure (R)-1-(4-brom-3,5-dimethyl-benzyl)-2-oxo-2-(4-piperidin-4-yl-piperazin-1-yl)-ethyl ester |
| (65) | 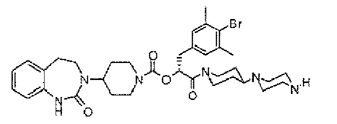 | 4-(2-Oxo-1,2,4,5-tetrahydro-1,3-benzdiazepin-3-yl)-piperidin-1-carbonsäure (R)-1-(4-brom-3,5-dimethyl-benzyl)-2-oxo-2-(4-piperazin-1-yl-piperidin-1-yl)-ethyl ester |
| (66) | 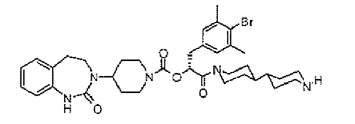 | 4-(2-Oxo-1,2,4,5-tetrahydro-1,3-benzdiazepin-3-yl)-piperidin-1-carbonsäure (R)-2-4,4'-bipiperidinyl-1-yl-1-(4-brom-3,5-dimethyl-benzyl)-2-oxo-ethyl ester |
| (67) | 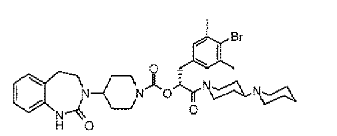 | 4-(2-Oxo-1,2,4,5-tetrahydro-1,3-benzdiazepin-3-yl)-piperidin-1-carbonsäure (R)-2-1,4'-bipiperidinyl-1'-yl-1-(4-brom-3,5-dimethylbenzyl)-2-oxo-ethyl ester |
| (68) | 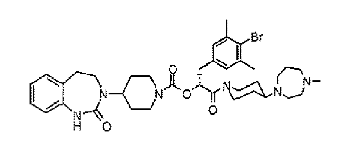 | 4-(2-Oxo-1,2,4,5-tetrahydro-1,3-benzdiazepin-3-yl)-piperidin-1-carbonsäure (R)-1-(4-brom-3,5-dimethyl-benzyl)-2-[4-(4-methyl-perhydro-1,4-diazepin-1-yl)-piperidin-1-yl]-2-oxo-ethyl ester |
| (69) | 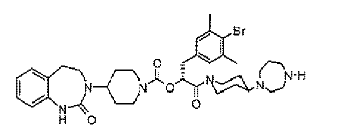 | 4-(2-Oxo-1,2,4,5-tetrahydro-1,3-benzdiazepin-3-yl)-piperidin-1-carbonsäure (R)-1-(4-brom-3,5-dimethyl-benzyl)-2-oxo-2-(4-perhydro-1,4-diazepin-1-yl-piperidin-1-yl)-ethyl ester |
| (70) | 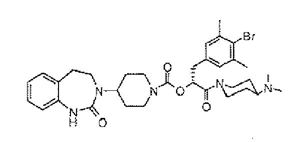 | 4-(2-Oxo-1,2,4,5-tetrahydro-1,3-benzdiazepin-3-yl)-piperidin-1-carbonsäure (R)-1-(4-brom-3,5-dimethyl-benzyl)-2-(4-dimethylamino-piperidin-1-yl)-2-oxo-ethyl ester |
| (71) | 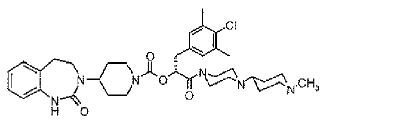 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-[4-(1-methyl-piperidin-4-yl)-piperazin-1-yl]-2-oxo-ethyl ester |
| (72) | 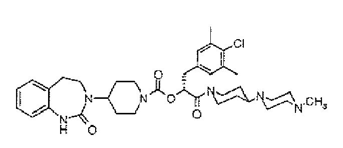 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-[4-(4-methyl-piperazin-1-yl)-piperidin-1-yl]-2-oxo-ethyl ester |
| (73) | 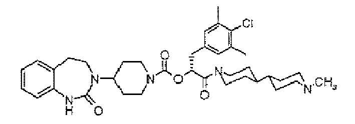 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-(1'-methyl-4,4'-bipiperidinyl-1-yl)-2-oxo-ethyl ester |
| (74) | 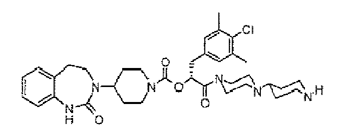 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-oxo-2-(4-piperidin-4-yl-piperazin-1-yl)-ethyl ester |
| (75) | 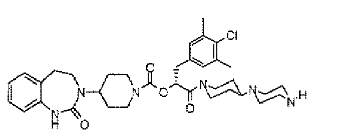 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-oxo-2-(4-piperazin-1-yl-piperidin-1-yl)-ethyl ester |
| (76) | 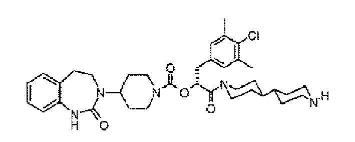 | 4-(2-Oxo-1,2,4,5-tetrahydro-1,3-benzdiazepin-3-yl)-piperidin-1-carbonsäure (R)-2-4,4'-bipiperidinyl-1-yl-1-(4-chlor-3,5-dimethyl-benzyl)-2-oxo-ethyl ester |
| (77) | 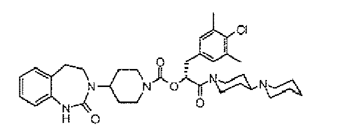 | 4-(2-Oxo-1,2,4,5-tetrahydro-1,3-benzdiazepin-3-yl)-piperidin-1-carbonsäure (R)-2-1,4'-bipiperidinyl-1'-yl-1-(4-chlor-3,5-dimethylbenzyl)-2-oxo-ethyl ester |
| (78) | 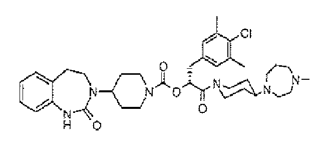 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-[4-(4-methyl-perhydro-1,4-diazepin-1-yl)-piperidin-1-yl]-2-oxo-ethyl ester |
| (79) | 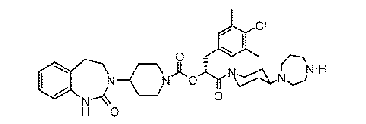 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-oxo-2-(4-perhydro-1,4-diazepin-1-yl-piperidin-1-yl)-ethyl ester |
| (80) | 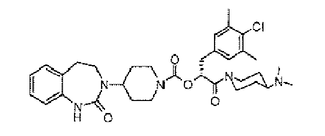 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-(4-dimethylamino-piperidin-1-yl)-2-oxo-ethyl ester |
| (81) | 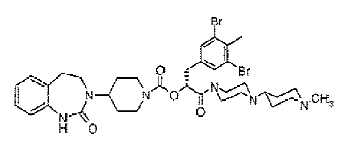 | 4-(2-Oxo-1,2,4,5-tetrahydro-1,3-benzdiazepin-3-yl)-piperidin-1-carbonsäure (R)-1-(3,5-dibrom-4-methyl-benzyl)-2-[4-(1-methyl-piperidin-4-yl)-piperazin-1-yl]-2-oxo-ethyl ester |
| (82) | 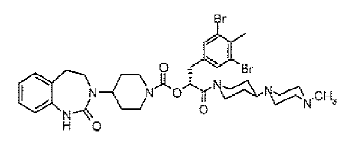 | 4-(2-Oxo-1,2,4,5-tetrahydro-1,3-benzdiazepin-3-yl)-piperidin-1-carbonsäure (R)-1-(3,5-dibrom-4-methyl-benzyl)-2-[4-(4-methyl-piperazin-1-yl)-piperidin-1-yl]-2-oxo-ethyl ester |
| (83) | 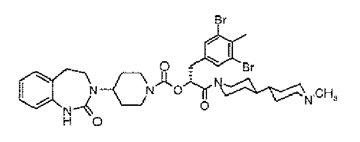 | 4-(2-Oxo-1,2,4,5-tetrahydro-1,3-benzdiazepin-3-yl)-piperidin-1-carbonsäure (R)-1-(3,5-dibrom-4-methyl-benzyl)-2-(1'-methyl-4,4'-bipiperidinyl-1-yl)-2-oxo-ethyl ester |
| (84) | 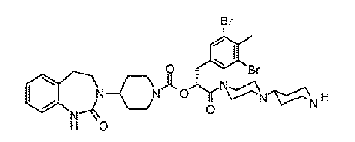 | 4-(2-Oxo-1,2,4,5-tetrahydro-1,3-benzdiazepin-3-yl)-piperidin-1-carbonsäure (R)-1-(3,5-dibrom-4-methyl-benzyl)-2-oxo-2-(4-piperidin-4-yl-piperazin-1-yl)-ethyl ester |
| (85) | 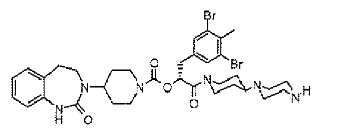 | 4-(2-Oxo-1,2,4,5-tetrahydro-1,3-benzdiazepin-3-yl)-piperidin-1-carbonsäure (R)-1-(3,5-dibrom-4-methyl-benzyl)-2-oxo-2-(4-piperazin-1-yl-piperidin-1-yl)-ethyl ester |
| (86) | 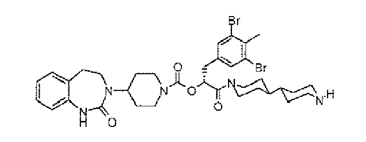 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-dibrom-4-methyl-benzyl)-2-oxo-ethyl ester |
| (87) | 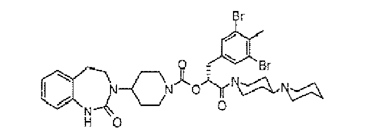 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-dibrom-4-methyl-benzyl)-2-oxo-ethyl ester |
| (88) | 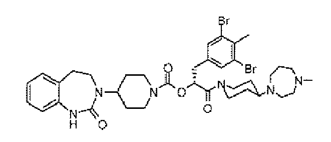 | 4-(2-Oxo-1,2,4,5-tetrahydro-1,3-benzdiazepin-3-yl)-piperidin-1-carbonsäure (R)-1-(3,5-dibrom-4-methyl-benzyl)-2-[4-(4-methyl-perhydro-1,4-diazepin-1-yl)-piperidin-1-yl]-2-oxo-ethyl ester |
| (89) | 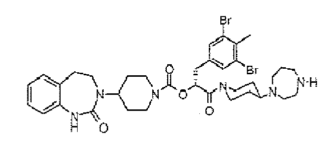 | 4-(2-Oxo-1,2,4,5-tetrahydro-1,3-benzdiazepin-3-yl)-piperidin-1-carbonsäure (R)-1-(3,5-dibrom-4-methyl-benzyl)-2-oxo-2-(4-perhydro-1,4-diazepin-1-yl-piperidin-1-yl)-ethyl ester |
| (90) | 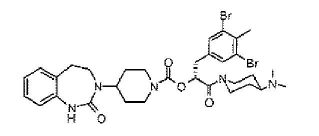 | 4-(2-Oxo-1,2,4,5-tetrahydro-1,3-benzdiazepin-3-yl)-piperidin-1-carbonsäure (R)-1-(3,5-dibrom-4-methyl-benzyl)-2-(4-dimethylamino-piperidin-1-yl)-2-oxo-ethyl ester |
| (91) | 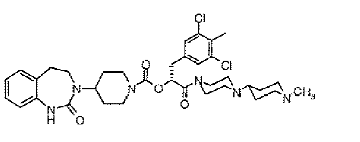 | 4-(2-Oxo-1,2,4,5-tetrahydro-1,3-benzdiazepin-3-yl)-piperidin-1-carbonsäure (R)-1-(3,5-dichlor-4-methyl-benzyl)-2-[4-(1-methyl-piperidin-4-yl)-piperazin-1-yl]-2-oxo-ethyl ester |
| (92) | 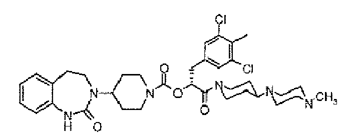 | 4-(2-Oxo-1,2,4,5-tetrahydro-1,3-benzdiazepin-3-yl)-piperidin-1-carbonsäure (R)-1-(3,5-dichlor-4-methyl-benzyl)-2-[4-(4-methyl-piperazin-1-yl)-piperidin-1-yl]-2-oxo-ethyl ester |
| (93) | 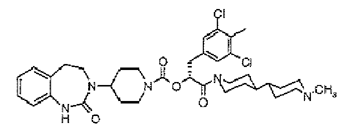 | 4-(2-Oxo-1,2,4,5-tetrahydro-1,3-benzdiazepin-3-yl)-piperidin-1-carbonsäure (R)-1-(3,5-dichlor-4-methyl-benzyl)-2-(1'-methyl-4,4'-bipiperidinyl-1-yl)-2-oxo-ethyl ester |
| (94) | 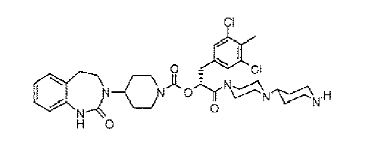 | 4-(2-Oxo-1,2,4,5-tetrahydro-1,3-benzdiazepin-3-yl)-piperidin-1-carbonsäure (R)-1-(3,5-dichlor-4-methyl-benzyl)-2-oxo-2-(4-piperidin-4-yl-piperazin-1-yl)-ethyl ester |
| (95) | 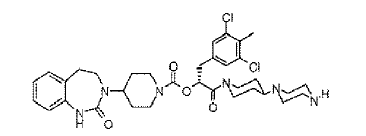 | 4-(2-Oxo-1,2,4,5-tetrahydro-1,3-benzdiazepin-3-yl)-piperidin-1-carbonsäure (R)-1-(3,5-dichlor-4-methyl-benzyl)-2-oxo-2-(4-piperazin-1-yl-piperidin-1-yl)-ethyl ester |
| (96) | 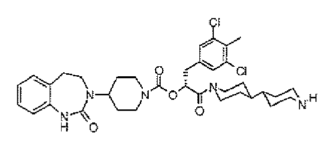 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-dichlor-4-methyl-benzyl)-2-oxo-ethyl ester |
| (97) | 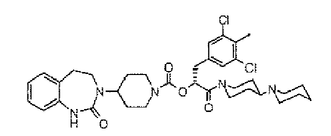 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-dichlor-4-methylbenzyl)-2-oxo-ethyl ester |
| (98) | 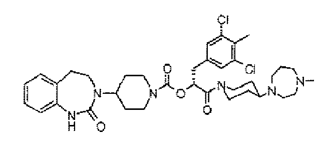 | 4-(2-Oxo-1,2,4,5-tetrahydro-1,3-benzdiazepin-3-yl)-piperidin-1-carbonsäure (R)-1-(3,5-dichlor-4-methyl-benzyl)-2-[4-(4-methyl-perhydro-1,4-diazepin-1-yl)-piperidin-1-yl]-2-oxo-ethyl ester |
| (99) | 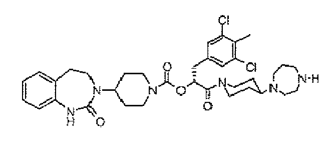 | 4-(2-Oxo-1,2,4,5-tetrahydro-1,3-benzdiazepin-3-yl)-piperidin-1-carbonsäure (R)-1-(3,5-dichlor-4-methyl-benzyl)-2-oxo-2-(4-perhydro-1,4-diazepin-1-yl-pipendin-1-yl)-ethyl ester |
| (100) | 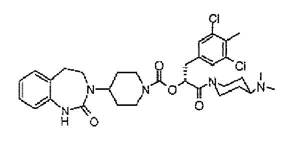 | 4-(2-Oxo-1,2,4,5-tetrahydro-1,3-benzdiazepin-3-yl)-piperidin-1-carbonsäure (R)-1-(3,5-dichlor-4-methyl-benzyl)-2-(4-dimethylamino-piperidin-1-yl)-2-oxo-ethyl ester |
| (101) | 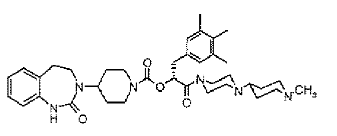 | 4-(2-Oxo-1,2,4,5-tetrahydro-1,3-benzdiazepin-3-yl)-piperidin-1-carbonsäure (R)-2-[4-(1-methyl-piperidin-4-yl)-piperazin-1-yl]-2-oxo-1-(3,4,5-trimethyl-benzyl)-ethyl ester |
| (102) | 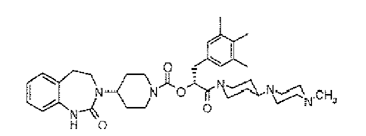 | 4-(2-Oxo-1,2,4,5-tetrahydro-1,3-benzdiazepin-3-yl)-piperidin-1-carbonsäure (R)-2-[4-(4-methyl-piperazin-1-yl)-piperidin-1-yl]-2-oxo-1-(3,4,5-trimethyl-benzyl)-ethyl ester |
| (103) | 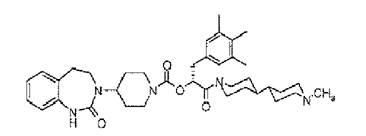 | 4-(2-Oxo-1,2,4,5-tetrahydro-1,3-benzdiazepin-3-yl)-piperidin-1-carbonsäure (R)-2-(1'-methyl-4,4'-bipiperidinyl-1-yl)-2-oxo-1-(3,4,5-trimethyl-benzyl)-ethyl ester |
| (104) | 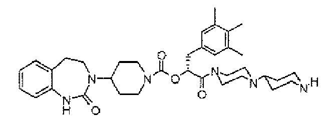 | 4-(2-Oxa-1,2,4,5-tetrahydro-1,3-benzdiazepin-3-yl)-piperidin-1-carbonsäure (R)-2-oxo-2-(4-piperidin-4-yl-piperazin-1-yl)-1-(3,4,5-trimtethyl-benzyl)-ethyl ester |
| (105) | 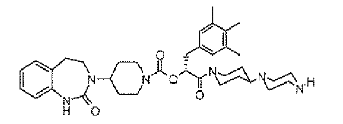 | 4-(2-Oxo-1,2,4,5-tetrahydro-1,3-benzdiazepin-3-yl)-piperidin-1-carbonsäure (R)-2-oxo-2-(4-piperazin-1-yl-piperidin-1-yl)-1-(3,4,5-trimethyl-benzyl)-ethyl ester |
| (106) | 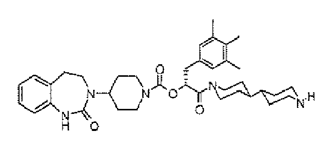 | 4-(2-Oxo-1,2,4,5-tetrahydro-1,3-benzdiazepin-3-yl)-piperidin-1-carbonsäure (R)-2-4,4'-bipiperidinyl-1-yl-2-oxo-1-(3,4,5-trimethyl-benzyl)-ethyl ester |
| (107) | 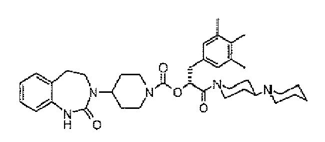 | 4-(2-Oxo-1,2,4,5-tetrahydro-1,3-benzdiazepin-3-yl)-piperidin-1-carbonsäure (R)-2-1,4'-bipiperidinyl-1'-yl-2-oxo-1-(3,4,5-trimethyl-benzyl)-ethyl ester |
| (108) | 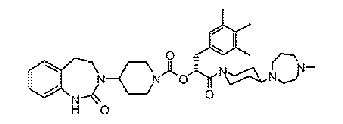 | 4-(2-Oxo-1,2,4,5-tetrahydro-1,3-benzdiazepin-3-yl)-piperidin-1-carbonsäure (R)-2-[4-(4-methyl-perhydro-1,4-diazepin-1-yl)-piperidin-1-yl]-2-oxo-1-(3,4,5-trimethyl-benzyl)-ethyl ester |
| (109) | 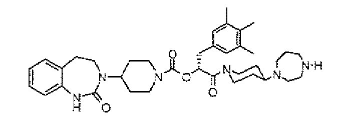 | 4-(2-Oxo-1,2,4,5-tetrahydro-1,3-benzdiazepin-3-yl)-piperidin-1-carbonsäure (R)-2-oxo-2-(4-perhydro-1,4-diazepin-1-yl-piperidin-1-yl)-1-(3,4,5-trimethyl-benzyl)-ethyl ester |
| (110) | 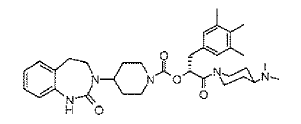 | 4-(2-Oxo-1,2,4,5-tetrahydro-1,3-benzdiazepin-3-yl)-piperidin-1-carbonsäure (R)-2-(4-dimethylamino-piperidin-1-yl)-2-oxo-1-(3,4,5-trimethyl-benzyl)-ethyl ester |
| (111) | 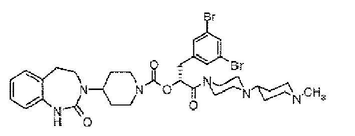 | 4-(2-Oxo-1,2,4,5-tetrahydro-1,3-benzdiazepin-3-yl)-piperidin-1-carbonsäure (R)-1-(3,5-dibrom-benzyl)-2-[4-(1-methyl-piperidin-4-yl)-piperazin-1-yl]-2-oxo-ethyl ester |
| (112) | 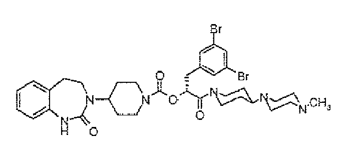 | 4-(2-Oxo-1,2,4,5-tetrahydro-1,3-benzdiazepin-3-yl)-piperidin-1-carbonsäure (R)-1-(3,5-dibrom-benzyl)-2-[4-(4-methyl-piperazin-1-yl)-piperidin-1-yl]-2-oxo-ethyl ester |
| (113) | 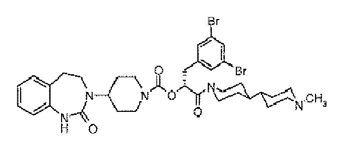 | 4-(2-Oxo-1,2,4,5-tetrahydro-1,3-benzdiazepin-3-yl)-piperidin-1-carbonsäure (R)-1-(3,5-dibrom-benzyl)-2-(1'-methyl-4,4'-bipiperidinyl-1-yl)-2-oxo-ethyl ester |
| (114) | 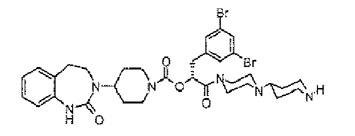 | 4-(2-Oxo-1,2,4,5-tetrahydro-1,3-benzdiazepin-3-yl)-piperidin-1-carbonsäure (R)-1-(3,5-dibrom-benzyl)-2-oxo-2-(4-piperidin-4-yl-piperazin-1-yl)-ethyl ester |
| (115) | 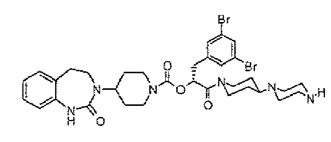 | 4-(2-Oxo-1,2,4,5-tetrahydro-1,3-benzdiazepin-3-yl)-piperidin-1-carbonsäure (R)-1-(3,5-dibrom-benzyl)-2-oxo-2-(4-piperazin-1-yl-piperidin-1-yl)-ethyl ester |
| (116) | 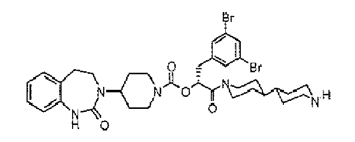 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-dibrom-benzyl)-2-oxo-ethyl ester |
| (117) | 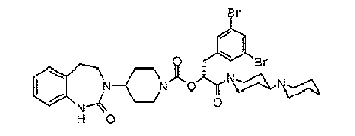 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-dibrom-benzyl)-2-oxo-ethyl ester |
| (118) | 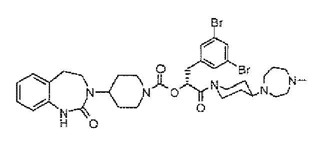 | 4-(2-Oxo-1,2,4,5-tetrahydro-1,3-benzdiazepin-3-yl)-piperidin-1-carbonsäure (R)-1-(3,5-dibrom-benzyl)-2-[4-(4-methyl-perhydro-1,4-diazepin-1-yl)-piperidin-1-yl]-2-oxo-ethyl ester |
| (119) | 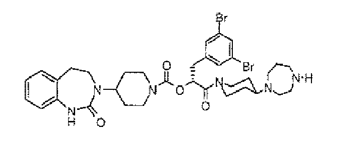 | 4-(2-Oxo-1,2,4,5-tetrahydro-1,3-benzdiazepin-3-yl)-piperidin-1-carbonsäure (R)-1-(3,5-dibrom-benzyl)-2-oxo-2-(4-perhydro-1,4-diazepin-1-yl-piperidin-1-yl)-ethyl ester |
| (120) | 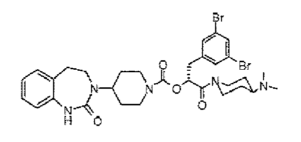 | 4-(2-Oxo-1,2,4,5-teirahydro-1,3-benzdiazepin-3-yl)-piperidin-1-carbonsäure (R)-1-(3,5-dibrom-benzyl)-2-(4-dimethylamino-piperidin-1-yl)-2-oxo-ethyl ester |
| (121) | 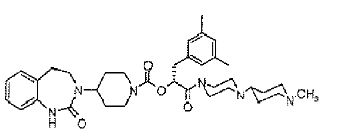 | 4-(2-Oxo-1,2,4,5-tetrahydro-1,3-benzdiazepin-3-yl)-piperidin-1-carbonsäure (R)-1-(3,5-dimethyl-benzyl)-2-[4-(1-methyl-piperidin-4-yl)-piperazin-1-yl]-2-oxo-ethyl ester |
| (122) | 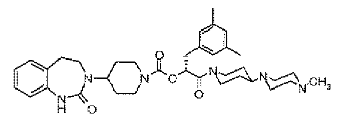 | 4-(2-Oxo-1,2,4,5-tetrahydro-1,3-benzdiazepin-3-yl)-piperidin-1-carbonsäure (R)-1-(3,5-dimethyl-benzyl)-2-[4-(4-methyl-piperazin-1-yl)-piperidin-1-yl]-2-oxo-ethyl ester |
| (123) | 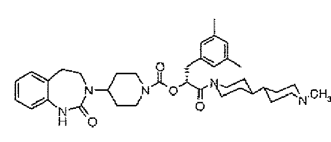 | 4-(2-Oxo-1,2,4,5-tetrahydro-1,3-benzdiazepin-3-yl)-piperidin-1-carbonsäure (R)-1-(3,5-dimethyl-benzyl)-2-(1'-methyl-4,4'-bipiperidinyl-1-yl)-2-oxo-ethyl ester |
| (124) | 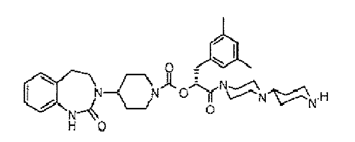 | 4-(2-Oxo-1,2,4,5-tetrahydro-1,3-benzdiazepin-3-yl)-piperidin-1-carbonsäure (R)-1-(3,5-dimethyl-benzyl)-2-oxo-2-(4-piperidin-4-yl-piperazin-1-yl)-ethyl ester |
| (125) | 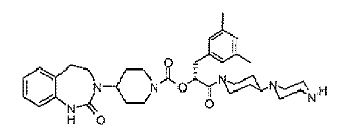 | 4-(2-Oxo-1,2,4,5-tetrahydro-1,3-benzdiazepin-3-yl)-piperidin-1-carbonsäure (R)-1-(3,5-dimethyl-benzyl)-2-oxo-2-(4-piperazin-1-yl-piperidin-1-yl)-ethyl ester |
| (126) | 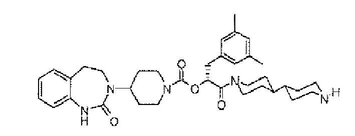 | 4-(2-Oxo-1,2,4,5-tetrahydro-1,3-benzdiazepin-3-yl)-piperidin-1-carbonsäure (R)-2-4,4'-bipiperidinyl-1-yl-1-(3,6-dimethyl-benzyl)-2-oxo-ethyl ester |
| (127) | 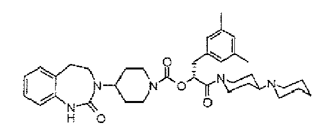 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-dimethyl-benzyl)-2-oxo-ethyl ester |
| (128) | 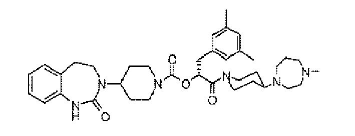 | 4-(2-Oxo-1,2,4,5-tetrahydro-1,3-benzdiazepin-3-yl)-piperidin-1-carbonsäure (R)-1-(3,5-dimethyl-benzyl)-2-[4-(4-methyl-perhydro-1,4-diazepin-1-yl)-piperidin-1-yl]-2-oxo-ethyl ester |
| (129) | 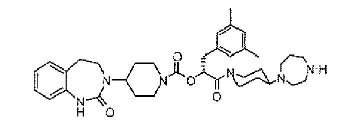 | 4-(2-Oxo-1,2,4,5-tetrahydro-1,3-benzdiazepin-3-yl)-piperidin-1-carbonsäure (R)-1-(3,5-dimethyl-benzyl)-2-oxo-2-(4-perhydro-1,4-diazepin-1-yl-piperidin-1-yl)-ethyl ester |
| (130) | 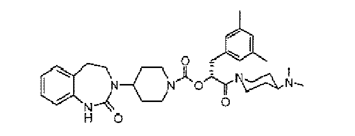 | 4-(2-Oxo-1,2,4,5-tetrahydro-1,3-benzdiazepin-3-yl)-piperidin-1-carbonsäure (R)-2-(4-dimethylamino-piperidin-1-yl)-1-(3,5-dimethyl-benzyl)-2-oxo-ethyl ester |
| (131) | 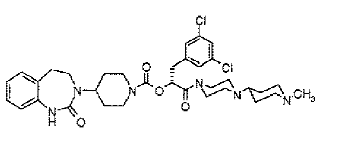 | 4-(2-Oxo-1,2,4,5-tetrahydro-1,3-benzdiazepin-3-yl)-piperidin-1-carbonsäure (R)-1-(3,5-dichlor-benzyl)-2-[4-(1-methyl-piperidin-4-yl)-piperazin-1-yl]-2-oxo-ethyl ester |
| (132) | 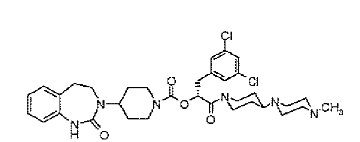 | 4-(2-Oxo-1,2,4,5-tetrahydro-1,3-benzdiazepin-3-yl)-piperidin-1-carbonsäure (R)-1-(3,5-dichlor-benzyl)-2-[4-(4-methyl-piperazin-1-yl)-piperidin-1-yl]-2-oxo-ethyl ester |
| (133) | 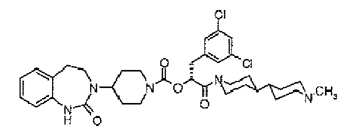 | 4-(2-Oxo-1,2,4,5-tetrahydro-1,3-benzdiazepin-3-yl)-piperidin-1-carbonsäure (R)-1-(3,5-dichlor-benzyl)-2-(1'-methyl-4,4'-bipiperidinyl-1-yl)-2-oxo-ethyl ester |
| (134) | 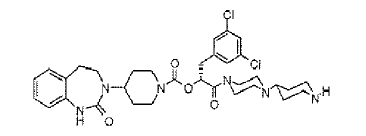 | 4-(2-Oxo-1,2,4,5-tetrahydro-1,3-benzdiazepin-3-yl)-piperidin-1-carbonsäure (R)-1-(3,5-dichlor-benzyl)-2-oxo-2-(4-piperidin-4-yl-piperazin-1-yl)-ethyl ester |
| (135) | 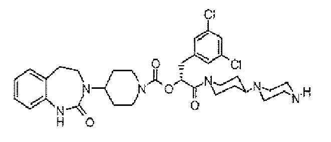 | 4-(2-Oxo-1,2,4,5-tetrahydro-1,3-benzdiazepin-3-yl)-piperidin-1-carbonsäure (R)-1-(3,5-dichlor-benzyl)-2-oxo-2-(4-piperazin-1-yl-piperidin-1-yl)-ethyl ester |
| (136) | 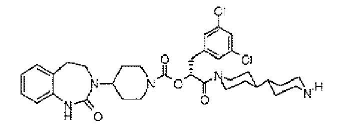 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-dichlor-benzyl)-2-oxo-ethyl ester |
| (137) | 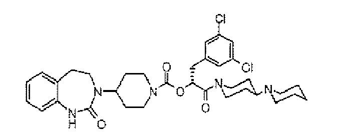 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-dichlor-benzyl)-2-oxo-ethyl ester |
| (138) | 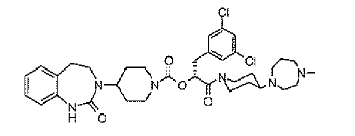 | 4-(2-Oxo-1,2,4,5-tetrahydro-1,3-benzdiazepin-3-yl)-piperidin-1-carbonsäure (R)-1-(3,5-dichlor-benzyl)-2-[4-(4-methyl-perhydro-1,4-diazepin-1-yl)-piperidin-1-yl]-2-oxo-ethyl ester |
| (139) | 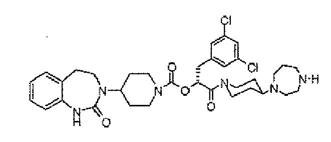 | 4-(2-Oxo-1,2,4,5-tetrahydro-1,3-benzdiazepin-3-yl)-piperidin-1-carbonsäure (R)-1-(3,5-dichlor-benzyl)-2-oxo-2-(4-perhydro-1,4-diazepin-1-yl-piperidin-1-yl)-ethyl ester |
| (140) | 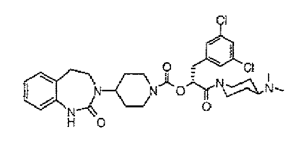 | 4-(2-Oxo-1,2,4,5-tetrahydro-1,3-benzdiazepin-3-yl)-piperidin-1-carbonsäure (R)-1-(3,5-dichlor-benzyl)-2-(4-dimethylamino-piperidin-1-yl)-2-oxo-ethyl ester |
| (141) | 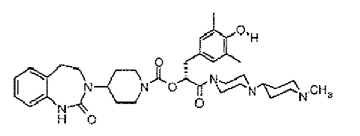 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-(1-methyl-piperidin-4-yl)-piperazin-1-yl]-2-oxo-ethyl ester |
| (142) | 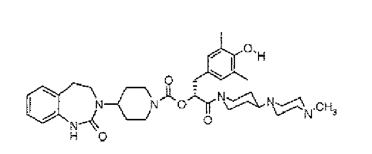 | 4-(2-Oxo-1,2,4,5-tetrahydro-1,3-benzdiazepin-3-yl)-pi peridin-1-carbonsäure (R)-1-(4-hydroxy-3,5-dimethyl-benzyl)-2-[4-(4-methyl-piperazin-1-yl)-piperidin-1-yl]-2-oxo-ethyl ester |
| (143) | 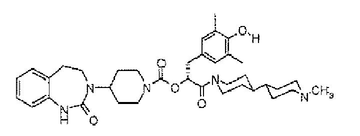 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-(1'-methyl-4,4'-bipiperidinyl-1-yl)-2-oxo-ethyl ester |
| (144) | 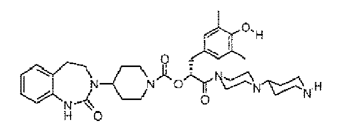 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-oxo-2-(4-piperidin-4-yl-piperazin-1-yl)-ethyl ester |
| (145) | 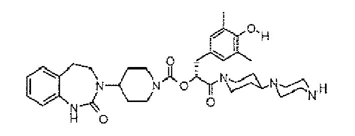 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-oxo-2-(4-piperazin-1-yl-piperidin-1-yl)-ethyl ester |
| (146) | 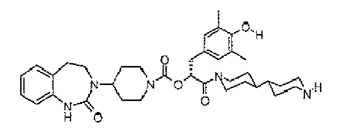 | 4-(2-Oxo-1,2,4,5-tetrahydro-1,3-benzdiazepin-3-yl)-piperidin-1-carbonsäure (R)-2-4,4'-bipiperidinyl-1-yl-1-(4-hydroxy-3,5-dimethyl-benzyl)-2-oxo-ethyl ester |
| (147) | 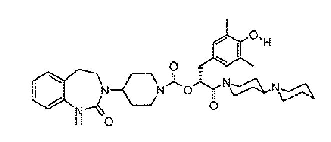 | 4-(2-Oxo-1,2,4,5-tetrahydro-1,3-benzdiazepin-3-yl)-piperidin-1-carbonsäure (R)-2-1,4'-bipiperidinyl-1'-yl-1-(4-hydroxy-3,5-dimethyl-benzyl)-2-oxo-ethyl ester |
| (148) | 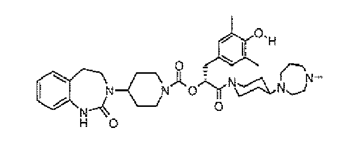 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-(4-methyl-perhydro-1,4-diazepin-1-yl)-piperidin-1-yl]-2-oxo-ethyl ester |
| (149) | 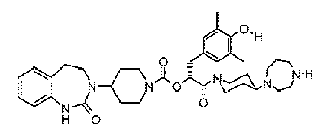 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-oxo-2-(4-perhydro-1,4-diazepin-1-yl-piperidin-1-yl)-ethyl ester |
| (150) | 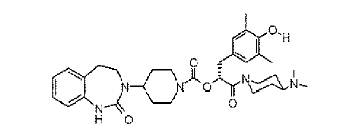 | 4-(2-Oxo-1,2,4,5-tetrahydro-1,3-benzdiazepin-3-yl)-piperidin-1-carbonsäure (R)-2-(4-dimethylamino-piperidin-1-yl)-1-(4-hydroxy-3,5-dimethyl-benzyl)-2-oxo-ethyl ester |
| (151) | 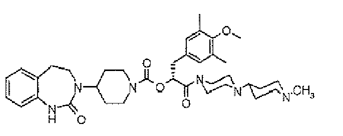 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-[4-(1-methyl-piperidin-4-yl)-piperazin-1-yl]-2-oxo-ethyl ester |
| (152) | 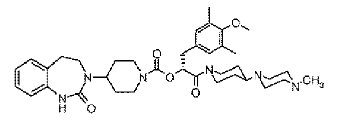 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-[4-(4-methyl-piperazin-1-yl)-piperidin-1-yl]-2-oxo-ethyl ester |
| (153) | 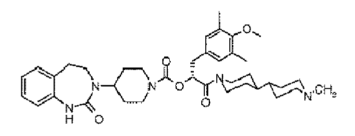 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-(1'-methyl-4,4'-bipiperidinyl-1-yl)-2-oxo-ethyl ester |
| (154) | 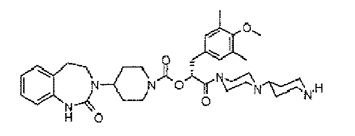 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-oxo-2-(4-piperidin-4-yl-piperazin-1-yl)-ethyl ester |
| (155) | 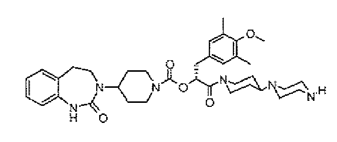 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-oxo-2-(4-piperazin-1-yl-piperidin-1-yl)-ethyl ester |
| (156) | 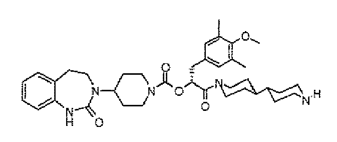 | 4-(2-Oxo-1,2,4,5-tetrahydro-1,3-benzdiazepin-3-yl)-piperidin-1-carbonsäure (R)-2-4,4'-bipiperidinyl-1-yl-1-(4-methoxy-3,5-dimethyl-benzyl)-2-oxo-ethyl ester |
| (157) | 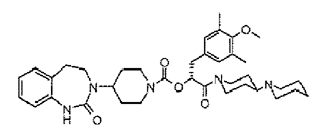 | 4-(2-Oxo-1,2,4,5-tetrahydro-1,3-benzdiazepin-3-yl)-piperidin-1-carbonsäure (R)-2-1,4'-bipiperidinyl-1'-yl-1-(4-methoxy-3,5-dimethyl-benzyl)-2-oxo-ethyl ester |
| (158) | 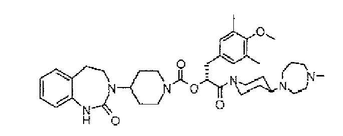 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-[4-(4-methyl-perhydro-1,4-diazepin-1-yl)-piperidin-1-yl]-2-oxo-ethyl ester |
| (159) | 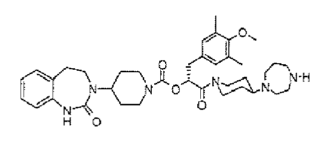 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-oxo-2-(4-perhydro-1,4-diazepin-1-yl-piperidin-1-yl)-ethyl ester |
| (160) | 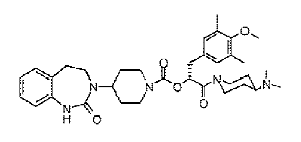 | 4-(2-Oxo-1,2,4,5-tetrahydro-1,3-benzdiazepin-3-yl)-piperidin-1-carbonsäure (R)-2-(4-dimethylamino-piperidin-1-yl)-1-(4-methoxy-3,5-dimethyl-benzyl)-2-oxo-ethyl ester |
| (161) | 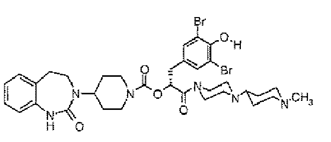 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(1-methylpiperidin-4-yl)-piperazin-1-yl]-2-oxo-ethyl ester |
| (162) | 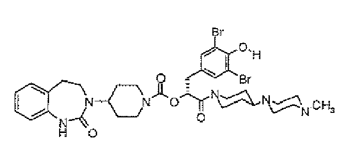 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(4-methyl-piperazin-1-yl)-piperidin-1-yl]-2-oxo-ethyl ester |
| (163) | 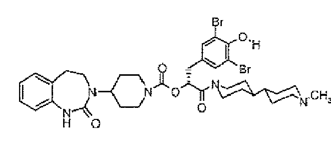 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(1'-methyl-4,4'-bipiperidinyl-1-yl)-2-oxo-ethyl ester |
| (164) | 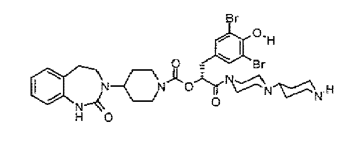 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-oxo-2-(4-piperidin-4-yl-piperazin-1-yl)-ethyl ester |
| (165) | 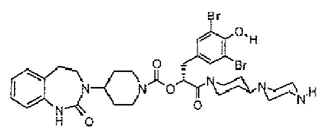 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-oxo-2-(4-piperazin-1-yl-piperidin-1-yl)-ethyl ester |
| (166) | 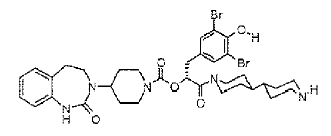 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-dibrom-4-hydroxy-benzyl)-2-oxo-ethyl ester |
| (167) | 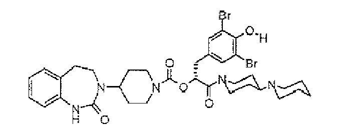 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-dibrom-4-hydroxy-benzyl)-2-oxo-ethyl ester |
| (168) | 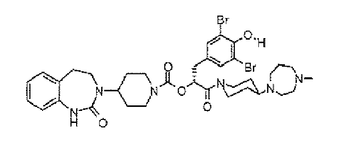 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(4-methyl-perhydro-1,4-diazepin-1-yl)-piperidin-1-yl]-2-oxo-ethyl ester |
| (169) | 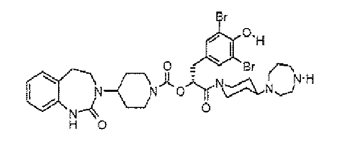 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-oxo-2-(4-perhydro-1,4-diazepin-1-yl-piperidin-1-yl)-ethyl ester |
| (170) | 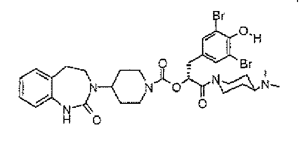 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(4-dimethylamino-piperidin-1-yl)-2-oxo-ethyl ester |
| (171) | 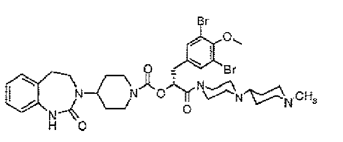 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-[4-(1-methyl-piperidin-4-yl)-piperazin-1-yl]-2-oxo-ethyl ester |
| (172) | 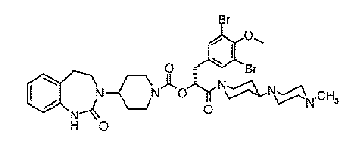 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-[4-(4-methyl-piperazin-1-yl)-piperidin-1-yl]-2-oxo-ethyl ester |
| (173) | 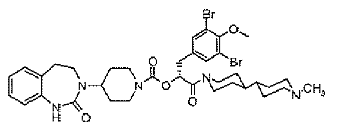 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-(1'-methyl-4,4'-bipiperidinyl-1-yl)-2-oxo-ethyl ester |
| (174) | 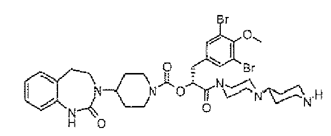 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-oxo-2-(4- piperidin-4-yl-piperazin-1-yl)-ethyl ester |
| (175) | 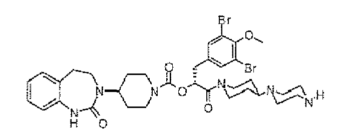 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-oxo-2-(4-piperazin-1-yl-piperidin-1-yl)-ethyl ester |
| (176) | 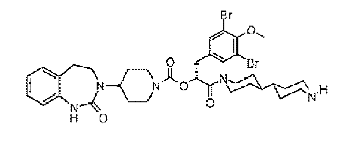 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-dibrom-4-methoxy-benzyl)-2-oxo-ethyl ester |
| (177) | 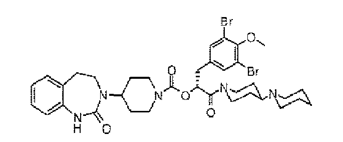 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-dibrom-4-methoxy-benzyl)-2-oxo-ethyl ester |
| (178) | 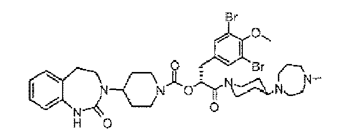 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-[4-(4-methyl-perhydro-1,4-diazepin-1-yl)-piperidin-1-yl]-2-oxo-ethyl ester |
| (179) | 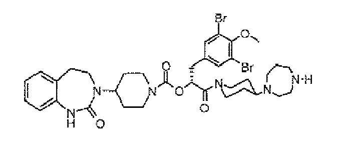 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-oxo-2-(4-perhydro-1,4-diazepin-1-yl-piperidin-1-yl)-ethyl ester |
| (180) | 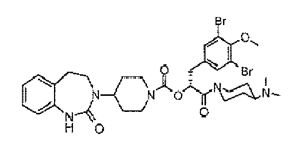 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-(4-dimethylamino-piperidin-1-yl)-2-oxo-ethyl ester |
| (181) | 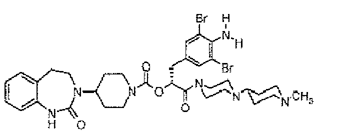 | 4-(2-Oxo-1,2,4,5-tetrahydro-1,3-benzdiazepin-3-yl)-piperidin-1-carbonsäure (R)-1-(4-amino-3,5-dibrom-benzyl)-2-[4-(1-methyl-piperidin-4-yl)-piperazin-1-yl]-2-oxo-ethyl ester |
| (182) | 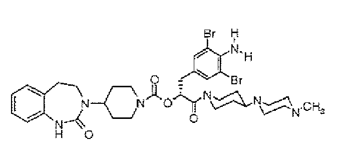 | 4-(2-Oxo-1,2,4,5-tetrahydro-1,3-benzdiazepin-3-yl)-piperidin-1-carbonsäure (R)-1-(4-amino-3,5-dibrom-benzyl)-2-[4-(4-methyl-piperazin-1-yl)-piperidin-1-yl]-2-oxo-ethyl ester |
| (183) | 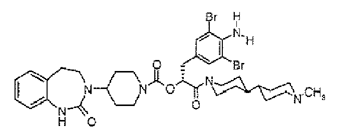 | 4-(2-Oxo-1,2,4,5-tetrahydro-1,3-benzdiazepin-3-yl)-piperidin-1-carbonsäure (R)-1-(4-amino-3,5-dibrom-benzyl)-2-(1'-methyl-4,4'-bipiperidinyl-1-yl)-2-oxo-ethyl ester |
| (184) | 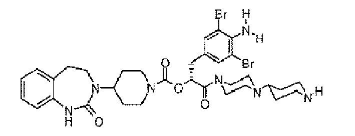 | 4-(2-Oxo-1,2,4,5-tetrahydro-1,3-benzdiazepin-3-yl)-piperidin-1-carbonsäure (R)-1-(4-amino-3,5-dibrom-benzyl)-2-oxo-2-(4-piperidin-4-yl-piperazin-1-yl)-ethyl ester |
| (185) | 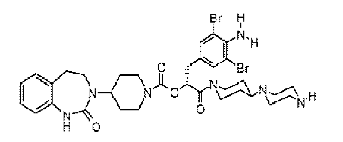 | 4-(2-Oxo-1,2,4,5-tetrahydro-1,3-benzdiazepin-3-yl)-piperidin-1-carbonsäure (R)-1-(4-amino-3,5-dibrom-benzyl)-2-oxo-2-(4-piperazin-1-yl-piperidin-1-yl)-ethyl ester |
| (186) | 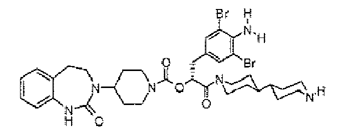 | 4-(2-Oxo-1,2,4,5-tetrahydro-1,3-benzdiazepin-3-yl)-piperidin-1-carbonsäure (R)-1-(4-amino-3,5-dibrom-benzyl)-2-4,4'-bipiperidinyl-1-yl-2-oxo-ethyl ester |
| (187) | 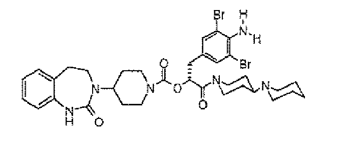 | 4-(2-Oxo-1,2,4,5-tetrahydro-1,3-benzdiazepin-3-yl)-piperidin-1-carbonsäure (R)-1-(4-amino-3,5-dibrom-benzyl)-2-1,4'-bipiperidinyl-1'-yl-2-oxo-ethyl ester |
| (188) | 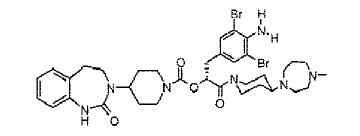 | 4-(2-Oxo-1,2,4,5-tetrahydro-1,3-benzdiazepin-3-yl)-piperidin-1-carbonsäure (R)-1-(4-amino-3,5-dibrom-benzyl)-2-[4-(4-methyl-perhydro-1,4-diazepin-1-yl)-piperidin-1-yl]-2-oxo-ethyl ester |
| (189) | 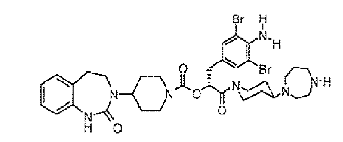 | 4-(2-Oxo-1,2,4,5-tetrahydro-1,3-benzdiazepin-3-yl)-piperidin-1-carbonsäure (R)-1-(4-amino-3,5-dibrom-benzyl)-2-oxo-2-(4-perhydro-1,4-diazepin-1-yl-piperidin-1-yl)-ethyl ester |
| (190) | 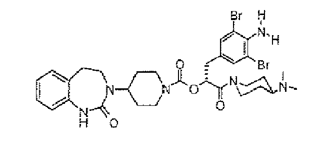 | 4-(2-Oxo-1,2,4,5-tetrahydro-1,3-benzdiazepin-3-yl)-pipendin-1-carbonsäure (R)-1-(4-amino-3,5-dibrom-benzyl)-2-(4-dimethylamino-piperidin-1-yl)-2-oxo-ethyl ester |
| (191) | 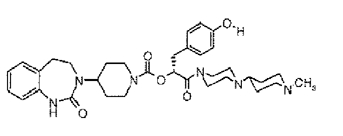 | 4-(2-Oxo-1,2,4,5-tetrahydro-1,3-benzdiazepin-3-yl)-piperidin-1-carbonsäure (R)-1-(4-hydroxy-benzyl)-2-[4-(1-methyl-piperidin-4-yl)-piperazin-1-yl]-2-oxo-ethyl ester |
| (192) | 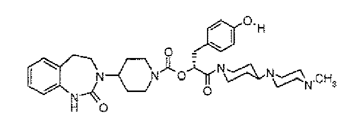 | 4-(2-Oxo-1,2,4,5-tetrahydro-1,3-benzdiazepin-3-yl)-piperidin-1-carbonsäure (R)-1-(4-hydroxy-benzyl)-2-[4-(4-methyl-piperazin-1-yl)-piperidin-1-yl]-2-oxo-ethyl ester |
| (193) | 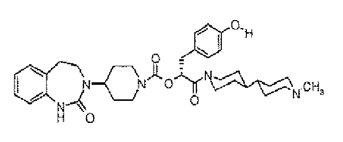 | 4-(2-Oxo-1,2,4,5-tetrahydro-1,3-benzdiazepin-3-yl)-piperidin-1-carbonsäure (R)-1-(4-hydroxy-benzyl)-2-(1'-methyl-4,4'-bipiperidinyl-1-yl)-2-oxo-ethyl ester |
| (194) | 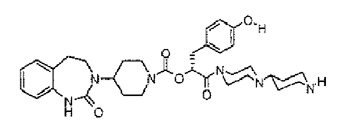 | 4-(2-Oxo-1,2,4,5-tetrahydro-1,3-benzdiazepin-3-yl)-piperidin-1-carbonsäure (R)-1-(4-hydroxy-benzyl)-2-oxo-2-(4-piperidin-4-yl-piperazin-1-yl)-ethyl ester |
| (195) | 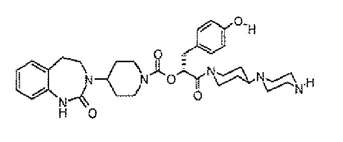 | 4-(2-Oxo-1,2,4,5-tetrahydro-1,3-benzdiazepin-3-yl)-piperidin-1-carbonsäure (R)-1-(4-hydroxy-benzyl)-2-oxo-2-(4-piperazin-1-yl-piperidin-1-yl)-ethyl ester |
| (196) | 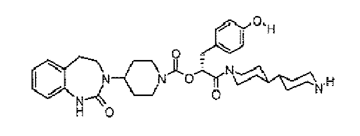 | 4-(2-Oxo-1,2,4,5-tetrahydro-1,3-benzdiazepin-3-yl)-piperidin-1-carbonsäure (R)-2-4,4'-bipiperidinyl-1-yl-1-(4-hydroxy-benzyl)-2-oxo-ethyl ester |
| (197) | 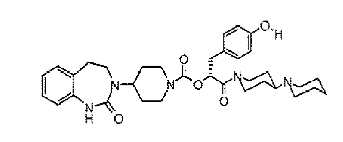 | 4-(2-Oxo-1,2,4,5-tetrahydro-1,3-benzdiazepin-3-yl)-piperidin-1-carbonsäure (R)-2-1,4'-bipiperidinyl-1'-yl-1-(4-hydroxy-benzyl)-2-oxo-ethyl ester |
| (198) | 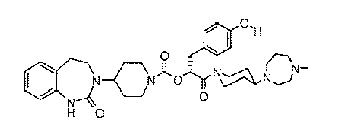 | 4-(2-Oxo-1,2,4,5-tetrahydro-1,3-benzdiazepin-3-yl)-piperidin-1-carbonsäure (R)-1-(4-hydroxy-benzyl)-2-[4-(4-methyl-perhydro-1,4-diazepin-1-yl)-piperidin-1-yl]-2-oxo-ethyl ester |
| (199) | 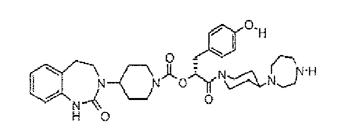 | 4-(2-Oxo-1,2,4,5-tetrahydro-1,3-benzdiazepin-3-yl)-piperidin-1-carbonsäure (R)-1-(4-hydroxy-benzyl)-2-oxo-2-(4-perhydro-1,4-diazepin-1-yl-piperidin-1-yl)-ethyl ester |
| (200) | 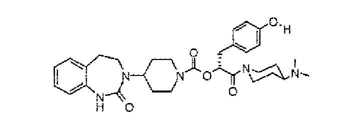 | 4-(2-Oxo-1,2,4,5-tetrahydro-1,3-benzdiazepin-3-yl)-piperidin-1-carbonsäure (R)-2-(4-dimethylamino-piperidin-1-yl)-1-(4-hydroxy-benzyl)-2-oxo-ethyl ester |
| (201) | 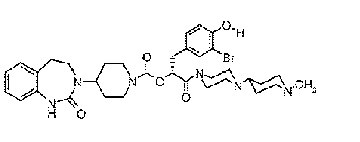 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(1-methyl-piperidin-4-yl)-piperazin-1-yl]-2-oxo-ethyl ester |
| (202) | 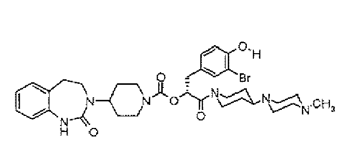 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(4-methyl-piperazin-1-yl)-piperidin-1-yl]-2-oxo-ethyl ester |
| (203) | 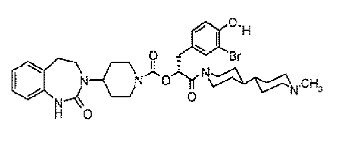 | 4-(2-Oxo-1,2,4,5-tetrahydro-1,3-benzdiazepin-3-yl)-piperidin-1-carbonsäure (R)-1-(3-brom-4-hydroxy-benzyl)-2-(1'-methyl-4,4'-bipiperidinyl-1-yl)-2-oxo-ethyl ester |
| (204) | 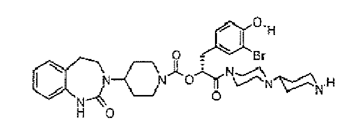 | 4-(2-Oxo-1,2,4,5-tetrahydro-1,3-benzdiazepin-3-yl)-piperidin-1-carbonsäure (R)-1-(3-brom-4-hydroxy-benzyl)-2-oxo-2-(4-piperidin-4-yl-piperazin-1-yl)-ethyl ester |
| (205) | 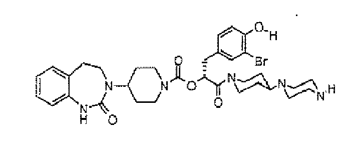 | 4-(2-Oxo-1,2,4,5-tetrahydro-1,3-benzdiazepin-3-yl)-piperidin-1-carbonsäure (R)-1-(3-brom-4-hydroxy-benzyl)-2-oxo-2-(4-piperazin-1-yl-piperidin-1-yl)-ethyl ester |
| (206) | 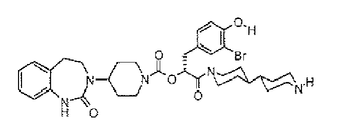 | 4-(2-Oxo-1,2,4,5-tetrahydro-1,3-benzdiazepin-3-yl)-piperidin-1-carbonsäure (R)-2-4,4'-bipiperidinyl-1-yl-1-(3-brom-4-hydroxy-benzyl)-2-oxo-ethyl ester |
| (207) | 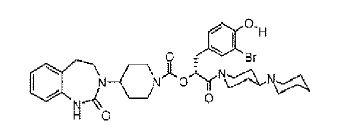 | 4-(2-Oxo-1,2,4,5-tetrahydro-1,3-benzdiazepin-3-yl)-piperidin-1-carbonsäure (R)-2-1,4'-bipiperidinyl-1'-yl-1-(3-brom-4-hydroxy-benzyl)-2-oxo-ethyl ester |
| (208) | 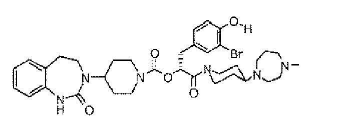 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(4-methyl-perhydro-1,4-diazepin-1-yl)-piperidin-1-yl]-2-oxo-ethyl ester |
| (209) | 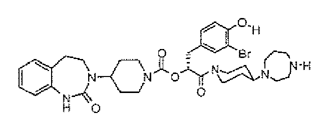 | 4-(2-Oxo-1,2,4,5-tetrahydro-1,3-benzdiazepin-3-yl)-piperidin-1-carbonsäure (R)-1-(3-brom-4-hydroxy-benzyl)-2-oxo-2-(4-perhydro-1,4-diazepin-1-yl-piperidin-1-yl)-ethyl ester |
| (210) | 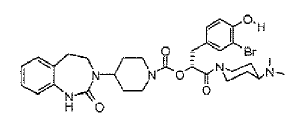 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-dimethylamino-piperidin-1-yl)-2-oxo-ethyl ester |
| (211) | 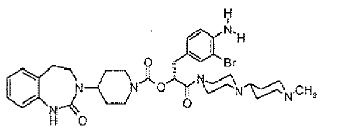 | 4-(2-Oxo-1,2,4,5-tetrahydro-1,3-benzdiazepin-3-yl)-piperidin-1-carbonsäure (R)-1-(4-amino-3-brom-benzyl)-2-[4-(1-methyl-piperidin-4-yl)-piperazin-1-yl]-2-oxo-ethyl ester |
| (212) | 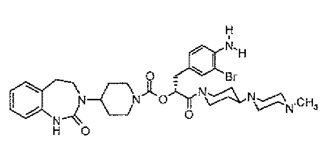 | 4-(2-Oxo-1,2,4,5-tetrahydro-1,3-benzdiazepin-3-yl)-piperidin-1-carbonsäure (R)-1-(4-amino-3-brom-benzyl)-2-[4-(4-methyl-piperazin-1-yl)-piperidin-1-yl]-2-oxo-ethyl ester |
| (213) | 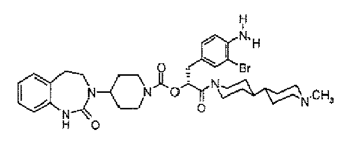 | 4-(2-Oxo-1,2,4,5-tetrahydro-1,3-benzdiazepin-3-yl)-piperidin-1-carbonsäure (R)-1-(4-amino-3-brom-benzyl)-2-(1'-methyl-4,4'-bipiperidinyl-1-yl)-2-oxo-ethyl ester |
| (214) | 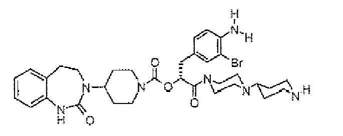 | 4-(2-Oxo-1,2,4,5-tetrahydro-1,3-benzdiazepin-3-yl)-piperidin-1-carbonsäure (R)-1-(4-amino-3-brom-benzyl)-2-oxo-2-(4-piperidin-4-yl-piperazin-1-yl)-ethyl ester |
| (215) | 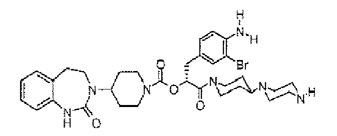 | 4-(2-Oxo-1,2,4,5-tetrahydro-1,3-benzdiazepin-3-yl)-piperidin-1-carbonsäure (R)-1-(4-amino-3-brom-benzyl)-2-oxo-2-(4-piperazin-1-yl-piperidin-1-yl)-ethyl ester |
| (216) | 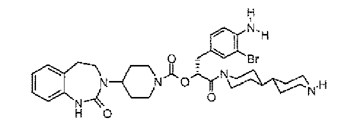 | 4-(2-Oxo-1,2,4,5-tetrahydro-1,3-benzdiazepin-3-yl)-piperidin-1-carbonsäure (R)-1-(4-amino-3-brom-benzyl)-2-4,4'-bipiperidinyl-1-yl-2-oxo-ethyl ester |
| (217) | 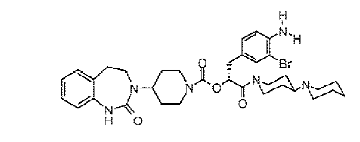 | 4-(2-Oxo-1,2,4,5-tetrahydro-1,3-benzdiazepin-3-yl)-piperidin-1-carbonsäure (R)-1-(4-amino-3-brom-benzyl)-2-1,4'-bipiperidinyl-1'-yl-2-oxo-ethyl ester |
| (218) | 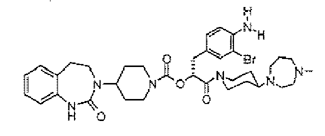 | 4-(2-Oxo-1,2,4,5-tetrahydro-1,3-benzdiazepin-3-yl)-piperidin-1-carbonsäure (R)-1-(4-amino-3-brom-benzyl)-2-[4-(4-methyl-perhydro-1,4-diazepin-1-yl)-piperidin-1-yl]-2-oxo-ethyl ester |
| (219) | 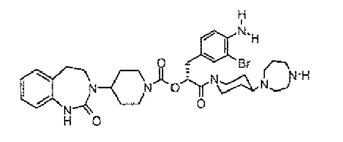 | 4-(2-Oxo-1,2,4,5-tetrahydro-1,3-benzdiazepin-3-yl)-piperidin-1-carbonsäure (R)-1-(4-amino-3-brom-benzyl)-2-oxo-2-(4-perhydro-1,4-diazepin-1-yl-piperidin-1-yl)-ethyl ester |
| (220) | 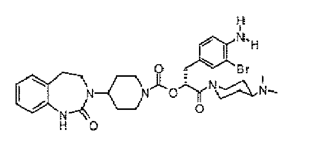 | 4-(2-Oxo-1,2,4,5-tetrahydro-1,3-benzdiazepin-3-yl)-piperidin-1-carbonsäure (R)-1-(4-amino-3-brom-benzyl)-2-(4-dimethylamino-piporidin-1-yl)-2-oxo-ethyl ester |
| (221) | 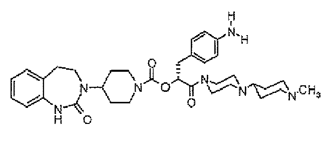 | 4-(2-Oxo-1,2,4,5-tetrahydro-1,3-benzdiazepin-3-yl)-piperidin-1-carbonsäure (R)-1-(4-amino-benzyl)-2-[4-(1-methyl-piperidin-4-yl)-piperazin-1-yl]-2-oxo-ethyl ester |
| (222) | 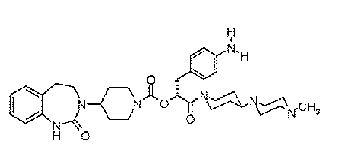 | 4-(2-Oxo-1,2,4,5-tetrahydro-1,3-benzdiazepin-3-yl)-piperidin-1-carbonsäure (R)-1-(4-amino-benzyl)-2-[4-(4-methyl-piperazin-1-yl)-piperidin-1-yl]-2-oxo-ethyl ester |
| (223) | 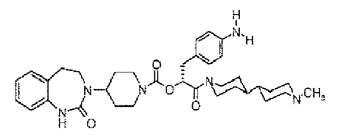 | 4-(2-Oxo-1,2,4,5-tetrahydro-1,3-benzdiazepin-3-yl)-piperidin-1-carbonsäure (R)-1-(4-amin-benzyl)-2-(1'-methyl-4,4'-bipiperidinyl-1-yl)-2-oxo-ethyl ester |
| (224) | 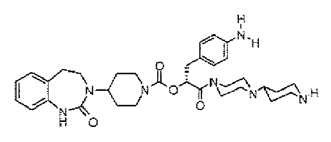 | 4-(2-Oxo-1,2,4,5-tetrahydro-1,3-benzdiazepin-3-yl)-piperidin-1-carbonsäure (R)-1-(4-amino-benzyl)-2-oxo-2-(4-piperidin-4-yl-piperazin-1-yl)-ethyl ester |
| (225) | 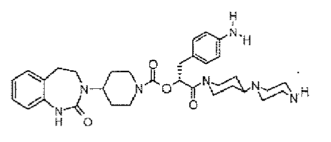 | 4-(2-Oxo-1,2,4,5-tetrahydro-1,3-henzdiazepin-3-yl)-piperidin-1-carbonsäure (R)-1-(4-amino-benzyl)-2-oxo-2-(4-piperazin-1-yl-piperidin-1-yl)-ethyl ester |
| (226) | 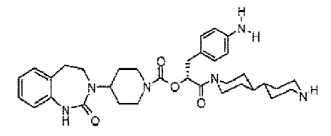 | 4-(2-Oxo-1,2,4,5-tetrahydro-1,3-benzdiazepin-3-yl)-piperidin-1-carbonsäure (R)-1-(4-amino-benzyl)-2-4,4'-bipiperidinyl-1-yl-2-oxo-ethyl ester |
| (227) | 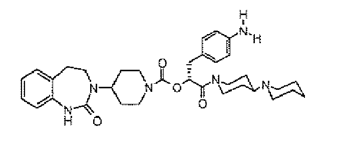 | 4-(2-Oxo-1,2,4,5-tetrahydro-1,3-benzdiazepin-3-yl)-piperidin-1-carbonsäure (R)-1-(4-amino-benzyl)-2-1,4'-bipiperidinyl-1'-yl-2-oxo-ethyl ester |
| (228) | 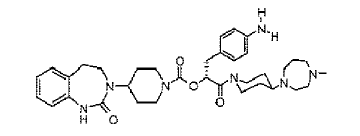 | 4-(2-Oxo-1,2,4,5-tetrahydro-1,3-benzdiazepin-3-yl)-piperidin-1-carbonsäure (R)-1-(4-amino-benzyl)-2-[4-(4-methyl-perhydro-1,4-diazepin-1-yl)-piperidin-1-yl]-2-oxo-ethyl ester |
| (229) | 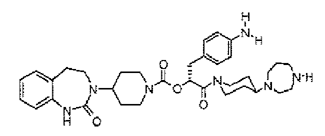 | 4-(2-Oxo-1,2,4,5-tetrahydro-1,3-benzdiazepin-3-yl)-piperidin-1-earbonsäure (R)-1-(4-amino-benzyl)-2-oxo-2-(4-perhydro-1,4-diazepin-1-yl-piperidin-1-yl)-ethyl ester |
| (230) | 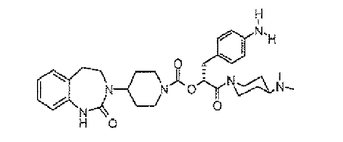 | 4-(2-Oxo-1,2,4,5-tetrahydro-1,3-benzdiazepin-3-yl)-piperidin-1-carbonsäure (R)-1-(4-amino-benzyl)-2-(4-dimethylamino-piperidin-1-yl)-2-oxo-ethyl ester |
| (231) | 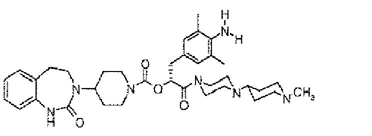 | 4-(2-Oxo-1,2,4,5-tetrahydro-1,3-benzdiazepin-3-yl)-piperidin-1-carbonsäure (R)-1-(4-amino-3,5-dimethyl-benzyl)-2-[4-(1-methyl-piperidin-4-yl)-piperazin-1-yl]-2-oxo-ethyl ester |
| (232) | 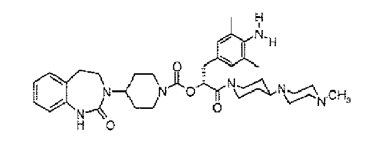 | 4-(2-Oxo-1,2,4,5-tetrahydro-1,3-benzdiazepin-3-yl)-piperidin-1-carbonsäure (R)-1-(4-amino-3,5-dimethyl-benzyl)-2-[4-(4-methyl-piperazin-1-yl)-piperidin-1-yl]-2-oxo-ethyl ester |
| (233) | 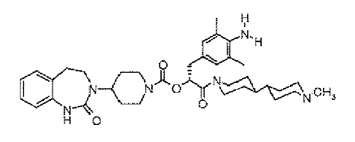 | 4-(2-Oxo-1,2,4,5-tetrahydro-1,3-benzdiazepin-3-yl)-piperidin-1-carbonsäure (R)-1-(4-amino-3,5-dimethyl-benzyl)-2-(1'-methyl-4,4'-bipiparidinyl-1-yl)-2-oxo-ethyl ester |
| (234) | 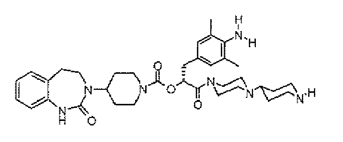 | 4-(2-Oxo-1,2,4,5-tetrahydro-1,3-benzdiazepin-3-yl)-piperidin-1-carbonsäure (R)-1-(4-amino-3,5-dimethyl-benzyl)-2-oxo-2-(4-piperidin-4-yl-piperazin-1-yl)-ethyl ester |
| (235) | 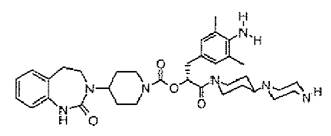 | 4-(2-Oxo-1,2,4,5-tetrahydro-1,3-benzdiazepin-3-yl)-piperidin-1-carbonsäure (R)-1-(4-amino-3,5-dimethyl-benzyl)-2-oxo-2-(4-piperazin-1-yl-piperidin-1-yl)-ethyl ester |
| (236) | 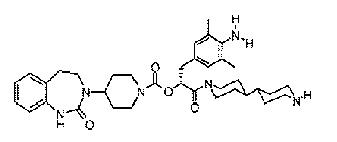 | 4-(2-Oxo-1,2,4,5-tetrahydro-1,3-benzdiazepin-3-yl)-piperidin-1-carbonsäure (R)-1-(4-amino-3,5-dimethyl-benzyl)-2-4,4'-bipiperidinyl-1-yl-2-oxo-ethyl ester |
| (237) | 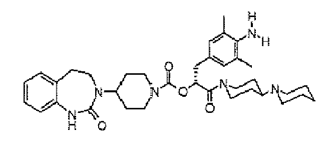 | 4-(2-Oxo-1,2,4,5-tetrahydro-1,3-benzdiazepin-3-yl)-piperidin-1-carbonsäure (R)-1-(4-amino-3,5-dimethyl-benzyl)-2-1,4'-bipiperidinyl-1'-yl-2-oxo-ethyl ester |
| (238) | 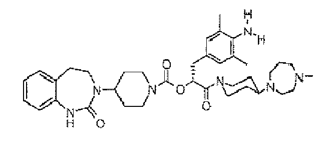 | 4-(2-Oxo-1,2,4,5-tetrahydro-1,3-benzdiazepin-3-yl)-piperidin-1-carbonsäure (R)-1-(4-amino-3,5-dimethyl-benzyl)-2-[4-(4-methyl-perhydro-1,4-diazepin-1-yl)-piperidin-1-yl]-2-oxo-ethyl ester |
| (239) | 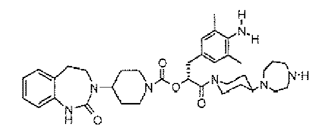 | 4-(2-Oxo-1,2,4,5-tetrahydro-1,3-benzdiazepin-3-yl)-piperidin-1-carbonsäure (R)-1-(4-amino-3,5-dimethyl-benzyl)-2-oxo-2-(4-perhydro-1,4-diazepin-1-yl-piperidin-1-yl)-ethyl ester |
| (240) | 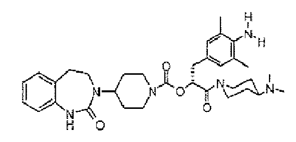 | 4-(2-Oxo-1,2,4,5-tetrahydro-1,3-benzdiazepin-3-yl)-piperidin-1-carbonsäure (R)-1-(4-amino-3,5-dimethyl-benzyl)-2-(4-dimethylamino-piperidin-1-yl)-2-oxo-ethyl ester |
| (241) | 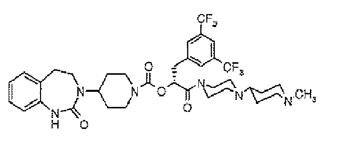 | 4-(2-Oxo-1,2,4,5-tetrahydro-1,3-benzdiazepin-3-yl)-piperidin-1-carbonsäure (R)-1-(3,5-bis-trifluormethyl-benzyl)-2-[4-(1-methyl-piperidin-4-yl)-piperazin-1-yl]-2-oxo-ethyl ester |
| (242) | 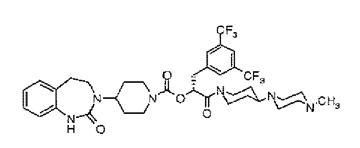 | 4-(2-Oxo-1,2,4,5-tetrahydro-1,3-benzdiazepin-3-yl)-piperidin-1-carbonsäure (R)-1-(3,5-bis-trifluormethyl-benzyl)-2-[4-(4-methyl-piperazin-1-yl)-pipendin-1-yl]-2-oxo-ethyl ester |
| (243) | 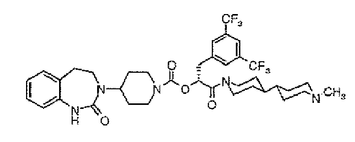 | 4-(2-Oxo-1,2,4,5-tetrahydro-1,3-benzdiazepin-3-yl)-piperidin-1-carbonsäure (R)-1-(3,5-bis-trifluormethyl-benzyl)-2-(1'-methyl-4,4'-bipiperidinyl-1-yl)-2-oxo-ethyl ester |
| (244) | 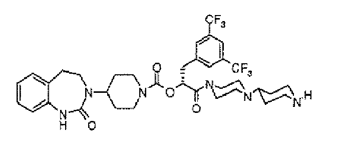 | 4-(2-Oxo-1,2,4,5-tetrahydro-1,3-banzdiazepin-3-yl)-piperidin-1-carbonsäure (R)-1-(3,5-bis-trifluormethyl-benzyl)-2-oxo-2-(4-piperidin-4-yl-piperazin-1-yl)-ethyl ester |
| (245) | 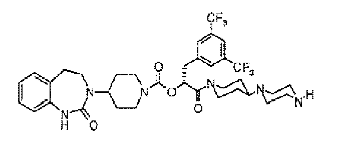 | 4-(2-Oxo-1,2,4,5-tetrahydro-1,3-benzdiazepin-3-yl)-piperidin-1-carbonsäure (R)-1-(3,5-bis-trifluormethyl-benzyl)-2-oxo-2-(4-piperazin-1-yl-piperidin-1-yl)-ethyl ester |
| (246) | 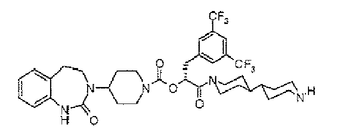 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-bis-trifluormethyl-benzyl)-2-oxo-ethylester |
| (247) | 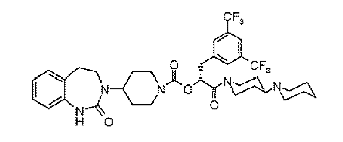 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-bis-thifluormethyl-benzyl)-2-oxo-ethylester |
| (248) | 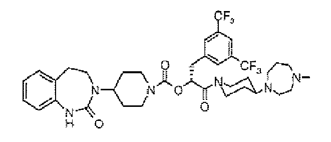 | 4-(2-Oxo-1,2,4,5-tetrahydro-1,3-benzdiazepin-3-yl)-piperidin-1-carbonsäure (R)-1-(3,5-bis-trifluormethyl-benzyl)-2-[4-(4-methyl-perhydro-1,4-diazepin-1-yl)-piperidin-1-yl]-2-oxo-ethylester |
| (249) | 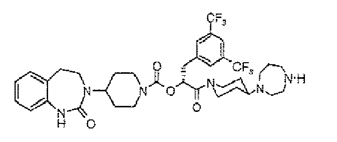 | 4-(2-Oxo-1,2,4,5-tetrahydro-1,3-benzdiazepin-3-yl)-piperidin-1-carbonsäure (R)-1-(3,5-bis-trifluormethyl-benzyl)-2-oxo-2-(4-perhydro-1,4-diazepin-1-yl-piperidin-1-yl)-ethylester |
| (250) | 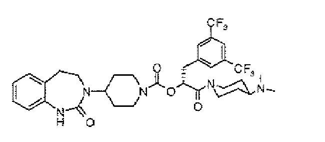 | 4-(2-Oxo-1,2,4,5-tetrahydro-1,3-benzdiazepin-3-yl)-piperidin-1-carbonsäure (R)-1-(3,5-bis-trifluormethyl-benzyl)-2-(4-dimethylamino-piperidin-1-yl)-2-oxo-ethylester |
| (251) | 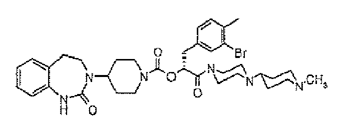 | 4-(2-Oxo-1,2,4,5-tetrahydro-1,3-benzdiazepin-3-yl)-piperidin-1-carbonsäure (R)-1-(3-brom-4-methyl-benzyl)-2-[4-(1-methyl-piperidin-4-yl)-piperazin-1-yl] -2-oxo-ethyl ester |
| (252) | 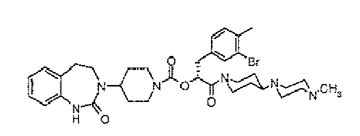 | 4-(2-Oxo-1,2,4,5-tetrahydro-1,3-benzdiazepin-3-yl)-piperidin-1-carbonsäure (R)-1-(3-brom-4-methyl-benzyl)-2-[4-(4-methyl-piperazin-1-yl)-piperidin-1-yl]-2-oxo-ethyl ester |
| (253) | 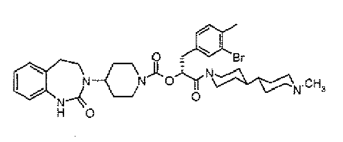 | 4-(2-Oxo-1,2,4,5-tetrahydro-1,3-benzdiazepin-3-yl)-piperidin-1-carbonsäure (R)-1-(3-brom-4-methyl-benzyl)-2-(1'-methyl-4,4'-bipiperidinyl-1-yl)-2-oxo-ethyl ester |
| (254) | 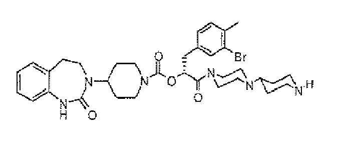 | 4-(2-Oxo-1,2,4,5-tetrahydro-1,3-benzdiazepin-3-yl)-piperidin-1-carbonsäure (R)-1-(3-brom-4-methyl-benzyl)-2-Oxo-2-(4-piperidin-4-yl-piperazin-1-yl)-ethyl ester |
| (255) | 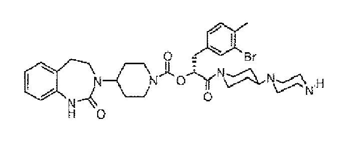 | 4-(2-Oxo-1,2,4,5-tetrahydro-1,3-benzdiazepin-3-yl)-piperidin-1-carbonsäure (R)-1-(3-brom-4-methyl-benzyl)-2-oxo-2-(4-piperazin-1-yl-piperidin-1-yl)-ethyl ester |
| (256) | 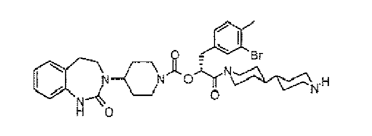 | 4-(2-Oxo-1,2,4,5-tetrahydro-1,3-benzdiazepin-3-yl)-piperidin-1-carbonsäure (R)-2-4,4'-bipiperidinyl-1-yl-1-(3-brom-4-methyl-benzyl)-2-oxo-ethyl ester |
| (257) | 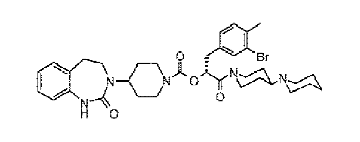 | 4-(2-Oxo-1,2,4,5-tetrahydro-1,3-benzdiazepin-3-yl)-piperidin-1-carbonsäure (R)-2-1,4'-bipiperidinyl-1'-yl-1-(3-brom-4-methyl-benzyl)-2-oxo-ethyl ester |
| (258) | 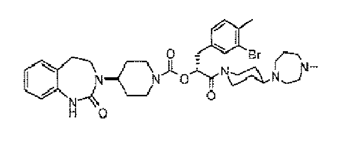 | 4-(2-Oxo-1,2,4,5-tetrahydro-1,3-benzdiazepin-3-yl)-piperidin-1-carbonsäure (R)-1-(3-brom-4-methyl-benzyl)-2-(4-(4-methyl-perhydro-1,4-diazepin-1-yl)-piperidin-1-yl)-2-oxo-ethyl ester |
| (259) | 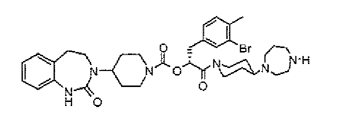 | 4-(2-Oxo-1,2,4,5-tetrahydro-1,3-benzdiazepin-3-yl)-piperidin-1-carbonsäure (R)-1-(3-brom-4-methyl-benzyl)-2-oxo-2-(4-perhydro-1,4-diazepin-1-yl-piperidin-1-yl)-ethyl ester |
| (260) | 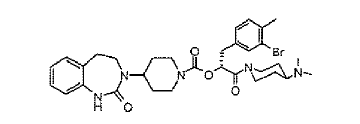 | 4-(2-Oxo-1,2,4,5-tetrahydro-1,3-benzdiazepin-3-yl)-piperidin-1-carbonsäure (R)-1-(3-brom-4-methyl-benzyl)-2-(4-dimethylamino-piperidin-1-yl)-2-oxo-ethyl ester |
| (261) | 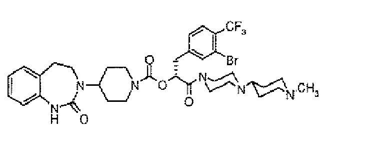 | 4-(2-Oxo-1,2,4,5-tetrahydro-1,3-benzdiazepin-3-yl)-piperidin-1-carbonsäure (R)-1-(3-brom-4-trifluormethyl-benzyl)-2-[4-(1-methyl-piperidin-4-yl)-piperazin-1-yl]-2-oxo-ethyl ester |
| (262) | 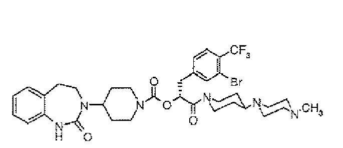 | 4-(2-Oxo-1,2,4,5-tetrahydro-1,3-benzdiazepin- 3-yl)-piperidin-1-carbonsäure (R)-1-(3-brom-4-trifluormethyl-benzyl)-2-[4-(4-methyl-piperazin-1-yl)-piperidin-1-yl]-2-oxo-ethyl ester |
| (263) | 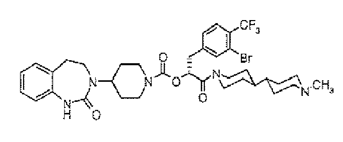 | 4-(2-Oxo-1,2,4,5-tetrahydro-1,3-benzdiazepin-3-yl)-piperidin-1-carbonsäure (R)-1-(3-brom-4-trifluormethyl-benzyl)-2-(1'-methyl-4,4'-bipiperidinyl-1-yl)-2-oxo-ethyl ester |
| (264) | 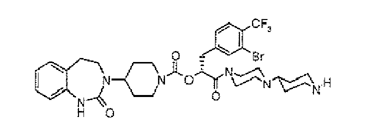 | 4-(2-Oxo-1,2,4,5-tetrahydro-1,3-benzdiazepln-3-yl)-piperidin-1-carbonsäure (R)-1-(3-brom-4-trifluormethyl-benzyl)-2-oxo-2-(4-piperidin-4-yl-piperazin-1-yl)-ethyl ester |
| (265) | 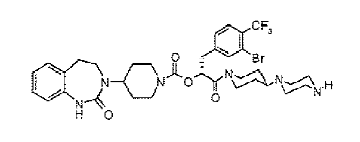 | 4-(2-Oxo-1,2,4,5-tetrahydre-1,3-benzdiazepin-3-yl)-piperidin-1-carbonsäure (R)-1-(3-brom-4-trifluormethyl-benzyl)-2-oxo-2-(4-piperazin-1-yl-piperidin-1-yl)-ethyl ester |
| (266) | 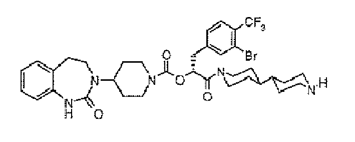 | 4-(2-Oxo-1,2,4,5-tetrahydro-1,3-benzdiazepin-3-yl)-piperidin-1-carbonsäure (R)-2-4,4'-bipiperidinyl-1-yl-1-(3-brom-4-trifluormethyl-benzyl)-2-oxo-ethyl ester |
| (267) | 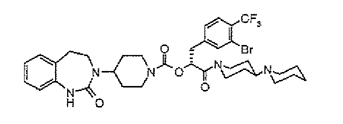 | 4-(2-Oxo-1,2,4,5-tetrahydro-1,3-benzdiazepin-3-yl)-piperidin-1-carbonsäure (R)-2-1,4'-bipiperidinyl-1'-yl-1-(3-brom-4-trifluormethyl-benzyl)-2-oxo-ethyl ester |
| (268) | 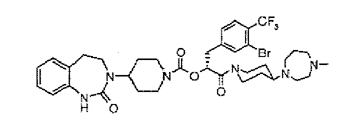 | 4-(2-Oxo-1,2,4,5-tetrahydro-1,3-benzdiazepin-3-yl)-piperidin-1-carbonsäure (R)-1-(3-brom-4-trifluormethyl-benzyl)-2-[4-(4-methyl-perhydro-1,4-diazepin-1-yl)-piperidin-1-yl]-2-oxo-ethyl ester |
| (269) | 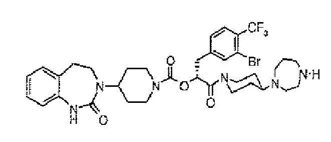 | 4-(2-Oxo-1,2,4,5-tetrahydro-1,3-benzdiazepin-3-yl)-piperidin-1-carbonsäure (R)-1-(3-brom-4-trifluormethyl-henzyl)-2-oxo-2-(4-perhydro-1,4-diazepin-1-yl-piperidin-1-yl)-ethyl ester |
| (270) | 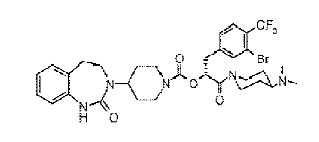 | 4-(2-Oxo-1,2,4,5-tetrahydro-1,3-benzdiazepin-3-yl)-piperidin-1-carbonsäure (R)-1-(3-brom-4-trifluormethyl-benzyl)-2-(4-dimethylamino-piperidin-1-yl)-2-oxo-ethyl ester |
| (271) | 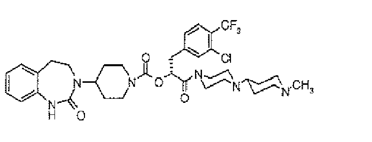 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-[4-(1-methyl-piperidin-4-yl)-piperazin-1-yl]-2-oxo-ethyl ester |
| (272) | 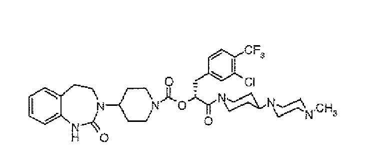 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-[4-(4-methyl-piperazin-1-yl)-piperidin-1-yl]-2-oxo-ethyl ester |
| (273) | 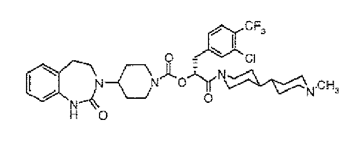 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-(1'-methyl-4,4'-bipiperidinyl-1-yl)-2-oxo-ethyl ester |
| (274) | 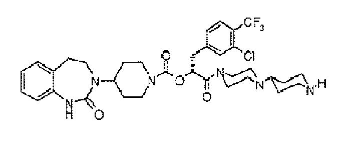 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-oxo-2-(4-piperidin-4-yl-piperazin-1-yl)-ethyl ester |
| (275) | 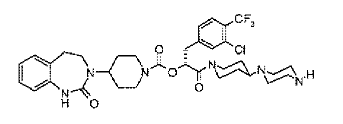 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-oxo-2-(4-piperazin-1-yl-piperidin-1-yl)-ethyl ester |
| (276) | 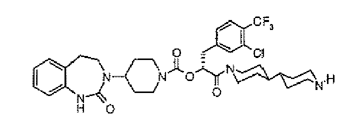 | 4-(2-Oxo-1,2,4,5-tetrahydro-1,3-benzdiazepin-3-yl)-piperidin-1-carbonsäure (R)-2-4,4'-bipiperidinyl-1-yl-1-(3-chlor-4-trifluormethyl-benzyl)-2-oxo-ethyl ester |
| (277) | 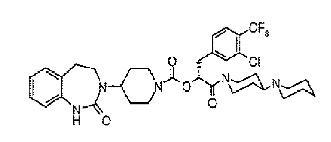 | 4-(2-Oxo-1,2,4,5-tetrahydro-1,3-benzdiazepin-3-yl)-piperidin-1-carbonsäure (R)-2-1,4'-bipiperidinyl-1'-yl-1-(3-chlor-4-trifluormethyl-benzyl)-2-oxo-ethyl ester |
| (278) | 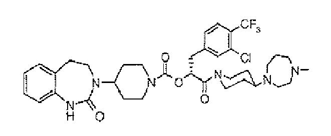 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-[4-(4-methyl-perhydro-1,4-diazepin-1-yl)-piporidin-1-yl]-2-oxo-ethyl ester |
| (279) | 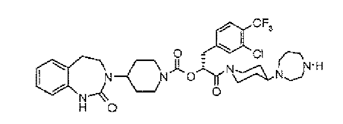 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-oxo-2-(4-perhydro-1,4-diazepin-1-yl-piperidin-1-yl)-ethyl ester |
| (280) | 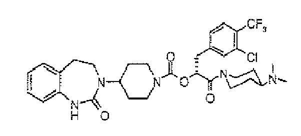 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-(4-dimethylamino-piperidin-1-yl)-2-oxo-ethyl ester |
| (281) | 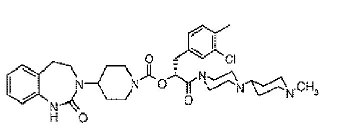 | 4-(2-Oxo-1,2,4,5-tetrahydro-1,3-benzdiazepin-3-yl)-piperidin-1-carbonsäure (R)-1-(3-chlor-4-methyl-benzyl)-2-[4-(1-methyl-piperidin-4-yl)-piperazin-1-yl]-2-oxo-ethyl ester |
| (282) | 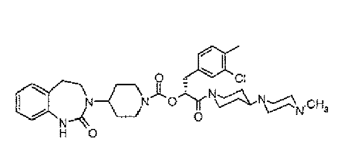 | 4-(2-Oxo-1,2,4,5-tetrahydro-1,3-benzdiazepin-3-yl)-piperidin-1-carbonsäure (R)-1-(3-chlor-4-methyl-benzyl)-2-[4-(4-methyl-piperazin-1-yl)-piperidin-1-yl]-2-oxo-ethyl ester |
| (283) | 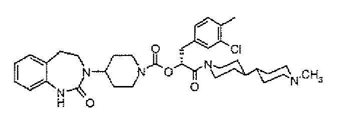 | 4-(2-Oxo-1,2,4,5-tetrahydro-1,3-benzdiazepin-3-yl)-piperidin-1-carbonsäure (R)-1-(3-chlor-4-methyl-benzyl)-2-(1'-methyl-4,4'-bipiperidinyl-1-yl)-2-oxo-ethyl ester |
| (284) | 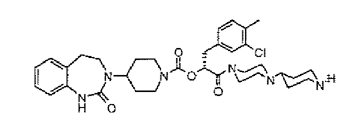 | 4-(2-Oxo-1,2,4,5-tetrahydro-1,3-benzdiazepin-3-yl)-piperidin-1-carbonsäure (R)-1-(3-chlor-4-methyl-benzyl)-2-oxo-2-(4-piperidin-4-yl-piperazin-1-yl)-ethyl ester |
| (285) | 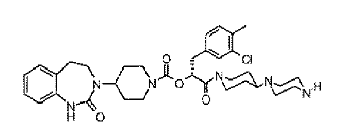 | 4-(2-Oxo-1,2,4,5-tetrahydro-1,3-benzdiazepin-3-yl)-piperidin-1-carbonsäure (R)-1-(3-chlor-4-methyl-benzyl)-2-oxo-2-(4-piperazin-1-yl-piperidin-1-yl)-ethyl ester |
| (286) | 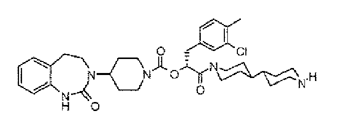 | 4-(2-Oxo-1,2,4,5-tetrahydro-1,3-benzdiazepin-3-yl)-piperidin-1-carbonsäure (R)-2-4,4'-bipiperidinyl-1-yl-1-(3-chlor-4-methyl-benzyl)-2-oxo-ethyl ester |
| (287) | 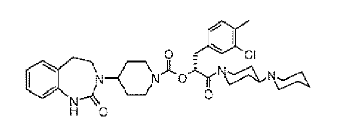 | 4-(2-Oxo-1,2,4,5-tetrahydro-1,3-benzdiazepin-3-yl)-piperidin-1-carbonsäure (R)-2-1,4'-bipiperidinyl-1'-yl-1-(3-chlor-4-methyl-benzyl)-2-oxo-ethyl ester |
| (288) | 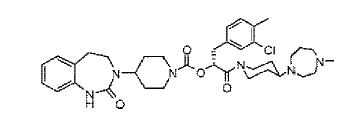 | 4-(2-Oxo-1,2,4,5-tetrahydro-1,3-benzdiazepin-3-yl)-piperidin-1-carbonsäure (R)-1-(3-chlor-4-methyl-benzyl)-2-[4-(4-methyl-perhydro-1,4-diazepin-1-yl)-piperidin-1-yl]-2-oxo-ethyl ester |
| (289) | 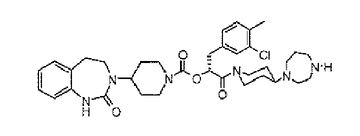 | 4-(2-Oxo-1,2,4,5-tetrahydro-1,3-benzdiazepin-3-yl)-piperidin-1-carbonsäure (R)-1-(3-chlor-4-methyl-benzyl)-2-oxo-2-(4-perhydro-1,4-diazepin-1-yl-piperidin-1-yl)-ethyl ester |
| (290) | 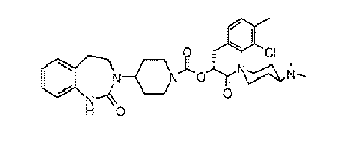 | 4-(2-Oxo-1,2,4,5-tetrahydro-1,3-benzdiazepin-3-yl)-piperidin-1-carbonsäure (R)-1-(3-chlor-4-methyl-benzyl)-2-(4-dimethylamino-piperidin-1-yl)-2-oxo-ethyl ester |
| (291) | 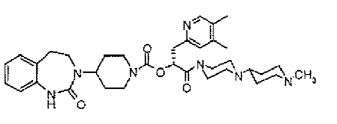 | 4-(2-Oxo-1,2,4,5-tetrahydro-1,3-benzdiazepin-3-yl)-piperidin-1-carbonsäure (R)-1-(4,5-dimethyl-pyridin-2-ylmethyl)-2-[4-(1-methyl-piperidin-4-yl)-piperazin-1-yl]-2-oxo-ethyl ester |
| (292) | 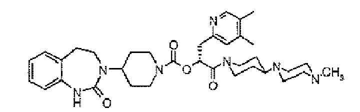 | 4-(2-Oxo-1,2,4,5-tetrahydro-1,3-benzdiazepin-3-yl)-piperidin-1-carbonsäure (R)-1-(4,5-dimethyl-pyridin-2-ylmethyl)-2-[4-(4-methyl-piperazin-1-yl)-piperidin-1-yl]-2-oxo-ethyl ester |
| (293) | 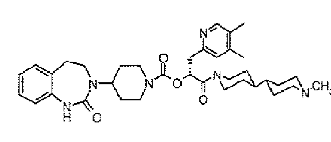 | 4-(2-Oxa-1,2,4,5-tetrahydro-1,3-benzdiazepin-3-yl)-piperidin-1-carbonsäure (R)-1-(4,5-dimethyl-pyridin-2-ylmethyl)-2-(1'-methyl-4,4'-bipiperidinyl-1-yl)-2-oxo-ethyl ester |
| (294) | 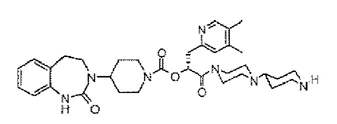 | 4-(2-Oxo-1,2,4,5-tetrahydro-1,3-benzdiazepin-3-yl)-piperidin-1-carbonsäure (R)-1-(4,5-dimethyl-pyridin-2-ylmethyl)-2-oxo-2-(4-piperidin-4-yl-piperazin-1-yl)-ethyl ester |
| (295) | 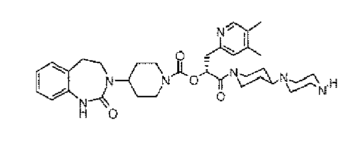 | 4-(2-Oxo-1,2,4,5-tetrahydro-1,3-benzdiazepin-3-yl)-piperidin-1-carbonsäure (R)-1-(4,5-dimethyl-pyridin-2-ylmethyl)-2-oxo-2-(4-piperazin-1-yl-piperidin-1-yl)-ethyl ester |
| (296) | 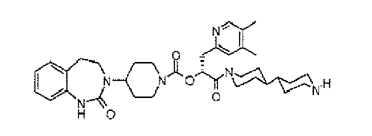 | 4-(2-Oxo-1,2,4,5-tetrahydro-1,3-benzdiazepin-3-yl)-piperidin-1-carbonsäure (R)-2-4,4'-bipiperidinyl-1-yl-1-(4,5-dimethyl-pyridin-2-ylmethyl)-2-oxo-ethyl ester |
| (297) | 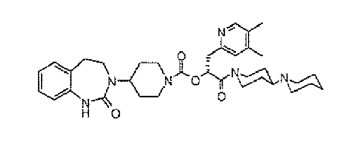 | 4-(2-Oxo-1,2,4,5-tetrahydro-1,3-benzdiazepin-3-yl)-piperidin-1-carbonsäure (R)-2-1,4'-bipiperidinyl-1'-yl-1-(4,5-dimethyl-pyridin-2-ylmethyl)-2-oxo-ethyl ester |
| (298) | 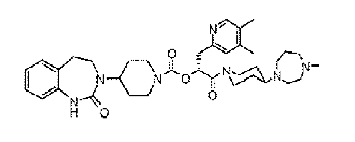 | 4-(2-Oxo-1,2,4,5-tetrahydro-1,3-benzdiazepin-3-yl)-piperidin-1-carbonsäure (R)-1-(4,5-dimethyl-pyridin-2-ylmethyl)-2-[4-(4-methyl-perhydro-1,4-diazepin-1-yl)-piperidin-1-yl]-2-oxo-ethyl ester |
| (299) | 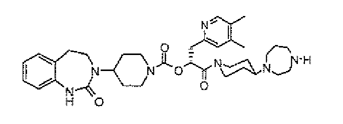 | 4-(2-Oxo-1,2,4,5-tetrahydro-1,3-benzdiazepin-3-yl)-piperidin-1-carbonsäure (R)-1-(4,5-dimethyl-pyridin-2-ylmethyl)-2-oxo-2-(4-perhydro-1,4-diazepin-1-yl-piperidin-1-yl)-ethyl ester |
| (300) | 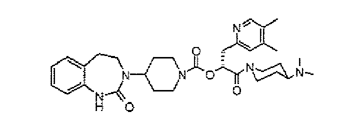 | 4-(2-Oxo-1,2,4,5-tetrahydro-1,3-benzdiazepin-3-yl)-piperidin-1-carbonsäure (R)-2-(4-dimethylamino-piperidin-1-yl)-1-(4,5-dimethyl-pyridin-2-ylmethyl)-2-oxo-ethyl ester |
| (301) | 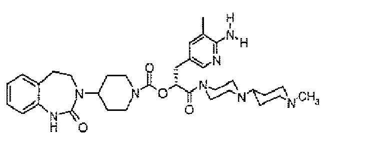 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-[4-(1-methyl-piperidin-4-yl)-piperazin-1-yl]-2-oxo-ethyl ester |
| (302) | 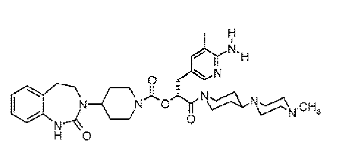 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-[4-(4-methyl-piperazin-1-yl)-piperidin-1-yl]-2-oxo-ethyl ester |
| (303) | 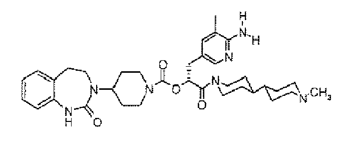 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-(1'-methyl-4,4'-bipiperidinyl-1-yl)-2-oxo-ethyl ester |
| (304) | 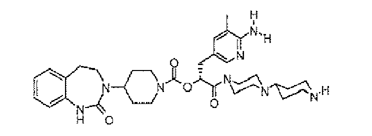 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-oxo-2-(4-piperidin-4-yl-piperazin-1-yl)-ethyl ester |
| (305) | 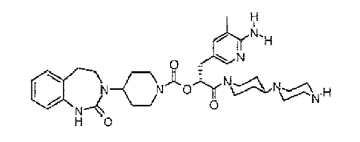 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-oxo-2-(4-piperazin-1-yl-piperidin-1-yl)-ethyl ester |
| (306) | 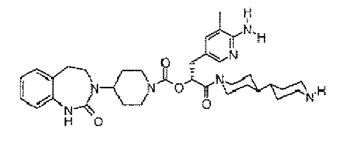 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-4,4'-bipiperidinyl-1-y[-2-oxo-ethyl ester |
| (307) | 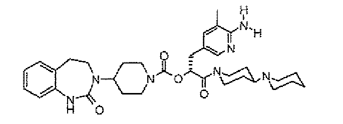 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-1,4'-bipiperidinyl-1'-yl-2-oxo-ethyl ester |
| (308) | 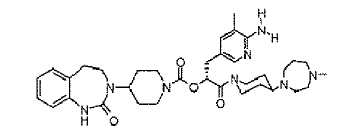 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-[4-(4-methyl-perhydro-1,4-diazepin-1-yl)-piperidin-1-yl]-2-oxo-ethyl ester |
| (309) | 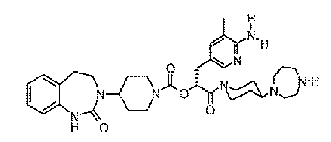 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-oxo-2-(4-perhydro-1,4-diazepin-1-yl-piperidin-1-yl)-ethyl ester |
| (310) | 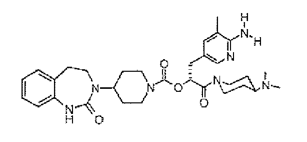 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-(4-dimethylamino-piperidin-1-yl)-2-oxo-ethyl ester |
| (311) | 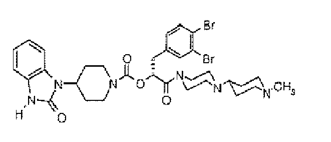 | 4-(2-Oxo-2,3-dihydro-benzimidazol-1-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (312) | 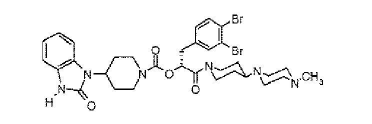 | 4-(2-Oxo-2,3-dihydro-benzimidazol-1-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (313) | 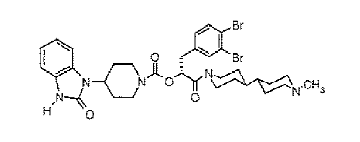 | 4-(2-Oxo-2,3-dihydro-benzimidazol-1-yl)-piperidin-1-carbonsäure (R)-1-(3,4-dibrom-benzyl)-2-(1'-methyl-4,4'-bipiperidinyl-1-yl)-2-oxo-ethyl ester |
| (314) | 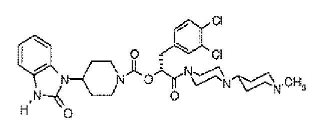 | 4-(2-Oxo-2,3-dihydro-benzimidazol-1-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (315) | 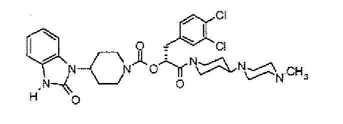 | 4-(2-Oxo-2,3-dihydro-benzimidazol-1-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (316) | 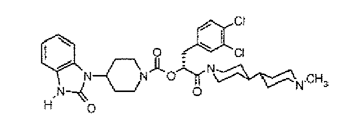 | 4-(2-Oxo-2,3-dihydro-benzimidazol-1-yl)-piperidin-1-carbonsäure (R)-1-(3,4-dichlor-benzyl)-2-(1'-methyl-4,4'-bipiperidinyl-1-yl)-2-oxo-ethyl ester |
| (317) | 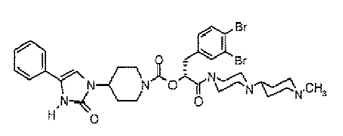 | 4-(2-Oxo-4-phenyl-2,3-dihydro-imidazol-1-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (318) | 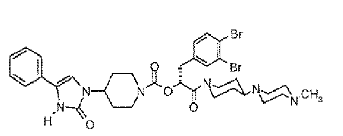 | 4-(2-Oxo-4-phenyl-2,3-dihydro-imidazol-1-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (319) | 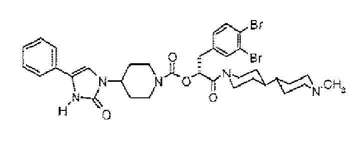 | 4-(2-Oxo-4-phenyl-2,3-dihydro-imidazol-1-yl)-piperidin-1-carbonsäure (R)-1-(3,4-dibrom-benzyl)-2-(1'-methyl-[4,4']bipiperidinyl-1-yl)-2-oxo-ethyl ester |
| (320) | 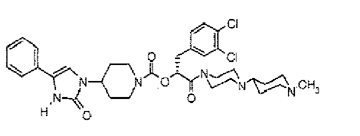 | 4-(2-Oxo-4-phenyl-2,3-dihydro-imidazol-1-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (321) | 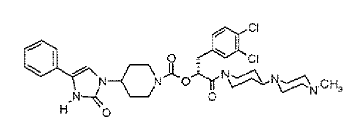 | 4-(2-Oxo-4-phenyl-2,3-dihydro-imidazol-1-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (322) | 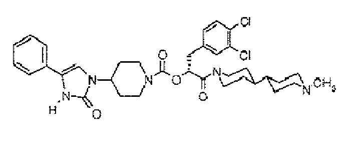 | 4-(2-Oxo-4-phenyl-2,3-dihydro-imidazol-1-yl)-piperidin-1-carbonsäure (R)-1-(3,4-dichlor-benzyl)-2-(1'-methyl-[4,4']bipiperidinyl-1-yl)-2-oxo-ethyl ester |
| (323) | 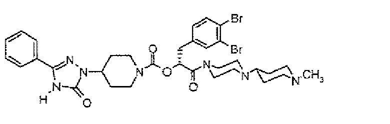 | 4-(5-Oxo-3-phonyl-4,5-dihydro-1,2,4-triazol-1-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (324) | 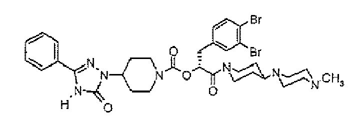 | 4-(5-Oxo-3-phenyl-4,5-dihydro-1,2,4-triazol-1-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (325) | 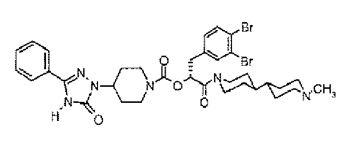 | 4-(5-Oxo-3-phenyl-4,5-dihydro-1,2,4-triazol-1-yl)-piperidin-1-carbonsäure (R)-1-(3,4-dibrom-benzyl)-2-(1'-methyl-4,4'-bipiperidinyl-1-yl)-2-oxo-ethyl ester |
| (326) | 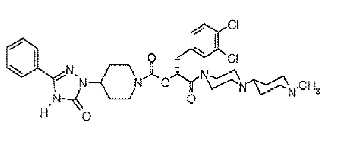 | 4-(5-Oxo-3-phenyl-4,5-dihydro-1,2,4-triazol-1- yl)-piperidin-1-carbonsäure (R)-1-(3,4-dichlor-benzyl)-2-[4-(1-methyl-piperidin-4-yl)-piperazin-1-yl]-2-oxo-ethyl ester |
| (327) | 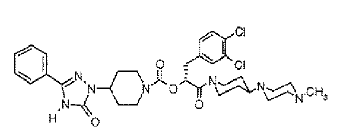 | 4-(5-Oxo-3-phenyl-4,5-dihydro-1,2,4-triazol-1-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (328) | 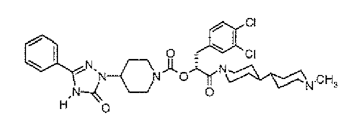 | 4-(5-Oxo-3-phenyl-4,5-dihydro-1,2,4-triazol-1-yl)-piperidin-1-carbonsäure (R)-1-(3,4-dichlor-benzyl)-2-(1'-methy-4,4'-bipiperidinyl-1-yl)-2-oxo-ethyl ester |
| (329) | 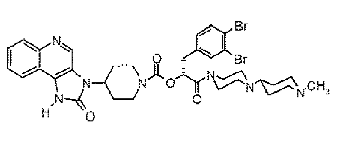 | 4-(2-Oxo-1,2-dihydro-imidazo[4,5-*c*]chinolin-3-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (330) | 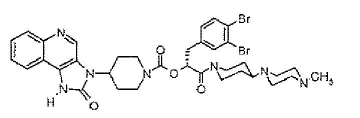 | 4-(2-Oxo-1,2-dihydro-imidazo[4,5-*c*]chinolin-3-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (331) | 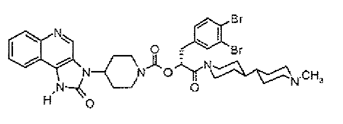 | 4-(2-Oxo-1,2-dihydro-imidazo[4,5-*c*]chinolin-3-yl)-piperidin-1-carbonsäure (R)-1-(3,4-dibrom-benzyl)-2-(1'-methyl-[4,4']bipiperidinyl-1-yl)-2-oxo-ethyl ester |
| (332) | 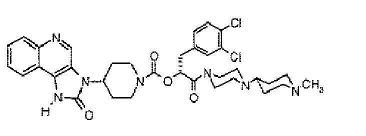 | 4-(2-Oxo-1,2-dihydro-imidazo[4,5-*c*]chinolin-3-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (333) | 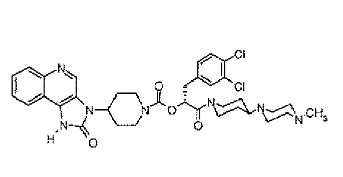 | 4-(2-Oxo-1,2-dihydro-imidazo[4,5-*c*]chinolin-3-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (334) | 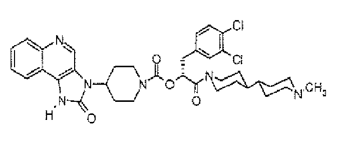 | 4-(2-Oxo-1,2-dihydro-imidazo[4,5-*c*]chinolin-3-yl)-piperidin-1-carbonsäure (R)-1-(3,4-dichlor-benzyl)-2-(1'-methyl-[4,4']bipiperidinyl-1-yl)-2-oxo-ethyl ester |
| (335) | 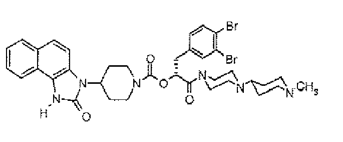 | 4-(2-Oxo-1,2-dihydro-naphth[1,2-*d*]imidazol-3-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (336) | 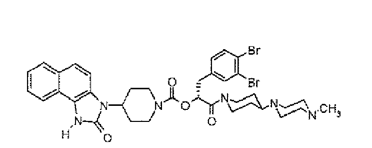 | 4-(2-Oxo-1,2-dihydro-naphth[1,2-*d*]imidazol-3-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (337) | 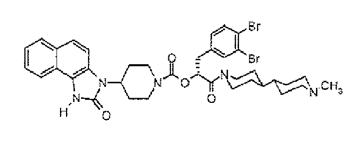 | 4-(2-Oxo-1,2-dihydro-naphth[1,2-*d*]imidazol-3-yl)-piperidin-1-carbonsäure (R)-1-(3,4-dibrom-benzyl)-2-(1'-methyl-4,4'-bipiperidinyl-1-yl)-2-oxo-ethyl ester |
| (338) | 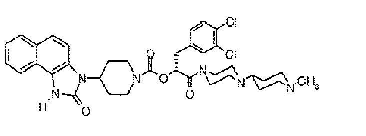 | 4-(2-Oxo-1,2-dihydro-naphth[1,2-*d*]imidazol-3-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (339) | 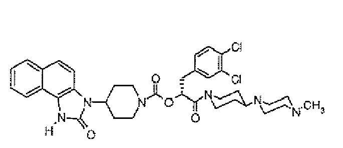 | 4-(2-Oxo-1,2-dihydro-naphth[1,2-*d*]imidazol-3-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (340) | 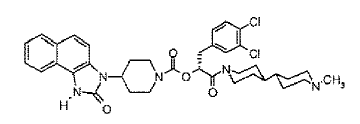 | 4-(2-Oxo-1,2-dihydro-naphth[1,2-*d*]umidazol-3-yl)-piperidin-1-carbonsäure (R)-1-(3,4-dichlor-benzyl)-2-(1'-methyl-4,4'-bipiperidinyl-1-yl)-2-oxo-ethyl ester |
| (341) | 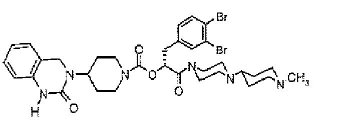 | 4-(2-Oxo-1,4-dihydro-2*H*-chinazolin-3-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (342) | 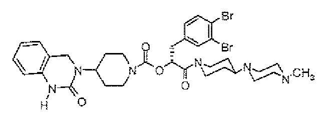 | 4-(2-Oxo-1,4-dihydro-2*H*-chinazolin-3-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (343) | 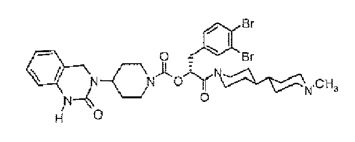 | 4-(2-Oxo-1,4-dihydro-2*H*-chinazolin-3-yl)-piperidin-1-carbonsäure (R)-1-(3,4-dibrom-benzyl)-2-(1'-mothyl-[4,4']bipiperidinyl-1-yl)-2-oxo-ethyl ester |
| (344) | 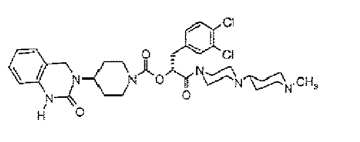 | 4-(2-Oxo-1,4-dihydro-2*H*-chinazolin-3-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (345) | 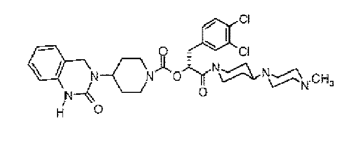 | 4-(2-Oxo-1,4-dihydro-2*H*-chinazolin-3-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (346) | 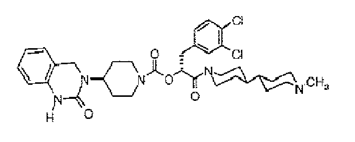 | 4-(2-Oxo-1,4-dihydro-2*H*-chinazolin-3-yl)-piperidin-1-carbonsäure (R)-1-(3,4-dichlor-benzyl)-2-(1'-methyl-[4,4']bipiperidinyl-1-yl)-2-oxo-ethyl ester |
| (347) | 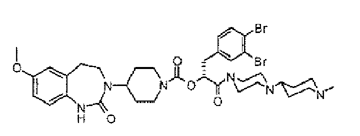 | 4-(7-Methoxy-2-oxo-1,2,4,5-tetrahydro-1,3-benzdiazepin-3-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (348) | 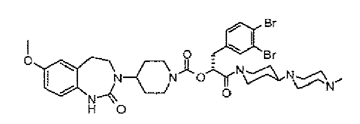 | 4-(7-Methoxy-2-oxo-1,2,4,5-tetrahydro-1,3-benzdiazepin-3-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (349) | 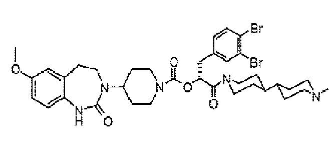 | 4-(7-Methoxy-2-oxo-1,2,4,5-tetrahydro-1,3-benzdiazepin-3-yl)-piperidin-1-carbonsäure (R)-1-(3,4-dibrom-benzyl)-2-(1'-methyl-4,4'-bipiperidinyl-1-yl)-2-oxo-ethyl ester |
| (350) | 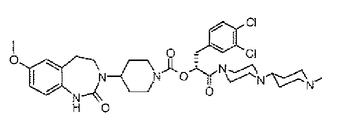 | 4-(7-Methoxy-2-oxo-1,2,4,5-tetrahydro-1,3-benzdiazepin-3-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (351) | 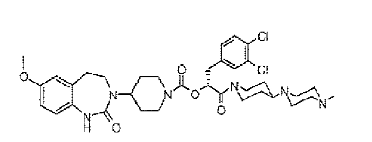 | 4-(7-Methoxy-2-oxo-1,2,4,5-tetrahydro-1,3-benzdiazepin-3-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (352) | 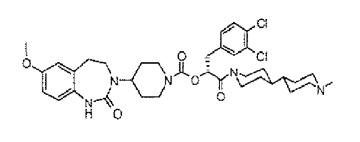 | 4-(7-Methoxy-2-oxo-1,2,4,5-tetrahydro-1,3-benzdiazepin-3-yl)-piperidin-1-carbonsäure (R)-1-(3,4-dichlor-benzyl)-2-(1'-methyl-4,4'-bipiperidinyl-1-yl)-2-oxo-ethyl ester |
| (353) | 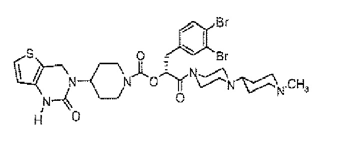 | 4-(2-Oxo-1,4-dihydro-2H-thieno[3,2-*c*]pyrimidin-3-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (354) | 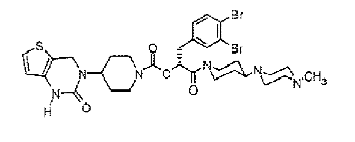 | 4-(2-Oxo-1,4-dihydro-2*H*-thieno[3,2-*d*]pyrimidin-3-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (355) | 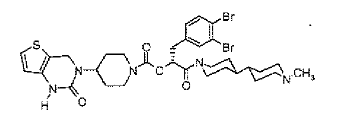 | 4-(2-Oxo-1,4-dihydro-2*H*-thieno[3,2-*d*]pyrimidin-3-yl)-piperidin-1-carbonsäure (R)-1-(3,4-dibrom-benzyl)-2-(1'-methyl-[4,4']bipiperidinyl-1-yl)-2-oxo-ethyl ester |
| (356) | 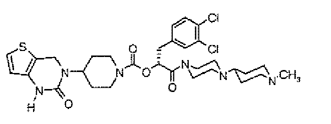 | 4-(2-Oxo-1,4-dihydro-2*H*-thieno[3,2-*d*]pyrimidin-3-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (357) | 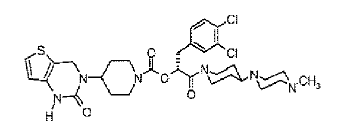 | 4-(2-Oxo-1,4-dihydro-2*H*-thieno[3,2-*d*]pyrimidin-3-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (358) | 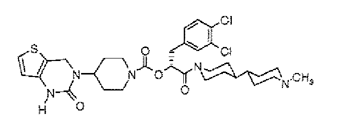 | 4-(2-Oxo-1,4-dihydro-2*H*-thieno[3,2-*d*]pyrimidin-3-yl)-piperidin-1-carbonsäure (R)-1-(3,4-dichlor-benzyl)-2-(1'-methyl-[4,4']bipiperidinyl-1-yl)-2-oxo-ethyl ester |
| (359) | 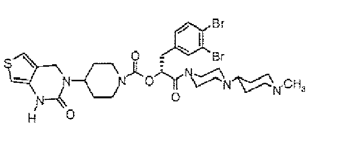 | 4-(2-Oxo-1,2-dihydro-4*H*-thieno[3,4-*d*]pyrimidin-3-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (360) | 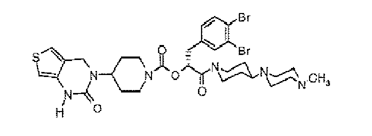 | 4-(2-Oxo-1,2-dihydro-4*H*-thieno[3,4-*d*]pyrimidin-3-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (361) | 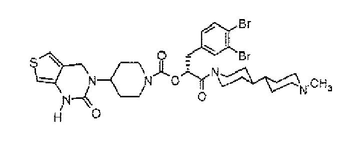 | 4-(2-Oxo-1,2-dihydro-4*H*-thieno[3,4-*d*]pyrimidin-3-yl)-piperidin-1-carbonsäure (R)-1-(3,4-dibrom-benzyl)-2-(1'-methyl-[4,4']bipiperidinyl-1-yl)-2-oxo-ethyl ester |
| (362) | 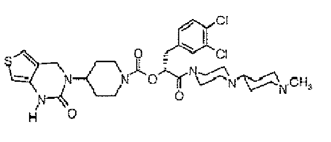 | 4-(2-Oxo-1,2-dihydro-4*H*-thieno[3,4-*d*]pyrimidin-3-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (363) | 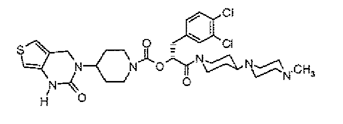 | 4-(2-Oxo-1,2-dihydro-4*H*-thieno[3,4-*d*]pyrimidin-3-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (364) | 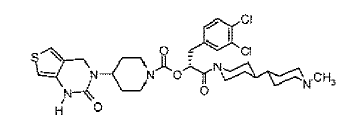 | 4-(2-Oxo-1,2-dihydro-4*H*-thieno[3,4-*d*]pyrimidin-3-yl)-piperidin-1-carbonsäure (R)-1-(3,4-dichlor-benzyl)-2-(1'-methyl-[4,4']bipiperidinyl-1-yl)-2-oxo-ethyl ester |
| (365) | 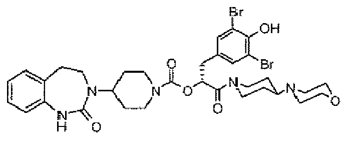 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(4-morpholin-4-yl-piperidin-1-yl)-2-oxo-ethyl ester |
| (366) | 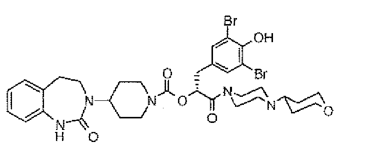 | 4-(2-Oxo-1,2,4,5-ietrahydro-1,3-benzdiazepin-3-yl)-piperidin-1-carbonsäure (R)-1-(3,5-dibrom-4-hydroxy-benzyl)-2-oxo-2-[4-(tetrahydro-pyran-4-yl)-piperazin-1-yl]-ethyl ester |
| (367) | 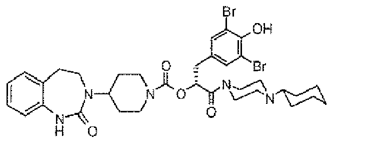 | 4-(2-Oxo-1,2,4,5-tetrahydro-1,3-benzdiazepin-3-yl)-piperidin-1-carbonsäure (R)-2-(4-cyclohexyl-piperazin-1-yl)-1-(3,5-dibrom-4-hydroxy-benzyl)-2-oxo-ethyl ester |
| (368) | 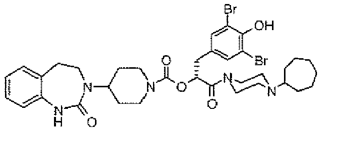 | 4-(2-Oxo-1,2,4,5-tetrahydro-1,3-benzdiazepin-3-yl)-piperidin-1-carbonsäure (R)-2-(4-cycloheptyl-piperazin-1-yl)-1-(3,5-dibrom-4-hydroxy-benzyl)-2-oxo-ethyl ester |
| (369) | 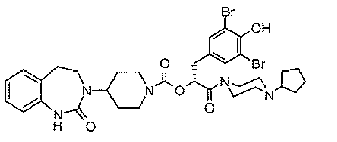 | 4-(2-Oxo-1,2,4,5-tetrahydro-1,3-benzdiazepin-3-yl)-piperidin-1-carbonsäure (R)-2-(4-cyclopentyl-piperazin-1-yl)-1-(3,5-dibrom-4-hydroxy-benzyl)-2-oxo-ethyl ester |
| (370) | 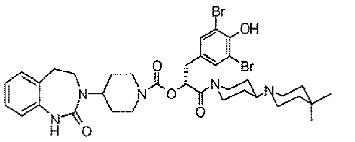 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(4,4-dimethyl-1,4'-bipiperidinyl-1'-yl)-2-oxo-ethyl ester |
| (371) | 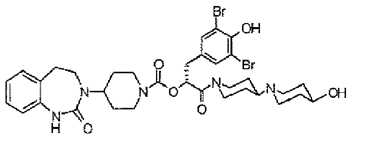 | 4-(2-Oxo-1,2,4,5-tetrahydro-1,3-benzdiazepin-3-yl)-piparidin-1-carbonsäure (R)-1-(3,5-dibrom-4-hydroxy-benzyl)-2-(4-hydroxy-1,4'-bipiperidinyl-1'-yl)-2-oxo-ethyl ester |
| (372) | 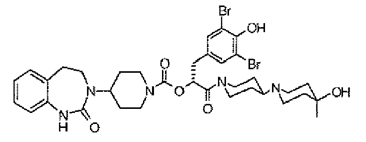 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(4-hydroxy-4-methyl-1,4'-bipiperidinyl-1'-yl)-2-oxo-ethyl ester |
| (373) | 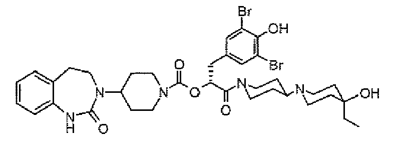 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(4-ethyl-4-hydroxy-1,4'-bipiperidinyl-1'-yl)-2-oxo-ethyl ester |
| (374) | 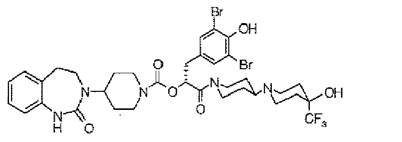 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(4-hydroxy-4-trifluormethyl-1,4'-bipiperidinyl-1'-yl)-2-oxo-ethyl ester |
| (375) | 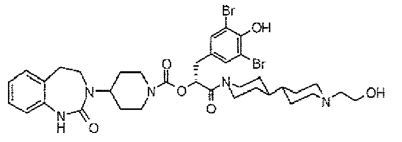 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[1'-(2-hydroxy-ethyl)-4,4'-bipiperidinyl-1-yl]-2-oxo-ethyl ester |
| (376) | 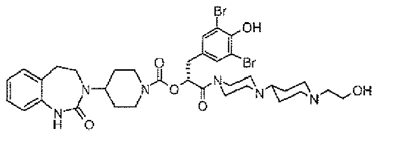 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-{4-[1-(2-hydroxy-ethyl)-piperidin-4-yl]-piperazin-1-yl}-2-oxo-ethyl ester |
| (377) | 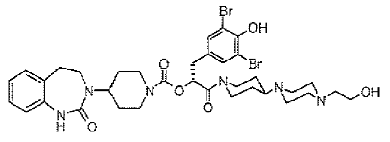 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-{4-[4-(2-hydroxy-ethyl)-piperazin-1-yl]-piperidin-1-yl}-2-oxo-ethyl ester |
| (378) | 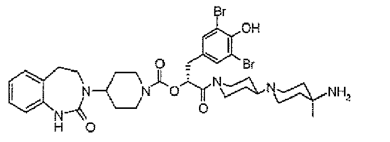 | 4-(2-Oxo-1,2,4,5-tetrahydro-1,3-benzdiazepin-3-yl)-piperidin-1-carbonsäure (R)-2-(4-amino-4-methyl-1,4'-bipiperidinyl-1'-yl)-1-(3,5-dibrom-4-hydroxy-benzyl)-2-oxo-ethyl ester |
| (379) | 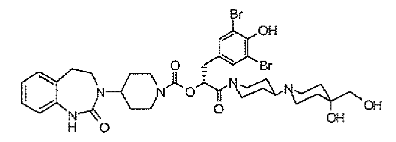 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(4-hydroxy-4-hydroxymethyl-1,4'-bipiperidinyl-1'-yl)-2-oxo-ethyl ester |
| (380) | 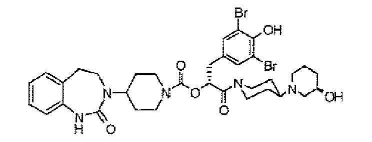 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-((R)-3-hydroxy-1,4'-bipiperidinyl-1'-yl)-2-oxo-ethyl ester |
| (381) | 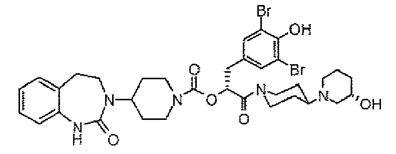 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-((S)-3-hydroxy-1,4'-bipiperidinyl-1'-yl)-2-oxo-ethyl ester |
| (382) | 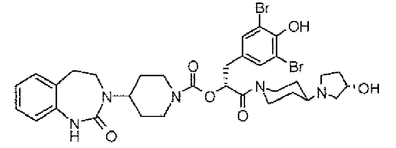 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-((S)-3-hydroxy-pyrrolidin-1-yl)-piperidin-1-yl]-2-oxo-ethyl ester |
| (383) | 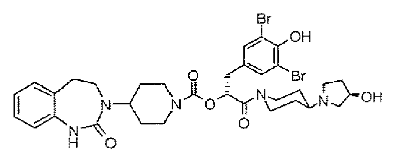 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-((R)-3-hydroxy-pyrrolidin-1-yl)-piperidin-1-yl]-2-oxo-ethyl ester |
| (384) | 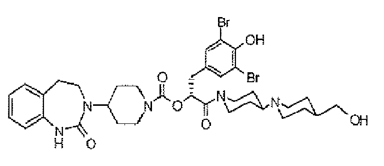 | 4-(2-Oxo-1,2,4,5-tetrahydro-1,3-benzdiazepin-3-yl)-piporidin-1-carbonsäure (R)-1-(3,5-dibrom-4-hydroxy-benzyl)-2-(4-hydroxymethyl-1,4'-bipiperidinyl-1'-yl)-2-oxo-ethyl ester |
| (385) | 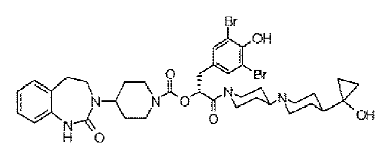 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(1-hydroxy-cyclopropyl)-1,4'-bipiperidinyl-l'-yl]-2-oxo-ethyl ester |
| (386) | 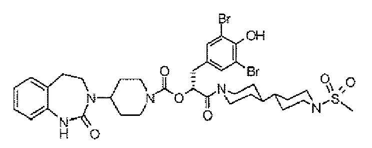 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(1'-methansulfonyl-4,4'-bipiperidinyl-1-yl)-2-oxo-ethyl ester |
| (387) | 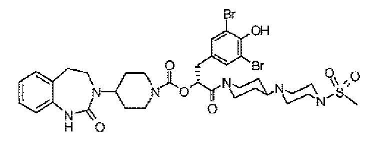 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(4-methansulfonyl-piperazin-1-yl)-piperidin-1-yl]-2-oxo-ethyl ester |
| (388) | 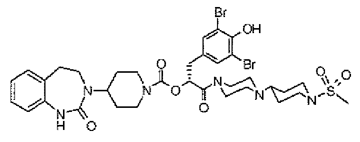 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(1-methansulfonyl-piperidin-4-yl)-piperazin-1-yl]-2-oxo-ethyl ester |
| (389) | 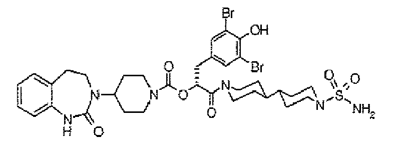 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-oxo-2-(1'-sulfamoyl-4,4'-bipiperidinyl-1-yl)-ethyl ester |
| (390) | 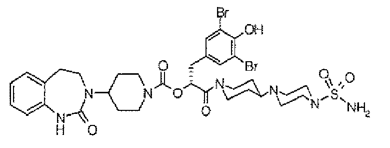 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-oxo-2-[4-(4-sulfamoyl-piperazin-1-yl)-piperidin-1-yl]-ethyl ester |
| (391) | 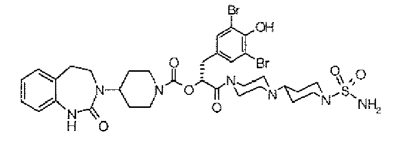 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-oxo-2-[4-(1-sulfamoyl-piperidin-4-yl)-piperazin-1-yl]-ethyl ester |
| (392) | 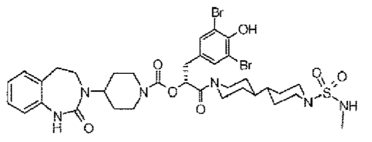 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(1'-methylsulfamoyl-4,4'-bipiperidinyl-1-yl)-2-oxo-ethyl ester |
| (393) | 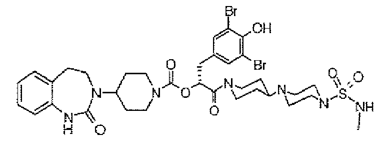 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(4-mothylsulfamoyl-piperazin-1-yl)-piperidin-1-yl]-2-oxo-ethyl ester |
| (394) | 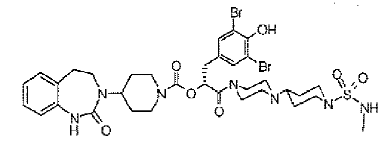 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(1-methylsulfamoyl-piperidin-4-yl)-piperazin-1-yl]-2-oxo-ethyl ester |
| (395) | 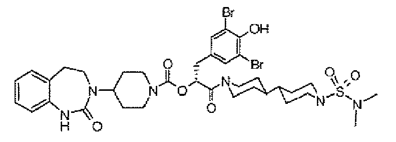 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(1'-dimethylsulfamoyl-4,4'-bipiperidinyl-1-yl)-2-oxo-ethyl ester |
| (396) | 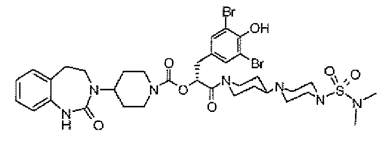 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(4-dimethylsulfamoyl-piperazin-1-yl)-piperidin-1-yl]-2-oxo-ethyl ester |
| (397) | 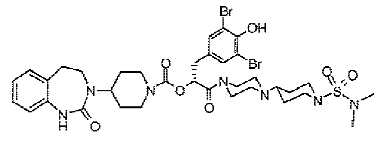 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(1-dimethylsulfamoyl-piperidin-4-yl)-piperazin-1-yl]-2-oxo-ethyl ester |
| (398) | 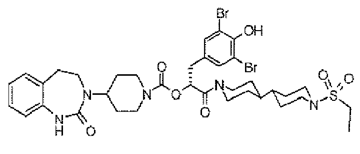 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(1'-ethansulfonyl-4,4'-bipiperidinyl-1-yl)-2-oxo-ethyl ester |
| (399) | 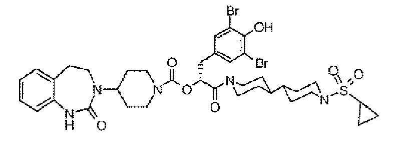 | 4-(2-Oxo-1,2,4,5-tetrahydro-1,3-benzdiazepin-3-yl)-piperidin-1-carbonsäure (R)-2-(1'-cyclopropansulfonyl-4,4'-bipiperidinyl-1-yl)-1-(3,5-dibrom-4-hydroxy-benzyl)-2-oxo-ethyl ester |
| (400) | 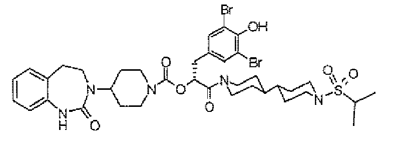 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-oxo-2-[1'-(propan-2-sulfonyl)-4,4'-bipiperidinyl-1-yl]-ethyl ester |
| (401) | 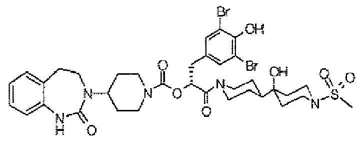 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(4'-hydroxy-1'-methansulfonyl-4,4'-bipiperidinyl-1-yl)-2-oxo-ethyl ester |
| (402) | 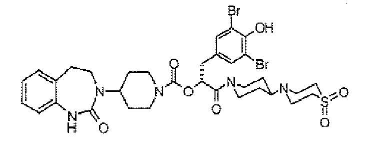 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(1,1-dioxo-1λ⁶-thiomorpholin-4-yl)-piperidin-1-yl]-2-oxo-ethyl ester |
| (403) | 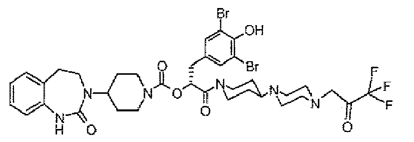 | 4-(2-Oxo-1,2,4,5-tetrahydro-1,3-benzdiazepin-3-yl)-piporidin-1-carbonsäure (R)-1-(3,5-dibrom-4-hydroxy-benzyl)-2-oxo-2-{4-[4-(3,3,3-trifluor-2-oxo-propyl)-piperazin-1-yl]-piperidin-1-yl}-ethyl ester |
| (404) | 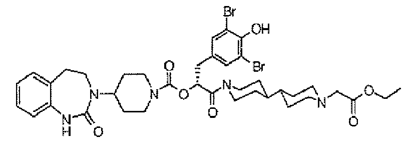 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(1'-ethoxycarbonylmethyl-4,4'-bipiperidinyl-1-yl)-2-oxo-ethyl ester |
| (405) | 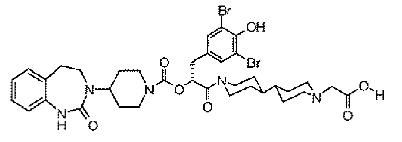 | 4-(2-Oxo-1,2,4,5-tetrahydro-1,3-benzdiazepin-3-yl)-piperidin-1-carbonsäure (R)-2-(1'-carboxymethyl-4,4'-bipiperidinyl-1-yl)-1-(3,5-dibrom-4-hydroxy-benzyl)-2-oxo-ethyl ester |
| (406) | 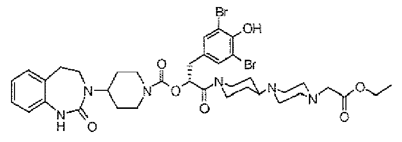 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(4-ethoxycarbonylmethyl-piperazin-1-yl)-piperidin-1-yl]-2-oxo-ethyl ester |
| (407) | 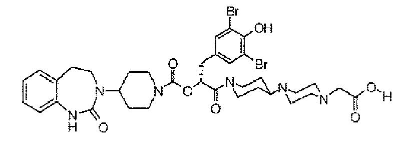 | 4-(2-Oxo-1,2,4,5-tetrahydro-1,3-benzdiazepin-3-yl)-piperidin-1-carbonsäure (R)-2-[4-(4-carboxymethyl-piperazin-1-yl)-piperidin-1-yl]-1-(3,5-dibrom-4-hydroxy-benzyl)-2-oxo-ethyl ester |
| (408) | 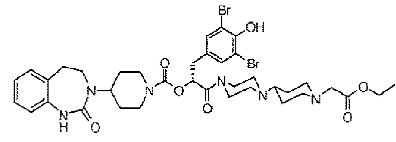 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(1-ethoxycarbonylmethyl-piperidin-4-yl)-piperazin-1-yl]-2-oxo-ethyl ester |
| (409) | 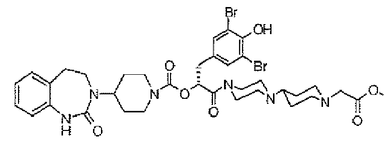 | 4-(2-Oxo-1,2,4,5-tetrahydro-1,3-benzdiazepin-3-yl)-piperidin-1-carbonsäure (R)-2-[4-('1-carboxymethyl-piparidin-4-yl)-piperazin-1-yl]-1-(3,5-dibrom-4-hydroxy-benzyl)-2-oxo-ethyl ester |
| (410) | 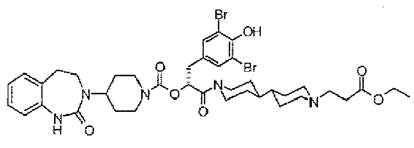 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[1'-(2-ethoxycarbonyl-ethyl)-4,4'-bipiperidinyl-1-yl]-2-oxo-ethyl ester |
| (411) | 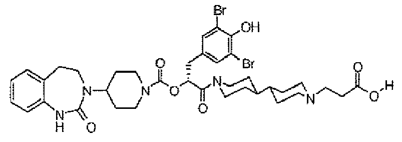 | 4-(2-Oxo-1,2,4,5-tetrahydro-1,3-benzdiazepin-3-yl)-piperidin-1-carbonsäure (R)-2-[1'-(2-carboxy-ethyl)-4,4'-bipiperidinyl-1-yl]-1-(3,5-dibrom-4-hydroxy-benzyl)-2-oxo-ethyl ester |
| (412) | 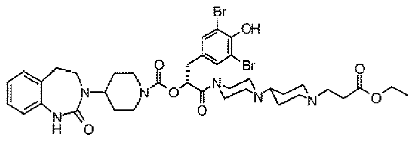 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-{4-[1-(2-ethoxycarbonyl-ethyl)-piperidin-4-yl]-piperazin-1-yl}-2-oxo-ethyl ester |
| (413) | 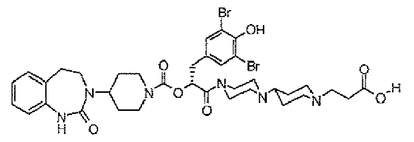 | 4-(2-Oxo-1,2,4,5-tetrahydro-1,3-benzdiazepin-3-yl)-piperidin-1-carbonsäure (R)-2-{4-[1-(2-carboxy-ethyl)-piperidin-4-yl]-piperazin-1-yl}-1-(3,5-dibrom-4-hydroxy-benzyl)-2-oxo-ethyl ester |
| (414) | 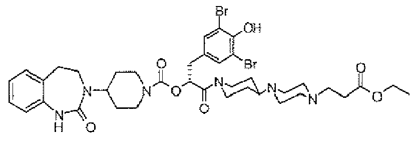 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-{4-[4-(2-ethoxycarbonyl-ethyl)-piperazin-1-yl]-piperidin-1-yl}-2-oxo-ethyl ester |
| (415) | 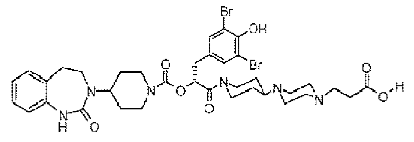 | 4-(2-Oxo-1,2,4,5-tetrahydro-1,3-benzdiazepin-3-yl)-piperidin-1-carbonsäure (R)-2-{4-[4-(2-carboxy-ethyl)-piperazin-1-yl]-piperidin-1-yl}-1-(3,5-dibrom-4-hydroxy-benzyl)-2-oxo-ethyl ester |
| (416) | 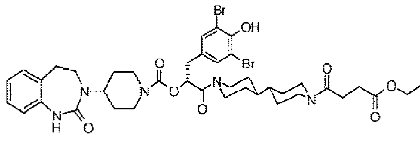 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[1'-(3-ethoxycarbonyl-propionyl)-4,4'-bipiperidinyl-1-yl]-2-oxo-ethyl ester |
| (417) | 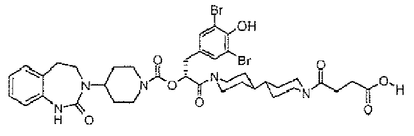 | 4-(2-Oxo-1,2,4,5-tetrahydro-1,3-benzdiazepin-3-yl)-piperidin-1-carbonsäure (R)-2-[1'-(3-carboxy-propionyl)-4,4'-bipiperidinyl-1-yl]-1-(3,5-dibrom-4-hydroxy-benzyl)-2-oxo-ethyl ester |
| (418) | 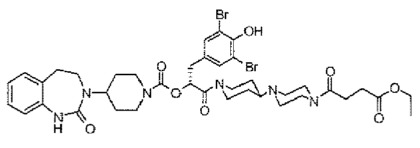 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-{4-[4-(3-ethoxycarbonyl-propionyl)-piperazin-1-yl]-piperidin-1-yl}-2-oxo-ethyl ester |
| (419) | 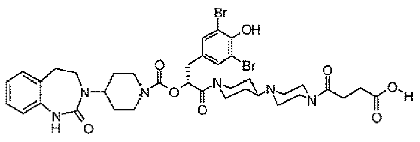 | 4-(2-Oxo-1,2,4,5-tetrahydro-1,3-benzdiazepin-3-yl)-piperidin-1-carbonsäure (R)-2-{4-[4-(3-carboxy-propionyl)-piperazin-1-yl]-piperidin-1-yl}-1-(3,5-dibrom-4-hydroxy-benzyl)-2-oxo-ethyl ester |
| (420) | 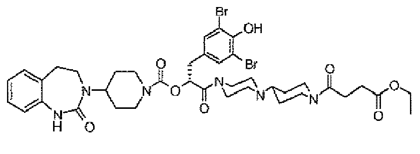 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-{4-[1-(3-ethoxycarbonyl-propionyl)-piperidin-4-yl]-piperazin-1-yl}-2-oxo-ethyl ester |
| (421) | 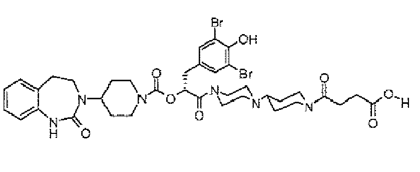 | 4-(2-Oxo-1,2,4,5-tetrahydro-1,3-benzdiazepin-3-yl)-piperidin-1-carbonsäure (R)-2-{4-[1-(3-carboxy-propionyl)-piperidin-4-yl]-piperazin-1-yl}-1-(3,5-dibrom-4-hydroxy-benzyl)-2-oxo-ethyl ester |
| (422) | 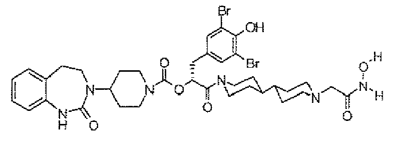 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(1'-hydroxycarbamoylmethyl-4,4'-bipiperidinyl-1-yl)-2-oxo-ethyl ester |
| (423) | 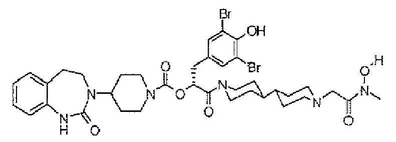 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-{1'-[(hydroxy-methyl-carbamoyl)-mothyl]-4,4'-bipiperidinyl-1-yl}-2-oxo-ethyl ester |
| (424) | 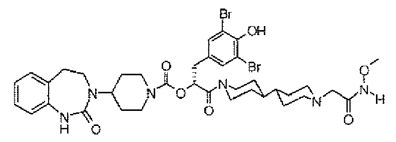 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[1'-(methoxycarbamoyl-methyl)-4,4'-bipiperidinyl-1-yl]-2-oxo-ethyl ester |
| (425) | 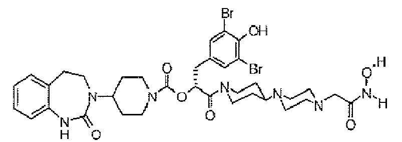 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(4-hydroxycarbamoylmethyl-piperazin-1-yl)-piperidin-1-yl]-2-oxo-ethyl ester |
| (426) | 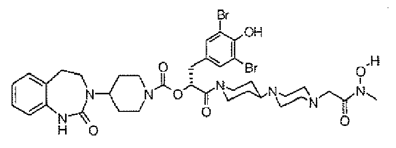 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(4-{4-[(hydroxy-methyl-carbamoyl)-methyl]-piperazin-1-yl}-piperidin-1-yl)-2-oxo-ethyl ester |
| (427) | 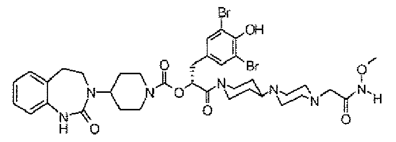 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-{4-[4-(methoxycarbamoyl-methyl)-piperazin-1-yl]-piperidin-1-yl}-2-oxo-ethyl ester |
| (428) | 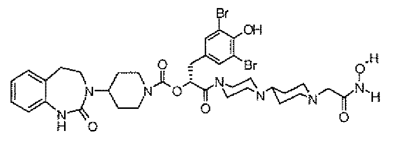 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(1-hydroxycarbamoylmethyl-piperidin-4-yl)-piperazin-1-yl]-2-oxo-ethyl ester |
| (429) | 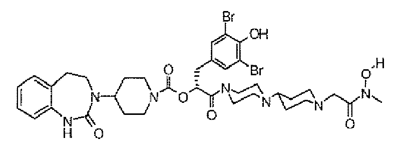 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(4-{1-[(hydroxy-methyl-carbamoyl)-methyl]-piperidin-4-yl}-piperazin-1-yl)-2-oxo-ethyl ester |
| (430) | 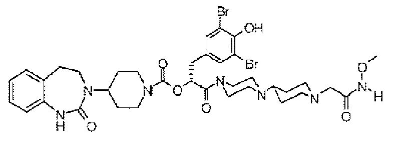 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-{4-[1-(methoxycarbamoyl-methyl)-piperidin-4-yl]-piperazin-1-yl}-2-oxo-ethyl ester |
| (431) | 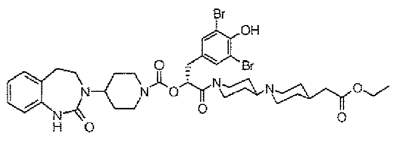 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(4-ethoxycarbonylmethyl-1,4'-bipiperidinyl-1'-yl)-2-oxo-ethyl ester |
| (432) | 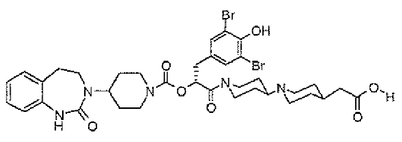 | 4-(2-Oxo-1,2,4,5-tetrahydro-1,3-benzdiazepin-3-yl)-piperidin-1-carbonsäure (R)-2-(4-carboxymethyl-1,4'-bipiperidinyl-1'-yl)-1-(3,5-dibrom-4-hydroxy-benzyl)-2-oxo-ethyl ester |
| (433) | 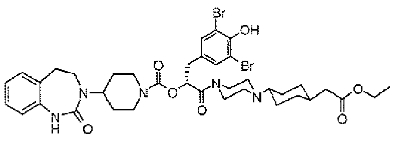 | cis-4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(4-ethoxycarbonylmethyl-cyclohexyl)-piperazin-1-yl]-2-oxo-ethyl ester |
| (434) | 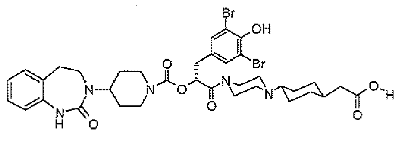 | cis-4-(2-Oxo-1,2,4,5-tetrahydro-1,3-benzdiazepin-3-yl)-piperidin-1-carbonsäure (R)-2-[4-(4-carboxymethyl-cyclohexyl)-piperazin-1-yl]-1-(3,5-dibrom-4-hydroxy-benzyl)-2-oxo-ethyl ester |
| (435) | 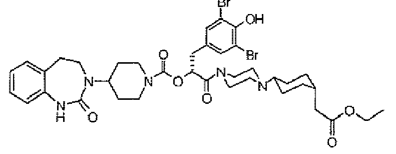 | trans-4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(4-ethoxycarbonylmethyl-cyclohexyl)-piperazin-1-yl]-2-oxo-ethyl ester |
| (436) | 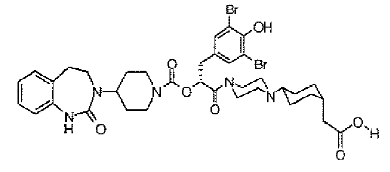 | trans-4-(2-Oxo-1,2,4,5-tetrahydro-1,3-benzdiazepin-3-yl)-piperidin-1-carbonsäure (R)-2-[4-(4-carboxymethyl-cyclohexyl)-piperazin-1-yl]-1-(3,5-dibrom-4-hydroxy-benzyl)-2-oxo-ethyl ester |
| (437) | 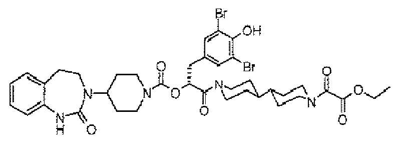 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(1'-ethoxyoxalyl-4,4'-bipiperidinyl-1-yl)-2-oxo-ethyl ester |
| (438) | 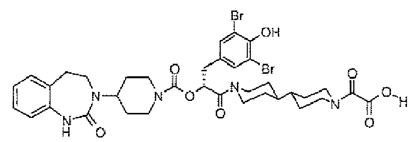 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(1'-oxalyl-4,4'-bipiperidinyl-1-yl)-2-oxo-ethyl ester |
| (439) | 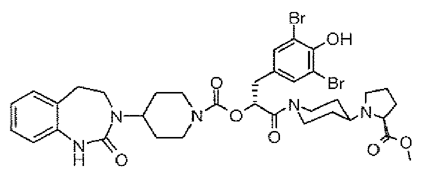 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-((S)-2-methoxycarbonyl-pyrrolidin-1-yl)-piperidin-1-yl]-2-oxo-ethyl ester |
| (440) | 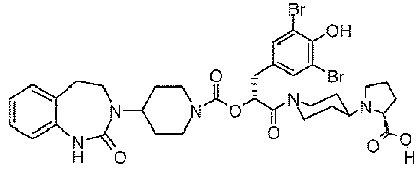 | 4-(2-Oxo-1,2,4,5-tetrahydro-1,3-benzdiazepin-3-yl)-piperidin-1-carbonsäure (R)-2-[4-((S)-2-carboxy-pyrrolidin-1-yl)-piperidin-1-yl]-1-(3,5-dibrom-4-hydroxy-benzyl)-2-oxo-ethyl ester |
| (441) | 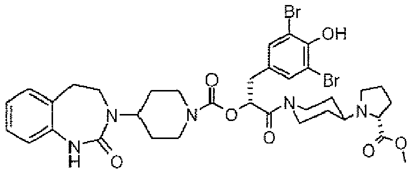 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-((R)-2-methoxycarbonyl-pyrrolidin-1-yl)-piperidin-1-yl]-2-oxo-ethyl ester |
| (442) | 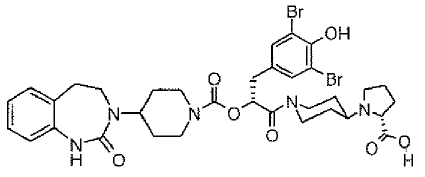 | 4-(2-Oxo-1,2,4,5-tetrahydro-1,3-benzdiazepin-3-yl)-piperidin-1-carbonsäure (R)-2-[4-((R)-2-carboxy-pyrrolidin-1-yl)-piperidin-1-yl]-1-(3,5-dibrom-4-hydroxy-benzyl)-2-oxo-ethyl ester |
| (443) | 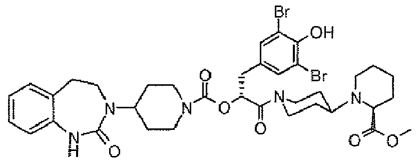 | (S)-1'-{(R)-3-(3,5-Dibrom-4-hydroxy-phenyl)-2-[4-(2-oxo-1,2,4,5-tetrahydro-1,3-benzdiazepin-3-yl)-piperidin-1-carbonyloxy]-propionyl}-1,4'-bipiperidinyl-2-carbonsäure methyl ester |
| (444) | 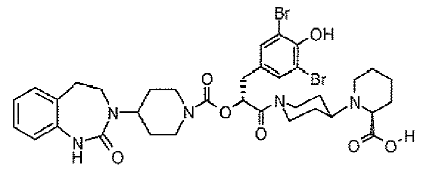 | (S)-1'-{(R)-3-(3,5-Dibrom-4-hydroxy-phenyl)-2-[4-(2-oxo-1,2,4,5-tetrahydro-1,3-benzdiazepin-3-yl)-piperidin-1-carbonyloxy]-propionyl}-1,4'-bipiperidinyl-2-carbonsäure |
| (445) | 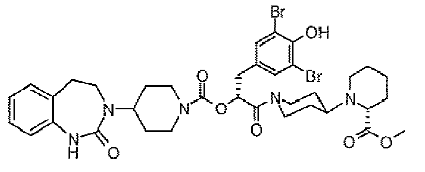 | (R)-1'-{(R)-3-(3,5-Dibrom-4-hydroxy-phenyl)-2-[4-(2-oxo-1,2,4,5-tetrahydro-1,3-benzdiazepin-3-yl)-piperidin-1-carbonyloxy]-propionyl}-1,4'-bipiperidinyl-2-carbonsäure methyl ester |
| (446) | 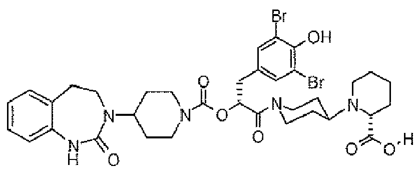 | (R)-1'-{(R)-3-(3,5-Dibrom-4-hydroxy-phenyl)-2- [4-(2-oxo-1,2,4,5-tetrahydro-1,3-benzdiazepin-3-yl)-piperidin-1-carbonyloxy]-propionyl}-1,4'-bipiperidinyl-2-carbonsäure |
| (447) | 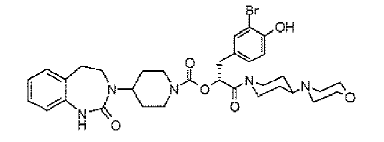 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-morpholin-4-yl-piperidin-1-yl)-2-oxo-ethyl ester |
| (448) | 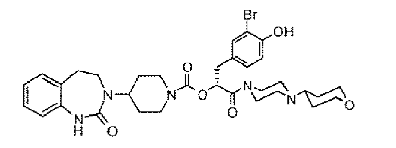 | 4-(2-Oxo-1,2,4,5-tetrahydro-1,3-benzdiazepin-3-yl)-piperidin-1-carbonsäure (R)-1-(3-brom-4-hydroxy-benzyl)-2-oxo-2-[4-(tetrahydro-pyran-4-yl)-piperazin-1-yl]-ethyl ester |
| (449) | 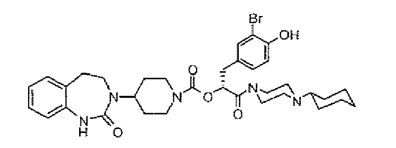 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-cyclohexyl-piperazin-1-yl)-2-oxo-ethyl ester |
| (450) | 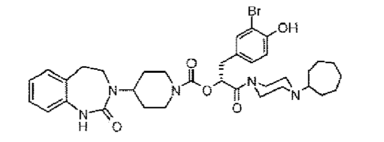 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-cycloheptyl-piperazin-1-yl)-2-oxo-ethyl ester |
| (451) | 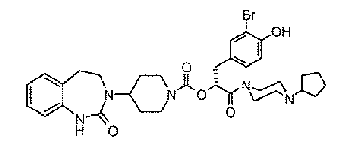 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-cyclopentyl-piparazin-1-yl)-2-oxo-ethyl ester |
| (452) | 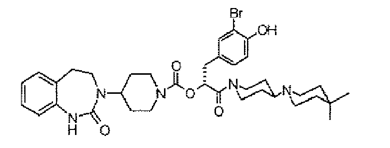 | 4-(2-Oxo-1,2,4,5-tetrahydro-1,3-benzdiazepin-3-yl)-piperidin-1-carbonsäure (R)-1-(3-brom-4-hydroxy-benzyl)-2-(4,4-dimethyl-1,4'-bipiperidinyl-1'-yl)-2-oxo-ethyl ester |
| (453) | 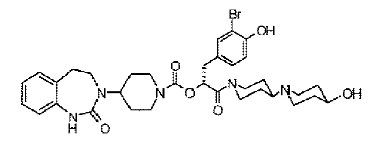 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-hydroxy-1,4'-bipiperidinyl-1'-yl)-2-oxo-ethyl ester |
| (454) | 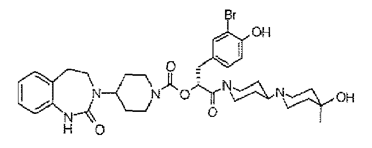 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-hydroxy-4-methyl-1,4'-bipiperidinyl-1'-yl)-2-oxo-ethyl ester |
| (455) | 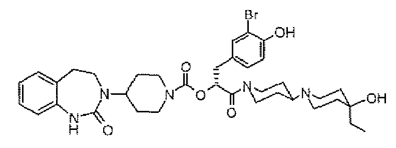 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-ethyl-4-hydroxy-1,4'-bipiperidinyl-1'-yl)-2-oxo-ethyl ester |
| (456) | 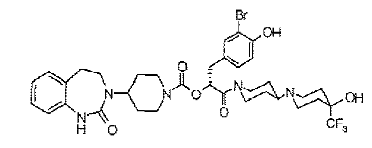 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-hydroxy-4-trifluormethyl-1,4'-bipiperidinyl-1'-yl)-2-oxo-ethyl ester |
| (457) | 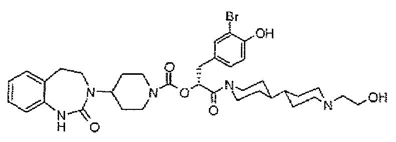 | 4-(2-Oxo-1,2,4,5-tetrahydro-1,3-benzdiazepin-3-yl)-piperidin-1-carbonsäure (R)-1-(3-brom-4-hydroxy-benzyl)-2-[1'-(2-hydroxy-ethyl)-4,4'-bipiperidinyl-1-yl]-2-oxo-ethyl ester |
| (458) | 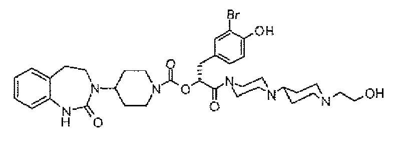 | 4-(2-Oxo-1,2,4,5-tetrahydro-1,3-benzdiazepin-3-yl)-piperidin-1-carbonsäure (R)-1-(3-brom-4-hydroxy-benzyl)-2-{4-[1-(2-hydroxy-ethyl)-piperidin-4-yl]-piperazin-1-yl}-2-oxo-ethyl ester |
| (459) | 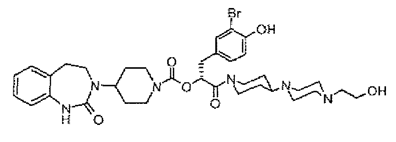 | 4-(2-Oxo-1,2,4,5-tetrahydro-1,3-benzdiazepin-3-yl)-piperidin-1-carbonsäure (R)-1-(3-brom-4-hydroxy-benzyl)-2-{4-[4-(2-hydroxy-ethyl)-piperazin-1-yl]-piperidin-1-yl}-2-oxo-ethyl ester |
| (460) | 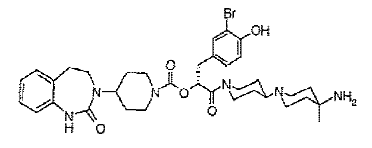 | 4-(2-Oxo-1,2,4,5-tetrahydro-1,3-benzdiazepin-3-yl)-piperidin-1-carbonsäure (R)-2-(4-amino-4-methyl-1,4'-bipiperidinyl-l'-yl)-1 -(3-brom-4-hydroxy-benzyl)-2-oxo-ethyl ester |
| (461) | 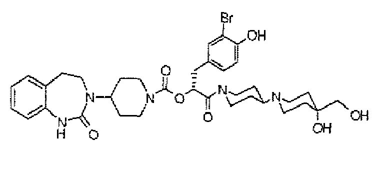 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-hydroxy-4-hydroxymethyl-1,4'-bipiperidinyl-1'-yl)-2-oxo-ethyl ester |
| (462) | 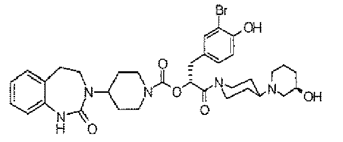 | 4-(2-Oxo-1,2,4,5-tetrahydro-1,3-benzdiazepin-3-yl)-piperidin-1-carbonsäure (R)-1-(3-brom-4-hydroxy-benzyl)-2-((R)-3-hydroxy-1,4'-bipiperidinyl-1'-yl)-2-oxo-ethyl ester |
| (463) | 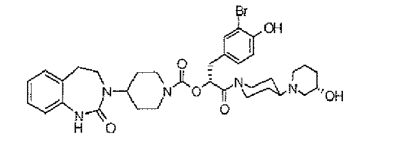 | 4-(2-Oxo-1,2,4,5-tetrahydro-1,3-benzdiazepin-3-yl)-piperidin-1-carbonsäure (R)-1-(3-brom-4-hydroxy-benzyl)-2-((S)-3-hydroxy-1,4'-bipiperidinyl-1'-yl)-2-oxo-ethyl ester |
| (464) | 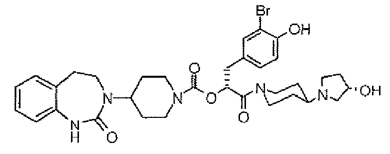 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-((S)-3-hydroxy-pyrrolidin-1-yl)-piperidin-1-yl]-2-oxo-ethyl ester |
| (465) | 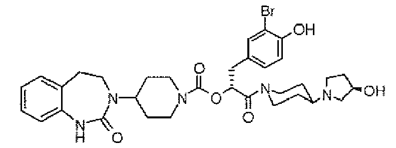 | 4-(2-Oxo-1,2,4,5-tetrahydro-1,3-benzdiazepin-3-yl)-piperidin-1 -carbonsäure (R)-1-(3-brom-4-hydroxy-benzyl)-2-[4-((R)-3-hydroxy-pyrrolidin-1-yl)-piperidin-1-yl]-2-oxo-ethyl ester |
| (466) | 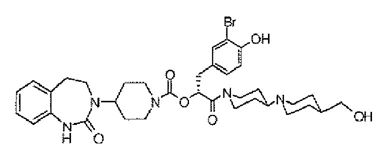 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-hydroxymethyl-1,4'-bipiperidinyl-1'-yl)-2-oxo-ethyl ester |
| (467) | 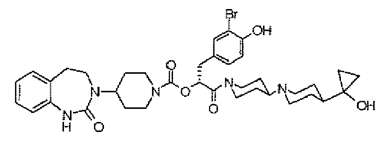 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(1-hydroxy-cyclopropyl)-1,4'-bipiperidinyl-1'-yl]-2-oxo-ethyl ester |
| (468) | 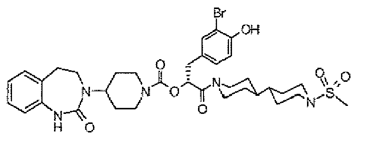 | 4-(2-Oxo-1,2,4,5-tetrahydro-1,3-benzdiazepin-3-yl)-piperidin-1-carbonsäure (R)-1-(3-brom-4-hydroxy-benzyl)-2-(1'-methansulfonyl-4,4'-bipiperidinyl-1-yl)-2-oxo-ethyl ester |
| (469) | 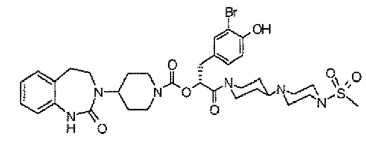 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(4-methansulfonyl-piperazin-1-yl)-piperidin-1-yl]-2-oxo-ethyl ester |
| (470) | 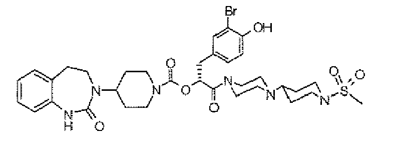 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(1-methansulfonyl-piperidin-4-yl)-piperazin-1-yl]-2-oxo-ethyl ester |
| (471) | 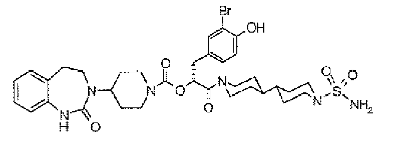 | 4-(2-Oxo-1,2,4,5-tetrahydro-1,3-benzdiazepin-3-yl)-piperidin-1-carbonsäure (R)-1-(3-brom-4-hydroxy-benzyl)-2-oxo-2-(1'-sulfamoyl-4,4'-bipiperidinyl-1-yl)-ethyl ester |
| (472) | 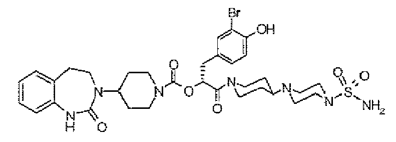 | 4-(2-Oxo-1,2,4,5-tetrahydro-1,3-benzdiazepin-3-yl)-piperidin-1-carbonsäure (R)-1-(3-brom-4-hydroxy-benzyl)-2-oxo-2-[4-(4-sulfamoyl-piperazin-1-yl)-piperidin-1-yl]-ethyl ester |
| (473) | 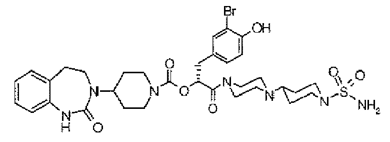 | 4-(2-Oxo-1,2,4,5-tetrahydro-1,3-benzdiazepin-3-yl)-piperidin-1-carbonsäure (R)-1-(3-brom-4-hydroxy-benzyl)-2-oxo-2-[4-(1-sulfamoyl-piperidin-4-yl)-piperazin-1-yl]-ethyl ester |
| (474) | 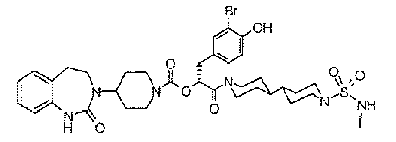 | 4-(2-Oxo-1,2,4,5-tetrahydro-1,3-benzdiazepin-3-yl)-piperidin-1-carbonsäure (R)-1-(3-brom-4-hydroxy-benzyl)-2-(1'-methylsulfamoyl-4,4'-bipiperidinyl-1-yl)-2-oxo-ethyl ester |
| (475) | 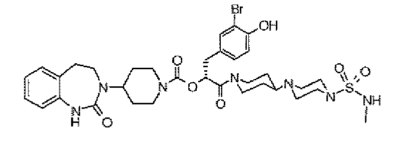 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(4-methylsulfamoyl-piperazin-1-yl)-piperidin-1-yl]-2-oxo-ethyl ester |
| (476) | 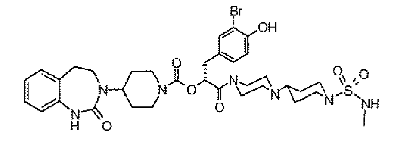 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(1-methylsulfamoyl-piperidin-4-yl)-piperazin-1-yl]-2-oxo-ethyl ester |
| (477) | 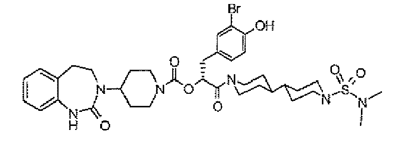 | 4-(2-Oxo-1,2,4,5-tetrahydro-1,3-benzdiazepin-3-yl)-piperidin-1-carbonsäure (R)-1-(3-brom-4-hydroxy-benzyl)-2-(1'-dimethylsulfamoyl-4,4'-bipiperidinyl-1-yl)-2-oxo-ethyl ester |
| (478) | 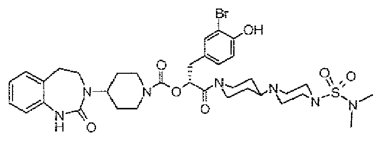 | 4-(2-Oxo-1,2,4,5-tetrahydro-1,3-benzdiazepin-3-yl)-piporidin-1-carbonsäure (R)-1-(3-brom-4-hydroxy-benzyl)-2-[4-(4-dimethylsulfamoyl-piperazin-1-yl)-piperidin-1-yl]-2-oxo-ethyl ester |
| (479) | 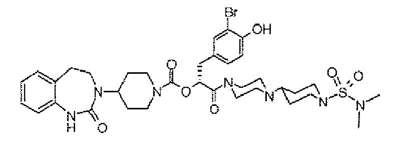 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(1-dimethylsulfamoyl-piperidin-4-yl)-piperazin-1-yl]-2-oxo-ethyl ester |
| (480) | 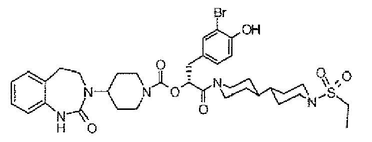 | 4-(2-Oxo-1,2,4,5-tetrahydro-1,3-benzdiazepin-3-yl)-piperidin-1-carbonsäure (R)-1-(3-brom-4-hydroxy-benzyl)-2-(1'-ethansulfonyl-4,4'-bipiperidinyl-1-yl)-2-oxo-ethyl ester |
| (481) | 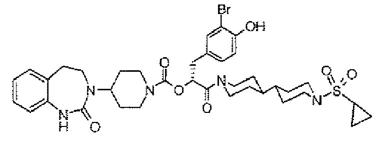 | 4-(2-Oxo-1,2,4,5-tetrahydro-1,3-benzdiazepin-3-yl)-piperidin-1-carbonsäure (R)-1-(3-brom-4-hydroxy-benzyl)-2-(1'-cyclopropansulfonyl-4,4'-bipiperidinyl-1-yl)-2-oxo-ethyl ester |
| (482) | 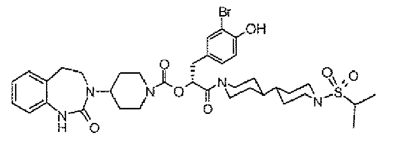 | 4-(2-Oxo-1,2,4,5-tetrahydro-1,3-benzdiazepin-3-yl)-piperidin-1-carbonsäure (R)-1-(3-brom-4-hydroxy-benzyl)-2-oxo-2-[1'-(propan-2-sulfonyl)-4,4'-bipiperidinyl-1-yl]-ethyl ester |
| (483) | 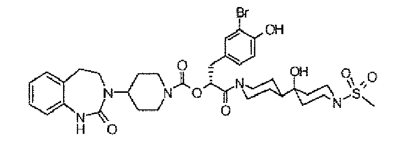 | 4-(2-Oxo-1,2,4,5-tetrahydro-1,3-benzdiazepin-3-yl)-piperidin-1-carbonsäure (R)-1-(3-brom-4-hydroxy-benzyl)-2-(4'-hydroxy-1'-methansulfonyf-4,4'-bipiperidinyl-1-yl)-2-oxo-ethyl ester |
| (484) | 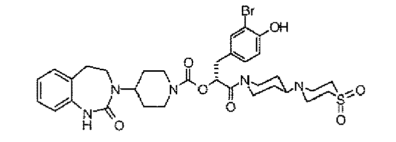 | 4-(2-Oxo-1,2,4,5-tetrahydro-1,3-benzdiazepin-3-y)-piperidin-1-carbonsäure (R)-1-(3-brom-4-hydroxy-benzyl)-2-[4-(1,1-dioxo-1λ⁶-thiomorpholin-4-yl)-piperidin-1-yl]-2-oxo-ethyl ester |
| (485) | 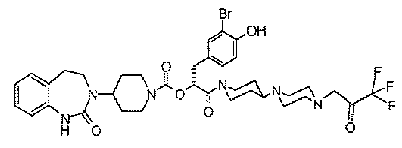 | 4-(2-Oxo-1,2,4,5-tetrahydro-1,3-benzdiazepin-3-yl)-piperidin-1-carbonsäure (R)-1-(3-brom-4-hydroxy-benzyl)-2-oxo-2-{4-[4-(3,3,3-trifluor-2-oxo-propyl)-piperazin-1-yl]-piperidin-1-yl}-ethyl ester |
| (486) | 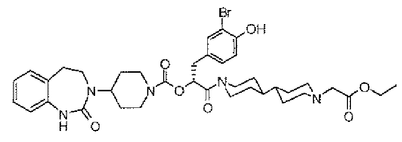 | 4-(2-Oxo-1,2,4,5-tetrahydro-,3-benzdiazepin-3-yl)-piperidin-1-carbonsäure (R)-1-(3-brom-4-hydroxy-benzyl)-2-(1'-ethoxycarbonylmethyl-4,4'-bipiperidinyl-1-yl)-2-oxo-ethyl ester |
| (487) | 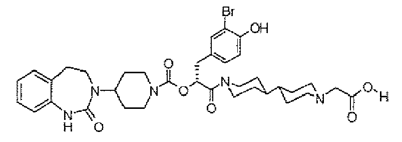 | 4-(2-Oxo-1,2,4,5-tetrahydro-1,3-benzdiazepin-3-yl)-piperidin-1-carbonsäure (R)-1-(3-brom-4-hydroxy-benzyl)-2-(1'-carboxymethyl-4,4'-bipiperidinyl-1-yl)-2-oxo-ethyl ester |
| (488) | 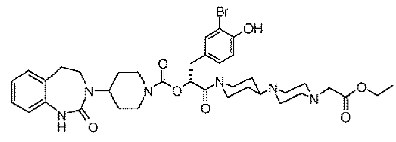 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(4-ethoxycarbonylmethyl-piperazin-1-yl)-piperidin-1-yl]-2-oxo-ethyl ester |
| (489) | 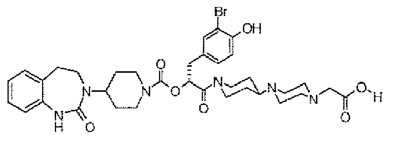 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(4-carboxymethyl-piperazin-1-yl)-piperidin-1-yl]-2-oxo-ethyl ester |
| (490) | 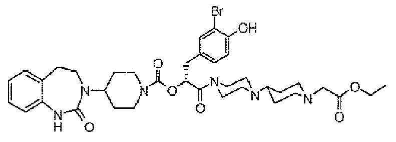 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(1-ethoxycarbonylmethyl-piperidin-4-yl)-piperazin-1-yl]-2-oxo-ethyl ester |
| (491) | 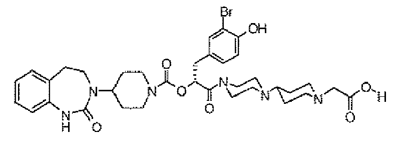 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(1-carboxymethyl-piperidin-4-yl)-piperazin-1-yl]-2-oxo-ethyl ester |
| (492) | 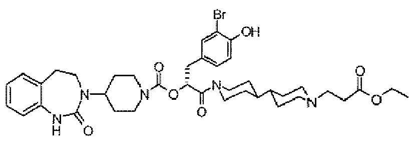 | 4-(2-Oxo-1,2,4,5-tetrahydro-1,3-benzdiazepin-3-yl)-piperidin-1-carbonsäure (R)-1-(3-brom-4-hydroxy-benzyl)-2-[1'-(2-ethoxycarbonyl-ethyl)-4,4'-bipiperidinyl-1-yl]-2-oxo-ethyl ester |
| (493) | 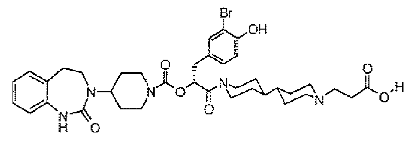 | 4-(2-Oxo-1,2,4,5-tetrahydro-1,3-benzdiazepin-3-yl)-piperidin-1-carbonsäure (R)-1-(3-brom-4-hydroxy-benzyl)-2-[1'-(2-carboxy-ethyl)-4,4'-bipiperidinyl-1-yl]-2-oxo-ethyl ester |
| (494) | 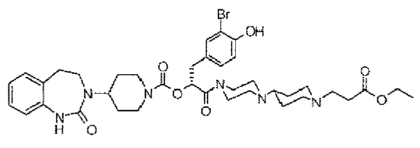 | 4-(2-Oxo-1,2,4,5-tetrahydro-1,3-benzdiazepin-3-yl)-piperidin-1-carbonsäure (R)-1-(3-brom-4-hydroxy-benzyl)-2-{4-[1-(2-ethoxycarbonyl-ethyl)-piperidin-4-yl]-piperazin-1-yl}-2-oxo-ethyl ester |
| (495) | 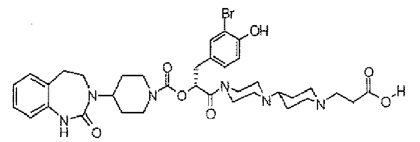 | 4-(2-Oxo-1,2,4,5-tetrahydro-1,3-benzdiazepin-3-yl)-piperidin-1-carbonsäure (R)-1-(3-brom-4-hydroxy-benzyl)-2-{4-[1-(2-carboxy-ethyl)-piperidin-4-yl]-piperazin-1-yl}-2-oxo-ethyl ester |
| (496) | 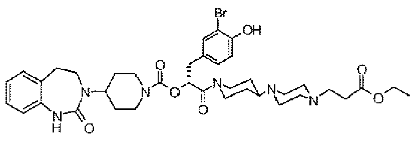 | 4-(2-Oxo-1,2,4,5-tetrahydro-1,3-benzdiazepin-3-yl)-piperidin-1-carbonsäure (R)-1-(3-brom-4-hydroxy-benzyl)-2-{4-[4-(2-ethoxycarbonyl-eihyl)-piperazin-1-yl]-piperidin-1-yl}-2-oxo-ethyl ester |
| (497) | 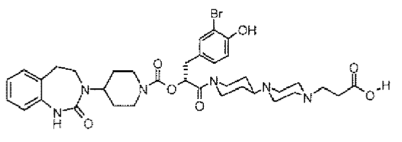 | 4-(2-Oxo-1,2,4,5-tetrahydro-1,3-benzdiazepin-3-yl)-piperidin-1-carbonsäure (R)-1-(3-brom-4-hydroxy-benzyl)-2-{4-[4-(2-carboxy-ethyl)-piperazin-1-yl]-piperidin-1-yl}-2-oxo-ethyl ester |
| (498) | 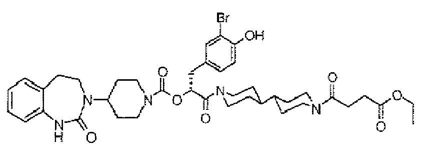 | 4-(2-Oxo-1,2,4,5-tetrahydro-1,3-benzdiazepin-3-yl)-piperidin-1-carbonsäure (R)-1-(3-brom-4-hydroxy-benzyl)-2-[1'-(3-ethoxycarbonyl-propionyl)-4,4'-bipiperidinyl-1-yl]-2-oxo-ethyl ester |
| (499) | 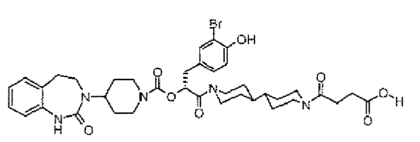 | 4-(2-Oxo-1,2,4,5-tetrahydro-1,3-benzdiazepin-3-yl)-piperidin-1-carbonsäure (R)-1-(3-brom-4-hydroxy-benzyl)-2-[1'-(3-carboxy-propionyl)-4,4'-bipiperidinyl-1-yl]-2-oxo-ethyl ester |
| (500) | 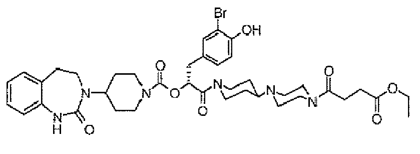 | 4-(2-Oxo-1,2,4,5-tetrahydro-1,3-benzdiazepin-3-yl)-piperidin-1-carbonsäure (R)-1-(3-brom-4-hydroxy-benzyl)-2-{4-[4-(3-ethoxycarbonyl-propionyl)-piperazin-1-yl]-piperidin-1-yl}-2-oxo-ethyl ester |
| (501) | 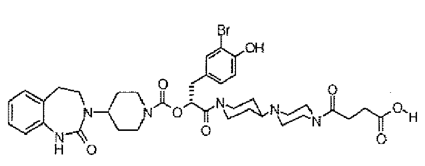 | 4-(2-Oxo-1,2,4,5-tetrahydro-1,3-benzdiazepin-3-yl)-piperidin-1-carbonsäure (R)-1-(3-brom-4-hydroxy-benzyl)-2-{4-[4-(3-carboxy-propionyl)-piperazin-1-yl]-piperidin-1-yl}-2-oxo-ethyl ester |
| (502) | 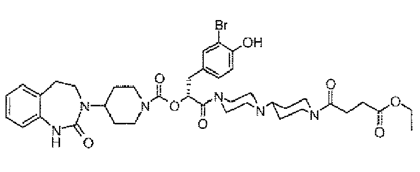 | 4-(2-Oxo-1,2,4,5-tetrahydro-1,3-benzdiazepin-3-yl)-piperidin-1-carbonsäure (R)-1-(3-brom-4-hydroxy-benzyl)-2-{4-[-(3-ethoxycarbonyl-propionyl)-piperidin-4-yl]-piperazin-1-yl}-2-oxo-ethyl ester |
| (503) | 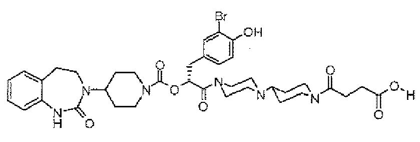 | 4-(2-Oxo-1,2,4,5-tetrahydro-1,3-benzdiazepin-3-yl)-piperidin-1-carbonsäure (R)-1-(3-brom-4-hydroxy-benzyl)-2-{4-[1-(3-carboxy-propionyl)-piperidin-4-yl]-piperazin-1-yl}-2-oxo-ethyl ester |
| (504) | 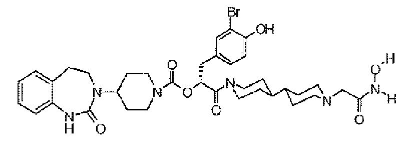 | 4-(2-Oxo-1,2,4,5-tetrahydro-1,3-benzdiazepin-3-yl)-piperidin-1-carbonsäure (R)-1-(3-brom-4-hydroxy-benzyl)-2-(1'-hydroxycarbamoylmethyl-4,4'-bipiperidinyl-1-yl)-2-oxo-ethyl ester |
| (505) | 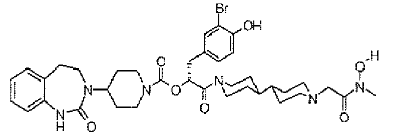 | 4-(2-Oxo-1,2,4,5-tetrahydro-1,3-benzdiazepin-3-yl)-piperidin-1-carbonsäure (R)-1-(3-brom-4-hydroxy-benzyl)-2-{1'-[(hydroxy-methyl-carbamoyl)-methyl]-4,4'-bipiperidinyl-1-yl}-2-oxo-ethyl ester |
| (506) | 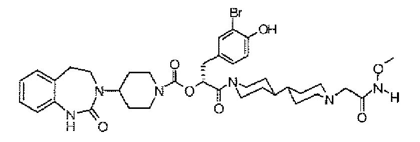 | 4-(2-Oxo-1,2,4,5-tetrahydro-1,3-benzdiazepin-3-yl)-piporidin-1 -carbonsäure (R)-1-(3-brom-4-hydroxy-benzyl)-2-[1'-(methoxycarbamoyl-methyl)-4,4'-bipiperidinyl-1-yl]-2-oxo-ethyl ester |
| (507) | 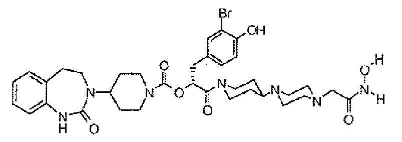 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(4-hydroxycarbamoylmethyl-piperazin-1-yl)-piperidin-1-yl]-2-oxo-ethyl ester |
| (508) | 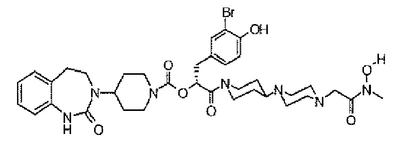 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-{4-[(hydroxy-methyl-carbamoyl)-mothyl]-piperazin-1-yl}-piperidin-1-yl)-2-oxo-ethyl ester |
| (509) | 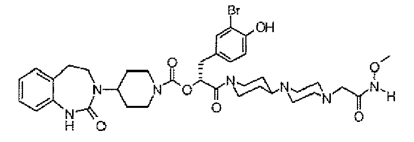 | 4-(2-Oxo-1,2,4,5-tetrahydro-1,3-benzdiazepin-3-yl)-piperidin-1-carbonsäure (R)-1-(3-brom-4-hydroxy-benzyl)-2-{4-[4-(methoxycarbamoyl-methyl)-piperazin-1-yl]-piperidin-1-yl}-2-oxo-ethyl ester |
| (510) | 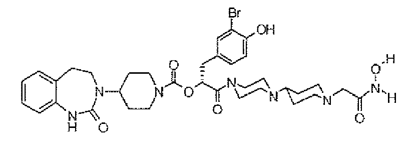 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(1-hydroxycarbamoylmethyl-piperidin-4-yl)-piperazin-1-yl]-2-oxo-ethyl ester |
| (511) | 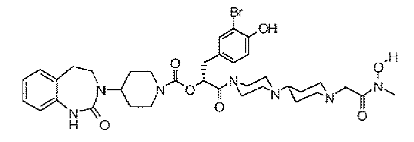 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-{1-[(hydroxy-methyl-carbamoyl)-methyl]-piperidin-4-yl}-piperazin-1-yl)-2-oxo-ethyl ester |
| (512) | 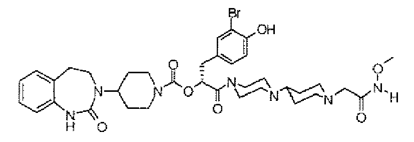 | 4-(2-Oxo-1,2,4,5-tetrahydro-1,3-benzdiazepin-3-yl)-piperidin-1-carbonsäure (R)-1-(3-brom-4-hydroxy-benzyl)-2-{4-[1-(methoxycarbamoyl-methyl)-piperidin-4-yl]-piperazin-1-yl}-2-oxo-ethyl ester |
| (513) | 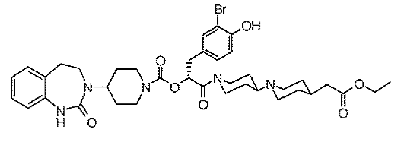 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-ethoxycarbonylmethyl-1,4'-bipiperidinyl-1'-yl)-2-oxo-ethyl ester |
| (514) | 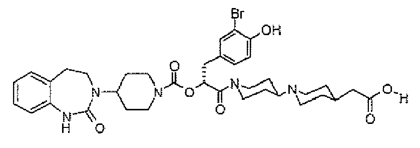 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-carboxymethyl-1,4'-bipiperidinyl-1'-yl)-2-oxo-ethyl ester |
| (515) | 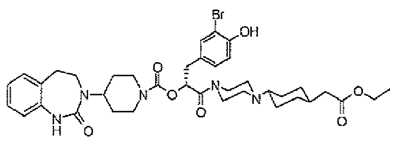 | trans-4-(2-Oxo-1,2,4,5-tetrahydro-1,3-benzdiazepin-3-yl)-piperidin-1-carbonsäure (R)-1-(3-brom-4-hydroxy-benzyl)-2-[4-(4-ethoxycarbonylmethyl-cyclohexyl)-piperazin-1-yl]-2-oxo-ethyl ester |
| (516) | 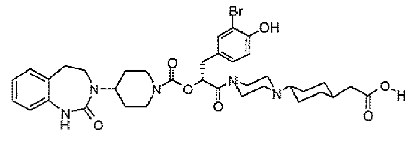 | trans-4-(2-Oxo-1,2,4,5-tetrahydro-1,3-benzdiazepin-3-yl)-piperidin-1-carbonsäure (R)-1-(3-brom-4-hydroxy-benzyl)-2-[4-(4-carboxymethyl-cyclohexyl)-piperazin-1-yl]-2-oxo-ethyl ester |
| (517) | 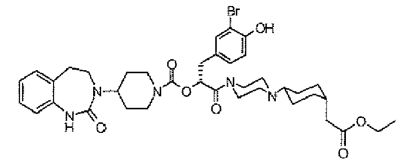 | cis-4-(2-Oxo-1,2,4,5-tetrahydro-1,3-benzdiazepin-3-yl)-piperidin-1-carbonsäure (R)-1-(3-brom-4-hydroxy-benzyl)-2-[4-(4-ethoxycarbonylmethyl-cyclohexyl)-piperazin-1-yl]-2-oxo-ethyl ester |
| (518) | 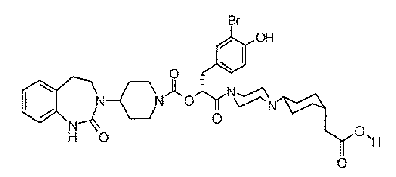 | cis-4-(2-Oxo-1,2,4,5-tetrahydro-1,3-benzdiazepin-3-yl)-piperidin-1-carbonsäure (R)-1-(3-brom-4-hydroxy-benzyl)-2-[4-(4-carboxymethyl-cyclohexyl)-piperazin-1-yl]-2-oxo-ethyl ester |
| (519) | 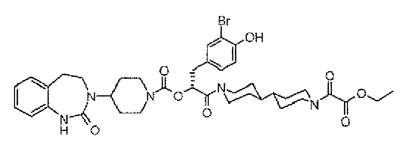 | 4-(2-Oxo-1,2,4,5-tetrahydro-1,3-benzdiazepin-3-yl)-piperidin-1-carbonsäure (R)-1-(3-brom-4-hydroxy-benzyl)-2-(1'-ethoxyoxalyl-4,4'-bipiperidinyl-1-yl)-2-oxo-ethyl ester |
| (520) | 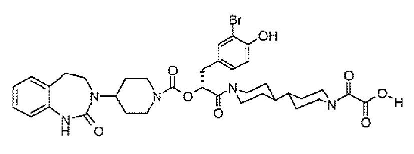 | 4-(2-Oxo-1,2,4,5-tetrahydro-1,3-benzdiazepin-3-yi)-piperidin-1-carbansäure (R)-1-(3-brom-4-hydroxy-benzyl)-2-(1'-oxalyl-4,4'-bipiperidinyl-1-yl)-2-oxo-ethyl ester |
| (521) | 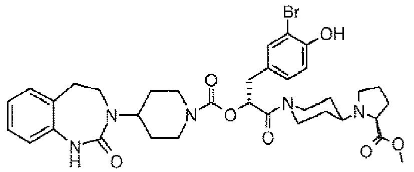 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-((S)-2-methoxycarbonyl-pyrrolidin-1-yl)-piperidin-1-yl]-2-oxo-ethyl ester |
| (522) | 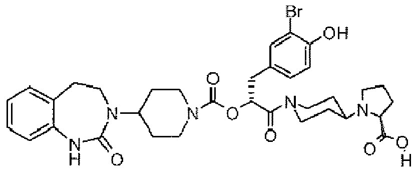 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-((S)-2-carboxy-pyrrolidin-1-yl)-piperidin-1-yl]-2-oxo-ethyl ester |
| (523) | 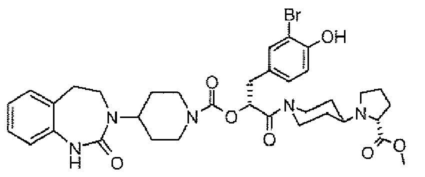 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-((R)-2-methoxycarbonyl-pyrrolidin-1-yl)-piperidin-1-yl]-2-oxo-ethyl ester |
| (524) | 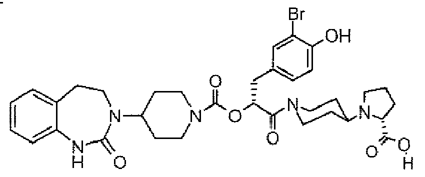 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-((R)-2-carboxy-pyrrolidin-1-yl)-piperidin-1-yl]-2-oxo-ethyl ester |
| (525) | 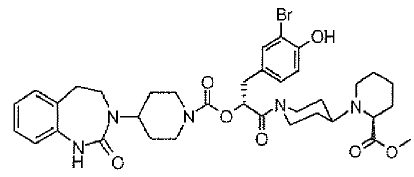 | (S)-1'-{(R)-3-(3-Brom-4-hydroxy-phenyl)-2-[4-(2-oxo-1,2,4,5-tetrahydro-1,3-benzdiazepin-3-yl)-piperidin-1-carbonyloxy]-propionyl}-1,4'-bipiperidinyl-2-carbonsäure methyl ester |
| (526) | 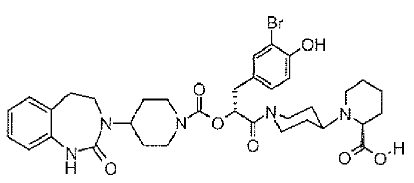 | S)-1'-{(R)-3-(3-Brom-4-hydroxy-phenyl)-2-[4- (2-oxo-1,2,4,5-tetrahydro-1,3-benzdiazepin-3-yl)-piporidin-1-carbonyloxy]-propionyl}-1,4'-bipiperidinyl-2-carbonsäure |
| (527) | 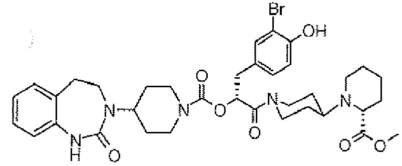 | (R)-1'-{(R)-3-(3-Brom-4-hydroxy-phenyl)-2-[4-(2-oxo-1,2,4,5-tetrahydro-1,3-benzdiazepin-3-yl)-piperidin-1-carbonyloxy]-propionyl}-1,4'-bipiperidinyl-2-carbonsäure methyl ester |
| (528) | 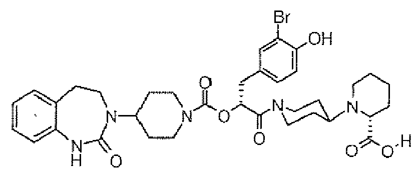 | (R)-1'-{(R)-3-(3-Brom-4-hydroxy-phenyl)-2-[4-(2-oxo-1,2,4,5-tetrahydro-1,3-benzdiazepin-3-yl)-piperidin-1-carbonyloxy]-propionyl}-1,4'-bipiperidinyl-2-carbonsäure |
| (529) | 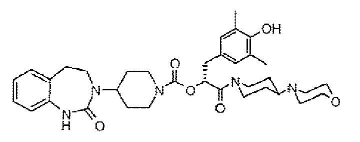 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-(4-morpholin-4-yl-piperidin-1-yl)-2-oxo-ethyl ester |
| (530) | 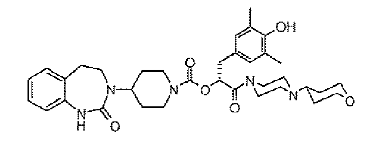 | 4-(2-Oxo-1,2,4,5-tetrahydro-1,3-benzdiazepin-3-yl)-piperidin-1-carbansäure (R)-1-(4-hydroxy-3,5-dimethyl-benzyl)-2-oxo-2-[4-(tetrahydro-pyran-4-yl)-piperazin-1-yl]-ethyl ester |
| (531) | 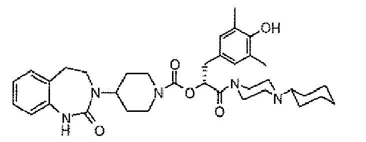 | 4-(2-Oxo-1,2,4,5-tetrahydro-1,3-benzdiazepin-3-yl)-piperidin-1-carbonsäure (R)-2-(4-cyclohexyl-piperazin-1-yl)-1-(4-hydroxy-3,5-dimethyl-benzyl)-2-oxo-ethyl ester |
| (532) | 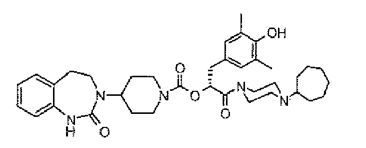 | 4-(2-Oxo-1,2,4,5-tetrahydro-1,3-benzdiazepin-3-yl)-piperidin-1-carbonsäure (R)-2-(4-cycloheptyl-piperazin-1-yl)-1-(4-hydroxy-3,5-dimethyl-benzyl)-2-oxo-ethyl ester |
| (533) | 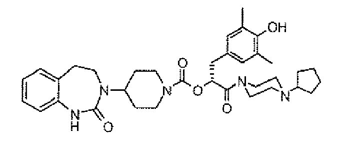 | 4-(2-Oxo-1,2,4,5-tetrahydro-1,3-benzdiazepin-3-yl)-piperidin-1-carbonsäure (R)-2-(4-cyclopentyl-piperazin-1-yl)-1-(4-hydroxy-3,5-dimethyl-benzyl)-2-oxo-ethyl ester |
| (534) | 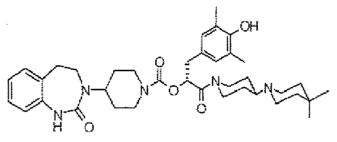 | 4-(2-Oxo-1,2,4,5-tetrahydro-1,3-benzdiazepin-3-yl)-piperidin-1-carbonsäure (R)-2-(4,4-dimethyl-1,4'-bipiperidinyl-1'-yl)-1-(4-hydroxy-3,5-dimethyl-benzyl)-2-oxo-ethyl ester |
| (535) | 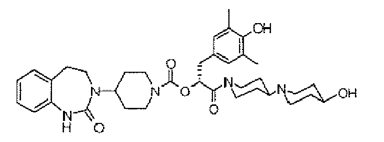 | 4-(2-Oxo-1,2,4,5-tetrahydro-1,3-benzdiazepin-3-yl)-piperidin-1-carbonsäure (R)-2-(4-hydroxy-1,4'-bipiperidinyl-1'-yl)-1-(4-hydroxy-3,5-dimethyl-benzyl)-2-oxo-ethyl ester |
| (536) | 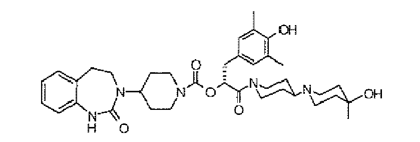 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-(4-hydroxy-4-methyl-1,4'-bipiperidinyl-1'-yl)-2-oxo-ethyl ester |
| (537) | 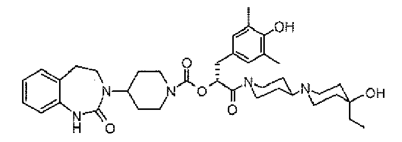 | 4-(2-Oxo-1,2,4,5-tetrahydro-1,3-benzdiazepin-3-yl)-piperidin-1-carbonsäure (R)-2-(4-ethyl-4-hydroxy-1,4'-bipiperidinyl-1'-yl)-1-(4-hydroxy-3,5-dimethyl-benzyl)-2-oxo-ethyl ester |
| (538) | 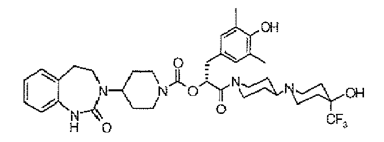 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-(4-hydroxy-4-trifluormethyl-1,4'-bipiperidinyl-1'-yl)-2-oxo-ethyl ester |
| (539) | 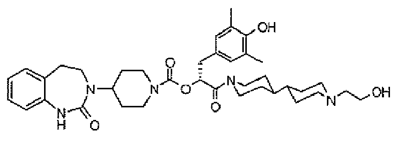 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[1'-(2-hydroxy-ethyl)-4,4'-bipiperidinyl-1-yl]-2-oxo-ethyl ester |
| (540) | 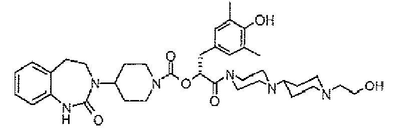 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-{4-[1-(2-hydroxy-ethyl)-piperidin-4-yl]-piperazin-1-yl}-2-oxo-ethyl ester |
| (541) | 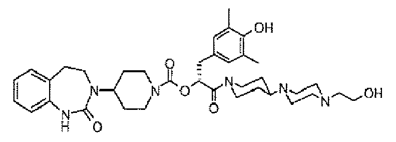 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-{4-[4-(2-hydroxy-ethyl)-piperazin-1-yl]-piperidin-1-yl}-2-oxo-ethyl ester |
| (542) | 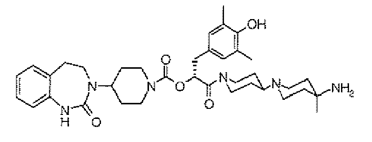 | 4-(2-Oxo-1,2,4,5-tetrahydro-1,3-benzdiazepin-3-yl)-piperidin-1-carbonsäure (R)-2-(4-amino-4-methyl-1,4'-bipiperidinyl-1'-yl)-1-(4-hydroxy-3,5-dimethyl-benzyl)-2-oxo-ethyl ester |
| (543) | 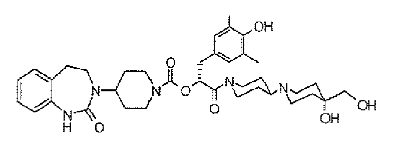 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-(4-hydroxy-4-hydroxymethyl-1,4'-bipiperidinyl-1'-yl)-2-oxo-ethyl ester |
| (544) | 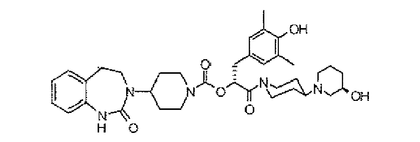 | 4-(2-Oxo-1,2,4,5-tetrahydro-1,3-benzdiazepin-3-yl)-piperidin-1-carbonsäure (R)-2-((R)-3-hydroxy-1,4'-bipiperidinyl-1'-yl)-1-(4-hydroxy-3,5-dimethyl-benzyl)-2-oxo-ethyl ester |
| (545) | 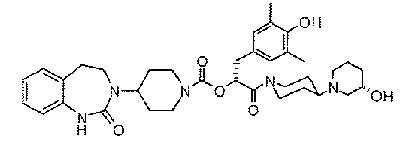 | 4-(2-Oxo-1,2,4,5-tetrahydro-1,3-benzdiazepin-3-yl)-piperidin-1-carbonsäure (R)-2-((S)-3-hydroxy-1,4'-bipiperidinyl-1'-yl)-1-(4-hydroxy-3,5-dimethyl-benzyl)-2-oxo-ethyl ester |
| (546) | 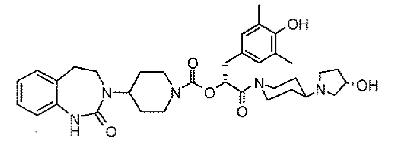 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-((S)-3-hydroxy-pyrrolidin-1-yl)-piperidin-1-yl]-2-oxo-ethyl ester |
| (547) | 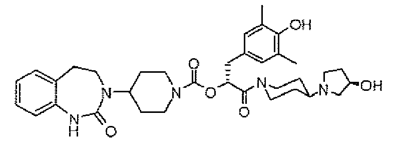 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-((R)-3-hydroxy-pyrrolidin-1-yl)-piperidin-1-yl]-2-oxo-ethyl ester |
| (548) | 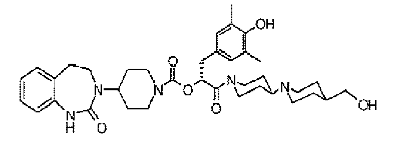 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-(4-hydroxymethyl-1,4'-bipiperidinyl-1'-yl)-2-oxo-ethyl ester |
| (549) | 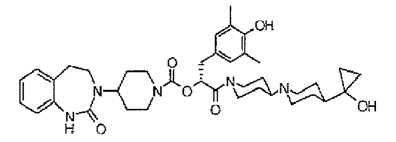 | 4-(2-Oxo-1,2,4,5-tetrahydro-1,3-benzdiazepin-3-yl)-piperidin-1-carbonsäure (R)-2-[4-(1-hydroxy-cyclopropyl)-1,4'-bipiperidinyl-1'-yl]-1-(4-hydroxy-3,5-dimethyl-benzyl)-2-oxo-ethyl ester |
| (550) | 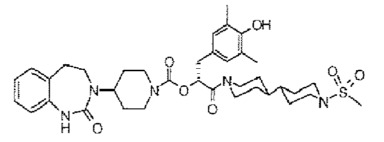 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-(1'-methansulfonyl-4,4'-bipiperidinyl-1-yl)-2-oxo-ethyl ester |
| (551) | 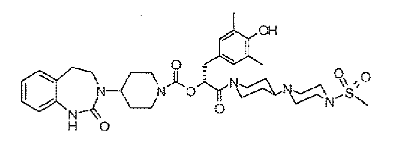 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-(4-methansulfonyl-piperazin-1-yl)-piperidin-1-yl]-2-oxo-ethyl ester |
| (552) | 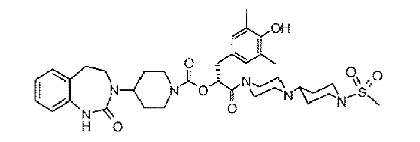 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-(1-methansulfonyl-piperidin-4-yl)-piperazin-1-yl]-2-oxo-ethyl ester |
| (553) | 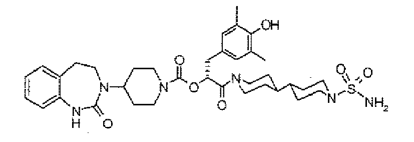 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-oxo-2-(1'-sulfamoyl-4,4'-bipiperidinyl-1-yl)-ethyl ester |
| (554) | 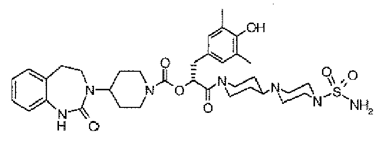 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-oxo-2-[4-(4-sulfamoyl-piperazin-1-yl)-piperidin-1-yl]-ethyl ester |
| (555) | 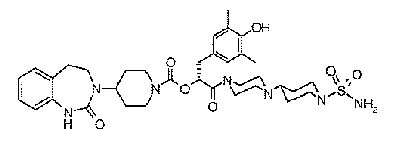 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-oxo-2-[4-(1-sulfamoyl-piperidin-4-yl)-piperazin-1 -yl]-ethyl ester |
| (556) | 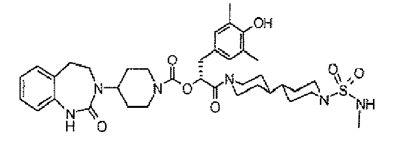 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-(1'-methylsulfamoyl-4,4'-bipiperidinyl-1-yl)-2-oxo-ethyl ester |
| (557) | 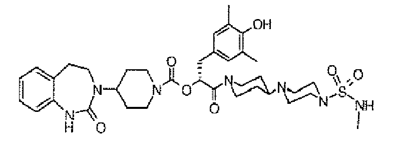 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-(4-methylsulfamoyl-piperazin-1-yl)-piperidin-1-yl]-2-oxo-ethyl ester |
| (558) | 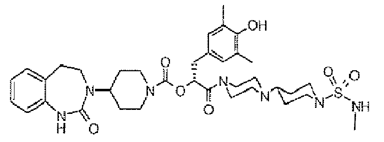 | 4-(2-Oxo-1,2,4,5-tetrahydro-1,3-benzdiazepin- 3-yl)-piperidin-1-carbonsäure (R)-1-(4-hydroxy-3,5-dimethyl-benzyl)-2-[4-(1-methylsulfamoyl-piperidin-4-yl)-piperazin-1-yl]-2-oxo-ethyl ester |
| (559) | 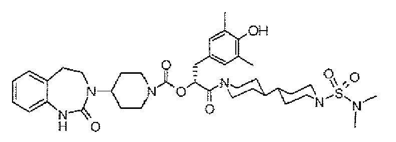 | 4-(2-Oxo-1,2,4,5-tetrahydro-1,3-benzdiazepin-3-yl)-piperidin-1-carbonsäure (R)-2-(1'-dimethylsulfamoyl-4,4'-bipiperidinyl-1-yl)-1-(4-hydroxy-3,5-dimethyl-benzyl)-2-oxo-ethyl ester |
| (560) | 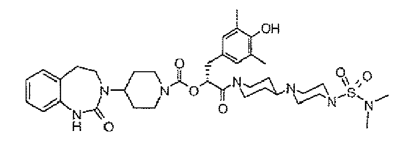 | 4-(2-Oxo-1,2,4,5-tetrahydro-1,3-benzdiazepin-3-yl)-piperidin-1-carbonsäure (R)-2-[4-(4-dimethylsulfamoyl-piperazin-1-yl)-piperidin-1-yl]-1-(4-hydroxy-3,5-dimethyl-benzyl)-2-oxo-ethyl ester |
| (561) | 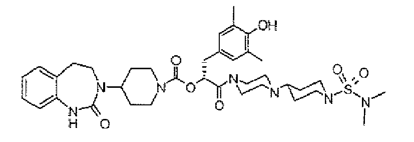 | 4-(2-Oxo-1,2,4,5-tetrahydro-1,3-benzdiazepin-3-yl)-piperidin-1-carbonsäure (R)-2-[4-(1-dimethylsulfamoyl-piperidin-4-yl)-piperazin-1-yl]-1-(4-hydroxy-3,5-dimethyl-benzyl)-2-oxo-ethyl ester |
| (562) | 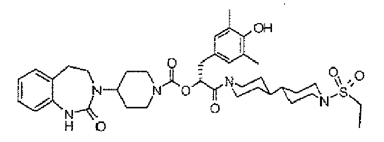 | 4-(2-Oxo-1,2,4,5-tetrahydro-1,3-benzdiazepin-3-yl)-piperidin-1-carbonsäure (R)-2-(1'-ethansulfonyl-4,4'-bipiperidinyl-1-yl)-1-(4-hydroxy-3,5-dimethyl-benzyl)-2-oxo-ethyl ester |
| (563) | 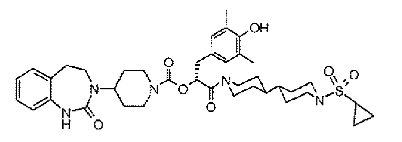 | 4-(2-Oxo-1,2,4,5-tetrahydro-1,3-benzdiazepin-3-yl)-piperidin-1-carbonsäure (R)-2-(1'-cyclopropansulfonyl-4,4'-bipiperidinyl-1-yl)-1-(4-hydroxy-3,5-dimethyl-benzyl)-2-oxo-ethyl ester |
| (564) | 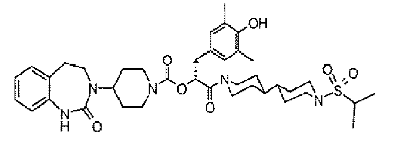 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-oxo-2-[1'-(propan-2-sulfonyl)-4,4'-bipiperidinyl-1-yl]-ethyl ester |
| (565) | 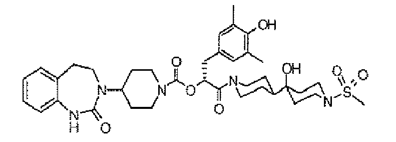 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-(4'-hydroxy-1'-methansulfonyl-4,4'-bipiperidinyl-1-yl)-2-oxo-ethyl ester |
| (566) | 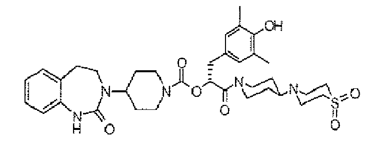 | 4-(2-Oxo-1,2,4,5-tetrahydro-1,3-benzdiazepin-3-yl)-piperidin-1-carbonsäure (R)-2-[4-(1,1-dioxo-1λ⁶-thiomorpholin-4-yl)-piperidin-1-yl]-1-(4-hydroxy-3,5-dimethyl-benzyl)-2-oxo-ethyl ester |
| (567) | 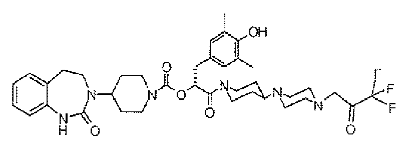 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-oxo-2-{4-[4-(3,3,3-trifluor-2-oxo-propyl)-piperazin-1-yl]-piperidin-1-yl}-ethyl ester |
| (568) | 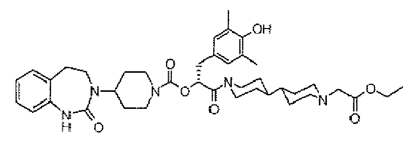 | 4-(2-Oxo-1,2,4,5-tetrahydro-1,3-benzdiazepin-3-yl)-piperidin-1-carbonsäure (R)-2-(1'-ethoxycarbonylmethyl-4,4'-bipiperidinyl-1-yl)-1-(4-hydroxy-3,5-dimethyl-benzyl)-2-oxo-ethyl ester |
| (569) | 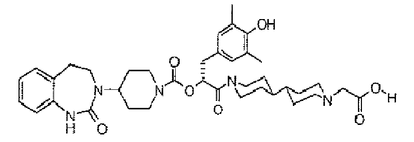 | 4-(2-Oxo-1,2,4,5-tetrahydro-1,3-benzdiazepin-3-yl)-piperidin-1-carbonsäure (R)-2-(1'-carboxymethyl-4,4'-bipiperidinyl-1-yl)-1-(4-hydroxy-3,5-dimethyl-benzyl)-2-oxo-ethyl ester |
| (570) | 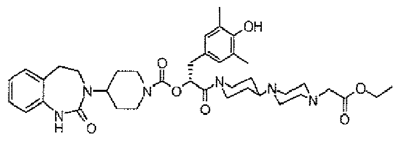 | 4-(2-Oxo-1,2,4,5-tetrahydro-1,3-benzdiazepin-3-yl)-piperidin-1-carbonsäure (R)-2-[4-(4-ethoxycarbonylmethyl-piperazin-1-yl)-piperidin-1-yl]-1-(4-hydroxy-3,5-dimethyl-benzyl)-2-oxo-ethyl ester |
| (571) | 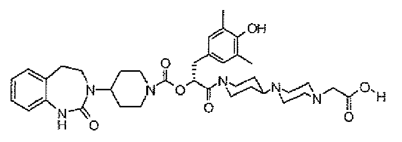 | 4-(2-Oxo-1,2,4,5-tetrahydro-1,3-benzdiazepin-3-yl)-piperidin-1-carbonsäure (R)-2-[4-(4-carboxymethyl-piperazin-1-yl)-piperidin-1-yl]-1-(4-hydroxy-3,5-dimethyl-benzyl)-2-oxo-ethyl ester |
| (572) | 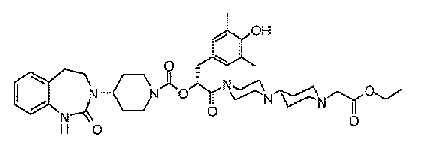 | 4-(2-Oxo-1,2,4,5-tetrahydro-1,3-benzdiazepin-3-yl)-piperidin-1-carbonsäure (R)-2-[4-(1-ethoxycarbonylmethyl-piperidin-4-yl)-piperazin-1-yl]-1-(4-hydroxy-3,5-dimethyl-benzyl)-2-oxo-ethyl ester |
| (573) | 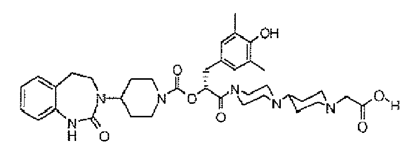 | 4-(2-Oxo-1,2,4,5-tetrahydro-1,3-benzdiazepin-3-yl)-piperidin-1-carbonsäure (R)-2-[4-(1-carboxymothyl-piperidin-4-yl)-piperazin-1-yl]-1-(4-hydroxy-3,5-dimethyl-benzyl)-2-oxo-ethyl ester |
| (574) | 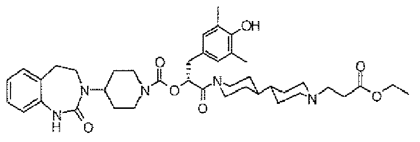 | 4-(2-Oxo-1,2,4,5-tetrahydro-1,3-benzdiazepin- 3-yl)-piperidin-1-carbonsäure (R)-2-[1'-(2-ethoxycarbonyl-ethyl)-4,4'-bipiperidinyl-1-yl]-1-(4-hydroxy-3,5-dimethyl-benzyl)-2-oxo-ethyl ester |
| (575) | 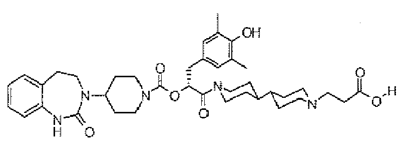 | 4-(2-Oxo-1,2,4,5-tetrahydro-1,3-benzdiazepin-3-yl)-piperidin-1-carbonsäure (R)-2-[1'-(2-carboxy-ethyl)-4,4'-bipiperidinyl-1-yl]-1-(4-hydroxy-3,5-dimethyl-benzyl)-2-oxo-ethyl ester |
| (576) | 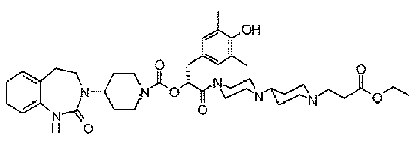 | 4-(2-Oxo-1,2,4,5-tetrahydro-1,3-benzdiazepin-3-yl)-piperidin-1-carbonsäure (R)-2-{4-[1-(2-ethoxycarbonyl-ethyl)-piperidin-4-yl]-piperazin-1-yl}-1-(4-hydroxy-3,5-dimethyl-benzyl)-2-oxo-ethyl ester |
| (577) | 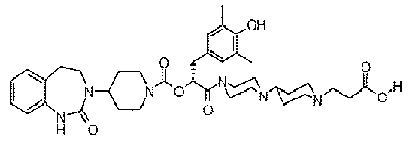 | 4-(2-Oxo-1,2,4,5-tetrahydro-1,3-benzdiazepin-3-yl)-piperidin-1-carbonsäure (R)-2-{4-[1-(2-carboxy-ethyl)-piperidin-4-yl]-piperazin-1-yl}-1-(4-hydroxy-3,5-dimethyl-benzyl)-2-oxo-ethyl ester |
| (578) | 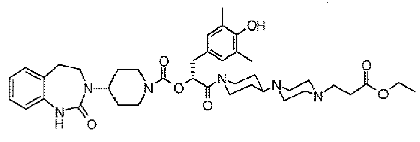 | 4-(2-Oxo-1,2,4,5-tetrahydro-1,3-benzdiazepin-3-yl)-piperidin-1-carbonsäure (R)-2-{4-[4-(2-ethoxycarbonyl-ethyl)-piperazin-1-yl]-piperidin-1-yl}-1-(4-hydroxy-3,5-dimethyl-benzyl)-2-oxo-ethyl ester |
| (579) | 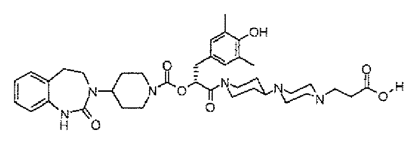 | 4-(2-Oxo-1,2,4,5-tetrahydro-1,3-benzdiazepin-3-yl)-piperidin-1-carbonsäure (R)-2-{4-[4-(2-carboxy-ethyl)-piperazin-1-yl]-piperidin-1-yl}-1-(4-hydroxy-3,5-dimethyl-benzyl)-2-oxo-ethyl ester |
| (580) | 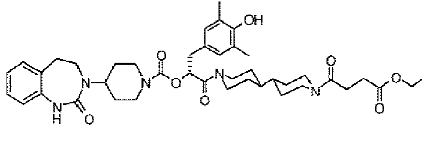 | 4-(2-Oxo-1,2,4,5-tetrahydro-1,3-benzdiazepin-3-yl)-piperidin-1-carbensäure (R)-2-[1'-(3-ethoxycarbonyl-propionyl)-4,4'-bipiperidinyl-1-yl]-1-(4-hydroxy-3,5-dimethyl-benzyl)-2-oxo-ethyl ester |
| (581) | 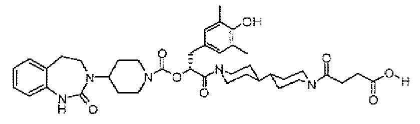 | 4-(2-Oxo-1,2,4,b-tetrahydro-1,3-benzdiazepin-3-yl)-piperidin-1-carbonsäure (R)-2-[1'-(3-carboxy-propionyl)-4,4'-bipiperdinyl-1-yl]-1-(4- hydroxy-3,5-dimethyl-benzyl)-2-oxo-ethyl ester |
| (582) | 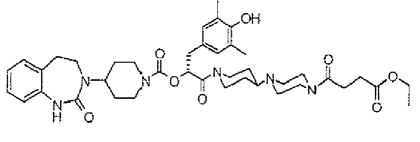 | 4-(2-Oxo-1,2,4,5-tetrahydro-1,3-benzdiazepin-3-yl)-piperidin-1-carbonsäure (R)-2-{4-[4-(3-ethoxycarbonyl-propionyl)-piperazin-1-yl]-piperidin-1-yl}-1-(4-hydroxy-3,5-dimethyl-benzyl)-2-oxo-ethyl ester |
| (583) | 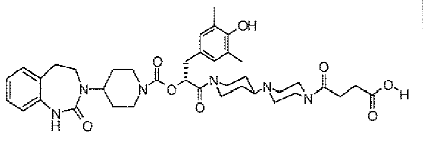 | 4-(2-Oxo-1,2,4,5-tetrahydro-1,3-benzdiazepin-3-yl)-piperidin-1-carbonsäure (R)-2-{4-[4-(3-carboxy-propionyl)-piperazin-1-yl]-piperidin-1-yl}-1-(4-hydroxy-3,5-dimethyl-benzyl)-2-oxo-ethyl ester |
| (584) | 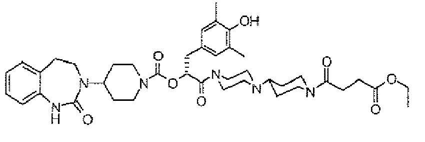 | 4-(2-Oxo-1,2,4,5-tetrahydro-1,3-benzdiazepin-3-yl)-piperidin-1-carbonsäure (R'-2-{4-[1-(3-ethoxycarbonyl-propionyl)-piperidin-4-yl]-piperazin-1-yl}-1-(4-hydroxy-3,5-dimethyl-benzyl)-2-oxo-ethyl ester |
| (585) | 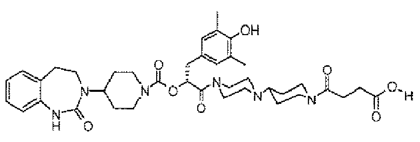 | 4-(2-Oxo-1,2,4,5-tetrahydro-1,3-benzdiazepin-3-yl)-piperidin-1-carbonsäure (R)-2-(4-[1-(3-carboxy-propionyl)-piperidin-4-yl]-piperazin-1-yl}-1-(4-hydroxy-3,5-dimethyl-benzyl)-2-oxo-ethyl ester |
| (586) | 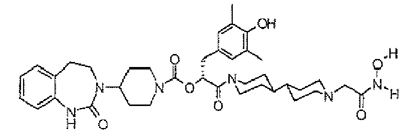 | 4-(2-Oxo-1,2,4,5-tetrahydro-1,3-benzdiazepin-3-yl)-piperidin-1-carbansäure (R)-2-(1'-hydroxycarbamoylmethyl-4,4'-bipiporidinyl-1-yl)-1-(4-hydroxy-3,5-dimethyl-benzyl)-2-oxo-ethyl ester |
| (587) | 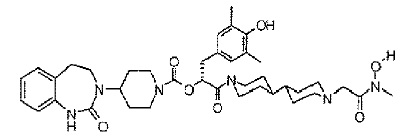 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-{1'-[(hydroxy-methyl-carbamoyl)-methyl]-4,4'-bipiperidinyl-1-yl}-2-oxo-ethyl ester |
| (588) | 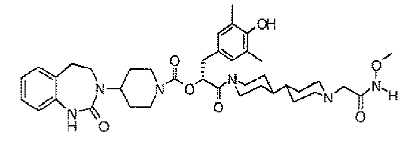 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[1'-(methoxycarbamoyl-methyl)-4,4'-bipiperidinyl-1-yl]-2-oxo-ethyl ester |
| (589) | 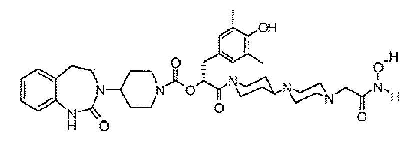 | 4-(2-Oxo-1,2,4,5-tetrahydro-1,3-benzdiazepin-3-yl)-piperidin-1-carbonsäure (R)-2-[4-(4-hydroxycarbamoylmethyl-piperazin-1-yl)-piperidin-1-yl]-1-(4-hydroxy-3,5-dimethyl-benzyl)-2-oxo-ethyl ester |
| (590) | 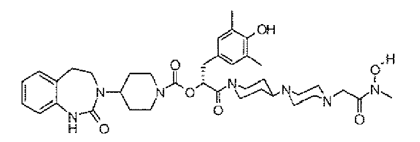 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-(4-{4-[(hydroxy-methyl-carbamoyl)-methyl]-piperazin-1-yl}-piperidin-1-yl)-2-oxo-ethyl ester |
| (591) | 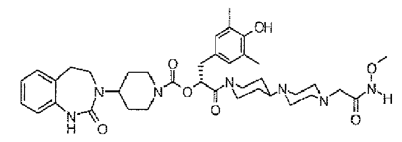 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-{4-[4-(methoxycarbamoyl-methyl)-piperazin-1-yl]-piperidin-1-yl}-2-oxo-ethyl ester |
| (592) | 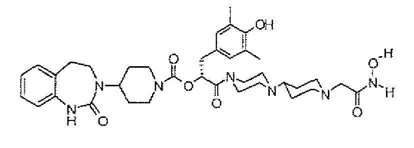 | 4-(2-Oxo-1,2,4,5-tetrahydro-1,3-benzdiazepin-3-yl)-piperidin-1-carbonsäure (R)-2-[4-(1-hydroxycarbamoylmethyl-piperidin-4-yl)-piperazin-1-yl]-1-(4-hydroxy-3,5-dimethyl-benzyl)-2-oxo-ethyl ester |
| (593) | 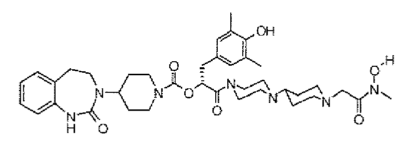 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-(4-{1-[(hydroxy-methyl-carbamoyl)-methyl]-piperidin-4-yl}-piperazin-1-yl)-2-oxo-ethyl ester |
| (594) | 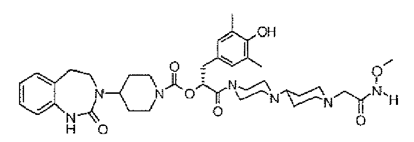 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-{4-[1-(methoxycarbamoyl-methyl)-piperidin-4-yl]-piperazin-1-yl}-2-oxo-ethyl ester |
| (595) | 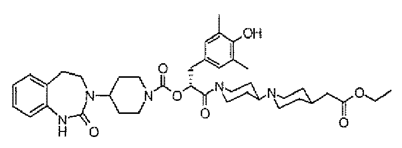 | 4-(2-Oxo-1,2,4,5-tetrahydro-1,3-benzdiazepin-3-yl)-piperidin-1-carbonsäure (R)-2-(4-ethoxycarbonylmethyl-1,4'-bipiperidinyl-1'-yl)-1-(4-hydroxy-3,5-dimethy-benzyl)-2-oxo-ethyl ester |
| (596) | 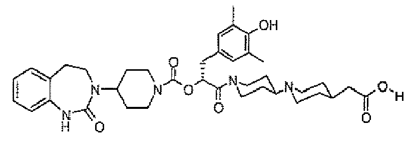 | 4-(2-Oxo-1,2,4,5-tetrahydro-1,3-benzdiazepin-3-yl)-piperidin-1-carbonsäure (R)-2-(4-carboxymethyl-1,4'-bipiperidinyl-1'-yl)-1-(4-hydroxy-3,5-dimethyl-benzyl)-2-oxo-ethyl ester |
| (597) | 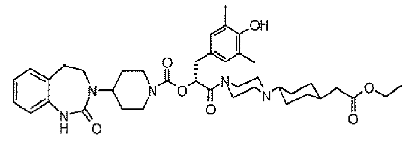 | trans-4-(2-Oxo-1,2,4,5-tetrahydro-1,3-benzdiazepin-3-yl)-piperidin-1-carbonsäure (R)-2-[4-(4-ethoxycarbonylmethyl-cyclohexyl)-piperazin-1-yl]-1-(4-hydroxy-3,5-dimethyl-benzyl)-2-oxo-ethyl ester |
| (598) | 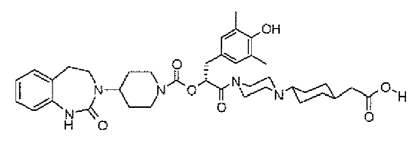 | trans-4-(2-Oxo-1,2,4,5-tetrahydro-1,3-benzdiazepin-3-yl)-piperidin-1-carbonsäure (R)-2-[4-(4-carboxymethyl-cyclohexyl)-piperazin-1-yl]-1-(4-hydroxy-3,5-dimethyl-benzyl)-2-oxo-ethyl ester |
| (599) | 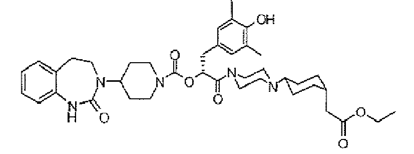 | cis-4-(2-Oxo-1,2,4,5-tetrahydro-1,3-benzdiazepin-3-yl)-piperidin-1-carbonsäure (R)-2-[4-(4-ethoxycarbonylmethyl-cyclohexyl)-piperazin-1-yl]-1-(4-hydroxy-3,5-dimethyl-benzyl)-2-oxo-ethyl ester |
| (600) | 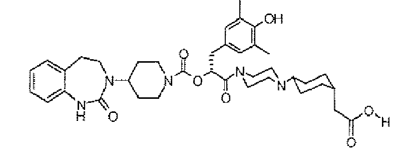 | cis-4-(2-Oxo-1,2,4,5-totrahydro-1,3-benzdiazepin-3-yl)-piperidin-1-carbonsäure (R)-2-[4-(4-carboxymethyl-cyclohexyl)-piperazin-1-yl]-1-(4-hydroxy-3,5-dimethyl-benzyl)-2-oxo-ethyl ester |
| (601) | 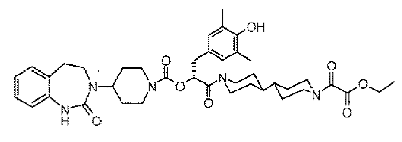 | 4-(2-Oxo-1,2,4,5-tetrahydro-1,3-benzdiazepin-3-yl)-piperidin-1-carbonsäure (R)-2-(1'-ethoxyoxalyl-4,4'-bipiperidinyl-1-yl)-1-(4-hydroxy-3,5-dimethyl-benzyl)-2-oxo-ethyl ester |
| (602) | 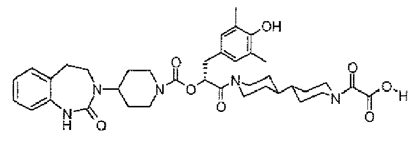 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-(1'-oxalyl-4,4'-bipiperidinyl-1-yl)-2-oxo-ethyl ester |
| (603) | 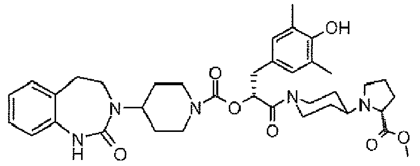 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-((S)-2-methoxycarbonyl-pyrrolidin-1-yl)-piperidin-1-yl]-2-oxo-ethyl ester |
| (604) | 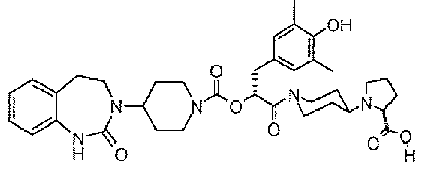 | 4-(2-Oxo-1,2,4,5-tetrahydro-1,3-benzdiazepin-3-yl)-piperidin-1-carbonsäure (R)-2-[4-((S)-2-carboxy-pyrrolidin-1-yl)-piperidin-1-yl]-1-(4-hydroxy-3,5-dimethyl-benzyl)-2-oxo-ethyl ester |
| (605) | 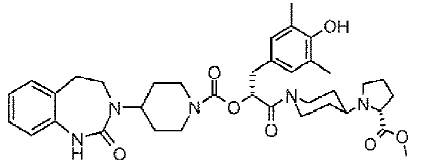 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-((R)-2-methoxycarbonyl-pyrrolidin-1-yl)-piperidin-1-yl]-2-oxo-ethyl ester |
| (606) | 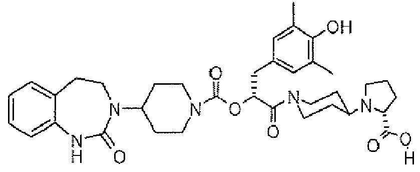 | 4-(2-Oxo-1,2,4,5-tetrahydro-1,3-benzdiazepin-3-yl)-piporidin-1-carbonsäure (R)-2-[4-((R)-2-carboxy-pyrrolidin-1-yi)-piperidin-1-yl]-1-(4-hydroxy-3,5-dimethyl-benzyl)-2-oxo-ethyl ester |
| (607) | 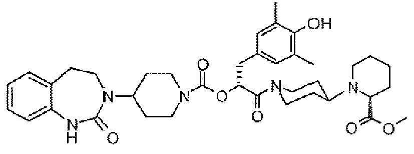 | (S)-1'-{(R)-3-(4-Hydroxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-2-carbonsäure methyl ester |
| (608) | 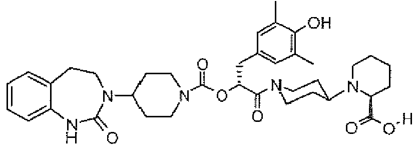 | (S)-1'-{(R)-3-(4-Hydroxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-2-carbonsäure |
| (609) | 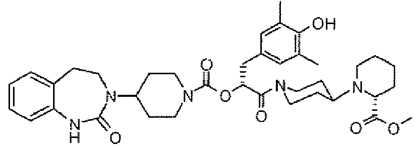 | (R)-1'-{(R)-3-(4-Hydroxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-2-carbonsäure methyl ester |
| (610) | 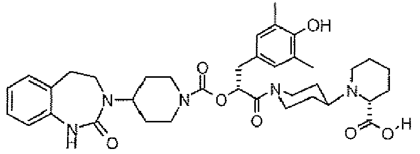 | (R)-1'-{(R)-3-(4-Hydroxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-2-carbonsäure |
| (611) | 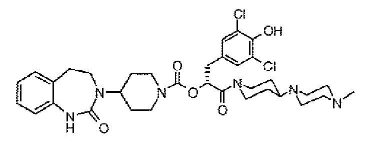 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-[4-(4-methyl-piperazin-1-yl)-piperidin-1-yl]-2-oxo-ethyl ester |
| (612) | 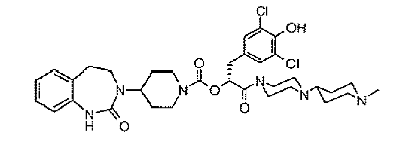 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-[4-(1-methyl-piperidin-4-yl)-piperazin-1-yl]-2-oxo-ethyl ester |
| (613) | 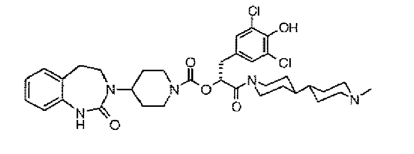 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-(1'-methyl-4,4'-bipiperidinyl-1-yl)-2-oxo-ethyl ester |
| (614) | 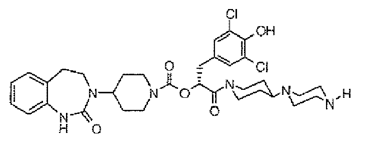 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-oxo-2-(4-piporazin-1-yl-piperidin-1-yl)-ethyl ester |
| (615) | 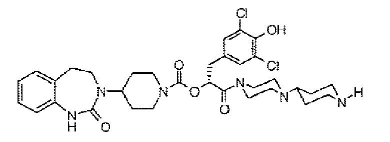 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-oxo-2-(4-piperidin-4-yl-piperazin-1-yl)-ethyl ester |
| (616) | 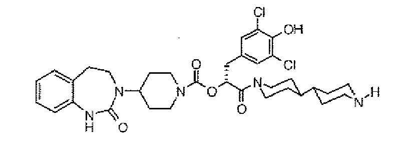 | 4-(2-Oxo-1,2,4,5-tetrahydro-1,3-benzdiazopin-3-yl)-piperidin-1-carbonsäure-(R)-2-4,4'-bipiperidinyl-1-yl-1-(3,5-dichlor-4-hydroxy-benzyl)-2-oxo-ethyl ester |
| (617) | 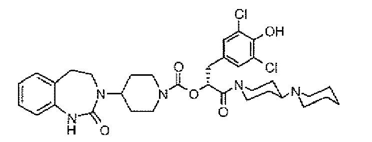 | 4-(2-Oxo-1,2,4,5-tetrahydro-1,3-benzdiazepin-3-yl)-piperidin-1-carbonsäure-(R)-2-1,4'-bipiperidinyl-1'-yl-1-(3,5-dichlor-4-hydroxy-benzyl)-2-oxo-ethyl ester |
| (618) | 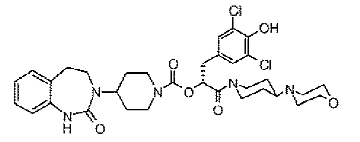 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-(4-morpholin-4-yl-piperidin-1-yl)-2-oxo-ethyl ester |
| (619) | 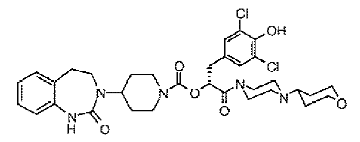 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-oxo-2-[4-(tetrahydro-pyran-4-yl)-piporazin-1-yl]-ethyl ester |
| (620) | 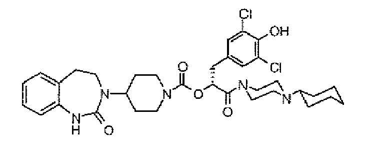 | 4-(2-Oxo-1,2,4,5-tetrahydro-1,3-benzdiazepin-3-yl)-piperidin-1-carbonsäure-(R)-2-(4-cyclohexyl-piperazin-1-yl)-1-(3,5-dichlor-4-hydroxy-benzyl)-2-oxo-ethyl ester |
| (621) | 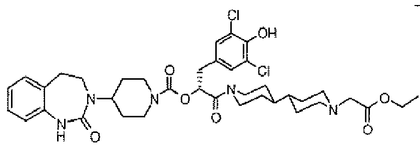 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-(1'-ethoxycarbonylmethyl-4,4'-bipiperidinyl-1-yl)-2-oxo-ethyl ester |
| (622) | 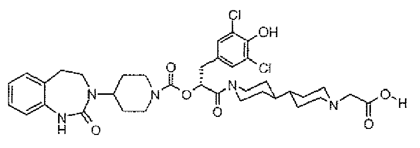 | 4-(2-Oxo-1,2,4,5-tetrahydro-1,3-benzdiazepin-3-yl)-piperidin-1-carbonsäure-(R)-2-(1'-carboxymethyl-4,4'-bipiperidinyl-1-yl)-1-(3,5-dichlor-4-hydroxy-benzyl)-2-oxo-ethyl ester |
| (623) | 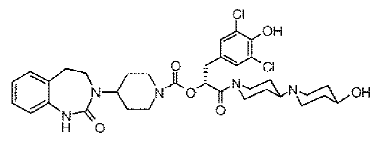 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-(4-hydroxy-1,4'-bipiperidinyl-1'-yl)-2-oxo-ethyl ester |
| (624) | 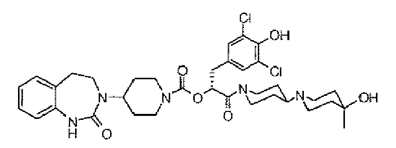 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-(4-hydroxy-4-methyl-1,4'-bipiperidinyl-1'-yl)-2-oxo-ethyl ester |
| (625) | 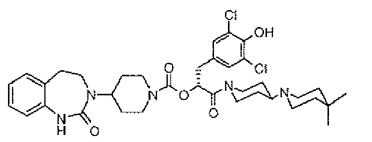 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-(4,4-dimethyl-1,4'-bipiperidinyl-1'-yl)-2-oxo-ethyl ester |
| (626) | 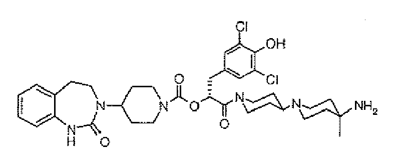 | 4-(2-Oxo-1,2,4,5-tetrahydro-1,3-benzdiazepin-3-yl)-piporidin-1-carbonsäure-(R)-2-(4-amino-4-methyl-1,4'-bipiperidinyl-1'-yl)-1-(3,5-dichlor-4-hydroxy-benzyl)-2-oxo-ethyl ester |
| (627) | 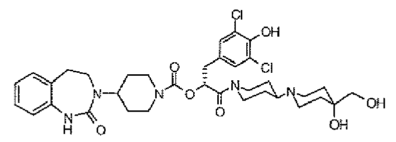 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-(4-hydroxy-4-hydroxymethyl-1,4'-bipiperidinyl-1'-yl)-2-oxo-ethyl ester |
| (628) | 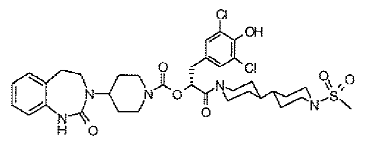 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-(1'-methansulfonyl-4,4'-bipiperidinyl-1-yl)-2-oxo-ethyl ester |
| (629) | 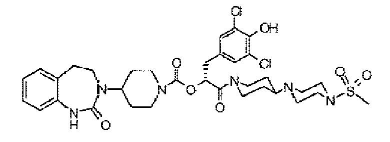 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-[4-(4-methan-sulfonyl-piperazin-1-yl)-piperidin-1-yl]-2-oxo-ethyl ester |
| (630) | 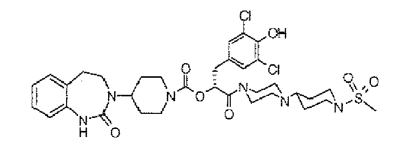 | 4-(2-Oxo-1,2,4,5-tetrahydro-1,3-benzdiazepin-3-yl)-piperidin-1-carbonsäure-(R)-1-(3,5-dichlor-4-hydroxy-benzyl)-2-[4-(1-methan-sulfonyl-piperidin-4-yl)-piperazin-1-yl]-2-oxo-ethyl ester |
| (631) | 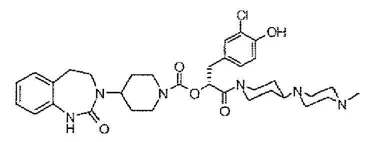 | 4-(2-Oxo-1,2,4,5-tetrahydro-1,3-benzdiazepin-3-yl)-piperidin-1-carbonsäure-(R)-1-(3-chlor-4-hydroxy-benzyl)-2-[4-(4-methyl-piperazin-1-yl)-piperidin-1-yl]-2-oxo-ethyl ester |
| (632) | 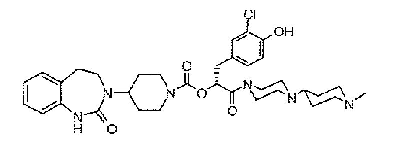 | 4-(2-Oxo-1,2,4,5-tetrahydro-1,3-benzdiazepin-3-yl)-piperidin-1-carbonsäure-(R)-1-(3-chlor-4-hydroxy-benzyl)-2-[4-(1 -methyl-piperidin-4-yl)-piperazin-1-yl]-2-oxo-ethyl ester |
| (633) | 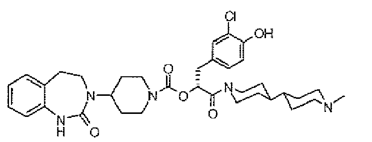 | 4-(2-Oxo-1,2,4,5-tetrahydro-1,3-benzdiazepin-3-yl)-piperidin-1-carbonsäure-(R)-1-(3-chlor-4-hydroxy-benzyl)-2-(1'-methyl-4,4'-bipiperidinyl-1-yl)-2-oxo-ethyl ester |
| (634) | 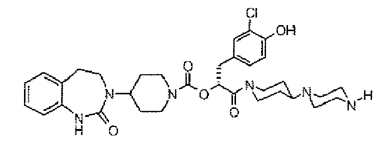 | 4-(2-Oxo-1,2,4,5-tetrahydro-1,3-benzdiazepin-3-yl)-piperidin-1-carbonsäure-(R)-1-(3-chlor-4-hydroxy-benzyl)-2-oxo-2-(4-piperazin-1-yl-piperidin-1-yl)-ethyl ester |
| (635) | 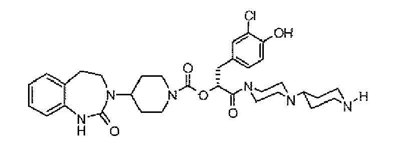 | 4-(2-Oxo-1,2,4,5-tetrahydro-1,3-benzdiazepin-3-yl)-piperidin-1-carbonsäure-(R)-1-(3-chlor-4-hydroxy-benzyl)-2-oxo-2-(4-piperidin-4-yl-piperazin-1-yl)-ethyl ester |
| (636) | 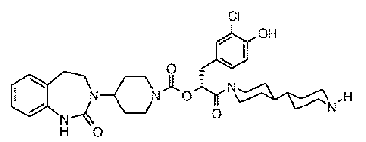 | 4-(2-Oxo-1,2,4,5-tetrahydro-1,3-benzdiazepin-3-yl)-piperidin-1-carbonsäure-(R)-2-4,4'-bipiperidinyl-1-yl-1-(3-chlor-4-hydroxy-benzyl)-2-oxo-ethyl ester |
| (637) | 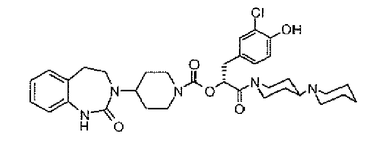 | 4-(2-Oxo-1,2,4,5-tetrahydro-1,3-benzdiazepin-3-yl)-piperidin-1-carbonsäure-(R)-2-1,4'-bipiperidinyl-1'-yl-1-(3-chlor-4-hydroxy-benzyl)-2-oxo-ethyl ester |
| (638) | 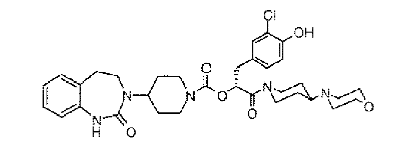 | 4-(2-Oxo-1,2,4,5-tetrahydro-1,3-benzdiazepin-3-yl)-piperidin-1-carbonsäure-(R)-1-(3-chlor-4-hydroxy-benzyl)-2-(4-morpholin-4-yl-piperidin-1-yl)-2-oxo-ethyl ester |
| (639) | 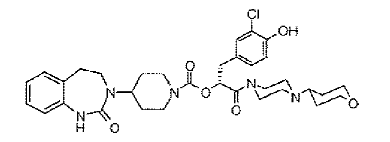 | 4-(2-Oxo-1,2,4,5-tetrahydro-1,3-benzdiazepin-3-yl)-piperidin-1-carbonsäure-(R)-1-(3-chlor-4-hydroxy-benzyl)-2-oxo-2-[4-(tetrahydro-pyran-4-yl)-piperazin-1-yl]-ethyl ester |
| (640) | 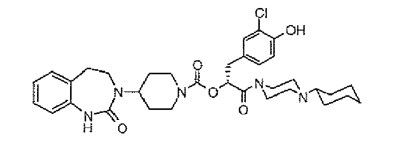 | 4-(2-Oxo-1,2,4,5-tetrahydro-1,3-benzdiazepin-3-yl)-piperidin-1-carbonsäure-(R)-1-(3-chlor-4-hydroxy-benzyl)-2-(4-cyclohexyl-piperazin-1-yl)-2-oxo-ethyl ester |
| (641) | 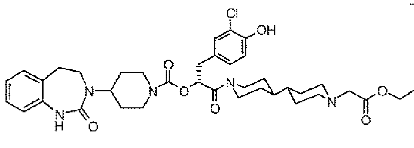 | 4-(2-Oxo-1,2,4,5-tetrahydro-1,3-benzdiazepin-3-yl)-piperidin-1-carbonsäure-(R)-1-(3-chlor-4-hydroxy-benzyl)-2-(1'-ethoxycarbonylmethyl-4,4'-bipiperidinyl-1-yl)-2-oxo-ethyl ester |
| (642) | 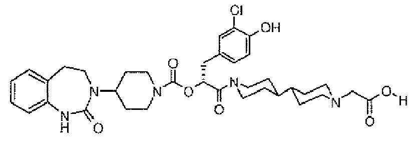 | 4-(2-Oxo-1,2,4,5-tetrahydro-1,3-benzdiazepin-3-yl)-piperidin-1-carbonsäure-(R)-2-('-carboxymethyl-4,4'-bipiperidinyl-1-yl)-1-(3-chlor-4-hydroxy-benzyl)-2-oxo-ethyl ester |
| (643) | 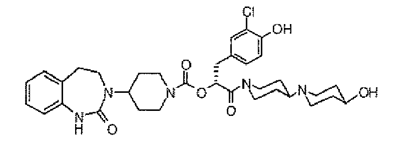 | 4-(2-Oxo-1,2,4,5-tetrahydro-1,3-benzdiazepin-3-yl)-piperidin-1-carbonsäure-(R)-1-(3-chlor-4-hydroxy-benzyl)-2-(4-hydroxy-1,4'-bipiperidinyl-1'-yl)-2-oxo-ethyl ester |
| (644) | 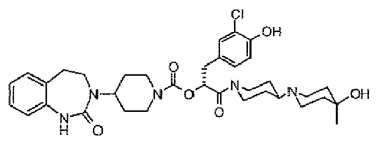 | 4-(2-Oxo-1,2,4,5-tetrahydro-1,3-benzdiazepin-3-yl)-piperidin-1-carbonsäure-(R)-1-(3-chlor-4-hydroxy-benzyl)-2-(4-hydroxy-4-methyl-1,4'-bipiperidinyl-1'-yl)-2-oxo-ethyl ester |
| (645) | 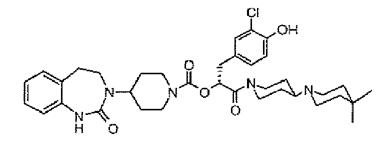 | 4-(2-Oxo-1,2,4,5-tetrahydro-1,3-benzdiazepin-3-yl)-piperidin-1-carbonsäure-(R)-1-(3-chlor-4-hydroxy-benzyl)-2-(4,4-dimethyl-1,4'-bipiperidinyl-1'-yl)-2-oxo-ethyl ester |
| (646) | 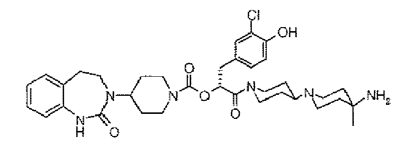 | 4-(2-Oxo-1,2,4,5-tetrahydro-1,3-benzdiazepin-3-yl)-piperidin-1-carbonsäure-(R)-2-(4-amino-4-methyl-1,4'-bipiperidinyl-1'-yl)-1-(3-chlor-4-hydroxy-benzyl)-2-oxo-ethyl ester |
| (647) | 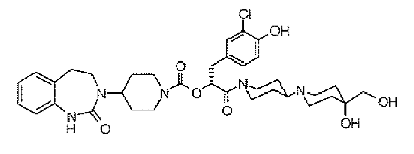 | 4-(2-Oxo-1,2,4,5-tetrahydro-1,3-benzdiazepin-3-yl)-piperidin-1-carbonsäure-(R)-1-(3-chlor-4-hydroxy-benzyl)-2-(4-hydroxy-4-hydroxymethyl-1,4'-bipiperidinyl-1'-yl)-2-oxo-ethyl ester |
| (648) | 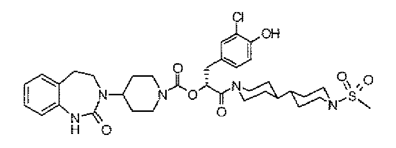 | 4-(2-Oxo-1,2,4,5-tetrahydro-1,3-benzdiazepin-3-yl)-piperidin-1-carbonsäure-(R)-1-(3-chlor-4-hydroxy-benzyl)-2-(1'-methansulfonyl-4,4'-bipiperidinyl-1-yl)-2-oxo-ethyl ester |
| (649) | 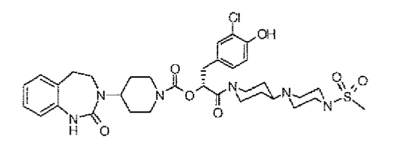 | 4-(2-Oxo-1,2,4,5-tetrahydro-1,3-benzdiazepin-3-yl)-piperidin-1-carbonsäure-(R)-1-(3-chlor-4-hydroxy-benzyl)-2-[4-(4-methansulfonyl-piperazin-1-yl)-piperidin-1-yl]-2-oxo-ethyl ester |
| (650) | 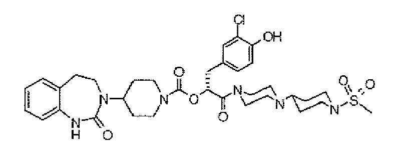 | 4-(2-Oxo-1,2,4,5-tetrahydro-1,3-benzdiazepin-3-yl)-piperidin-1-carbonsäure-(R)-1-(3-chlor-4-hydroxy-benzyl)-2-[4-(1-methansulfonyl-piperidin-4-yl)-piperazin-1-yl]-2-oxo-ethyl ester |
| (651) | 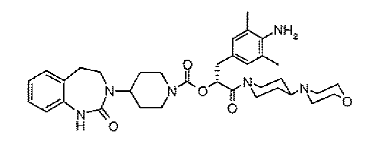 | 4-(2-Oxo-1,2,4,5-tetrahydro-1,3-benzdiazepin-3-yl)-piperidin-1-carbonsäure-(R)-1-(4-amino-3,5-dimethyl-benzyl)-2-(4-morpholin-4-yl-piperidin-1-yl)-2-oxo-ethyl ester |
| (652) | 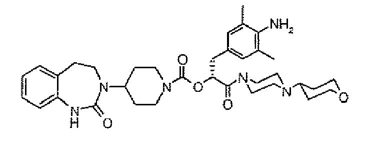 | 4-(2-Oxo-1,2,4,5-tetrahydro-1,3-benzdiazepin-3-yl)-piperidin-1-carbonsäure-(R)-1-(4-amino-3,5-dimethyl-benzyl)-2-oxo-2-[4-(tetrahydro-pyran-4-yl)-piperazin-1-yl]-ethyl ester |
| (653) | 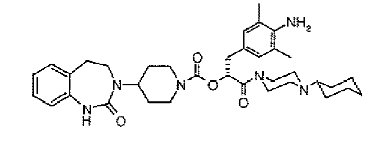 | 4-(2-Oxo-1,2,4,5-tetrahydro-1,3-benzdiazepin-3-yl)-piperidin-1-carbonsäure-(R)-1-(4-amino-3,5-dimethyl-benzyl)-2-(4-cyclohexyl-piperazin-1-yl)-2-oxo-ethyl ester |
| (654) | 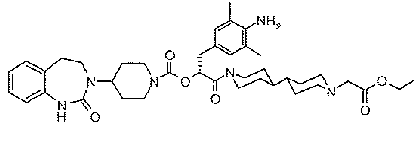 | 4-(2-Oxo-1,2,4,5-tetrahydro-1,3-benzdiazepin-3-yl)-piporidin-1-carbonsäure-(R)-1-(4-amino-3,5-dimethyl-benzyl)-2-(1'-ethoxycarbonylmethyl-4,4'-bipiperidinyl-1-yl)-2-oxo-ethyl ester |
| (655) | 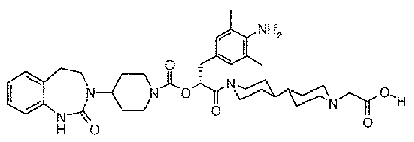 | 4-(2-Oxo-1,2,4,5-tetrahydro-1,3-benzdiazepin-3-yl)-piperidin-1-carbonsäure-(R)-1-(4-amino-3,5-dimethyl-benzyl)-2-(1'-carboxymethyl-4,4'-bipiperidinyl-1-yl)-2-oxo-ethyl ester |
| (656) | 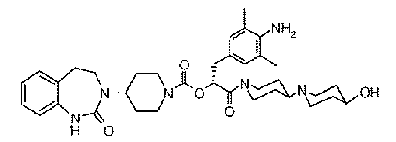 | 4-(2-Oxo-1,2,4,5-tetrahydro-1,3-benzdiazepin-3-yl)-piperidin-1-carbonsäure-(R)-1-(4-amino-3,5-dimethyl-benzyl)-2-(4-hydroxy-1,4'-bipiperidinyl-1'-yl)-2-oxo-ethyl ester |
| (657) | 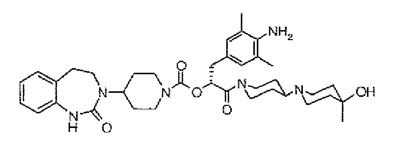 | 4-(2-Oxo-1,2,4,5-tetrahydro-1,3-benzdiazepin-3-yl)-piperidin-1-carbonsäure-(R)-1-(4-amino-3,5-dimethyl-benzyl)-2-(4-hydroxy-4-methyl-1,4'-bipiperidinyl-1'-yl)-2-oxo-ethyl ester |
| (658) | 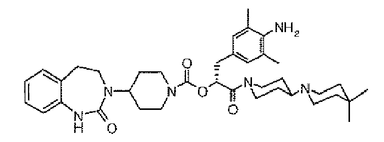 | 4-(2-Oxo-1,2,4,5-tetrahydro-1,3-benzdiazepin-3-yl)-piperidin-1-carbonsäure-(R)-1-(4-amino-3,5-dimethyl-benzyl)-2-(4,4-dimethyl-1,4'-bipiperidinyl-1'-yl)-2-oxo-ethyl ester |
| (659) | 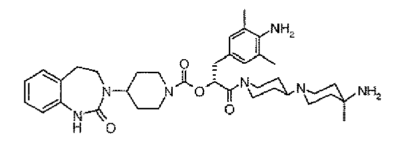 | 4-(2-Oxo-1,2,4,5-tetrahydro-1,3-benzdiazepin-3-yl)-piperidin-1-carbonsäure-(R)-1-(4-amino-3,5-dimethyl-benzyl)-2-(4-amino-4-methyl-1,4'-bipiperidinyl-1'-yl)-2-oxo-ethyl ester |
| (660) | 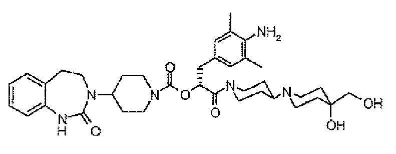 | 4-(2-Oxo-1,2,4,5-tetrahydro-1,3-benzdiazepin-3-yl)-piperidin-1-carbonsäure-(R)-1-(4-amino-3,5-dimethyl-benzyl)-2-(4-hydroxy-4-hydroxymethyl-1,4'-bipiperidinyl-1'-yl)-2-oxo-ethyl ester |
| (661) | 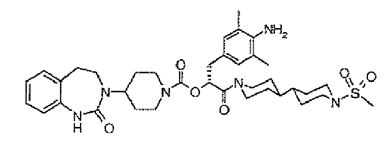 | 4-(2-Oxo-1,2,4,5-tetrahydro-1,3-benzdiazepin-3-yl)-piperidin-1-carbonsäure-(R)-1-(4-amino-3,5-dimethyl-benzyl)-2-(1'-methansulfonyl-4,4'-bipiperidinyl-1-yl)-2-oxo-ethyl ester |
| (662) | 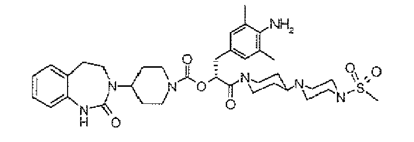 | 4-(2-Oxo-1,2,4,5-tetrahydro-1,3-benzdiazepin-3-yl)-piperidin-1-carbonsäure-(R)-1-(4-amino-3,5-dimethyl-benzyl)-2-[4-(4-methansulfonyl-piperazin-1-yl)-piperidin-1-yl]-2-oxo-ethyl ester |
| (663) | 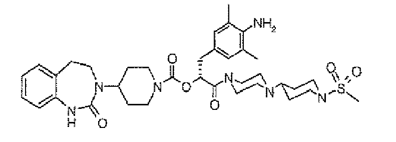 | 4-(2-Oxo-1,2,4,5-tetrahydro-1,3-benzdiazepin-3-yl)-piperidin-1-carbonsäure-(R)-1-(4-amino-3,5-dimethyl-benzyl)-2-[4-(1-methansulfonyl-piperidin-4-yl)-piperazin-1-yl]-2-oxo-ethyl ester |
| (664) | 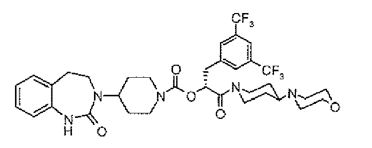 | 4-(2-Oxo-1,2,4,5-tetrahydro-1,3-benzdiazepin-3-yl)-piperidin-1-carbonsäure-(R)-1-(3,5-bis-trifluormethyl-benzyl)-2-(4-morpholin-4-yl-piperidin-1-yl)-2-oxo-ethyl ester |
| (665) | 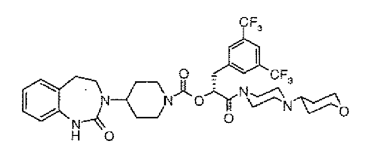 | 4-(2-Oxo-1,2,4,5-tetrahydro-1,3-benzdiazepin-3-yl)-piperidin-1-carbonsäure-(R)-1-(3,5-bis-trifluormethyl-benzyl)-2-oxo-2-[4-(tetrahydro-pyran-4-yl)-piperazin-1-yl]-ethyl ester |
| (666) | 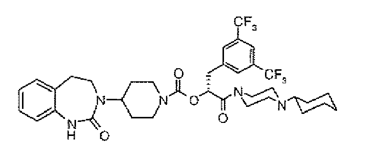 | 4-(2-Oxo-1,2,4,5-tetrahydro-1,3-benzdiazepin-3-yl)-piperidin-1-carbonsäure-(R)-1-(3,5-bis-trifluormethyl-benzyl)-2-(4-cyclohexyl-piperazin-1-yl)-2-oxo-ethyl ester |
| (667) | 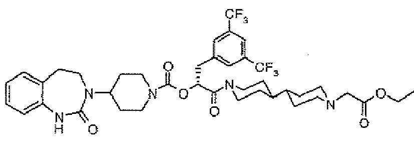 | 4-(2-Oxo-1,2,4,5-tetrahydro-1,3-berezdiazepin-3-yl)-piperidin-1-carbonsäure-(R)-1-(3,5-bis-trif luormethyl-benzyl)-2-(1'-ethoxycarbonylmethyl-4,4'-bipiperidinyl-1-yl)-2-oxo-ethyl ester |
| (668) | 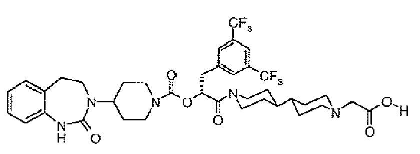 | 4-(2-Oxo-1,2,4,5-tetrahydro-1,3-benzdiazepin-3-yl)-piperidin-1-carbonsäure-(R)-1-(3,5-bis-trifluormethyl-benzyl)-2-(1'-carboxymethyl-4,4'-bipiperidinyl-1-yl)-2-oxo-ethyl ester |
| (669) | 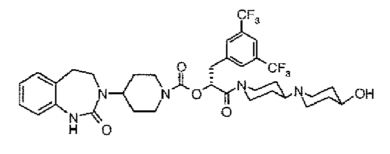 | 4-(2-Oxo-1,2,4,5-tetrahydro-1,3-benzdiazepin-3-yl)-piperidin-1-carbonsäure-(R)-1-(3,5-bis-trif luormethyl-benzyl)-2-(4-hydroxy-1,4'-bipiperidinyl-1'-yl)-2-oxo-ethyl ester |
| (670) | 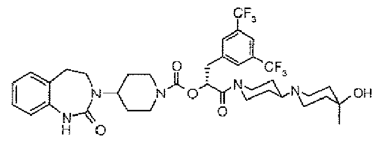 | 4-(2-Oxo-1,2,4,5-tetrahydro-1,3-benzdiazepin-3-yl)-piperidin-1-carbonsäure-(R)-1-(3,5-bis-trifluormethyl-benzyl)-2-(4-hydroxy-4-methyl-1,4'-bipiperidinyl-1'-yl)-2-oxo-ethyl ester |
| (671) | 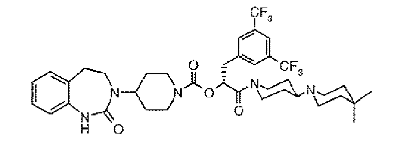 | 4-(2-Oxo-1,2,4,5-tetrahydro-1,3-benzdiazepin-3-yl)-piperidin-1-carbonsäure-(R)-1-(3,5-bis-trifluormethyl-benzyl)-2-(4,4-dimethyl-1,4'-bipiperidinyl-1'-yl)-2-oxo-ethyl ester |
| (672) | 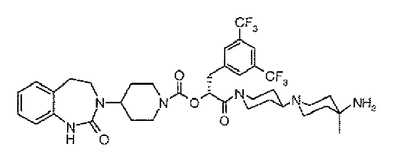 | 4-(2-Oxo-1,2,4,5-tetrahydro-1,3-benzdiazepin-3-yl)-piperidin-1-carbonsäure-(R)-2-(4-amino-4-methyl-1,4'-bipiperidinyl-1'-yl)-1-(3,5-bis-trifluormethyl-benzyl)-2-oxo-ethyl ester |
| (673) | 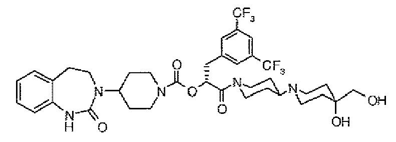 | 4-(2-Oxo-1,2,4,5-tetrahydro-1,3-benzdiazepin-3-yl)-piperidin-1-carbonsäure-(R)-1-(3,5-bis-trifluormethyl-benzyl)-2-(4-hydroxy-4-hydroxymethyl-1,4'-bipiperidinyl-1'-yl)-2-oxo-ethyl ester |
| (674) | 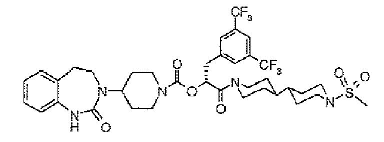 | 4-(2-Oxo-1,2,4,5-tetrahydro-1,3-benzdiazepin-3-yl)-piperidin-1-carbonsäure-(R)-1-(3,5-bis-trifluormethyl-benzyl)-2-(1'-methansulfonyl-4,4'-bipiporidinyl-1-yl)-2-oxo-ethyl ester |
| (675) | 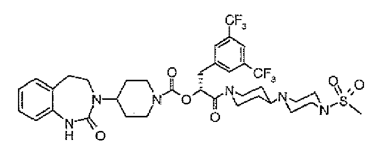 | 4-(2-Oxo-1,2,4,5-tetrahydro-1,3-benzdiazepin-3-yl)-piperidin-1-carbonsäure-(R)-1-(3,5-bis-trifluormethyl-benzyl)-2-[4-(4-methansulfonyl-piperazin-1-yl)-piperidin-1-yl]-2-oxo-ethyl ester |
| (676) | 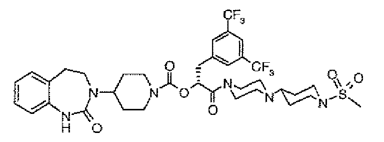 | 4-(2-Oxo-1,2,4,5-totrahydro-1,3-benzdiazepin-3-yl)-piperidin-1-carbonsäure-(R)-1-(3,5-bis-trifluormethyl-benzyl)-2-[4-(1-methansulfonyl-piperidin-4-yl)-piperazin-1-yl]-2-oxo-ethyl ester |
| (677) | 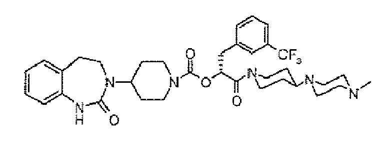 | 4-(2-Oxo-1,2,4,5-tetrahydro-1,3-benzdiazepin-3-yl)-piperidin-1-carbonsäure-(R)-2-[4-(4-methyl-piperazin-1-yl)-piperidin-1-yl]-2-oxo-1-(3-trifluormethyl-benzyl)-ethyl ester |
| (678) | 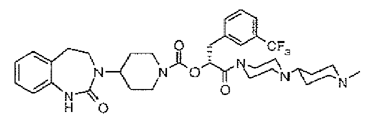 | 4-(2-Oxo-1,2,4,5-tetrahydro-1,3-benzdiazepin-3-yl)-piporidin-1-carbonsäure-(R)-2-[4-(1-mothyl-piporidin-4-yl)-piperazin-1-yl]-2-oxo-1-(3-trifluormethyl-benzyl)-ethyl ester |
| (679) | 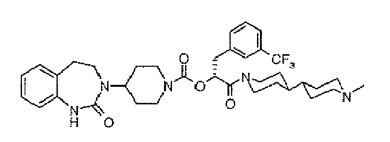 | 4-(2-Oxo-1,2,4,5-tetrahydro-1,3-benzdiazepin-3-yl)-piperidin-1-carbonsäure-(R)-2-(1'-methyl-4,4'-bipiperidinyl-1-yl)-2-oxo-1-(3-trifluormethyl-benzyl)-ethyl ester |
| (680) | 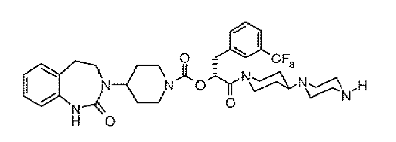 | 4-(2-Oxo-1,2,4,5-tetrahydro-1,3-benzdiazepin-3-yl)-piperidin-3-carbonsäure-(R)-2-oxo-2-(4-piperazin-1-yl-piperidin-1-yl)-1-(3-trifluormethyl-benzyl)-ethyl ester |
| (681) | 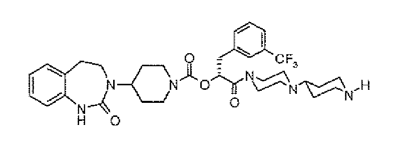 | 4-(2-Oxo-1,2,4,5-tetrahydro-1,3-benzdiazepin-3-yl)-piperidin-1-carbonsäure-(R)-2-oxo-2-(4-piperidin-4-yl-piperazin-1-yl)-1-(3-trifluormethyl-benzyl)-ethyl ester |
| (682) | 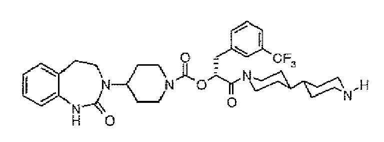 | 4-(2-Oxo-1,2,4,5-tetrahydro-1,3-benzdiazepin-3-yl)-piperidin-1-carbonsäure-(R)-2-4,4'-bipiperidinyl-1 -yl-2-oxo-1 -(3-trif luormethyl-benzyl)-ethyl ester |
| (683) | 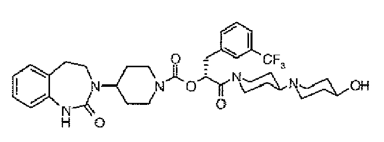 | 4-(2-Oxo-1,2,4,5-tetrahydro-1,3-benzdiazepin-3-yl)-piperidin-1-carbonsäure-(R)-2-(4-hydroxy-1,4'-bipiperidinyl-1'-yl)-2-oxo-1-(3-trifluormethyl-benzyl)-ethyl ester |
| (684) | 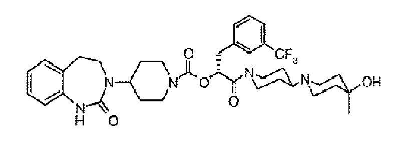 | 4-(2-Oxo-1,2,4,5-tetrahydro-1,3-benzdiazepin-3-yl)-piperidin-1-carbonsäure-(R)-2-(4-hydroxy-4-methyl-1,4'-bipiperidinyl-1'-yl)-2-oxo-1-(3-trifluormethyl-benzyl)-ethyl ester |
| (685) | 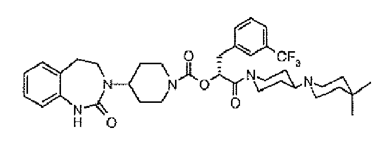 | 4-(2-Oxo-1,2,4,5-tetrahydro-1,3-benzdiazepin-3-yl)-piperidin-1-carbonsäure-(R)-2-(4,4-dimethyl-1,4'-bipiperidinyl-1'-yl)-2-oxo-1-(3-trifluormethyl-benzyl)-ethyl ester |
| (686) | 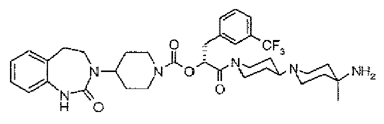 | 4-(2-Oxo-1,2,4,5-tetrahydro-1,3-benzdiazepin-3-yl)-piperidin-1-carbonsäure-(R)-2-(4-amino-4-methyl-1,4'-bipiperidinyl-1'-yl)-2-oxo-1-(3-trifluormethyl-benzyl)-ethyl ester |
| (687) | 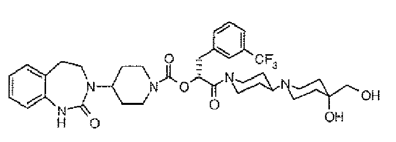 | 4-(2-Oxo-1,2,4,5-tetrahydro-1,3-benzdiazepin-3-yl)-piperidin-1-carbonsäure-(R)-2-(4-hydroxy-4-hydroxymethyl-1,4'-bipiperidinyl-1'-yl)-2-oxo-1-(3-trifluormethyl-benzyl)-ethyl ester |
| (688) | 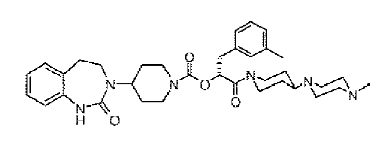 | 4-(2-Oxo-1,2,4,5-tetrahydro-1,3-benzdiazepin-3-yl)-piperidin-1-carbonsäure-(R)-1-(3-methyl-benzyl)-2-[4-(4-methyl-piperazin-1-yl)-piperidin-1-yl]-2-oxo-ethyl ester |
| (689) | 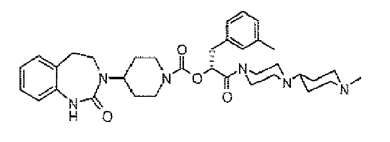 | 4-(2-Oxo-1,2,4,5-tetrahydro-1,3-benzdiazepin-3-yl)-piperidin-1-carbonsäure-(R)-1-(3-methyl-benzyl)-2-[4-(1-methyl-piporidin-4-yl)-piperazin-1-yl]-2-oxo-ethyl ester |
| (690) | 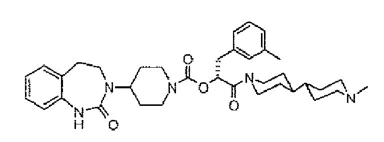 | 4-(2-Oxo-1,2,4,5-tetrahydro-1,3-benzdiazepin-3-yl)-piperidin-1-carbonsäure-(R)-1-(3-methyl-benzyl)-2-(1'-methyl-4,4'-bipiperidinyl-1-yl)-2-oxo-ethyl ester |
| (691) | 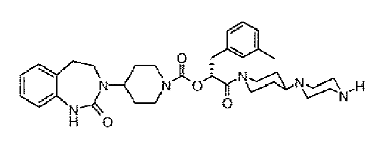 | 4-(2-Oxo-1,2,4,5-tetrahydro-1,3-benzdiazepin-3-yl)-piperidin-1-carbonsäure-(R)-1-(3-methyl-benzyl)-2-oxo-2-(4-piperazin-1-yl-piperidin-1-yl)-ethyl ester |
| (692) | 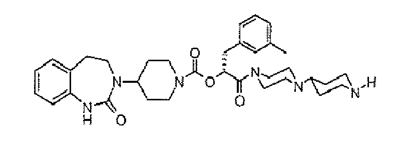 | 4-(2-Oxo-1,2,4,5-tetrahydro-1,3-benzdiazepin-3-yl)-piperidin-1-carbonsäure-(R)-1-(3-methyl-benzyl)-2-oxo-2-(4-piperidin-4-yl-piperazin-1-yl)-ethyl ester |
| (693) | 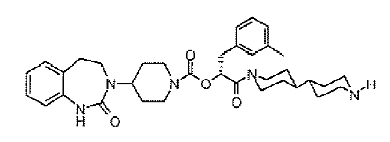 | 4-(2-Oxo-1,2,4,5-tetrahydro-1,3-benzdiazepin-3-yl)-piperidin-1-carbonsäure-(R)-2-4,4'-bipiperidinyl-1-yl-1-(3-methyl-benzyl)-2-oxo-ethyl ester |
| (694) | 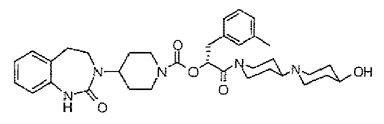 | 4-(2-Oxo-1,2,4,5-tetrahydro-1,3-benzdiazepin-3-yl)-piperidin-1-carbonsäure-(R)-2-(4-hydroxy-1,4'-bipiperidinyl-1'-yl)-1-(3-methyl-benzyl)-2-oxo-ethyl ester |
| (695) | 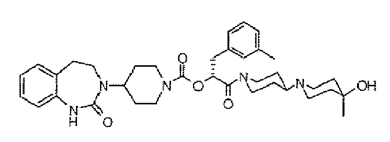 | 4-(2-Oxo-1,2,4,5-tetrahydro-1,3-benzdiazepin-3-yl)-piperidin-1-carbonsäure-(R)-2-(4-hydroxy-4-methyl-1,4'-bipiperidinyl-1'-yl)-1-(3-methyl-benzyl)-2-oxo-ethyl ester |
| (696) | 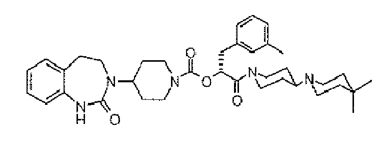 | 4-(2-Oxo-1,2,4,5-tetrahydro-1,3-benzdiazepin-3-yl)-piperidin-1-carbonsäure-(R)-2-(4,4-dimethyl-1,4'-bipiperidinyl-1'-yl)-1-(3-methyl-benzyl)-2-oxo-ethyl ester |
| (697) | 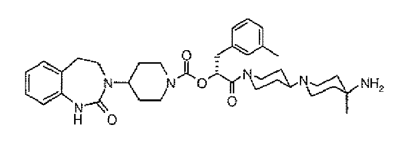 | 4-(2-Oxo-1,2,4,5-tetrahydro-1,3-benzdiazepin-3-yl)-piperidin-1-carbonsäure-(R)-2-(4-amino-4-methyl-1,4'-bipiperidinyl-1'-yl)-1-(3-methyl-benzyl)-2-oxo-ethyl ester |
| (698) | 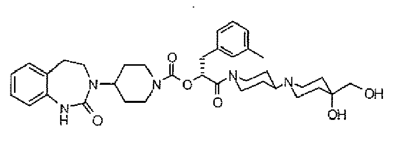 | 4-(2-Oxo-1,2,4,5-tetrahydro-1,3-benzdiazepin-3-yl)-piperidin-1-carbonsäure-(R)-2-(4-hydroxy-4-hydroxymethyl-1,4'-bipiperidinyl-1'-yl)-1-(3-methyl-benzyl)-2-oxo-ethyl ester |

deren Enantiomere, deren Diastereomere und deren Salze.

Die Verbindungen der allgemeinen Formel (I) werden nach prinzipiell bekannten Methoden hergestellt. Die folgenden Verfahren haben sich zur Herstellung der erfindungsgemäßen Verbindungen der allgemeinen Formel (I) besonders bewährt:
(a) Zur Herstellung von Verbindungen der allgemeinen Formel (I), in der alle Reste wie eingangs erwähnt definiert sind:
   Umsetzung von Piperidinen der allgemeinen Formel 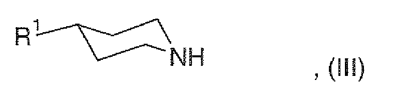
   in der R¹ wie eingangs erwähnt definiert ist,
   mit Kohlensäurederivaten der allgemeinen Formel 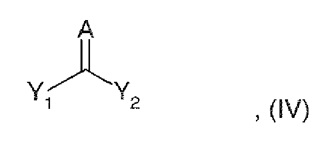
   in der Y₁ und Y₂ nucleofuge Gruppen bedeuten, die gleich oder verschieden sein können, bevorzugt das Chloratom, die *p*-Nitrophenoxy- oder Trichlormethoxygruppe, falls A das Sauerstoffatom darstellt, oder das Chloratom, falls A das Schwefelatom bedeutet,
   und mit Verbindungen der allgemeinen Formel 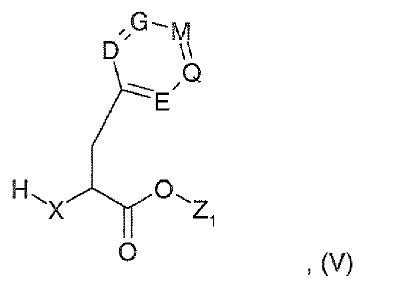
   in der X, D, E, G, M urad Q wie eingangs erwähnt definiert sind und Z₁ eine Schutzgruppe für eine Carboxygruppe darstellt, beispielsweise eine C₁₋₆-Alkyl- oder Benzylgruppe, wobei die Alkylgruppen linear oder verzweigt sein können und die Benzylgruppe durch ein oder zwei Methoxygruppen substituiert sein kann. Bevorzugt ist für Z₁ die Methyl-, Ethyl-, *tert*-Butyl oder Benzylgruppe. Vor Durchführung der Reaktion können im Rest R¹ einer Verbindung der Formel (III) und/oder in einer Verbindung der Formel (V) gegebenenfalls vorhandene Carbonsäurefunktionen, primäre oder sekundäre Aminofunktionen oder Hydroxyfunktionen durch übliche Schutzreste geschützt und gegebenenfalls verwendete Schutzreste nach Durchführung der Reaktion nach dem Fachmann geläufigen Methoden wieder abgespalten werden.
   In einer ersten Stufe werden die Verbindungen der allgemeinen Formen (III) in einem Lösungsmittel, beispielsweise in Dichlormethan, THF, Pyridin oder deren Mischungen, bei einer Ternperaturzwischen -20 bis 50°C in Gegenwart einer Base, beispielsweise Triethylamin, Pyridin oder Ethyldiisopropylamin, mit den Kohlensäurederivaten der allgemeinen Formel (IV) zur Reaktion gebracht. Die dabei entstehende Zwischenstufe kann aufgereinigt oder ohne Reinigung weiter umgesetzt werden.
   Die Umsetzung dieser Zwischenstufen mit Verbindungen der allgemeinen Formel (V) erfolgt ebenfalls in einem der oben genannten Lösungsmittel, und bei den oben genannten Temperaturen, in Gegenwart einer Base, wie Triethylamin oder Pyridin, mit oder ohne Zusatz eines Aktivierungsreagenz, wie z.B. 4-Dimethylaminopyridin. Zur Aktivierung können die Verbindungen der allgemeinen Formel (V) auch mittels eines Metallhydride, wie z.B. NaH oder KH, deprotoniert werden, wobei in diesem Fall auf die Gegenwart der Base oder des Aktivierungsreagenzes verzichtet werden kann.
(b) Zur Herstellung von Verbindungen der allgemeinen Formel (I), in der alle Reste wie eingangs erwähnt definiert sind:
   Kupplung einer Carbonsäure der allgemeinen Formel 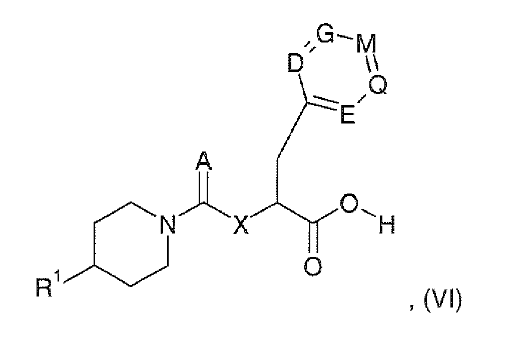
   in der alle Reste wie eingangs erwähnt definiert sind, mit einem Amin der allgemeinen Formel HNR²R³, in der R² und R³ wie eingangs definiert sind. Vor Durchführung der Reaktion können in einer Verbindung der Formel (VI) und/oder in den Resten R² und R³ des Amins der Formel HNR²R³ gegebenenfalls vorhandene Carbonsäurefunktionen, primäre oder sekundäre Aminofunktionen oder Hydroxyfunktionen durch übliche Schutzreste geschützt und gegebenenfalls verwendete Schutzreste nach Durchführung der Reaktion nach dem Fachmann geläufigen Methoden wieder abgespalten werden.
      Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z. B. Dicyclohexylcarbodiimid (DCC), Diisopropylcarbodiimid (DIC) oder Ethyl-(3-dimethylamino-propyl)-carbodiimid, O-(1*H*-Benzotriazol-1-yl)-*N,N-N,N'*-tetramethyluronium-hexa-fluorphosphat (HBTU) oder -tetrafluorborat (TBTU) oder 1 H-Benzotriazol-1-yl-oxy-tris-(dimethylamino)-phosphoniumhexafluorphosphat (BOP) eingesetzt werden. Durch Zugabe von 1-Hydroxybenzotriazol (HOBt) oder von 3-Hydroxy-4-oxo-3,4-dihydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösungsmitteln wie Dichlormethan, Tetrahydrofuran, Acetonitril, Dimethylformamid (DMF), Dimethylacetamid (DMA), *N*-Methylpyrrolidon (NMP) oder Gemischen aus diesen und bei Temperaturen zwischen -30 und +30°C, bevorzugt -20 und +25°C, durchgeführt. Sofern erforderlich wird als zusätzliche Hilfsbase *N*-Ethyldiisopropylamin (Hünig-Base) bevorzugt.
   Als weiteres Kupplungsverfahren zur Synthese von Verbindungen der allgemeinen Formel (I) wird das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J. Amer. Chem.Soc. 73, 3547 (1951)), bei der unter Verwendung von Chlorkohlensäureisobutylester in Gegenwart von Basen, wie 4-Methylmorpholin oder 4-Ethylmorpholin, das gemischte Anhydrid aus der zu kuppelnden Carbonsäure der allgemeinen Formel (VIII) und dem Kohlensäure-monoisobutylester erhalten wird. Die Herstellung dieses gemischten Anhydrids und die Kupplung mit den Aminen der allgemeinen Formel HNR²R³ erfolgt im Eintopfverfahren, unter Verwendung der vorstehend genannten Lösungsmittel und bei Temperaturen zwischen -20°C und +25°C, bevorzugt zwischen 0°C und +25°C.
(c) Zur Herstellung von Verbindungen der allgemeinen Formel (I), in der alle Reste wie eingangs erwähnt definiert sind:
   Kupplung einer Verbindung der allgemeinen Formel 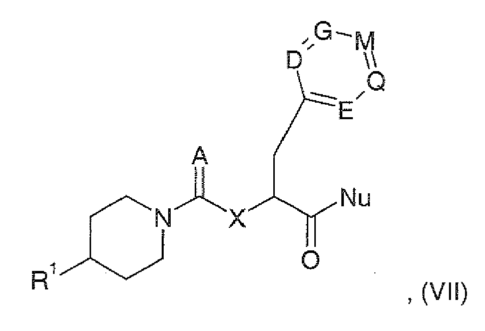
   mit einem Amin der allgemeinen Formel HNR²R³,
   in denen alle Reste wie eingangs erwähnt definiert sind und Nu eine Austrittsgruppe, beispielsweise ein Halogenatom, wie das Chlor-, Brom- oder lodatom, eine Alkylsulfonyloxygruppe mit 1 bis 10 Kohlenstoffatomen im Alkylteil, eine gegebenenfalls durch Chlor- oder Bromatome, durch Methyl- oder Nitrogruppen mono-, di- oder trisubstituierte Phenylsulfonyloxy- oder Naphthylsulfonyloxygruppe, wobei die Substituenten gleich oder verschieden sein können, eine 1*H*-Imidazol-1-yl-, eine gegebenenfalls durch eine oder zwei Methylgruppen im Kohlenstoffgerüst substituierte 1*H*-Pyrazol-1-yl-, eine 1*H*-1,2,4-Triazol-1-yl-, 1*H*-1,2,3-Triazol-1-yl-, 1*H-*1,2,3,4-Tetrazol-1-yl-, eine Vinyl-, Propargyl-, *p*-Nitrophenyl-, 2,4-Dinitrophenyl-, Trichlorphenyl-, Pentachlorphonyl-, Pentafluorphenyl-, Pyranyl- oder Pyridinyl-, eine Dimethylaminyloxy-, 2(1*H*)-Oxopyridin-1-yl-oxy-, 2,5-Dioxopyrrolidin-1-yloxy-, Phthalimidyloxy-, 1*H*-Benzo-triazol-1-yloxy- oder Azidgruppe bedeutet. Vor Durchführung der Reaktion können in einer Verbindung der Formel (VII) und/oder in den Resten R² und R³ des Amins der Formel HNR²R³ gegebenenfalls vorhandene Carbonsäurefunktionen, primäre oder sekundäre Aminofunktionen oder Hydroxyfunktionen durch übliche Schutzreste geschützt und gegebenenfalls verwendete Schutzreste nach Durchführung der Reaktion nach dem Fachmann geläufigen Methoden wieder abgespalten werden.

Die Umsetzung wird unter Schotten-Baumann- oder Einhorn-Bedingungen durchgeführt, das heißt, die Komponenten werden in Gegenwart von wenigstens einem Äquivalent einer Hilfsbase bei Temperaturen zwischen -50°C und +120°C, bevorzugt -10°C und +30°C, und gegebenenfalls in Gegenwart von Lösungsrrmitteln zur Reaktion gebracht. Als Hilfsbasen kommen bevorzugt Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Trimethylpyridin, Chinolin, Triethylamin, *N*-Ethyldiisopropylamin, *N*-Ethyldicyclohexylamin, 1,4-Di-azabicyclo[2,2,2]octan oder 1,8-Diaza-bicyclo[5,4,0]undec-7-en, als Lösungsmittel beispielsweise Dichlormethan, Tetrahydrofuran, 1,4-Dioxan, Acetonitril, Dimethylformamid, Dimethylacetamid, *N*-Methylpyrrolidon oder Gemische davon in Betracht; werden als Hilfsbasen Alkali-oder Erdalkalihydroxide, Alkalicarbonate oder -acetate verwendet, kann dem Reaktionsgemisch auch Wasser als Cosolvens zugesetzt werden.

Die erfindungsgemäßen neuen Verbindungen der allgemeinen Formel (I) enthalten ein oder mehrere Chiralitätszentren. Sind beispielsweise zwei Chiralitätszentren vorhanden, dann können die Verbindungen in Form zweier diastereomere Antipodenpaare auftreten. Die Erfindung umfaßt die einzelnen Isomeren ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel (I) fallenden Racematen gelingt beispielsweise durch HPLC an geeigneten chiralen stationären Phasen (z. B. Chiral AGP, Chlralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)- oder (-)-Camphersulfonsäure, bzw. einer optisch aktiven Base, beispielsweise mit (*R*)-(+)-1 - Phenylethylamin, (*S*)-(-)-1-Phenylethylamin oder (S)-Brucin, entstehen.

Nach einem üblichen Verfahren zur Isomerentrennung wird das Racemat einer Verbindung der allgemeinen Formel (I) mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Methanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)-oder (-)-Form erhalten.

Jeweils nur das (*R*)- oder (*S*)-Enantiomer bzw. ein Gemisch zweier optisch aktiver, unter die allgemeine Formel (I) fallender, diastereomerer Verbindungen wird auch dadurch erhalten, dass man die oben beschriebenen Synthesen mit jeweils einer geeigneten (*R*)- bzw. (*S*)-konfigurierten Reaktionskomponente durchführt.

Falls die Gruppe X in Verbindungen der allgemeinen Formel (V) das Sauerstoffatom darstellt, können die für die Synthese benötigten Hydroxycarbonsäuren der allgemeinen Formel 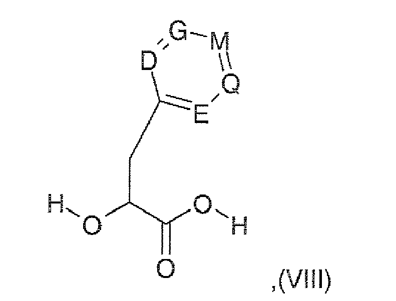 aus Verbindungen der allgemeinen Formel 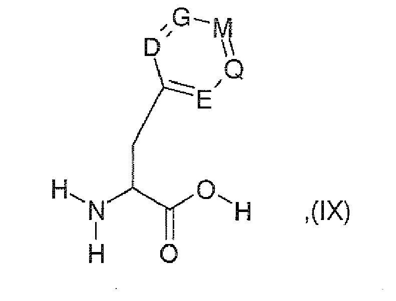 gewonnen werden, wobei D, E, G, M und Q in beiden Formeln wie eingangs erwähnt definiert sind.

Durch Diazotierung von Verbindungen der allgemeinen Formel (IX) mit einem geeignetem Diazotierungsreagenz, bevorzugt Natriumnitrit in sauren Milieu, können die Verbindungen der allgemeinen Formel (VIII) erhalten werden. Bei Einsatz enantiomerenreiner Verbindungen werden die entsprechenden enantiomerenreinen Hydroxycarbonsäureverbindungen erhalten, wobei die Reaktion unter Retention der Konfiguration abläuft.

Ein alternativer Zugang zu Verbindungen der allgemeinen Formel (VIII) besteht in der Umsetzung von Aldehyden der allgemeinen Formel (X) mit N-Acetylglycin in Acetanhydrid als Lösungsmittel in Gegenwart von Alkaliacetat, bevorzugt Natrium-oder Kaliumacetat bei geeigneter Temperatur, bevorzugt bei 80-130°C.

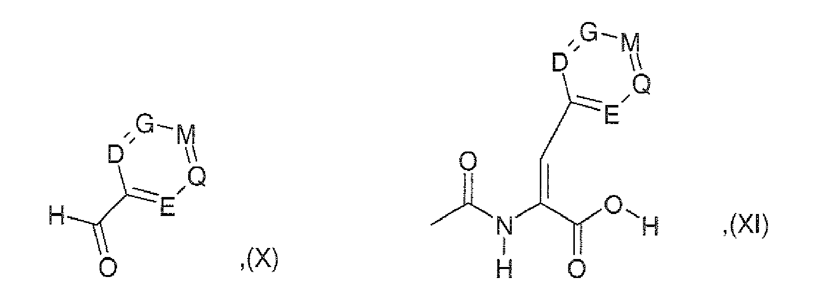

Die primär entstehenden Azlactone werden ohne Isolierung zu den Verbindungen der allgemeinen Formel (XI) hydrolysiert.
Durch weitere Umsetzung in Gegenwart von wässrigen Mineralsäuren, wie Schwefel-Phosphor- oder Chlorwasserstoffsäure, bevorzugt jedoch von Chlorwasserstoffsäure, werden Verbindungen der allgemeinen Formel (XII) erhalten. Diese werden dann mit geeigneten Reduktionsmitteln in die Verbindungen der allgemeinen Formel (VIII) überführt.

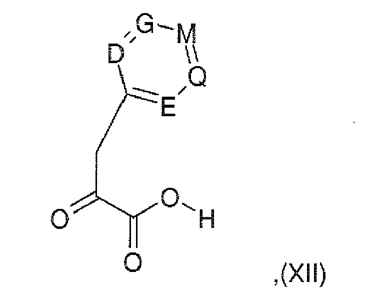

Als Reduktionsmittel können Alkaliborhydride, wie Natrium- oder Kaliumborhydrid verwendet werden. Weitere Reduktionsmittel stellen Chlordialkylborane, wie Chlordicyclohexylboran, dar. Werden chirale Chlordialkylborane, wie z.B. B-Chlordiisopinocampheylboran benutzt, können die Verbindungen der allgemeinen Formel (VIII) in enantiomerenreiner Form isoliert werden.

Ein weiterer Zugang zu Verbindungen der allgemeinen Formel (VIII) besteht in der Alkylierung der Verbindung (XIII) 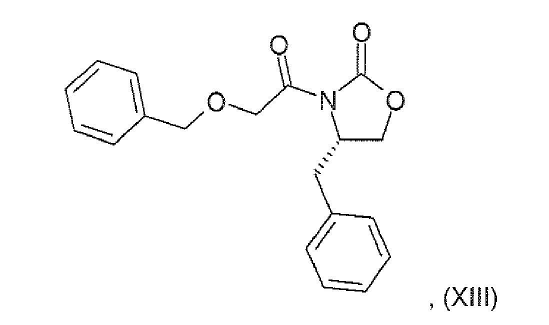 mit Aryl- oder Heteroaryl-methylhalogeniden der allgemeinen Formol 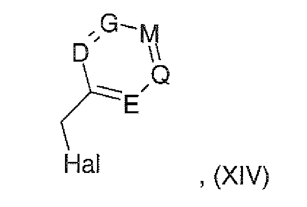 in der Hal ein Chlor-, Brom- oder Jodatom bedeutet, und D, E, G, Q und E wie eingangs erwähnt definiert sind, in Analogie zu literaturbekannten Methoden (Michael T. Crimmins, Kyle A. Emmitte und Jason D. Katz, Org. Lett. 2, 2165-2167 [2000]).
Die entstehenden diastereomeren Produkte können dann mit Hilfe physikochemischer Methoden, bevorzugt mit Hilfe chromatographischer Methode oder Umkristallisation, getrennt werden. Die hydrolytische Abspaltung des chiralen Auxiliars und Abspaltung der Benzylschutzgruppe eröffnet ebenfalls einen Zugang zu enantiomerenreinen Hydroxycarbonsäureverbindungen der allgemeinen Formel (V).
Die weitere Umsetzung von Verbindungen der allgemeinen Formel (VIII) zu Verbindungen der allgemeinen Formel (V) erfolgt im alkoholischen Milieu, bevorzugt in Ethanol oder Ethanol, in Gegenwart einer geeigneten Säure, wie Chlorwasserstoffsäure. Die Reaktion kann alternativ durch Umsetzung in alkoholischen Lösungsmittel bevorzugt Methanol, mit Thionychlorid erfolgen.

Falls die Gruppe X in Verbindungen der allgemeinen Formel (V) das Schwefelatom darstellt, können die für die Synthese benötigten Thiocarbonsäuren der allgemeinen Formel 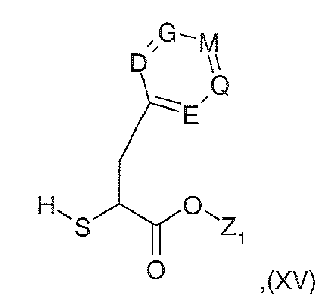 in der D, E, G, Mund Q wie eingangs erwähnt definiert sind und Z₁ eine wie unter Verfahren (a) beschriebene Schutzgruppe für eine Carboxygruppe darstellt, aus Verbindungen der allgemeinen Formel (V) gewonnen werden, in der X das Sauerstoffatom darstellt.

Durch eine Mitsunobu-Reaktion der Verbindungen der allgemeinen Formel (V) mit C₁-₆-Alkylthiocarbonsäuren, wobei die Alkylkette linear oder verzweigt sein kann diese bevorzugt jedoch die Methylgruppe darstellt, werden die entsprechenden Alkylthiocarbonsäureester dieser Verbindungen erhalten. Diese können nach bekannten Methoden zu den Verbindungen der allgemeinen Formel (XV) hydrolysiert werden (Bert Strijtveen und Richard M. Kellogg, J.Org. Chem. 51, 3664-3671 [1986]).

Alle Verbindungen der allgemeinen Formel (I), die primäre oder sekundäre Amino-, Hydroxy- oder Hydroxycarbonylfunktionen enthalten, werden bevorzugt aus mit Schutzgruppen versehenen Vorstufen gewonnen. Als Schutzgruppen für Aminofunktionen kommen beispielsweise eine Benzyloxycarbonyl-, 2-Nitro-benzyloxycarbonyl-, 4-Nitro-benzyloxycarbonyl-, 4-Methoxy-benzyloxycarbonyl-, 2-Chlor-benzyloxycarbonyl-, 3-Chlor-benzyloxycarbonyl-, 4-Chlor-benzyloxycarbonyl, 4-Biphenylyl-α,α-dimethyl-benzyloxycarbonyl- oder 3,5-Dimethoxy-α,α,-dimethyl-benzyloxycarbonylgruppe, eine Alkoxycarbonylgruppe mit insgesamt 1 bis 5 Kohlenstoffatomen im Alkylteil, beispielsweise die Methoxycarbonyl-, Ethoxycarbonyl-, *n*-Propoxycarbonyl-, Isopropoxycarbonyl-, *n*-Butoxycarbonyl-, 1-Methylpropoxycarbonyl-, 2-Methylpropoxy-carbonyl- oder *tert*-Butyloxycarbonylgruppe, die Allyloxycarbonyl-, 2,2,2-Trichlor-(1,1-dimethylethoxy)carbonyl- oder 9-Fluorenylmethoxycarbonylgruppe oder eine Formyl-, Acetyl- oder Trifluoracetylgruppe in Frage.
Als Schutzgruppe für Hydroxyfunktionen kommt beispielsweise eine Trimethylsilyl-, Triethylsilyl-, Triisopropyl-, *tert*-Butyldimethylsilyl- oder *tert*-Butyldiphenylsilylgruppe, eine *tert*-Butyl-, Benzyl-, 4-Methoxybenzyl- oder 3,4-Dimethoxybenzylgruppe in Frage.
Als Schutzgruppe für Hydroxycarbonylfunktionen kommt beispielsweise eine Alkylgruppe mit insgesamt 1 bis 5 Kohlenstoffatomen, beispielsweise die Methyl-, Ethyl-, *n*-Propyl-, Isopropyl-, *n*-Butyl-, *tert*-Butyl, Allyl-, 2,2,2-Trichlorethyl-, Benzyl-oder 4-Methoxybenzylgruppe in Frage.

Die erhaltenen Verbindungen der allgemeinen Formel (I) können, sofern sie geeignete basische Funktionen enthalten, insbesondere für pharmazeutische Anwendungen in ihre physiologisch verträglichen Salze mit anorganischen oder organischen Säuren übergeführt werden. Als Säuren kommen hierfür beispielsweise Salzsäure, Bromwasserstoffsäure, Phosphorsäure, Salpetersäure, Schwefelsäure, Methansulfonsäure, Ethansulfonsäure, Benzolsulfonsäure, *p*-Toluolsulfonsäure, Essigsäure, Fumarsäure, Bernsteinsäure, Milchsäure, Mandelsäure, Äpfelsäure, Zitronensäure, Weinsäure oder Maleinsäure in Betracht.

Außerdem lassen sich die neuen Verbindungen der Formel (I), falls sie Carbonsäurefunktion enthalten, in ihre Additionssalze mit anorganischen oder organischen Basen, insbesondere für die pharmazeutische Anwendung in ihre physiologisch verträglichen Additionssalze überführen. Als Basen kommen hierfür beispielsweise Natriumhydroxid, Kaliumhydroxid, Ammoniak, Cyclohexylamin, Dicyclohexylamin, Ethanolamin, Diethanolamin und Triethanolamin in Betracht.

Die vorliegende Erfindung betrifft Racemate, sofern die Verbindungen der allgemeinen Formel (I) nur ein Chiralitätselement besitzen. Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel (I) vorhanden ist, sowie die einzelnen optisch aktiven Enantiomeren, aus denen sich die erwähnten Racemate zusammensetzen.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Die neuen Verbindungen der allgemeinen Formel (I) und deren physiologisch verträgliche Salze weisen wertvolle pharmakologische Eigenschaften auf, die auf ihre selektiven CGRP-antagonistischen Eigenschaften zurückgehen. Ein weiterer Gegenstand der Erfindung sind diese Verbindungen enthaltende Arzneimittel, deren Verwendung und deren Herstellung.

Die voranstehend genannten neuen Verbindungen und deren physiologisch verträgliche Salze besitzen CGRP-antagonistische Eigenschaften und zeigen gute Affinitäten in CGRP-Fiezeptorbindungsstudien. Die Verbindungen weisen in den nachstehend beschriebenen pharmakologischen Testsystemen CGRP-antagonistische Eigenschaften auf.

Zum Nachweis der Affinität der voranstehend genannten Verbindungen zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC-Zellen werden in "Dulbecco's modified Eagle Medium" kultiviert. Das Medium konfluenter Kulturen wird entfernt. Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert. Nach Resuspension in 20 ml "Balanced Salts Solution" [BSS (in mM): NaCl 120, KCl 5.4, NaHCO₃ 16.2, MgSO₄ 0.8, NaHPO₄ 1.0, CaCl₂ 1.8, D-Glucose 5.5, HEPES 30, pH 7.40] werden die Zellen zweimal bei 100 x g zentrifugiert und in BSS resuspendiert. Nach Bestimmung der Zellzahl werden die Zellen mit Hilfe eines Ultra-Turrax homogenisiert und für 10 Minuten bei 3000 x g zentrifugiert. Der Überstand wird verworfen und das Pellet in Tris-Puffer (10 mM Tris, 50 mM NaCl, 5 mM MgCl₂, 1 mM EDTA, pH 7.40), angereichert mit 1% Rinderserum-Albumin und 0.1% Bacitracin, rezentrifugiert und resuspendiert (1 ml / 1000000 Zellen). Das Homogenat wird bei -80°C eingefroren. Die Membranpräparationen sind bei diesen Bedingungen für mehr als 6 Wochen stabil.

Nach Auftauen wird das Homogenat 1:10 mit Assay-Puffer (50 mM Tris, 150 mM NaCl, 5 mM MgCl₂, 1 mM EDTA, pH 7.40) verdünnt und 30 Sekunden lang mit einem Ultra-Turrax homogenisiert. 230 µl des Homogenats werden für 180 Minuten bei Raumtemperatur mit 50 pM ¹²⁵I-Iodotyrosyl-Calcitonin-Gene-Related Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM humanem CGRP-alpha während der Inkubation definiert.

Die Analyse der Konzentrations-Bindungskurven erfolgt mit Hilfe einer computergestützten nichtlinearen Kurvenanpassung.

Die eingangs erwähnten Verbindungen zeigen in dem beschriebenen Test IC₅₀-Werte ≤ 10000 nM.

### B. CGRP-Antagonismus in SK-N-MC-Zellen

SK-N-MC-Zellen (1 Mio. Zellen) werden zweimal mit 250 µl Inkubationspuffer (Hanks' HEPES, 1 mM 3-Isobutyl-1-methylxanthin, 1% BSA, pH 7.4) gewaschen und bei 37°C für 15 Minuten vorinkubiert. Nach Zugabe von CGRP (10 µl) als Agonist in steigenden Konzentrationen (10⁻¹¹ bis 10⁻⁶ M) bzw. zusätzlich von Substanz in 3 bis 4 verschiedenen Konzentrationen wird nochmals 15 Minuten inkubiert.

Intrazelluläres cAMP wird anschließend durch Zugabe von 20 µl 1M HCl und Zentrifugation (2000 x g, 4°C für 15 Minuten) extrahiert. Die Überstände werden in flüssigem Stickstoff eingefroren und bei -20°C gelagert.

Die cAMP-Gehalte der Proben werden mittels Radioimmunassay (Fa. Arnersham) bestimmt und die pA₂-Werte antagonistisch wirkender Substanzen graphisch ermittelt.

Die erfindungsgemäßen Verbindungen zeigen in dem beschriebenen *in vitro*-Testmodell CGRP-antagonistische Eigenschaften in einem Dosisbereich zwischen 10⁻¹² bis 10⁻⁵ M.

Aufgrund ihrer pharmakologischen Eigenschaften eignen sich die erfindungsgemäßen Verbindungen und deren Salze mit physiologisch verträgliche Säuren somit zur akuten und prophylaktischen Behandlung von Kopfschmerzen, insbesondere Migräne- bzw. Cluster-Kopfschmerz. Weiterhin beeinflussen die erfindungsgemäßen Verbindungen auch die folgenden Erkrankungen positiv:
nicht-insulinabhängigen Diabetes mellitus ("NIDDM"), complex regional pain syndrome (CRPS1), cardiovaskuläre Erkrankungen, Morphintoleranz, Clostritiumtoxin-bedingte Durchfallerkrankungen, Erkrankungen der Haut, insbesondere thermische und strahlenbedingte Hautschäden inklusive Sonnenbrand, entzündliche Erkrankungen, z.B. entzündliche Gelenkerkrankungen (Arthritis), neurogene Entzündungen der oralen Mucosa, entzündliche Lungenerkrankungen, allergische Rhinitis, Asthma, Erkrankungen, die mit einer überschießenden Gefäßerweiterung und dadurch bedingter verringerter Gewebedurchblutung einhergehen, z.B. Schock und Sepsis. Darüber hinaus zeigen die erfindungsgemäßen Verbindungen eine lindernde Wirkung auf Schmerzzustände im allgemeinen.
Die Symptomatik menopausaler, durch Gefäßerweiterung und erhöhten Blutfluß verursachter Hitzewallungen östrogendefizienter Frauen sowie hormonbehandelter Prostatakarzinompatienten wird durch die CGRP-Antagonisten der vorliegenden Anwendung präventiv und akut-therapeutisch günstig beeinflußt, wobei sich dieser Therapieansatz vor der Hormonsubstitution durch Nebenwirkungsarmut auszeichnet.

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0.01 bis 10 mg/kg Körpergewicht, vorzugsweise 0.1 bis 10 mg/kg Körpergewicht, jeweils 1 bis 3 x täglich.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Die erfindungsgemäß hergestellten Verbindungen können entweder alleine oder gegebenenfalls in Kombination mit anderen Wirksubstanzen zur Behandlung von Migräne intravenös, subkutan, intramuskulär, intrarektal, intranasal, durch Inhalation, transdermal oder oral erfolgen, wobei zur Inhalation insbesondere Aerosolformulierungen geeignet sind. Die Kombinationen können entweder simultan oder sequentiell verabreicht werden.

Als Kombinationspartner denkbare Wirkstoffklassen sind z.B. Antiemetica, Prokinetica, Neuroleptica, Antidepressiva, Neurokinin-Antagonisten, Angiotensin Rezeptor Blocker (Angiotensin II Antagonisten), iNOS Inhibitoren, AMPA Antagonisten, Anticonvulsiva, Histamin-H1-Rezeptorantagonisten, Antimuscarinika, β-Blocker, α-Agonisten und α-Antagonisten, Ergotalkaloiden, schwachen Analgetica, nichtsteroidalen Antiphlogistica, Corticosteroiden, Calcium-Antagonisten, 5-HT_{1B/1D}-Agonisten oder andere Antimigränemitteln, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Aceclofenac, Acemetacin, Acetylsalicylsäure, Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phonylbutazon, Piroxicam, Sulfasalazin, Zomepirac oder deren pharmazeutisch verträgliche Salze sowie Meloxicam und andere selektive COX2-Inhibitoren, wie beispielsweise Rafiecoxib und Celecoxib, in Betracht.

Weiterhin können Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Topiramat, Riboflavin, Montelukast, Lisinopril, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Lidocain oder Diitiazem und andere 5-HT_{1B/1D}-Agonisten wie z.B. Almotriptan, Avitriptan, Eietriptan, Frovatriptan, Naratriptan, Rizatriptan, Sumatriptan und Zolmitriptan verwendet werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatome durch Tritium, in RIA- und ELISA-Assays und als diagnostische bzw. analytische Hilfsmittel in der Neurotransmitter-Forschung.

### Experimenteller Teil

Für hergestellte Verbindungen liegen in der Regel IR-, ¹H-NMR und/oder Massenspektren vor.

Wenn nicht anders angegeben, werden R_{f}-Werte unter Verwendung von DC-Fertigplatten Kieselgel 60 F254 (E. Merck, Darmstadt, Artikel-Nr. 1.05714) ohne Kammersättigung bestimmt.
Die unter der Bezeichnung Polygram ermittelten R_{f}-Werte werden unter Verwendung von DC-Fertigfolien Polygram SIL G/UV₂₅₄ (beschichtet mit 0.2 mm Kieselgel) der Firma Macherey-Nagel (Düren, Artikel-Nr. 805 021) erhoben.
Die unter der Bezeichnung Polygram-Alox ermittelten R_{f}-Werte werden unter Verwendung von DC-Fertigfolien Polygram Alox N/UV₂₅₄ (beschichtet mit 0.2 mm Aluminiumoxid) der Firma Macherey-Nagel (Düren, Artikel-Nr. 802 021) erheben.
Die bei den Fliessmitteln angegebenen Verhältnisse beziehen sich auf Volumeneinheiten der jeweiligen Lösungsmittel. Die angegebenen Volumeneinheiten bei NH₃ beziehen sich auf eine konzentrierte Lösung von NH₃ in Wasser.
Soweit nicht anders vermerkt sind die bei den Aufarbeitungen der Reaktionslösungen verwendeten Säure-, Basen- und Salzlösungen wässrige Systeme der angebenen Konzentrationen.
Zu chromatographischen Reinigungen wird Kieselgel der Firma Millipore (MATREX^{™}, 35-70 µm) verwendet.
Zu chromatographischen Reinigungen wird Aluminiumoxid der Firma ICN Biomedicals (Eschwege, Artikelnummer 02090) verwendet. Gemäss Herstellerangaben wird vor Verwendung die benötigte Aktivitätsstufe erzeugt.
Die angegebenen HPLC-Daten werden unter nachstehend angeführten Parametern gemessen:

### Methode A:

| Zeit (min) | Volumenprozent Wasser (mit 0.1% Ameisensäure) | Volumenprozent Acetonitril (mit 0.1% Ameisensäure) |
|---|---|---|
| 0 | 95 | 5 |
| 9 | 10 | 90 |
| 10 | 10 | 90 |
| 11 | 95 | 5 |

Analytische Säule: Zorbax-Säule (Agilent Technologies), SB (Stable Bond) C18; 3.5 µm; 4.6 x 75 mm; Säulentemperatur: 30°C; Fluss: 0.8 mL / min; Injektionsvolumen: 5 µL; Detektion bei 254 nm

### Methode B:

| Zeit (min) | Volumenprozent Wasser (mit 0.1% Ameisensäure) | Volumenprozent Acetonitril (mit 0.1% Ameisensäure) |
|---|---|---|
| 0 | 95 | 5 |
| 9 | 10 | 90 |
| 10 | 10 | 90 |
| 11 | 95 | 5 |

Analytische Säule: Waters Symmetry C18; 3.5 µm; 4.6 x 75 mm; Säulentemperatur: 30°C; Fluss: 0.8 mL / min; Injektionsvolumen: 5 µL; Detektion bei 254 mm

### Methode C:

| Zeit (min) | Volumenprozent Wasser (mit 0.1% Ameisensäure) | Volumenprozent Acetonitril (mit 0.1% Ameisensäure) |
|---|---|---|
| 0 | 95 5 | |
| 8 | 50 | 50 |
| 9 | 10 | 90 |
| 10 | 10 | 90 |
| 11 | 90 | 10 |

Analytische Säule: Zorbax-Säule (Agilent Technologies), Bonus-RP C14; 3.5 µm; 4.6 x 75 mm; Säuientemperatur: 30°C; Fluss: 0.8 mL / min; Injektionsvolumen: 5 µL; Detektion bei 254 nm

### Methode D:

| Zeit (min) | Volumenprozent Wasser (mit 0.1% Ameisensäure) | Volumenprozent Acetonitril in (mit 0.1% Ameisensäure) |
|---|---|---|
| 0 | 95 | 5 |
| 8 | 50 | 50 |
| 9 | 10 | 90 |
| 10 | 10 | 90 |
| 11 | 90 | 10 |

Analytische Säule: Zorbax-Säule (Agilent Technologies), SB (Stable Bond) C18; 3.5 µm; 4.6 x 75 mm; Säulentemperatur: 30°C; Fluss: 0.8 mL / min; Injektionsvolumen- 5 µL; Detektion bei 254 nm

### Methode E:

| Zeit (min) | Volumenprozent Wasser (mit 0.1% Ameisensäure) | Volumenprozent Acetonitril (mit 0.1% Ameisensäure) |
|---|---|---|
| 0 | 95 | 5 |
| 4.5 | 10 | 90 |
| 5 | 10 | 90 |
| 5.5 | 90 | 10 |

Analytische Säule: Zorbax-Säule (Agilent Technologies), SB (Stable Bond) C18; 3.5 µm; 4.6 x 75 mm; Säufentemperatur: 30°C; Fluss: 1.6 mL / min; Injektionsvolumen: 5 µL; Detektion bei 254 nm

### Methode F:

| Zeit (min) | Volumenprozent Wasser (mit 0.04% Trifluoressigsäure) | Volumenprozent Acetonitril (mit 0.04% Trifluoressigsäure) |
|---|---|---|
| 0 | 80 | 20 |
| 30 | 20 | 80 |

Analytische Säule: Waters Symmetry C8; 5 µm; 4.6 x 150 mm; Säuientemperatur: 25°C; Fluss: 1.3 mL / min; Injektionsvolumen: 5 µL; Detektion bei 254 nm

Bei präparativen HPLC-Reinigungen werden in der Regel die gleichen Gradienten verwendet, die bei der Erhebung der analytischen HPLC-Daten benutzt wurden.
Die Sammlung der Produkte erfolgt massengesteuert, die Produkt enthaltenden Fraktionen werden vereinigt und gefriergetrocknet.
Falls nähere Angaben zur Konfiguration fehlen, bleibt offen, ob es sich um reine Enantiomere handelt oder ob partielle oder gar völlige Racemisierung eingetreten ist.
In den Versuchsbeschreibungen werden die folgenden Abkürzungen verwendet:
- Boc: *tert*-Butoxycarbonyl
- Cyc: Cyclohexan
- DCM: Dichlorethan
- DIPE: Diisopropylether
- DMF: *N*,*N*-Dimethylformamid
- EtOAc: Essigsäureethylester
- EtOH: Ethanol
- Fmoc: 9-Fluorenylmethoxycarbonyl
- halbkonz.: Halbkonzentriert
- HAT: O-(7-Azabenztriazol-1-yl)-*N*,*N*,*N*',*N*'-tetramethyluronium-hexafluorophosphat
- HCl: Salzsäure
- HOAc: Essigsäure
- HOBt: 1-Hydroxybenzotriazol-hydrat
- i. vac.: in vacuo (im Vakuum)
- KOCH: Kaliumhydroxid
- konz.: konzentriert
- LiOH: Lithiumhydroxid
- MeOH: Methanol
- NaOAc: Natriumacetat
- NaCl: Natriumchlorid
- NaOH: Natriumhydroxid
- n.e.: nicht erhoben
- PE: Petroether
- RT: Raumtemperatur
- TBME: *tert*-Butylmethylether
- TBTU: O-(Benzotriazol-1-yl)-*N*,*N*,*N*',*N*'-tetramethyluronium-tetrafluoroborat
- TFA: Trifluoressigsäure
- THF: Tetrahydrofuran

### Beispiel 1

### 4-(2-Oxo-1,2,4,5-tetrahydro-1,3-benzdiazepin-3-yl)-piperidin-1-carbonsäure-(R)-1-(3,4-dibrom-benzyl)-2-[4-(1-methyl-piperidin-4-yl)-piperazin-1-yl]-2-oxo-ethylester

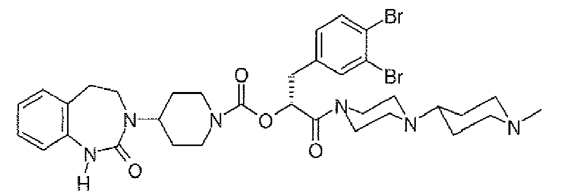

### 1a (Z, E)-2-Acotylamino-3-(3,4-dibrom-phenyl)-acryisäure

Eine Mischung aus 22.1 g (83.7 mmol) 3,4-Dibrombenzaldehyd, 14.7 g (128 mmol) N-Acetylglycin und 10.3 g (126 mmol) NaOAc in 100 mL Acetanhydrid wurden 1.5 h auf 118°C (Innentemperatur) erhitzt. Nach beendeter Umsetzung wurde das Reaktionsgemisch auf 100°C gekühlt und dann portionenweise mit 20 g Eis versetzt (exotherme Reaktion), wobei die Innentemperatur unter 120°C gehalten wurde. Das Reaktionsgemisch wurde weitere 2 h auf 95°C erhitzt, dann zu einer Mischung aus 240 mL Wasser und 120 mL Toluol gegeben und 1 h bei RT nachgerührt. Der Niederschlag wurde abgesaugt, mit jeweils 50 mL Toluol und Wasser nach gewaschen und über Nacht bei 40°C im Umlufttrockenschrank getrocknet.

| | |
|---|---|
| Ausbeute: | 20.8 g (69% der Theorie) |
| ESI-MS: | (M+H)⁺ = 362/364/366 (2 Br) |
| R_{f} = | 0.19 (Kieselgel, EtOAc/MeOH/NH₃ 90:10:1) |

### 1b 3-(3,4-Dibrom-phenyl)-2-oxo-propionsäure

Zu einer Lösung von 11.98 g (32.82 mol) (Z,E)-2-Acetylamino-3-(3,4-dibromphenyl)-acrylsäure in 90 mL N-Methyl-2-pyrrolidinon wurden 125 mL eisgekühlte 4 M HCl zugegeben und das Reaktionsgemisch anschliessend für 2 h unter Rückfluss erhitzt. Die auf 40°C gekühlte Reaktionslösung wurde auf 450 mL Wasser gegossen, die entstandene Suspension mit 300 mL Toluol versetzt und über Nacht nachgerührt. Die organische Phase wurde solange mit Wasser extrahiert bis sich zwischen den Phasen ein Niederschlag bildete. Dieser wurde abgesaugt, die Phasen getrennt, die Toluolphase zur Hälfte eingeengt, diese erneut mit Wasser versetzt und der entstandene Niederschlag abgesaugt. Dieser wurde dann mit dem ersten Niederschlag vereinigt und bei 50°C im Umlufttrockenschrank getrocknet.

| | |
|---|---|
| Ausbeute: | 5.73 g (54% der Theorie) |
| ESI-MS: | (M+H)⁺ = 319/321/323 (2 Br) |
| R_{f} = | 0.17 (Kieselgel , EtOAc/MeOH/NH₃ 80:20:2) |

### 1c (R)-3-(3,4-Dibrom-phonyl)-2-hydroxy-propionsäure

Zu einer auf -35°C gekühlten Lösung von 5.1 g (15.3 mmol) 3-(3,4-Dibrom-phenyl)-2-oxo-propionsäure und 2.2 mL (15.8 mmol) Triethylamin in 20 mL THF wurden innerhalb von 30 min eine Lösung von 6.1 g (19.0 mmol) (1R)-B-Chlordiisopinocampheylboran in 40 mL THF zugetropft und das Reaktionsgemisch 1 h bei dieser Temperatur gehalten. Die Reaktionslösung wurde vorsichtig mit 30 mL 1 M NaOH (exotherm) und 30 mL TBME versetzt, 15 min nachgerührt, die organische Phase abgetrennt, diese dann mit 25 mL Wasser und 15 mL 1 M NaOH extrahiert. Die vereinigten wässrigen Phasen wurden mit 2 M HCl sauer gestellt, dreimal mit jeweils 40 mL TBME extrahiert und die vereinigten organischen Phasen über Na₂SO₄ getrocknet. Nach Entfernen des Trockene- und Lösungsmittels wurde der Rückstand chromatographisch (Kieselgel, EtOAc/MeOH/NH₃ 70:30:3) gereinigt.

| | |
|---|---|
| Ausbeute: | 3.2 g (63% der Theorie) |
| ESI-MS: | (M+H)⁺ = 321/323/325 (2 Br) |
| Retentionszeit (HPLC): | 7.0 min (Methode A) |

### 1d (R)-3-(3,4-Dibrom-phenyl)-2-hydroxy-propionsäureethylester

Zu einer auf 0°C gekühlten Lösung von 3.2 g (9.9 mmol) (R)-3-(3,4-Dibrom-phenyl)-2-hydroxy-propionsäure in 40 mL trockenem EtOH wurden tropfenweise 0.8 mL (10.9 mmol) Thionylchlorid zugegeben und das Reaktionsgemisch 1 h bei RT nachgerührt. Die Reaktionslösung wurde i.vac. eingeengt, der Rückstand mit 30 mL DCM versetzt und vom unlösliche Niederschlag filtriert. Nach Entfernen des Lösungsmittels erhielt man das Produkt als zähes Öl, welches ohne Reinigung weiter umgesetzt wurde.

| | |
|---|---|
| Ausbeute: | 3.1 g (88% der Theorie) |
| ESI-MS: | (M+H)⁺ = 351/353/355 (2 Br) |
| Retentionszeit (HPLC): | 8.1 min (Methode A) |

1e 4-(2-Oxo-1,2,4,5-tetrahydro-1,3-benzdiazepin-3-yl)-piperidin-1-carbonylchlorid Zu einer auf 0°C gekühlten Lösung von 2.5 g (10.2 mmol) 3-Piperidin-4-yl-1,3,4,5-tetrahydro-1,3-benzdiazepin-2-on und 2.6 mL (14.9 mmol) Ethyldiisopropylamin in 75 mL DCM wurden 6.0 g (12.1 mmol) Phosgen (20 Gewichtsprozent in Toluoyl) zugegeben und das Reaktionsgemisch 30 min bei dieser Temperatur nachgerührt. Die Reaktionslösung wurde auf RT erwärmt, i.vac. auf ca. 50 mL eingeengt, über Kieselgel filtriert, dieses mit 200 mL DCM/EtOAc (1:1) gewaschen und die vereinigten organischen Filtrate erneut i.vac. eingeengt. Der Rückstand wurde mit DIPE verrührt, abgesaugt und i.vac. getrocknet.

| | |
|---|---|
| Ausbeute: | 2.4 g (77% der Theorie) |
| R_{f} = | 0.43 (Kieselgel, DCM/EtOAc 1:1) |

### 1f 4-(2-Oxo-1,2,4,5-tetrahydro-1,3-benzdiazepin-3-yl)-piperidin-1-carbonsäure-(R)-2-(3,4-dibrom-phenyl)-1-ethoxycarbonyl-ethylester

Zu einer auf 0°C gekühlten Lösung von 2.90 g (8.24 mol) (R)-3-(3,4-Dibromphenyl)-2-hydroxy-propionsäureethylester in 50 mL trockenem THF wurden 362 mg (55% in Mineralöl, 9.06 mol) NaH portionenweise zugegeben und weitere 30 min bei dieser Temperatur nachgerührt, wobei sich eine dunkelbraune Suspension bildete. Anschliessend wurden portionenweise unter Kühlung 2.15 g (6.99 mmol) 4-(2-Oxo-1,2,4,5-tetrahydro-1,3-benzdiazepin-3-yl)-piperidin-1-carbonylchlorid zugegeben und das Reaktionsgemisch 2 bei RT gerührt. Man versetzte mit 50 mL halbgesättigter NaHCO₃-Lösung, extrahierte zweimal mit jeweils 50 mL EtOAc, wusch die vereinigten organischen Phasen mit 50 mL gesättigter NaCl-Lösung und filtrierte die organische Phase über Na₂SO₄. Nach Entfernen des Lösungsmittels wurde der Rückstand chromatographisch (Kieselgel, EtOAc/Cyc 3:1) gereinigt.

| | |
|---|---|
| Ausbeute: | 3.64 g (84% der Theorie) |
| ESI-MS: | (M+H)⁺ = 622/624/626 (2 Br) |
| Retentionszeit (HPLC): | 10.0 min (Methode A) |

### 1g 4-(2-Oxo-1,2,4,5-tetrahydro-1,3-benzdiazepin-3-yl)-piperidin-1-carbonsäure-(R)-1 -carboxy-2-(3,4-dibrom-phenyl)-ethylester

Zu einer Lösung von 3.64 g (5.83 mmol) 4-(2-Oxo-1,2,4,5-tetrahydro-1,3-benzdiazepin-3-yl)-piperidin-1-carbonsäure-(R)-2-(3,4-dibrom-phenyl)-1-ethoxycarbonyl-ethylester in 70 mL THF wurde bei RT eine Lösung von 210 mg (9.0 mmol) LiOH*H₂O in 40 mL Wasser zugegeben und das Reaktionsgemisch 7 h bei RT gerührt. Man engte i.vac. ein, nahm den Rückstand in 100 mL Wasser auf, versetzte unter Rühren mit 1 M HCl bis zur sauren Reaktion, filtrierte den Niederschlag ab und trocknete diesen im Vakuumtrockenschrank bei 50°C. Das Produkt wurde ohne Reinigung weiter umgesetzt.

| | |
|---|---|
| Ausbeute: | 3.36 g (97% der Theorie) |
| ESI-MS: | (M+H)⁺ = 594/596/598 (2 Br) |
| Retentionszeit (HPLC): | 8.5 min (Methode A) |

### 1h 4-(2-Oxo-1,2,4,5-tetrahydro-1,3-benzdiazepin-3-yl)-piperidin-1-carbonsäure-(R)-1-(3,4-dibrom-benzyl)-2-[4-(1-methyl-piperidin-4-yl)-piperazin-1-yl]-2-oxo-ethylester

Eine Lösung von 80 mg (0.13 mmol) 4-(2-Oxo-1,2,4,5-tetrahydro-1,3-benzdiazepin-3-yl)-piperidin-1-carbonsäure-(R)-1-carboxy-2-(3,4-dibrom-phenyl)-ethylester, 43.2 mg (0.13 mmol) TBTU und 37 µL (0.27 mmol) Triethylamin in 1.5 mL DMF wurde 1 h bei RT gerührt. Dann erfolgte die Zugabe von 24.9 mg (0.134 mmol) 1-(1-Methylpiperidin-4-yl)-piperazin und das Reaktionsgemisch wurde anschliessend über Nacht bei RT gerührt. Die Reaktionslösung wurde über einen Spritzenfilter filtriert und ohne weitere Aufarbeitung direkt via HPLC gereinigt. Die das Produkt enthaltenden Fraktionen wurden vereinigt und lyophilisiert.

| | |
|---|---|
| Ausbeute: | 87.6 mg (87% der Theorie) |
| ESI-MS: | (M+H)⁺ = 759/761/763 (2 Br) |
| Retentionszeit (HPLC): | 5.0 min (Methode A) |

Analog wurden folgende Verbindungen aus jeweils 80 mg 4-(2-Oxo-1,2,4,5-tetrahydro-1,3-benzdiazepin-3-yl)-piperidin-1-carbonsäure-(R)-2-(3,4-dibrom-phenyl)-1-hydroxycarbonyl-ethylester und der entsprechenden Menge an Amin hergestellt:

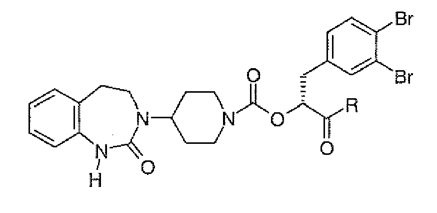

| **Beispiel** | **R** | **Ausbeute (%)** | **Massenspektrum** | **Retentionszeit HPLC** **(Methode)** |
|---|---|---|---|---|
| **2** | 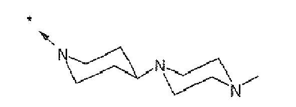 | 69 | 759/761/763 [M+H]⁺ | 5.6 min (A) |
| **3** | 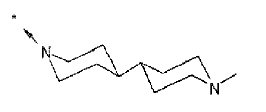 | 72 | 758/760/762 [M+H]⁺ | 6.0 min (A) |
| **4** | 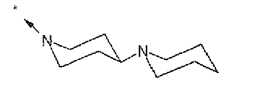 | 59 | 744/746/748 [M+H]⁺ | 6.2 min (A) |
| **5** | 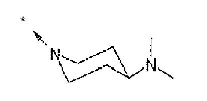 | 71 | 704/706/708 [M+H]⁺ | 5.8 min (A) |
| **6** | 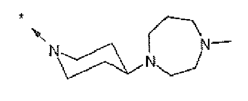 | 21 | 773/775/777 [M+H]⁺ | 5.4 min (A) |
| **7** | 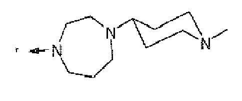 | 70 | 773/775/777 [M+H]⁺ | 5.1 min (A) |
| **8** | 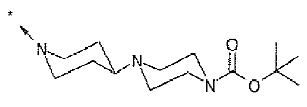 | 69 | 845/847/849 [M+H]⁺ | 6.6 min (A) |
| **9** | 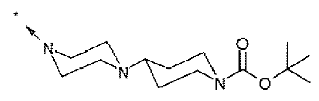 | 59 | 845/847/849 [M+H]⁺ | 6.7 min (A) |

Analog können folgende Verbindungen aus 4-(2-Oxo-1,2,4,5-tetrahydro-1,3-benzdiazepin-3-yl)-piperidin-1-carbonsäure-(R)-2-(3,4-dibrom-phenyl)-1-hydroxycarbonyl-ethylester und der entsprechenden Menge an Amin hergestellt werden:

| **Beispiel** | **R** |
|---|---|
| **10** | 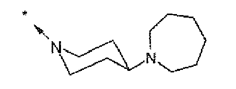 |
| **11** | 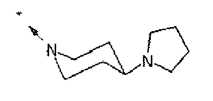 |
| **12** | 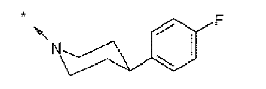 |
| **13** | 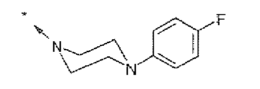 |
| **14** | 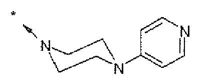 |
| **15** | 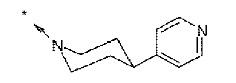 |
| **16** | 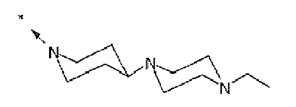 |
| **17** | 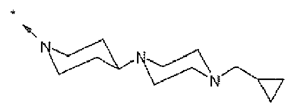 |
| **18** | 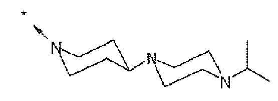 |

### Beispiel 19

### 4-(2-Oxo-1,2,4,5-tetrahvdro-1,3-benzdiazepin-3-yl)-piperidin-1-carbonsäure-(R)-1-(3,4-dibrom-benzyl)-2-oxo-2-(4-piperidin-4-yl-piperazin-1-yl)-ethylester

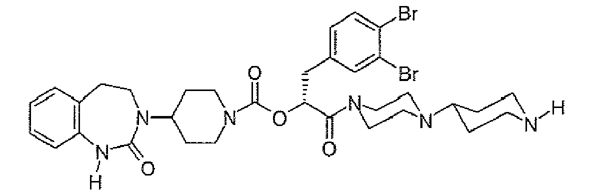

Eine Lösung von 125 mg (0.15 mmol) 4-(2-Oxo-1,2,4,5-tetrahydro-1,3-benzdiazepin-3-yl)-piperidin-1-carbonsäure-(R)-1-(3,4-dibrom-benzyl)-2-[4-(1-*tert*-butoxycarbonyl-piperidin-4-yl)-piperazin-1-yl]-2-oxo-ethylester in 20 mL 2 M HCl wurde 16 h bei RT gerührt. Das Reaktionsgemisch wurde lyophilisiert, wobei das Produkt als Bis-Hydrochlorid Salz anfiel.

| | |
|---|---|
| Ausbeute: | 110 mg (91% der Theorie) |
| ESI-MS: | (M+H)⁺ = 745/747/749 (2 Br) |
| Retentionszeit (HPLC): | 5.4 min (Methode A) |

### Beispiel 20

### 4-(2-Oxo-1,2,4,5-tetrahydro-1,3-benzdiazepin-3-yl)-piperidin-1-carbonsäure-(R)-1-(3,4-dibrom-benzyl)-2-oxo-2-(4-piperazin-1-yl-piperidin-1-yl)-ethylester

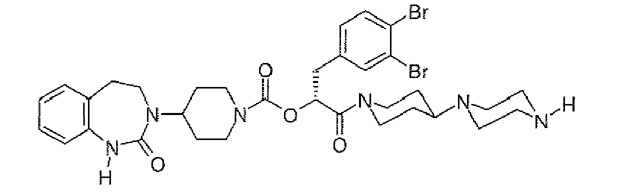

Eine Lösung von 79 mg (0.09 mmol) 4-(1-{(R)-3-(3,4-Dibrom-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-*tert*-butylester in 15 mL 2 M HCl wurde 16 h bei RT gerührt. Das Reaktionsgemisch wurde lyophilisiert, wobei das Produkt als Bis-Hydrochlorid Salz anfiel.

| | |
|---|---|
| Ausbeute: | 76 mg (100% der Theorie) |
| ESI-MS: | (M+H)⁺ = 745/747/749 (2 Br) |
| Retentionszeit (HPLC): | 5.7 min (Methode A) |

### Beispiel 21

### 4-(2-Oxo-1,2,4,5-tetrahydro-1,3-benzdiazepin-3-yl)-piperidin-1-carbonsäure-(R)-1-(3,4-dichlor-benzyl)-2-[4-(1-methyl-piperidin-4-yl)-piperazin-1-yl]-2-oxo-ethylester

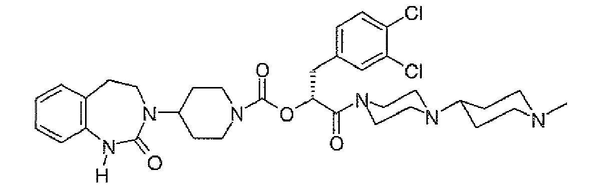

### 21a 2-Acetylamino-3-(3,4-dichlor-phenyl)-acrylsäure

Eine Mischung aus 20.0 g (112 mmol) 3,4-Dichlorbenzaldehyd, 19.7 g (168 mmol) N-Acetylglycin und 13.8 g (168 mmol) NaOAc in 80 mL Acetanhydrid wurden 5 h auf 120°C (Ölbadtemperatur) erhitzt. Nach beendeter Umsetzung wurde das Reaktionsgemisch mittels Eisbad abgekühlt und dann langsam mit 60 mL Wasser versetzt (leicht exotherme Reaktion). Das Reaktionsgemisch wurde weitere 1.5 h auf 80°C erhitzt, etwas abgekühlt, dann zu einer Mischung aus 400 mL Wasser und 200 mL Toluol gegeben und über Nacht bei RT nachgerührt. Der Niederschlag wurde abgesaugt, mit Toluol und Wasser nachgewaschen, anschließend mit Diethylether versetzt und abgesaugt.

| | |
|---|---|
| Ausbeute: | 21.0 g (68% der Theorie) |
| ESI-MS: | (M+H)⁺ = 274/276/278 (2 Cl) |
| R_{f} = | 0.16 (Kieselgel, DCM/MeOH/NH₃ 80:20:2) |

### 21b 3-(3,4-Dichlor-phenyl)-2-oxo-propionsäure

Zu einer Suspension von 21.0 g (76.6 mmol) 2-Acetylamino-3-(3,4-dichlor-phenyl)-acrylsäure in 100 mL N-Methyl-2-pyrrolidinon wurden 140 mL 4 M HCl zugegeben und das Reaktionsgemisch anschliessend für 4 h bei einer Ölbadtemperatur von 125°C erhitzt. Die abgekühlte Reaktionslösung wurde auf ein gekühltes Gemisch aus 350 mL Wasser und 120 mL Toluol gegossen. Die Phasen wurden getrennt, die wässrige Phase nochmals mit Toluol extrahiert, die vereinigten organischen Phasen mit Wasser extrahiert, über Na₂SO₄ filtriert und i. vac. eingeengt. Der Rückstand wurde in 1 M NaOH aufgenommen und zweimal mit Diethylether gewaschen. Die wässrige Phase wurde mit 2 M HCl sauer gestellt und dreimal mit EtOAc extrahiert. Die vereinigten organischen Phasen wurden über Na₂SO₄ filtriert und i. vac. eingeengt. Der Rückstand wurde mit Diethylether versetzt, abgesaugt und im Vakuumtrockenschrank getrocknet.

| | |
|---|---|
| Ausbeute: | 8.20 g (46% der Theorie) |
| ESI-MS: | (M+H)⁺ = 231/233/235 (2 Cl) |
| R_{f} = | 0.11 (Kieselgel, DCM/MeOH/Cyc/NH₃ 70:15:15:2) |

### 21c (R)-3-(3,4-Dichlor-phenyl)-2-hydroxy-propionsäure

Zu einer auf -35°C gekühlten Lösung von 8.0 g (34.3 mmol) 3-(3,4-Dichlor-phenyl)-2-oxo-propionsäure und 5.2 mL (38.0 mmol) Triethylamin in 40 mL THF wurden innerhalb von 30 min eine Lösung von 12.2 g (38.0 mmol) (1R)-B-Chlordiisopinocampheylboran in 20 mL THF zugetropft und das Reaktionsgemisch 1 h bei dieser Temperatur gehalten. Das Kühlband wurde entfernt und die Reaktionslösung 4 h bei RT gerührt. Anschließend wurde die Reaktionslösung bei 5-10°C vorsichtig mit 50 mL 1 M NaOH (exotherm) und 30 mL TBME versetzt und 15 min nachgerührt. Die organische Phase wurde abgetrennt, mit 25 mL Wasser und 15 mL 1 M NaOH extrahiert. Die vereinigten wässrigen Phasen wurden mit 2 M HCl sauer gestellt und dreimal mit jeweils 40 mL TBME extrahiert. Die vereinigten organischen Phasen wurden über Na₂SO₄ getrocknet und i. vac. eingeengt. Der Rückstand wurde in 80 mL kochendem Wasser gelöst und über Celite abgesaugt. Das Filtrat wurde mit NaCl gesättigt und dreimal mit EtOAc extrahiert. Die vereinigten organischen Phasen wurden über Na₂SO₄ filtriert und erneut i. vac. eingeengt. Das Rohprodukt wurde ohne Reingung weiter umgesetzt.

| | |
|---|---|
| Ausbeute: | 3.9 g (48% der Theorie) |
| ESI-MS: | (M+H)⁺ = 233/235/327 (2 Cl) |
| Retentionszeit (HPLC): | 6.8 min (Methode A) |
| R_{f} = | 0.87 (Kieselgel, DCM/MeOH/Cyc/NH₃ 70:15:15:2) |

### 21d (R)-3-(3,4-Dichlor-phenyl)-2-hydroxy-propionsäureethylester

Zu einer Lösung von 3.5 g (14.9 mmol) (R)-3-(3,4-Dichlor-phenyl)-2-hydroxy-propionsäure in 50 mL EtOH wurden 50 mL ethanolische HCl zugegeben und das Reaktionsgemisch 4 h bei RT nachgerührt. Die Reaktionslösung wurde i. vac. eingeengt, der Rückstand mit DCM versetzt, mit 15% K₂CO₃-Lösung extrahiert und die organische Phase über Na₂SO₄ getrocknet. Nach Entfernen des Trocken- und Lösungsmittels erhielt man das Produkt als Öl, welches ohne Reinigung weiter umgesetzt wurde.

| | |
|---|---|
| Ausbeute: | 2.6 g (66% der Theorie) |
| ESI-MS: | (M+H)⁺ = 263/265/267 (2 Cl) |
| R_{f} = | 0.91 (Kieselgel, DCM/MeOH/Cyc/NH₃ 70:15:15:2) |

### 21e 4-(2-Oxo-1,2,4,5-tetrahydro-1,3-benzdiazepin-3-yl)-piperidin-1-carbonsäure-(R)-2-(3,4-dichlor-phenyl)-1-ethoxycarbonyl-ethylester

Zu einer auf 0°C gekühlten Lösung von 2.6 g (9.9 mmol) (R)-3-(3,4-Dichlor-phenyl)-2-hydroxy-propionsäureethylester in 50 mL THF wurden 450 mg (55% in Mineralöl, 10.3 mmol) NaH portionenweise zugegeben und weitere 30 min bei dieser Temperatur nachgerührt. Anschliessend wurden portionenweise unter Kühlung 3.7 g (11.9 mmol) 4-(2-Oxo-1,2,4,5-tetrahydro-1,3-benzdiazepin-3-yl)-piperidin-1-carbonylchlorid zugegeben und das Reaktionsgemisch über Nacht bei RT gerührt. Die Reaktionslösung wurde i.vac. eingeengt, der Rückstand mit DCM versetzt, die organische Phase abgetrennt, diese mit 10% Zitronensäure-Lösung und 15% K₂CO₃-Lösung gewaschen und über Na₂SO₄ getrocknet. Nach Entfernen des Trocken- und Lösungsmittels erhielt man das Produkt, welches ohne weitere Reinigung umgesetzt wurde.

| | |
|---|---|
| Ausbeute: | 5.2 g (98% der Theorie) |
| ESI-MS: | (M+H)⁺ = 534/536/538 (2 Cl) |
| R_{f} = | 0.77 (Kieselgel, DCM/MeOH/Cyc/NH₃ 70:15:15:2) |

### 21f f 4-(2-Oxo-1,2,4,5-tetrahydro-1,3-benzdiazepin-3-yl)-piperidin-1-carbonsäure-1-carboxy-(R)-2-(3,4-dichlor-phenyl)-ethylester

Zu einer Lösung von 5.2 g (9.7 mmol) 4-(2-Oxo-1,2,4,5-tetrahydro-1,3-benzodiazepin-3-yl)-piperidin-1-(R)-carbonsäure-2-(3,4-dichlor-phenyl)-1-ethoxycarbonyl-ethylester in 30 mL THF wurde bei RT eine Lösung von 348 mg (14.5 mmol) Lithiumhydroxid in 10 mL Wasser zugegeben und das Reaktionsgemisch über Nacht bei RT gerührt. Man engte i. vac. ein, nahm den Rückstand in 15% K₂CO₃-Lösung auf und wusch die wässrige Phase dreimal mit EtOAc. Die wässrige Phase versetzte man unter Rühren mit 5 M HCl bis zur sauren Reaktion und extrahierte erschöpfend mit DCM. Die vereinigten organischen Phasen filtrierte man über Na₂SO₄ und entfernte das Lösungsmittel i. vac. Man nahm den Rückstand in Isopropanol auf und filtrierte den Niederschlag ab. Das Filtrat engte man i.vac. ein, reinigte den Rückstand chromatographisch (Kieselgel, Gradient DCM/MeOH/NH₃ 10:0:0 zu 75:25:5), vereinigte die entsprechende Fraktionen, entfernte das Lösungsmittel, versetzte den Rückstand mit Diethylether und saugte ab.

| | |
|---|---|
| Ausbeute: | 2.2 g (45% der Theorie) |
| ESI-MS: | (M+H)⁺ = 506/508/510 (2 Cl) |
| R_{f} = | 0.51 (Kieselgel, DCM/MeOH/Cyc/NH₃ 70:15:15:2) |

### 21g 4-(2-Oxo-1,2,4,5-tetrahydro-benzodiazepin-3-yl)-piperidin-1-carbonsäure-(R)-1-(3,4-dichlor-benzyl)-2-[4-(1-methyl-piperidin-4-yl)-piperazin-1-yl]-2-oxo-ethylester

Eine Lösung von 80 mg (0.16 mmol) 4-(2-Oxo-1,2,4,5-tetrahydro-1,3-benzdiazepin-3-yl)-piperidin-1-carbonsäure-1-carboxy-(R)-2-(3,4-dichlor-phenyl)-ethylester, 58 mg (0.18 mmol) TBTU und 25 µL (0.18 mmol) Triethylamin in 2 mL DMF wurde 10 min bei RT gerührt. Dann erfolgte die Zugabe von 33 mg (0.18 mmol) 1-(1-Methylpiperidin-4-yl)-piperazin und das Reaktionsgemisch wurde über Nacht bei RT gerührt. Die Reaktionslösung wurde über einen Spritzenfilter filtriert und ohne weitere Aufarbeitung direkt via HPLC gereinigt. Die das Produkt enthaltenden Fraktionen wurden vereinigt und lyophilisiert.

| | |
|---|---|
| Ausbeute: | 35.0 mg (33% der Theorie) |
| ESI-MS: | (M+H)⁺ = 671/673/675 (2 Cl) |
| Retentionszeit (HPLC): | 5.3 min (Methode A) |

Analog wurden folgende Verbindungen aus jeweils 80 mg (Beispiele 22 bis 24) bzw. aus jeweils 160 mg (Beispiele 25 bis 27) 4-(2-Oxo-1,2,4,5-tetra-hydro-1,3-benzdiazepin-3-yl)-piperidin-1-carbonsäure-1-carboxy-(R)-2-(3,4- dichlor-phenyl)-ethylester und der entsprechenden Menge an Amin hergestellt:

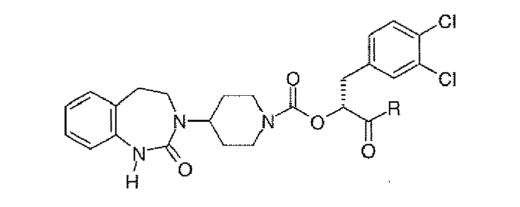

| **Beispiel** | **R** | **Ausbeute (%)** | **Massenspektrum** | **Retentionszeit HPLC** **(Methode)** |
|---|---|---|---|---|
| **22** | 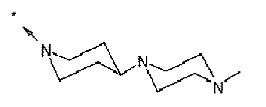 | 33 | 671/673/675 [M+H]⁺ | 5.8 min (A) |
| **23** | 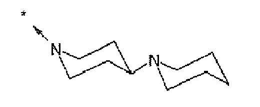 | 29 | 656/658/660 [M+H]⁺ | 6.5 min (A) |
| **24** | 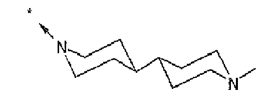 | 38 | 670/672/674 [M+H]⁺ | 6.2 min (A) |
| **25** | 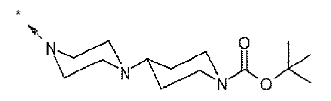 | 13 | 757/759/761 [M+H]⁺ | 7.0 min (A) |
| **26** | 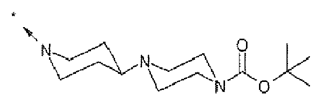 | 15 | 757/759/761 [M+H]⁺ | 6.9 min (A) |
| **27** | 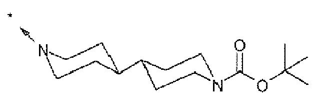 | 13 | n.e. | 5.0 min (A) |

### Beispiel 28

### 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-piperidin-4-yl-piperazin-1-yl)-ethylester

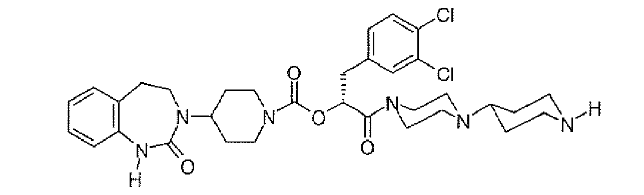

Eine Lösung von 30 mg (0.04 mmol) 4-(2-Oxo-1,2,4,5-tetrahydro-1,3-benzdiazepin-3-yl)-piperidin-1-carbonsäure-(R)-1-(3,4-dichlor-benzyl)-2-[4-(1-*tert*-butoxycarbonyl-piperidin-4-yl)-piperazin-1-yl]-2-oxo-ethylester (Beispiel 25) in 5 mL 4 M HCl wurde über Nacht bei RT gerührt. Das Reaktionsgemisch wurde lyophilisiert, wobei das Produkt als HCl-Salz anfiel.
Ausbeute: 18 mg (66% der Theorie)
ESI-MS: (M+H)⁺ = 657/659/661 (2 Cl)
R_{f} = 0.33 (Kieselgel, DCM/MeOH/Cyc/NH₃ 70:15:15:2)

### Beispiel 29

### 4-(2-Oxo-1,2,4,5-tetrahydro-benzdiazepin-3-yl)-piperidin-1-carbonsäure-(R)-1-(3,4-dichlor-benzyl)-2-oxo-2-(4-piperazin-1-yl-piperidin-1-yl)-ethylester

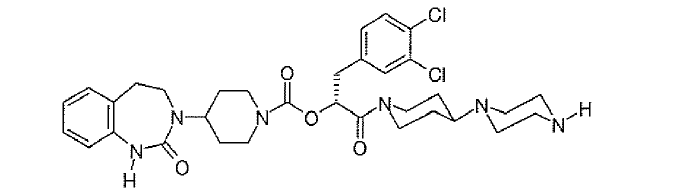

Eine Lösung von 35 mg (0.05 mmol) 4-(1-{(R)-3-(3,4-Dichlor-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-*tert*-butylester (Beispiel 26) in 5 mL 4 M HCl wurde über Nacht bei RT gerührt. Das Reaktionsgemisch wurde lyophilisiert, wobei das Produkt als HCl-Salz anfiel.

| | |
|---|---|
| Ausbeute: | 24 mg (75% der Theorie) |
| ESI-MS: | (M+H)⁺ = 657/659/661 (2 Cl) |
| R_{f} = | 0.30 (Kieselgel, DCM/MeOH/Cyc/NH₃ 70:15:15:2) |

### Beispiel 30

### 4-(2-Oxo-1,2,4,5-tetrahydro-benzdiazepin-3-yl)-piperidin-1-carbonsäure-(R)-2-4,4'-bipiperidinyl-1-yl-1-(3,4-dichlor-benzyl)-2-oxo-ethylester

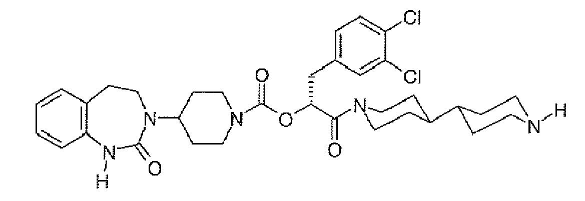

Eine Lösung von 30 mg (0.04 mmol) 1'-{(R)-3-(3,4-Dichlor-phenyl)-2-[4-(2-oxo-1,2,4,5-tetrahydro-1,3-benzdiazepin-3-yl)-piperidin-1-carbonyloxy]-propionyl}-4,4'-bipiperidinyl-1-carbonsäure-*tert*-butylester (Beispiel 27) in 5 mL 4 M HCl wurde über Nacht bei RT gerührt. Das Reaktionsgemisch wurde lyophilisiert, wobei das Produkt als HCl-Salz anfiel.

| | |
|---|---|
| Ausbeute: | 16 mg (58% der Theorie) |
| ESI-MS: | (M+H)⁺ = 656/658/660 (2 Cl) |

### Beispiel 31

### 4-(2-Oxo-1,2,4,5-tetrahydro-1,3-benzdiazepin-3-yl)-piperidin-1-carbonsäure-(R)-1-(4-chlor-3,5-dimethyl-benzyl)-2-[4-(4-methyl-piperazin-1-yl)-piperidin-1-yl]-2-oxo-ethylester

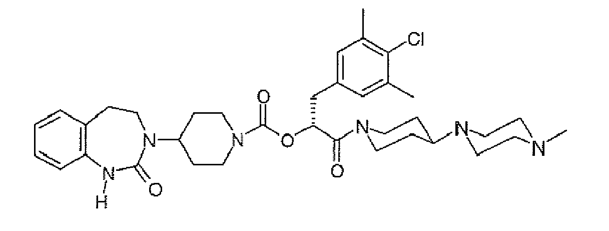

### 31a 5-Brom-2-chlor-1,3-dimethylbenzol

40.0 g (200 mmol) 4-Brom-2,6-dimethylamin in 100 mL halbkonz. HCl wurden bei 0°C mit 15.2 g (220 mmol) NaNO₂ in 90 mL Wasser versetzt, 20 min nachgerührt, mit einer Lösung aus 21.7 g (220 mmol) CuCl in 90 mL halbkonz. HCl versetzt und 2 h bei 70°C und 15 h bei RT gerührt. Nach beendeter Umsetzung wurde das Reaktionsgemisch auf 200 mL Wasser gegossen und mit TBME extrahiert. Die organischen Phasen wurden vereint, solange mit 2 M NaOH extrahiert, bis die organische Phase farblos blieb und die vereinigten organischen Phasen dann über Na₂SO₄ getrocknet. Nach Entfernen des Trocken- und Lösungsmittels wurde der Rückstand chromatographisch (Kieselgel, Cyc/DCM 1:1) gereinigt.

| | |
|---|---|
| Ausbeute: | 30.7 g (70% der Theorie) |
| ESI-MS: | (M+H)⁺ = 218/220/222 (Br, Cl) |
| R_{f} = | 0.84 (Kieselgel, Cyc/DCM 1:1) |

### 31b 2-Acetylamino-3-(4-chlor-3,5-dimethyl-phenyl)-acrylsäuremethylester

Unter Stickstoffatmosphäre wurden 30.0 g (136.7 mmol) 5-Brom-2-chlor-1,3-dimethylbenzol, 24.0 g (164.0 mmol) 2-Acetylamino-acrylsäuremethylester in 420 mL Triethylamin und 200 mL Acetonitril mit 3.4 g (10.9 mmol) Tri-*o*-tolyl-phosphan und 2.4 g (10.9 mmol) Pd(OAc)₂ versetzt und 18 h bei 80°C gerührt. Der Niederschlag wurde abgesaugt, das Filtrat i. vac. eingeengt, mit 800 mL DCM und 800 mL Wasser versetzt, die organische Phase abgetrennt und über Na₂SO₄ getrocknet. Nach Entfernen des Trocken- und Lösungsmittels wurde der Rückstand mit EtOAc verrührt, abgesaugt und i. vac getrocknet.

| | |
|---|---|
| Ausbeute: | 29.4 g (76% der Theorie) |
| ESI-MS: | (M+H)⁺ = 282/284 (Cl) |
| Retentionszeit (HPLC-MS): | 7.8 min (Methode A) |

### 31c 3-(4-Chlor-3,5-dimethyl-phenyl)-2-oxo-propionsäure

29.4 g (105 mmol) 2-Acetylamino-3-(4-chlor-3,5-dimethylphenyl)-acrylsäuremethylester in 330 mL N-Methyl-2-pyrrolidinon wurden mit 500 mL gekühlter 4 M HCl versetzt, 6 h unter Rückfluß und 16 h bei RT gerührt. Nach beendeter Umsetzung wurde das Reaktionsgemisch auf 1650 mL Wasser gegossen, 1 h nachgerührt, abgesaugt und die Kristalle bei 50°C im Vakuumtrockenschrank getrocknet. Das Produkt wurde aus Toluol umkristallisiert.

| | |
|---|---|
| Ausbeute: | 12.3 g (52% der Theorie) |
| ESI-MS: | (M+H)⁺ = 225/227 (Cl) |
| Retentionszeit (HPLC-MS): | 7.9 min (Methode A) |

### 31d (R)-3-(4-Chlor-3,5-dimethyl-phenyl)-2-hydroxy-propionsäure

12.3 g (54.4 mmol) 3-(4-Chlor-3,5-dimethyl-phenyl)-2-oxo-propionsäure in 130 mL THF und 7.6 mL (54.4 mmol) Triethylamin wurden bei -35°C mit einer Lösung aus 21.0 g (65.3 mmol) (1 R)-B-Chlordiisopinocampheylboran in 65 mL THF innerhalb von 30 min versetzt und 2 h bei dieser Temperatur nachgerührt. Nach beendeter Umsetzung wurde das Reaktionsgemisch bei 0°C mit 50 mL 1 M NaOH alkalisch gestellt (exotherm), 3 h nachgerührt, mit 30 mL TBME versetzt und die Phasen getrennt. Die organische Phase wurde mit 50 mL Wasser und 30 mL 1 M NaOH gewaschen. Die vereinigten wässrigen Phasen wurden mit 2 M HCl sauer gestellt und mit TBME extrahiert. Die organischen Phasen wurden über Na₂SO₄ getrocknet, und i. vac. eingeengt. Das Produkt wurde ohne Reinigung weiter umgesetzt.

| | |
|---|---|
| Ausbeute: | 12.5 g (100% der Theorie) |
| ESI-MS: | (M+H)⁺ = 227/229 (Cl) |
| Retentionszeit (HPLC-MS): | 7.1 min (Methode A) |

### 31e (R)-3-(4-Chlor-3,5-dimethyl-phenyl)-2-hydroxy-propionsäuremethylester

Zu einer auf 0°C gekühlten Lösung von 12.45 g (54.4 mmol) (R)-3-(4-Chlor-3,5-dimethyl-phenyl)-2-hydroxy-propionsäure in 300 mL MeOH wurden tropfenweise 4.4 mL (59.9 mmol) SOCl₂ zugegeben und das Reaktionsgemisch 1 h bei RT nachgerührt. Die Reaktionslösung wurde i. vac. eingeengt und der Rückstand chromatographisch (Kieselgel, Cyc/EtOAc 4:1) gereinigt.

| | |
|---|---|
| Ausbeute: | 10.1 g (76% der Theorie) |
| ESI-MS: | (M+NH₄)⁺ = 260/262 (Cl) |
| Retentionszeit (HPLC-MS): | 8.1 min (Methode A) |

### 31f 4-(2-Oxo-1,2,4,5-tetrahydro-1,3-benzdiazepin-3-yl)-piperidin-1-carbonsäure-(R)-2-(4-chlor-3,5-dimethyl-phenyl)-1-methoxycarbonyl-ethylester

Unter Stickstoffatmospäre wurden 1.0 g (8.2 mmol) 4-Dimethylaminopyridin in 30 mL Pyridin mit 1.7 g (8.2 mmol) Chlorameisensäure-4-nitrophenylester versetzt, 40 min bei RT gerührt, dann wurden 2.0 g (8.2 mmol) (R)-3-(4-Chlor-3,5-dimethyl-phenyl)-2-hydroxy-propionsäuremethylester zugegeben, nochmals 20 min bei RT gerührt und anschließend mit 2.0 g ( 8.2 mmol) 3-Piperidin-4-yl-1,3,4,5-tetrahydro-1,3-benzdiazepin-2-on versetzt und das Reaktionsgemisch 20 h bei RT gerührt. Man engte i.vac. ein, nahm den Rückstand in EtOAc auf, wusch mit 10% KHSO₄- und gesättigter NaHCO₃-Lösung und trocknete die organische Phase über Na₂SO₄. Nach Entfernen des Trocken- und Lösungsmittels wurde der Rückstand chromatographisch (Kieseigel,Cyc/EtOAc 1:1 bis 1:2 ) gereinigt.

| | |
|---|---|
| Ausbeute: | 2.16 g (51% der Theorie) |
| ESI-MS: | (M+H)⁺ = 514/516 (Cl) |
| Retentionszeit (HPLC-MS): | 10.1 min (Methode A) |

### 31g 4-(2-Oxo-1,2,4,5-tetrahydro-1,3-benzdiazepin-3-yl)-piperidin-1-carbonsäure-(R)-1-carboxy-2-(4-chlor-3,5-dimethyl-phenyl)-ethylester

Zu einer Lösung von 2.15 g (4.18 mmol) 4-(2-Oxo-1,2,4,5-tetrahydro-1,3-benzdiazepin-3-yl)-piperidin-1-carbonsäure-(R)-2-(4-chlor-3,5-dimethyl-phenyl)-1-methoxycarbonyl-ethylester in 60 mL THF wurde eine Lösung von 150 mg (0.60 mmol) LiOH in 30 mL Wasser gegeben und das Reaktionsgemisch 1 h bei RT gerührt. Man engte i. vac. ein, nahm den Rückstand in 100 mL Wasser auf, säuerte mit 1 M HCl an, filtrierte den Niederschlag und trocknete diesen im Vakuumtrockenschrank bei 40°C.

| | |
|---|---|
| Ausbeute: | 2.05 g (98% der Theorie) |
| ESI-MS: | (M+H)⁺ = 500/502 (Cl) |
| Retentionszeit (HPLC-MS): | 8.8 min (Methode A) |

### 31h 4-(2-Oxo-1,2,4,5-tetrahydro-1,3-benzdiazepin-3-yl)-piperidin-1-carbonsäure-(R)-1-(4-chlor-3,5-dimethyl-benzyl)-2-[4-(4-methyl-piperazin-1-yl)-piperidin-1-yl]-2-oxo-ethylester

Eine Lösung von 80 mg (0.16 mmol) 4-(2-Oxo-1,2,4,5-tetrahydro-benzdiazepin-3-yl)-piperidin-1-carbonsäure-(R)-2-(4-chlor-3,5-dimethyl-phenyl)-ethylester, 51 mg (0.16 mmol) TBTU und 28 µL (0.20 mmol) Triethylamin in 1.5 mL DMF wurde 1 h bei RT gerührt. Dann erfolgte die Zugabe von 30 mg (0.16 mmol) 1-Methyl-4-(piperidin-4-yl)-piperazin und das Reaktionsgemisch wurde 16 h bei RT gerührt. Die Reaktionslösung wurde über einen Spritzenfilter filtriert und ohne weitere Aufarbeitung direkt via HPLC gereinigt. Die das Produkt enthaltenden Fraktionen wurden vereinigt und lyophilisiert.

| | |
|---|---|
| Ausbeute: | 18 mg (17 % der Theorie) |
| ESI-MS: | (M+H)⁺ = 665/667 (Cl) |
| Retentionszeit (HPLC-MS): | 5.6 min (Methode A) |

Analog wurden folgende Verbindungen aus jeweils 80 mg (Beispiele 32 bis 34) bzw. aus jeweils 140 mg (Beispiele 35 und 36) 4-(2-Oxo-1,2,4,5-tetrahydro-1,3-benzdiazepin-3-yl)-piperidin-1-carbonsäure-(R)-1-carboxy-2-(4-chlor-3,5-dimethylphenyl)-ethylester und der entsprechenden Menge an Amin hergestellt:

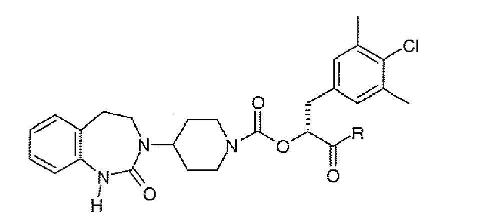

| **Beispiel** | **R** | **Ausbeute (%)** | **Massen-spektrum** | **Retentionszeit HPLC** **(Methode)** |
|---|---|---|---|---|
| **32** | 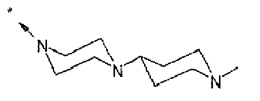 | 40 | 665/667(Cl) [M+H]⁺ | 5.3 min (A) |
| **33** | 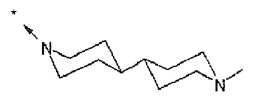 | 42 | 664/666(Cl) [M+H]⁺ | 6.8 min (A) |
| **34** | 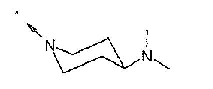 | 44 | 610/612(Cl) [M+H]⁺ | 6.5 min (A) |
| **35** | 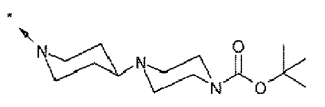 | 30 | 751/753(Cl) [M+H]+ | 7.5 min (A) |
| **36** | 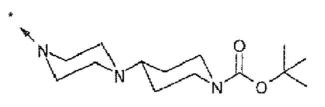 | 28 | 751/753(Cl) [M+H]+ | 7.7 min (A) |

### Beispiel 37

### 4-(2-Oxo-1,2,4,5-tetrahydro-1,3-benzdiazepin-3-yl)-piperidin-1-carbonsäure-(R)-1-(4-chlor-3,5-dimethyl-benzyl)-2-oxo-2-(4-piperazin-1-yl-piperidin-1-yl)-ethylester

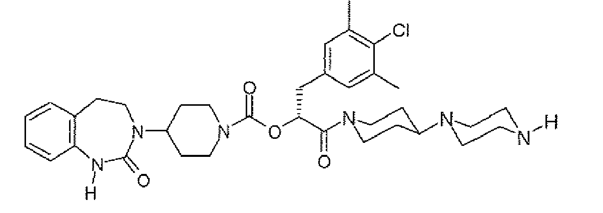

Eine Lösung von 64.0 mg (0.09 mmol) 4-(1-{(R)-3-(4-Chlor-3,5-dimethyl-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-*tert*-butylester (Beispiel 35) in 5 mL 2 M HCl wurde über Nacht bei RT gerührt. Das Reaktionsgemisch wurde lyophilisiert, wobei das Produkt als Bis-Hydrochlorid Salz anfiel.

| | |
|---|---|
| Ausbeute: | 61.2 mg (99% der Theorie) |
| ESI-MS: | (M+H)⁺ = 651/653 (Cl) |
| Retentionszeit (HPLC-MS): | 5.9 min (Methode A) |

### Beispiel 38

### 4-(2-Oxo-1,2,4,5-tetrahydro-1,3-benzdiazepin-3-yl)-piperidin-1-carbonsäure-(R)-1-(4-chlor-3,5-dimethyl-benzyl)-2-oxo-2-(4-piperidin-4-yl-piperazin-1-yl)-ethylester

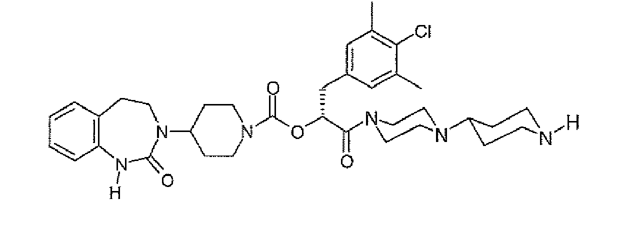

Eine Lösung von 59 mg (0.08 mmol) 4-(2-Oxo-1,2,4,5-tetrahydro-1,3-benzdiazepin-3-yl)-piperidin-1-carbonsäure-(R)-1-(4-chlor-3,5-dimethyl-benzyl)-2-[4-(1-*tert*-butoxycarbonyl-piperidin-4-yl)-piporazin-1-yl]-2-oxo-ethylester (Beispiel 36) in 5 mL 2 M HCl wurde über Nacht bei RT gerührt. Das Reaktionsgemisch wurde lyophilisiert, wobei das Produkt als Bis-Hydrochlorid Salz anfiel.

| | |
|---|---|
| Ausbeute: | 55.7 mg (57% der Theorie) |
| ESI-MS: | (M+H)⁺ = 651/653 (Cl) |
| Retentionszeit (HPLC-MS): | 5.5 min (Methode A) |

### Beispiel 39

### 4-(2-Oxo-1,2,4,5-tetrahydro-1,3-benzdiazepin-3-yl)-piperidin-1-carbonsäure-(R)-1-(3,5-dimethyl-benzyl)-2-[4-(1-methyl-piperidin-4-yl)-piperazin-1-yl]-2-oxo-ethylester

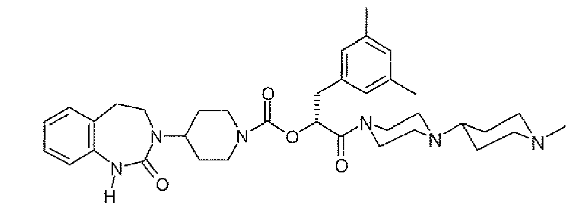

### 39a 4-(2-Oxo-1,2,4,5-tetrahydro-1,3-benzdiazepin-3-yl)-piperidin-1-carbonsäure-(R)-1-carboxy-2-(3,5-dimethyl-phenyl)-ethylester

500 mg (1.0 mmol) 4-(2-Oxo-1,2,4,5-tetrahydro-benzdiazepin-3-yl)-piperidin-1-carbonsäure-1-carboxy-(R)-2-(4-chlor-3,5-dimethyl-phenyl)-ethylester (Beispiel 31g) in 20 mL MeOH wurden mit 100 mg 10% Pd/C und 2 mL Triethylamin versetzt und 10 Tage bei RT und 3 bar hydriert. Nach beendeter Umsetzung wurde das Reaktionsgemisch i.vac. eingeengt, der Rückstand in 25 mL Wasser aufgenommen, mit 1 M HCl sauer gestellt, der Niederschlag abgesaugt und im Vakuumtrockenschrank bei 40°C getrocknet.

| | |
|---|---|
| Ausbeute: | 418 mg (90% der Theorie) |
| ESI-MS: | (M+H)⁺ = 466 |
| Retentionszeit (HPLC-MS): | 8.3 min (Methode A) |

### 39b 4-(2-Oxo-1,2,4,5-tetrahydro-1,3-benzdiazepin-3-yl)-piperidin-1-carbonsäure-(R)-1-(3,5-dimethyl-benzyl)-2-[4-(1-methyl-piperidin-4-yl)-piporazin-1-yl]-2-oxo-ethylester

Eine Lösung von 50 mg (0.16 mmol) 4-(2-Oxo-1,2,4,5-tetrahydro-1,3-benzdiazepin-3-yl)-piporidin-1-carbonsäure-(R)-1-carboxy-2-(3,5-dimethyl-phenyl)-ethylester, 35 mg (0.11 mmol) TBTU und 19 µL (0.13 mmol) Triethylamin in 1 mL DMF wurde 1 h bei RT gerührt. Dann erfolgte die Zugabe von 20 mg (0.11 mmol) 1-(1-Methyl-piperidin-4-yl)-piperazin und das Reaktionsgemisch wurde 16 h bei RT gerührt. Die Reaktionslösung wurde über einen Spritzenfilter filtriert und ohne weitere Aufarbeitung direkt via HPLC gereinigt. Die das Produkt enthaltenden Fraktionen wurden vereinigt und lyophilisiert.

| | |
|---|---|
| Ausbeute: | 33 mg (49 % der Theorie) |
| ESI-MS: | (M+H)⁺ = 631 |
| Retentionszeit (HPLC-MS): | 5.3 min (Methode A) |

Analog wurden folgende Verbindungen aus jeweils 50 mg (Beispiele 40 und 41) bzw. aus jeweils 80 mg (Beispiele 42 und 43) 4-(2-Oxo-1,2,4,5-tetrahydro-1,3-benzdiazepin-3-yl)-piperidin-1-carbonsäure-(R)-1-carboxy-2-(3,5-dimethyl-phenyl)-ethylester und der entsprechenden Menge an Amin hergestellt:

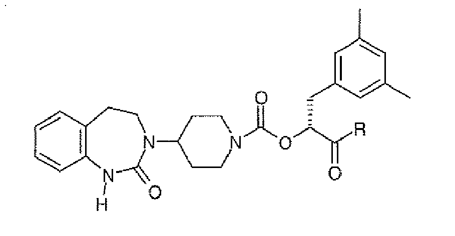

| **Beispiel** | **R** | **Ausbeute (%)** | **Massen-spektrum** | **Retentionszeit HPLC** **(Methode)** **oder R_{f}** **(Kieselgel, Laufmittel)** |
|---|---|---|---|---|
| **40** | 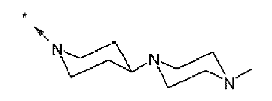 | 76 | 631 [M+H]⁺ | 5.8 min (A) |
| **41** | 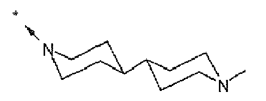 | 55 | 630 [M+H]⁺ | 6.5 min (A) |
| **42** | 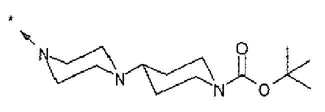 | 37 | 717 [M+H]⁺ | 4.7 min (A) |
| **43** | 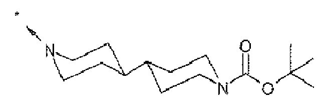 | 73 | 716 [M+H]⁺ | 0.59 (EtOAc) |

### Beispiel 44

### 4-(2-Oxo-1,2,4,5-tetrahydro-1,3-benzdiazepin-3-yl)-piperidin-1-carbonsäure-(R)-1-(3,5-dimethyl-benzyl)-2-oxo-2-(4-piperidin-4-yl-piperazin-1-yl)-ethylester

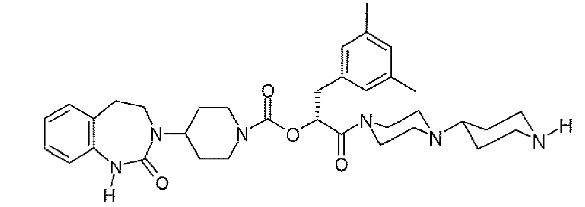

Eine Lösung von 45 mg (0.06 mmol) 4-(2-Oxo-1,2,4,5-tetrahydro-1,3-benzdiazepin-3-yl)-piperidin-1-carbonsäure-(R)-1-(3,5-dimethyl-benzyl)-2-[4-(1-*tert*-butoxy-carbonyl-piperidin-4-yl)-piperazin-1-yl]-2-oxo-ethylester (Beispiel 42) in 10 mL 2 M HCl wurde über Nacht bei RT gerührt. Das Reaktionsgemisch wurde lyophilisiert. Das Rohprodukt wurde in 1 mL DMF aufgenommen, mit 0.6 mL gesättigter K₂CO₃-Lösung alkalisch gestellt und via HPLC chromatographisch gereinigt.

| | |
|---|---|
| Ausbeute: | 26.8 mg (69% der Theorie) |
| ESI-MS: | (M+H)⁺ = 617 |
| Retentionszeit (HPLC-MS): | 5.4 min (Methode A) |

### Beispiel 45

### 4-(2-Oxo-1,2,4,5-tetrahydro-1,3-benzdiazepin-3-yl)-piperidin-1-carbonsäure-(R)-2-[4,4']bipiperidinyl-1-yl-1-(3,5-dimethyl-benzyl)-2-oxo-ethylester

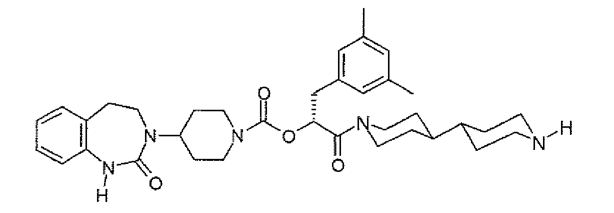

Eine Lösung von 101 mg (0.14 mmol) 1'-{(R)-3-(3,5-Dimethyl-phenyl)-2-[4-(2-oxo-1,2,4,5-tetrahydro-1,3-benzdiazepin-3-yl)-piperidin-1-carbonyloxy]-propionyl}-4,4'-bipiperidinyl-1-carbonsäure-*tert*-butylester (Beispiel 43) in 10 mL 2 M HCl wurde über Nacht bei RT gerührt. Das Reaktionsgemisch wurde lyophilisiert. Das Rohprodukt wurde in 1 mL DMF aufgenommen, mit 0.6 mL gesättigter K₂CO₃-Lösung alkalisch gestellt und via HPLC chromatographisch gereinigt.

| | |
|---|---|
| Ausbeute: | 18.7 mg (22% der Theorie) |
| ESI-MS: | (M+H)⁺=616 |
| Retentionszeit (HPLC-MS): | 6.5 min (Methode A) |

### Beispiel 46

### 4-(2-Oxo-1,2,4,5-tetrahydro-1,3-benzdiazepin-3-yl)-piperidin-1-carbonsäure-(R)-1-(3,5-bis-trifluormethylbenzyl)-2-[4-(1-methyl-piperidin-4-yl)-piperazin-1-yl]-2-oxo-ethylester

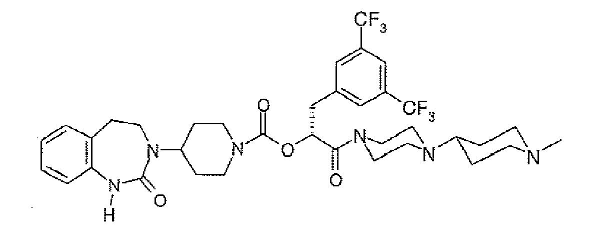

### 46a 2-Acetylamino-3-(3,5-bis-trifluormethyl-phenyl)acrylsäuremethylester

Unter Stickstoffatmosphäre wurden 50.0 g (171 mmol) 3,5-Bis-(trifluormethyl)-brombenzol, 25.0 g (171 mmol) 2-Acetylamino-acrylsäuremethylester in 475 mL Triethylamin und 250 mL Acetonitril mit 3.9 g (12.4 mmol) Tri-*o*-tolyl-phosphan und 2.8 g (12.5 mmol) Pd(OAc)₂ versetzt und 18 h bei 80°C gerührt. Nach beendeter Umsetzung wurde das Reaktionsgemisch i. vac. auf ca. 200 mL eingeengt, mit 400 mL EtOAc und 400 mL Wasser versetzt, der Niederschlag abgesaugt und die Phasen getrennt. Die organische Phase wurde über Na₂SO₄ getrocknet, mit Aktivkohle versetzt, filtriert und zur Trockene eingeengt. Der Rückstand wurde mit DIPE verrührt, abgesaugt und i. vac getrocknet.

| | |
|---|---|
| Ausbeute: | 19.5 g (32 % der Theorie) |
| ESI-MS: | (M+H)⁺ = 356 |
| R_{f} = | 0.76 (Kieselgel, PE/EtOAc 1:1) |

### 46b 3-(3,5-Bis-trifluormethyl-phenyl)-2-oxo-propionsäure

19.5 g (54.9 mmol) 2-Acetylamino-3-(3,5-bis-trifluormethyl-phenyl)-acrylsäuremethylester in 100 mL 1,4-Dioxan wurden auf 100°C Badtemperatur erwärmt, mit 100 mL 4 M HCl versetzt und 8 h bei 100°C Badtemperatur gerührt. Das Reaktionsgemisch wurde i. vac. eingeengt, die Kristalle abgesaugt, mit Wasser gewaschen und diese im Trockenschrank bei 50°C getrocknet.

| | |
|---|---|
| Ausbeute: | 16.1 g (98 % der Theorie) |
| ESI-MS: | (M-H)⁻ = 299 |
| R_{f} = | 0.18 (Kieselgel, EtOAc) |

### 46c (R)-3-(3,5-Bis-trifluormethyl-phenyl)-2-hydroxy -propionsäure

16.1 g (53.6 mmol) 3-(3,5-Bis-trifluormethyl-phenyl)-2-oxo-propionsäure in 9.5 (70.0 mmol) Triethylamin und 100 mL THF wurden bei -35°C mit einer Lösung aus 26.0 (81.1 mol) (1R)-B-Chlordiisopinocampheylboran in 40 mL THF innerhalb von 30 min versetzt, 1 h bei dieser Temperatur und über Nacht bei RT nachgerührt. Nach beendeter Umsetzung wurde das Reaktionsgemisch bei 0°C mit 160 mL 1 M NaOH alkalisch gestellt, 15 min nachgerührt, mit 100 mL TBME versetzt und die Phasen getrennt. Die organische Phase wurde mit 50 mL Wasser und 50 mL 1 M NaOH gewaschen. Die vereinigten wässrigen Phasen wurden mit 4 M HCl sauer gestellt, erschöpfend mit TBME extrahiert, die vereinigten organischen Phasen über Na₂SO₄ getrocknet, über Aktivkohle abgesaugt und i.vac. eingeengt. Das Produkt wurde ohne Reinigung weiter umgesetzt.

| | |
|---|---|
| Ausbeute: | 12.5 g (77% der Theorie) |
| ESI-MS: | (M-H)⁻ = 301 |
| R_{f} = | 0.45 (Kieselgel, EtOAc) |

### 46d (R)-3-(3,5-Bis-trifluormethyl-phenyl)-2-hydroxy-propionsäuremethylester

12.5 g (41.4 mmol) (R)-3-(3,5-Bis-trifluormethyl-phenyl)-2-hydroxy-propionsäure in 150 mL methanolischer HCl (1.25 M) wurden 4 h bei RT gerührt und anschliessend i. vac. eingeengt. Der Rückstand wurde in EtOAc aufgenommen und mit gesättigter NaHCO₃-Lösung gewaschen, die organische Phase über Na₂SO₄ getrocknet, über Aktivkohle abgesaugt und i. vac. eingeengt. Der Rückstand wurde mit PE verrührt, abgesaugt und i. vac. eingeengt. Das Produkt wurde ohne Reinigung weiter umgesetzt.

| | |
|---|---|
| Ausbeute: | 11.4 g (87% der Theorie) |
| ESI-MS: | (M+H)⁺ = 316 |
| R_{f} = | 0.80 (Kieselgel, PE/EtOAc 1:1) |

### 46e 4-(2-Oxo-1,2,4,5-tetrahydro-1,3-benzdiazepin-3-yl)-piperidin-1-carbonsäure-(R)-2-(3,5-bis-trifluormethyl-phenyl)-1-methoxycarbonyl-ethylester

Analog Beispiel 31f konnte aus 6.0 g (8.2 mmol) (R)-3-(3,5-Bis-trifluormethyl-phenyl)-2-hydroxy-propionsäuremethylester und 5.13 g (20.9 mmol) 3-Piperidin-4-yl-1,3,4,5-tetrahydro-1,3-benzdiazepin-2-on das Produkt erhalten werden. Die Reinigung erfolgte chromatographisch (Kieselgel, Gradient PE/EtOAc 1:1 zu 1:9 ).

| | |
|---|---|
| Ausbeute: | 5.1 g (46 % der Theorie) |
| ESI-MS: | (M+H)⁺ = 588 |
| R_{f} = | 0.63 (Kieselgel, DCM/MeOH/Cyc/NH₃ 70:15:15:2) |

### 46f 4-(2-Oxo-1,2,4,5-tetrahydro-1,3-benzdiazepin-3-yl)-piperidin-1-carbonsäure-(R)-2-(3,5-bis-trifluormethyl-phenyl)-1-carboxy-ethylester

Zu einer Lösung von 5.0 g (8.5 mmol) 4-(2-Oxo-1,2,4,5-tetrahydro-1,3-benzdiazepin-3-yl)-piperidin-1-carbonsäure-(R)-2-(3,5-bis-trifluormethyl-phenyl)-1-methoxycarbonyl-ethylester in 50 mL THF wurde eine Lösung von 307 mg (12.8 mmol) LiOH in 5 mL Wasser gegeben und das Reaktionsgemisch über Nacht bei RT gerührt. Man engte i. vac. ein, nahm den Rückstand in Wasser auf, säuerte mit 1 M HCl an, filtrierte den Niederschlag ab und trocknete diesen im Vakuumtrockenschrank bei 40°C.

| | |
|---|---|
| Ausbeute: | 4.5 g (92% der Theorie) |
| ESI-MS: | (M+H)⁺ = 574 |
| R_{f} = | 0.32 (Kieselgel,DCM/MeOH/Cyc/NH₃ 70:15:15:2) |

### 46g 4-(2-Oxo-1,2,4,5-tetrahydro-1,3-benzdiazepin-3-yl)-piperidin-1-carbonsäure-(R)-1-(3,5-bis-trifluormethyl-benzyl)-2-[4-(1-methyl-piperidin-4-yl)-piperazin-1-yl]-2-oxo-ethylester

Eine Lösung von 80 mg (0.14 mmol) 4-(2-Oxo-1,2,4,5-tetrahydro-benzdiazepin-3-yl)-piporidin-1-carbonsäure-(R)-2-(3,5-bis-trifluormethyl-phenyl)-1-carboxy-ethylester, 51 mg (0.16 mmol) TBTU und 22 µL (0.16 mmol) Triethylamin in 1 mL DMF wurde 1 h bei RT gerührt. Dann erfolgte die Zugabe von 29 mg (0.16 mmol) 1-(1-Methylpiperidin-4-yl)-piperazin und das Reaktionsgemisch wurde über Nacht bei RT gerührt. Die Reaktionslösung wurde über einen Spritzenfilter filtriert und ohne weitere Aufarbeitung direkt via HPLC gereinigt. Die das Produkt enthaltenden Fraktionen wurden vereinigt und lyophilisiert.
Ausbeute: 56 mg (54% der Theorie)
ESI-MS: (M+H)⁺ = 739
Retentionszeit (HPLC-MS): 5.8 min (Methode A)

Analog wurden folgende Verbindungen aus jeweils 80 mg (Beispiele 47 bis 49) bzw. aus jeweils 100 mg (Beispiele 50 bis 53 ) 4-(2-Oxo-1,2,4,5-tetrahydro-benzdiazepin-3-yl)-piperidin-1-carbonsäure-(R)-2-(3,5-bis-trifluormethyl-phenyl)-1-carboxy-ethylester und der entsprechenden Menge an Amin hergestellt:

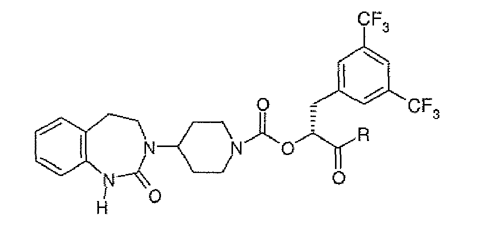

| **Beispiel** | **R** | **Ausbeute (%)** | **Massen-spektrum** | **Retentionszeit HPLC** **(Methode)** |
|---|---|---|---|---|
| **47** | 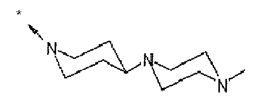 | 49 | 739 [M+H]⁺ | 6.5 min (A) |
| **48** | 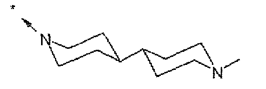 | 47 | 738 [M+H]⁺ | 7.1 min (A) |
| **49** | 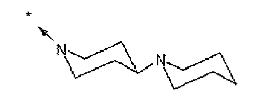 | 57 | 724 [M+H]⁺ | 7.1 min (A) |
| **50** | 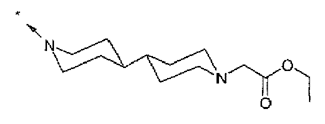 | 39 | 810 [M+H]+ | 7.3 min (A) |
| **51** | 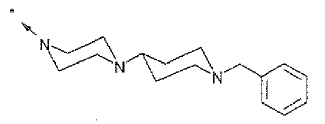 | 53 | 815 [M+H]+ | 6.4 min (A) |
| **52** | 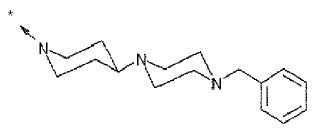 | 46 | 815 [M+H]+ | 6.1 min (A) |
| **53** | 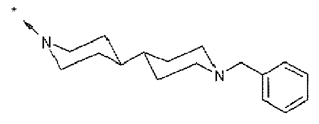 | 65 | 814 [M+H]+ | 7.9 min (A) |

### Beispiel 54

### 4-(2-Oxo-1,2,4,5-tetrahydro-1,3-benzdiazepin-3-yl)-piperidin-1-carbonsäure-(R)-1-(3,5-bis-trifluormethyl-benzyl)-2-oxo-2-(4-piperidin-4-yl-piperazin-1-yl)-ethylester

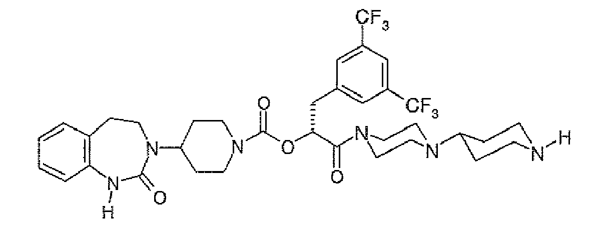

Eine Lösung von 77 mg (0.09 mmol) 4-(2-Oxo-1,2,4,5-tetrahydro-1,3-benzdiazepin-3-yl)-piperidin-1-carbonsäure-(R)-2-[4-(1-benzyl-piperidin-4-yl)-piperazin-1-yl]-1-(3,5-bis-trifluormethyl-benzyl)-2-oxo-ethylester (Beispiel 51) in 10 mL MeOH wurde mit 50 mg 10% Pd/C versetzt und 3 h bei RT und 50 psi Wasserstoff geschüttelt. Der Katalysator wurde abgesaugt, das Lösungsmittel i.vac. eingeengt, der Rückstand mit Acetonitril und Wasser versetzt und lyophilisiert.

| | |
|---|---|
| Ausbeute: | 46 mg (70% der Theorie) |
| ESI-MS: | (M+H)⁺ = 725 |
| Retentionszeit (HPLC-MS): | 5.7 min (Methode A) |

### Beispiel 55

### 4-(2-Oxo-1,2,4,5-tetrahydro-1,3-benzdiazepin-3-yl)-piperidin-1-carbonsäure-(R)-1-(3,5-bis-trifluormethyl-benzyl)-2-oxo-2-(4-piperazin-1-yl-piperidin-1-yl)-ethylester

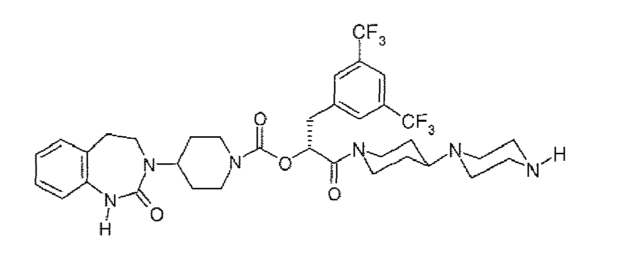

Eine Lösung von 64 mg (0.08 mmol) 4-(2-Oxo-1,2,4,5-tetrahydro-1,3-benzdiazepin-3-yl)-piperidin-1-carbonsäure-(R)-2-[4-(4-benzyl-piperazin-1-yl)-piperidin-1-yl]-1-(3,5-bis-trifluormethyl-benzyl)-2-oxo-ethylester (Beispiel 52) in 10 mL MeOH wurde mit 50 mg 10% Pd/C versetzt und 3 h bei RT und 50 psi Wasserstoff geschüttelt. Der Katalysator wurde abgesaugt, das Lösungsmittel i.vac. eingeengt, der Rückstand mit Acetonitril und Wasser versetzt und lyophilisiert.

| | |
|---|---|
| Ausbeute: | 43 mg (76% der Theorie) |
| ESI-MS: | (M+H)⁺ = 725 |
| Retentionszeit (HPLC-MS): | 5.7 min (Methode A) |

### Beispiel 56

### 4-(2-Oxo-1,2,4,5-tetrahydro-1,3-benzdiazepin-3-yl)-piperidin-1-carbonsäure-(R)-2-4,4'-bipiperidinyl-1-yl-1-(3,5-bis-trifluormethyl-benzyl)-2-oxo-2-ethylester

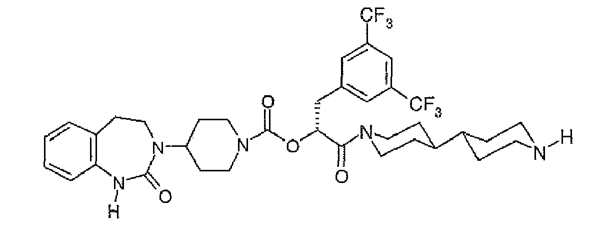

Eine Lösung von 92 mg (0.11 mmol) 4-(2-Oxo-1,2,4,5-tetrahydro-1,3-benzdiazepin-3-yl)-piperidin-1-carbonsäure-(R)-2-(1'-benzyl-4,4'-bipiperidinyl-1-yl)-1-(3,5-bis-trifluormethyl-benzyl)-2-oxo-ethylester (Beispiel 53) in 10 mL MeOH wurde mit 50 mg 10% Pd/C versetzt und 3 h bei RT und 50 psi Wasserstoff geschüttelt. Der Katalysator wurde abgesaugt, das Lösungsmittel i.vac. eingeengt, der Rückstand mit Acetonitril und Wasser versetzt und lyophilisiert.

| | |
|---|---|
| Ausbeute: | 55 mg (67 % der Theorie) |
| ESI-MS: | (M+H)⁺ = 724 |
| Retentionszeit (HPLC-MS): | 5.6 min (Methode A) |

### Beispiel 57

### 4-(2-Oxo-1,2,4,5-tetrahydro-1,3-benzdiazepin-3-yl)-piperidin-1-carbonsäure-(R)-1-(3,5-bis-trifluormethyl-benzyl)-2-(1'-carboxymethyl-4,4'-bipiperidinyl-1-yl)-2-oxo-ethyl-ester

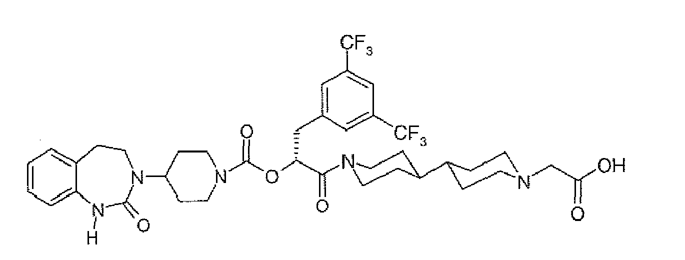

Zu einer Lösung von 35 mg (0.04 mmol) 4-(2-Oxo-1,2,4,5-tetrahydro-1,3-benzdiazepin-3-yl)-piperidin-1-carbonsäure-(R)-1-(3,5-bis-trifluormethyl-benzyl)-2-(1'-ethoxycarbonylmethyl-4,4'-bipiperidinyl-1-yl)-2-oxo-ethylester (Beispiel 50) in 5 mL THF wurde eine Lösung von 1.5 mg (0.06 mmol) LiOH in 1 mL Wasser gegeben und die Reaktionslösung über Nacht bei RT gerührt. Man engte i. vac. ein, nahm den Rückstand in Wasser auf, säuerte mit 1 N HCl an, filtrierte den Niederschlag ab und trocknete diesen im Vakuumtrockenschrank.

| | |
|---|---|
| Ausbeute: | 15 mg (44 % der Theorie) |
| ESI-MS: | (M+H)⁺ = 782 |
| R_{f} = | 0.41 (Kieselgel, DCM/MeOH/Cyc/NH₃ 70:15:15:2) |

### Beispiel 58

### 4-(2-Oxo-1,2,4,5-tetrahydro-1,3-benzdiazepin-3-yl)-piperidin-1-carbonsäure-(R)-1-(3,5-dibrom-benzyl)-2-(4-dimethylamino-piperidin-1-yl)-2-oxo-ethylester

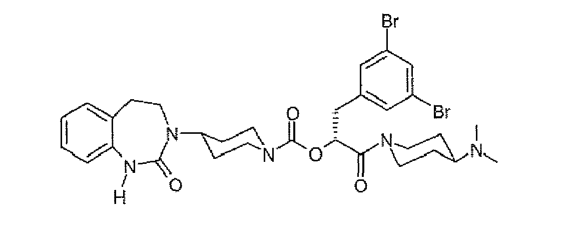

### 58a (Z,E)-2-Acetylamino-3-(3,5-dibrom-phenyl)-acrylsäure

Analog Beispiel 1a konnte aus 35.0 g (133 mmol) 3,5-Dibrom-benzaldehyd und 23.3 g (199 mmol) N-Acetyl-glycin das Produkt erhalten werden.

| | |
|---|---|
| Ausbeute: | 29.2 g (61% der Theorie) |
| Schmelzpunkt: | 248-249°C |
| ESI-MS | (M+H)⁺ = 362/364/366 (2 Br) |
| R_{f} = | 0.1 (Kieselgel, DCM/MeOH/AcOH 90:10:1) |

### 58b 3-(3,5-Dibrom-phenyl)-2-oxo-propionsäure

Analog Beispiel 1b konnte aus 29.0 g (80.0 mmol) (Z,E)-2-Acetylamino-3-(3,5-dibrom-phenyl)-acrylsäure das Produkt erhalten werden.

| | |
|---|---|
| Ausbeute: | 14.0 g (54% der Theorie) |
| ESI-MS | (M-H)⁻ = 318/320/322 (2 Br) |
| R_{f} = | 0.4 (Kieselgel, DCM/MeOH/AcOH 90:10:1) |

### 58c (R)-3-(3,5-Dibrom-phenyl)-2-hydroxy-propionsäure

Analog Beispiel 1c konnte aus 12.0 g (37.3 mmol) 3-(3,5-Dibrom-phenyl)-2-oxo-propionsäure und 15.1 g (47.1 mmol) (1R)-B-Chlordiisopinocampheylboran das Produkt erhalten werden.

| | |
|---|---|
| Ausbeute: | 4.1 g (34% der Theorie) |
| ESI-MS | (M-H)⁻ = 321/323/325 (2 Br) |

### 58d (R)-3-(3,5-Dibrom-phenyl)-2-hydroxy-propionsäuremethylester

Analog Beispiel 46d konnte aus 4.0 g (12.4 mmol) (R)-3-(3,5-Dibrom-phenyl)-2-hydroxy-propionsäure das Produkt erhalten werden, wobei zur Veresterung methanolische NCl (6 M) verwendet wurde.

| | |
|---|---|
| Ausbeute: | 4.0 g (96% der Theorie) |
| R_{f} = | 0.9 (Kieselgel, DCM/MeOH/AcOH 90:10:1) |

### 58e 4-(2-Oxo-1,2,4,5-tetrahydro-1,3-benzdiazepin-3-yl)-piperidin-1-carbonsäure-(R)-2-(3,5-dibrom-phenyl)-1-methoxycarbonyl-ethylester

Analog Beispiel 31f konnte aus 3.40 g (10.06 mmol) (R)-3-(3,5-Dibrom-phenyl)-2-hydroxy-propionsäuremethylester und 2.46 g (10.03 mmol) 3-Piperidin-4-yl-1,3,4,5-tetrahydro-1,3-benzdiazepin-2-on das Produkt erhalten werden.

| | |
|---|---|
| Ausbeute: | 2.0 g (33% der Theorie) |
| ESI-MS | (M+H)⁺ = 608/610/612 (2 Br) |
| R_{f} = | 0.2 (Kieselgel, *n*-Hexan/EtOAc 3:7) |
| Retentionszeit (HPLC): | 22.6 min (Methode F) |

### 58f 4-(2-Oxo-1,2,4,5-tetrahydro-1,3-benzdiazepin-3-yl)-piperidin-1-carbonsäure-(R)-1-carboxy-2-(3,5-dibrom-phenyl)-ethylester

Zu einer Lösung von 2.0 g (3.3 mmol) 4-(2-Oxo-1,2,4,5-tetrahydro-1,3-benzdiazepin-3-yl)-piperidin-1-carbonsäure-(R)-2-(3,5-dibrom-phenyl)-1-methoxycarbonyl-ethylester in 12.5 mL THF wurde eine Lösung von 118 mg (4.9 mmol) LiOH in 5 mL Wasser gegeben und 2 h bei RT gerührt. Das Reaktionsgemisch wurde i. vac. eingeengt, der Rückstand mit Wasser und TBME versetzt, die wässrige Phase mit konz. HCl auf pH 2-3 eingestellt und mit DCM extrahiert. Die vereinigten organischen Phasen wurden mit gesättigter NaCl-Lösung gewaschen, über Na₂SO₄ getrocknet und i. vac. bis zur Trockene eingeengt.

| | |
|---|---|
| Ausbeute: | 1.9 g (97% der Theorie) |
| ESI-MS | (M+H)⁺ = 594/596/598 (2 Br) |
| R_{f} = | 0.25 (Kieselgel, DCM/MeOH 9:1) |
| Retentionszeit (HPLC): | 19.1 min (Methode F) |

### 58g 4-(2-Oxo-1,2,4,5-tetrahydro-1,3-benzdiazepin-3-yl)-piperidin-1-carbonsäure-(R)-1-(3,5-dibrom-benzyl)-2-(4-dimethylamino-piperidin-1-yl)-2-oxo-ethylester

Eine Lösung von 150 mg (0.25 mmol) 4-(2-Oxo-1,2,4,5-tetrahydro-1,3-benzdiazepin-3-yl)-piperidin-1-carbonsäure-(R)-1-carboxy-2-(3,5-dibrom-phenyl)-ethylester, 90 mg (0.28 mmol) TBTU, 95 µL (0.55 mmol) Ethyldiisopropylamin und 38 mg (0.28 mmol) HOBt in 6 mL DMF wurde 90 min bei RT gerührt. Dann erfolgte die Zugabe von 42 mg (0.33 mmol) 4-Dimethylamino-piperidin und das Reaktionsgemisch wurde 16 h bei RT gerührt. Die Reaktionslösung wurde mit Wasser versetzt, die organische Phase eingeengt und der Rückstand chromatographisch (Kieselgel, DCM/MeOH/NH₃ 95:5:0.5) gereinigt.

| | |
|---|---|
| Ausbeute: | 140 mg (79 % der Theorie) |
| ESI-MS | (M+H)⁺ = 704/706/708 (2 Br) |
| R_{f} = | 0.35 (Kieselgel, DCM/MeOH/NH₃ 90:10:1) |
| Retentionszeit (HPLC): | 12.0 min (Methode F) |

Analog wurden folgende Verbindungen aus jeweils 150 mg 4-(2-Oxo-1,2,4,5-tetrahydro-1,3-benzdiazepin-3-yl)-piperidin-1-carbonsäure-(R)-1-carboxy-2-(3,5-dibrom-phenyl)-ethylester und der entsprechenden Menge an Amin hergestellt:

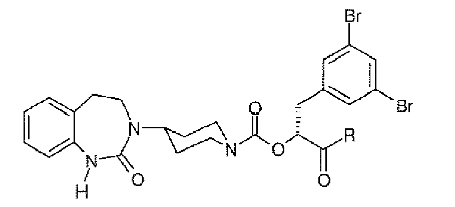

| **Beispiel** | **R** | **Ausbeute (%)** | **Massen-spektrum** | **Retentionszeit HPLC** **(Methode)** |
|---|---|---|---|---|
| **59** | 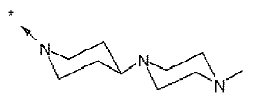 | 73 | 759/761/763 [M+H]⁺ | 10.4 min (F) |
| **60** | 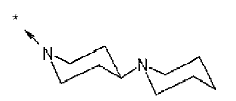 | 32 | 744/746/748 [M+H]⁺ | 12.8 min (F) |
| **61** | 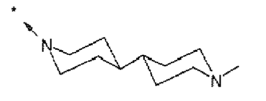 | 78 | 758/760/762 [M+H]⁺ | 12.9 min (F) |
| **62** | 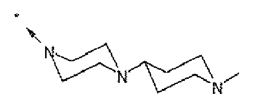 | 67 | 759/761/763 [M+H]⁺ | 9.3 min (F) |
| **63** | 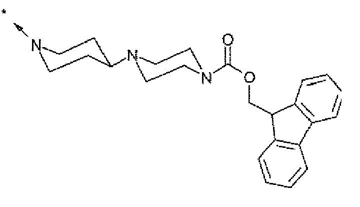 | 91 | 745/747/749 [M+H-Fmoc]⁺ | 17.6 min (F) |
| **64** | 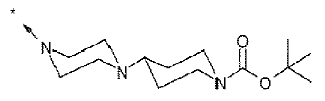 | 84 | 845/847/849 [M+H]⁺ | 14.9 min (F) |
| **65** | 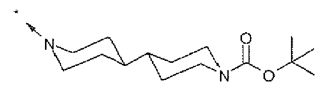 | 94 | 844/846/848 [M+H]⁺ | 27.2 min (F) |

### Beispiel 66

### 4-(2-Oxo-1,2,4,5-tetrahydro-1,3-benzdiazepin-3-yl)-piperidin-1-carbonsäure-(R)-1-(3,5-dibrom-benzyl)-2-oxo-2-(4-piperazin-1yl-piperidin-1-yl-ethylester

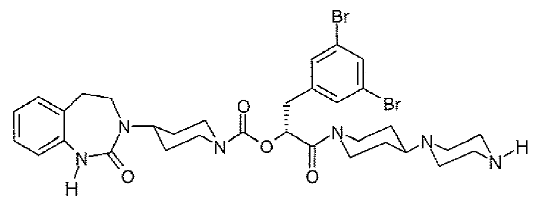

Eine Lösung von 300 mg (0.23 mmol) 4-(1-{(R)-3-(3,5-Dibrom-phenyl)-2-[4-(2-oxo-1,2,4,5-tetrahydro-1,3-benzdiazepin-3-yl)-piperidin-1-carbonyloxy]-propionyl}-piporidin-4-yl)-piperazin-1-carbonsäure-9*H*-fluoren-9-ylmethyloster (Beispiel 63) in 4 mL Piperidin wurde 1 h bei RT gerührt. Die Reaktionslösung wurde zur Trockene eingeengt und der Rückstand chromatographisch (Kieselgel, Gradient DCM zu DCM/MeOH/NH₃ 90: 10:1) gereinigt.

| | |
|---|---|
| Ausbeute: | 134 mg (79 % der Theorie) |
| ESI-MS: | (M+H)⁺ = 745/747/749 (2 Br) |
| Retentionszeit (HPLC): | 9.7 min (Methode F) |

### Beispiel 67

### 4-(2-Oxo-1,2,4,5-tetrahydro-1,3-benzdiazepin-3-yl)-piperidin-1-carbonsäure-(R)-1-(3,5-dibrom-benzyl)-2-oxo-2-(4-piperidin-4-yl-piperazin-1-yl)-ethylester

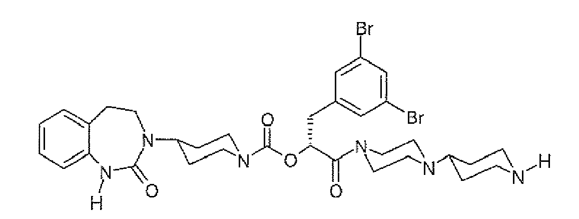

Zu einer Lösung von 180 mg (0.21 mmol) 4-(2-Oxo-1,2,4,5-tetrahydro-1,3-benzdiazepin-3-yl)-piperidin-1-carbonsäure-(R)-1-(3,5-dibrom-benzyl)-2-[4-(1-*tert-*butoxy-carbonyl-piperidin-4-yl)-piperazin-1-yl]-2-oxo-ethylester (Beispiel 64) in 2 mL Wasser wurden 1.8 mL HCl (3.2 M) gegeben und das Reaktionsgemisch 3 h bei RT gerührt. Man versetzte mit 25 mL EtOAc und 20 mL 17% Na₂CO₃-Lösung, trennte die organische Phase ab, extrahierte die wässrige Phase erneut mit 25 mL EtOAc, wusch die vereinigten organischen Phasen mit 10 mL gesättigter NaCl-Lösung und trocknete über Na₂SO₄. Nach Entfernen des Trocken- und Lösungsmittels wurde der Rückstand in Diethylether suspendiert, die organische Phase abdekantiert und der Rückstand getrocknet.

| | |
|---|---|
| Ausbeute: | 130 mg (82% der Theorie) |
| ESI-MS: | (M+H)⁺ = 745/747/749 (2 Br) |
| Retentionszeit (HPLC): | 9.3 min (Methode F) |

### Beispiel 68

### 4-(2-Oxo-1,2,4,5-tetrahydro-1,3-benzdiazepin-3-yl)-piperidin-1-carbonsäurg-(R)-2-4,4'-bipipendinyl-1-yl-1-(3,5-dibrom-benzyl)-2-oxo-2-ethylester

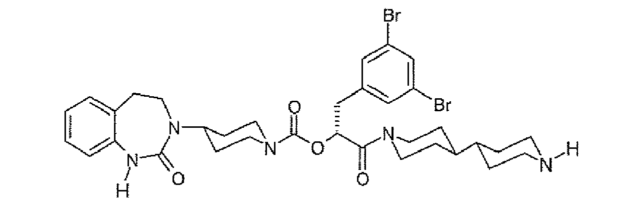

Eine Lösung von 160 mg (0.19 mmol) 1'-{(R)-3-(3,5-Dibrom-phenyl)-2-[4-(2-oxo-1,2,4,5-tetrahydro-1,3-benzdiazepin-3-yl)-piperidin-1-carbonyloxy]-propionyl}-4,4'-bipiperidinyl-1-carbon-säure-*tert*-butylester (Beispiel 65) in 2 mL Ameisensäure wurde 1 h bei RT gerührt. Die Reaktionslösung wurde i.vac. eingeengt, der Rückstand in DCM aufgenommen, die organische Phase mit 10% Na₂CO₃-Lösung gewaschen, filtriert und zur Trockene eingeengt. Der Rückstand wurde in 10% NaOH suspendiert, 1 h bei RT gerührt, der Niederschlag filtriert, mit wenig Wasser und Diethylether gewaschen und i.vac. getrocknet.

| | |
|---|---|
| Ausbeute: | 86 mg (61 % der Theorie) |
| ESI-MS: | (M+H)⁺ = 744/746/748 (2 Br) |
| Retentionszeit (HPLC): | 12.6 min (Methode F) |

### Beispiel 69

### 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-(4-methyl-piperazin-1-yl)-piperidin-1-yl]-2-oxo-ethylester

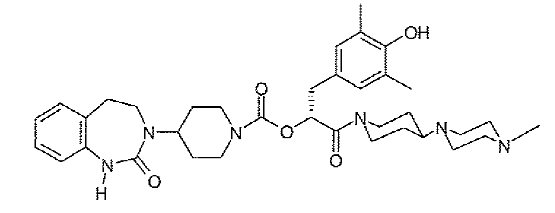

### 69a 2-Benzyloxy-5-brom-1,3-dimethylbenzol

Zu einer Lösung von 50.0 g (249 mmol) 2,6-Dimethyl-4-bromphenol in 500 mL DMF wurden 39.9 g (286 mmol) K₂CO₃ gegeben und 20 min nachgerührt. Dann wurden 34.0 mL (286 mmol) Benzylchlorid langsam zugetropft und das Reaktionsgemisch 3 h bei 100°C Badtemperatur gerührt. Nach beendeter Umsetzung wurde auf 500 mL Wasser gegossen und mit EtOAc erschöpfend extrahiert. Die organischen Phasen wurden vereint, über Na₂SO₄ getrocknet und i. vac. eingeengt.

| | |
|---|---|
| Ausbeute: | quantitativ |
| GC-MS: | (M⁺) = 290/292 (Br) |
| R_{f} = | 0.87 (Kieselgel, Cyc/EtOAc 3:1) |

### 69b 2-Acetylamino-3-(4-benzyloxy-3,5-dimethyl-phenyl)-acrylsäuremethylester

Unter Stickstoffatmosphäre wurde eine Mischung aus 40.0 g (137 mmol) 2-Benzyloxy-5-brom-1,3-dimethylbenzol und 24.1 g (165 mmol) 2-Acetylamino-acrylsäuremethylester in 420 mL Triethylamin und 200 mL Acetonitril mit 3.5 g (11.2 mmol) Tri-*o*-tolyl-phosphan und 2.5 g (11.1 mmol) Pd(OAc)₂ versetzt und 18 h bei 80°C gerührt. Der Niederschlag wurde abgesaugt, das Filtrat i. vac. eingeengt und mit 800 mL DCM und 800 mL Wasser versetzt. Die organische Phase wurde abgetrennt, über Na₂SO₄ abgesaugt, das Lösungsmittel i.vac. entfernt, der Rückstand mit EtOAc verrührt, abgesaugt und i. vac getrocknet.

| | |
|---|---|
| Ausbeute: | 31.1 g (64% der Theorie) |
| ESI-MS: | (M+H)⁺ = 354 |
| Retentionszeit (HPLC-MS): | 8.6 min (Methode A) |

### 69c 3-(4-Benzyloxy-3,5-dimethyl-phenyl)-2-oxo-propionsäure

31.1 g (88.1 mmol) 2-Acetylamino-3-(4-benzyloxy-3,5-dimethyl-phenyl)-acrylsäuremethylester in 150 mL 1,4-Dioxan wurden mit 125 mL 4 M HCl versetzt, 7 h unter Rückfluß und über Nacht bei RT gerührt. Der Niederschlag wurde abgesaugt, mit Wasser gewaschen und bei 45°C im Vakuumtrockenschrank getrocknet.

| | |
|---|---|
| Ausbeute: | 14.3 g (54 % der Theorie) |
| EI-MS: | (M)⁺ = 298 |
| Retentionszeit (HPLC-MS): | 9.0 min (Methode A) |

### 69d (R)-3-(4-Benzoyl-3,5-dimethyl-phenyl)-2-hydroxy-propionsäure

Unter einer Stickstoffatmosphäre wurde eine Lösung von 14.3 g (47.8 mmol) 3-(4-Benzyloxy-3,5-dimethyl-phenyl)-2-oxo-propionsäure und 8.3 mL (59.8 mmol) Triethylamin in 170 mL THF bei -35°C mit einer Lösung von 22.1 (69.0 mmol) (1R)-B-Chlordiisopinocampheylboran in 70 mL THF innerhalb von 30 min versetzt. Nach beendeter Zugabe wurde das Kältebad entfernt und die Reaktionslösung über Nacht bei RT gerührt. Das Reaktionsgemisch wurde bei 0°C mit 70 mL 1 M NaOH alkalisch gestellt, mit 100 mL TBME versetzt, 15 min nachgerührt und die Phasen getrennt. Die organische Phase wurde mit 50 mL Wasser und dreimal mit je 50 mL 1 M NaOH gewaschen. Die vereinigten wässrigen Phasen wurden mit halbkonz. HCl sauer gestellt, mit EtOAc erschöpfend extrahiert und die vereinigten organischen Phasen über Na₂SO₄ getrocknet. Nach Entfernen des Trocken- und Lösungsmittels wurde der Rückstand ohne Reinigung weiter umgesetzt.

| | |
|---|---|
| Ausbeute: | 14.0 g (98% der Theorie) |
| ESI-MS: | (M-H)⁻ = 299 |
| Retentionszeit (HPLC-MS): | 7.9 min (Methode A) |

### 69e (R)-3-(4-Benzyloxy-3,5-dimethyl-phenyl)-2-hydroxy-propionsäuremethylester

Zu einer auf 0°C gekühlten Lösung von 14.0 g (23.3 mmol) (R)-3-(4-Benzoyl-3,5-dimethyl-phenyl)-2-hydroxy-propionsäure in 150 mL MeOH wurden tropfenweise 2.0 mL (27.4 mmol) SOCl₂ zugegeben und das Reaktionsgemisch 1 h bei RT nachgerührt. Die Reaktionslösung wurde i. vac. eingeengt und der Rückstand chromatographisch (Kieselgel, Cyc/EtOAc 3:1) gereinigt.

| | |
|---|---|
| Ausbeute: | 5.7 g (78% der Theorie) |
| ESI-MS: | (M+NH₄)⁺ = 332 |
| Retentionszeit (HPLC-MS): | 9.1 min (Methode A) |

### 69f 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-methoxycarbonyl-ethylester

Unter Stickstoffatmosphäre wurde zu einer Lösung von 1.17 g (9.58 mmol) 4-Dimethylaminopyridin in 50 mL Pyridin 1.93 g (9.58 mmol) Chlorameisensäure-4-nitrophenylester gegeben, 1.5 h bei RT gerührt, mit 3.0 g (9.58 mmol) (R)-3-(4-Benzyloxy-3,5-dimethyl-phenyl)-2-hydroxy-propionsäuremethylester versetzt und 20 min bei RT gerührt. Anschließend wurden 2.35 g (9.58 mmol) 3-Piperidin-4-yl-1,3,4,5-tetrahydro-1,3-benzdiazepin-2-on zugegeben und 20 h bei RT gerührt. Das Reaktionsgemisch wurde i. vac. eingeengt, der Rückstand in EtOAc aufgenommen, die organische Phase mit 10% KHSO₄ und gesättigter NaHCO₃-Lösung gewaschen und über Na₂SO₄ getrocknet. Nach Entfernen des Trocken- und Lösungsmittels wurde der Rückstand chromatographisch (Kieselgel, Gradient Cyc/EtOAc 1:1 zu 1:2 ) gereinigt.

| | |
|---|---|
| Ausbeute: | 3.21 g (57% der Theorie) |
| ESI-MS: | (M+H)⁺ = 586 |
| Retentionszeit (HPLC-MS): | 10.4 min (Methode A) |

### 69g 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-carboxy-ethylester

Eine Lösung von 3.21 g (5.48 mmol) 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-methoxycarbonyl-ethylester in 80 mL THF wurde mit einer Lösung von 200 mg (8.35 mmol) LiOH in 40 mL Wasser versetzt und 1 h bei RT gerührt. Das Reaktionsgemisch wurde i. vac. eingeengt, der Rückstand in 100 mL Wasser aufgenommen, mit 2 M HCl sauer gestellt, der Niederschlag abgesaugt und im Vakuumtrockenschrank bei 40°C getrocknet.

| | |
|---|---|
| Ausbeute: | quantitativ |
| ESI-MS: | (M+H)⁺ = 572 |
| Retentionszeit (HPLC-MS): | 9.2 min (Methode A) |

### 69h 4-(2-Oxo-1,2,4,5-tetrahydro-1,3-benzdiazepin-3-yl)-piporidin-1-carbonsäure-(R)-2-(4-hydroxy-3,5-dimethyl-phenyl)-1-carboxy-ethylester

3.72 g (6.51 1 mmol) 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-carboxy-ethylester in 50 mL DCM wurden mit 300 mg 10% Pd/C versetzt und bei RT und 3 bar Wasserstoff bis zum Reaktionsstillstand geschüttelt. Der Katalysator wurde abgesaugt und das Lösungsmittel i. vac. eingeengt. Der Rückstand wurde mit DIPE verrieben und abgesaugt.

| | |
|---|---|
| Ausbeute: | 2.41 g (77% der Theorie) |
| ESI-MS: | (M+H)⁺ = 482 |
| Retentionszeit (HPLC-MS): | 7.0 min (Methode A) |

### 69i 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-(4-methyl-piperazin-1-yl)-piperidin-1 -yl]-2-oxo-ethylester

Eine Lösung von 70 mg (0.15 mmol) 4-(2-Oxo-1,2,4,5-tetrahydro-1,3-benzdiazepin-3-yl)-piperidin-1-carbonsäure-(R)-2-(4-hydroxy-3,5-dimethyl-phenyl)-1-carboxy-ethylester, 51 mg (0.16 mmol) TBTU und 26 µL (0.18 mmol) Triethylamin in 1 mL DMF wurde 1 h bei RT gerührt. Dann erfolgte die Zugabe von 27 mg (0.15 mmol) 1-Methyl-4-piperidin-4-yl-piperazin und das Reaktionsgemisch wurde 16 h bei RT gerührt. Die Reaktionslösung wurde über einen Spritzenfilter filtriert und ohne weitere Aufarbeitung direkt via HPLC gereinigt. Die das Produkt enthaltenden Fraktionen wurden vereinigt und lyophilisiert.

| | |
|---|---|
| Ausbeute: | 39 mg (42 % der Theorie) |
| ESI-MS: | (M+H)⁺ = 647 |
| Retentionszeit (HPLC-MS): | 5.3 min (Methode A) |

Analog wurden folgende Verbindungen aus jeweils 70 mg (Beispiele 70 bis 76), aus jeweils 100 mg (Beispiele 77 und 78) bzw. aus 400 mg (Beispiel 79) 4-(2-Oxo-1,2,4,5-tetrahydro-1,3-benzdiazepin-3-yl)-piperidin-1-carbonsäure-(R)-2-(4-hydroxy-3,5-dimethyl-phenyl)-1-carboxy-ethylester und der entsprechenden Menge an Amin hergestellt:

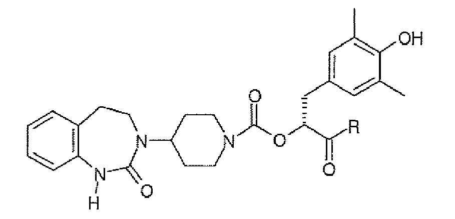

| **Beispiel** | **R** | **Ausbeute (%)** | **Massen-spektrum** | **Retentionszeit HPLC** **(Methode)** |
|---|---|---|---|---|
| **70** | 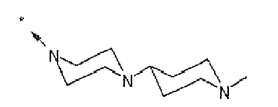 | 47 | 647 [M+H]⁺ | 4.9 min (A) |
| **71** | 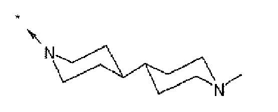 | 41 | 646 [M+H]⁺ | 5.8 min (A) |
| **72** | 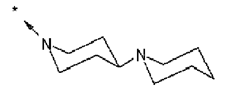 | 32 | 632 [M+H]⁺ | 5.8 min (A) |
| **73** | 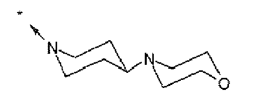 | 38 | 634 [M+H]⁺ | 5.6 min (A) |
| **74** | 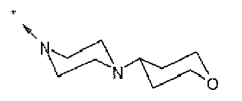 | 39 | 634 [M+H]⁺ | 5.7 min (A) |
| **75** | 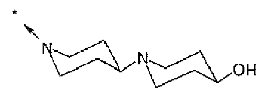 | 37 | 648 [M+H]⁺ | 5.5 min (A) |
| **76** | 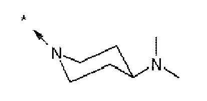 | 38 | 592 [M+H]⁺ | 5.6 min (A) |
| **77** | 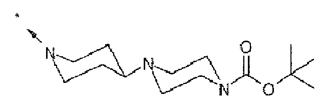 | 21 | 733 [M+H]⁺ | 6.4 min (A) |
| **78** | 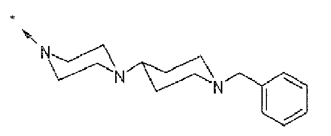 | 36 | 723 [M+H]⁺ | 5.3 min (A) |
| **79** | 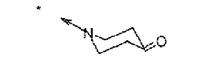 | 71 | 563 [M+H]⁺ | 6.9 min (A) |

### Beispiel 80

### 4-(2-Oxo-1,2,4,5-tetrahydro-1,3-benzdiazepin-3-yl)-pipendin-1-carbonsäure-(R)-1-(4-hydroxy-3,5-dimethyl-benzyl)-2-oxo-2-(4-piperazin-1-yl-piperidin-1-yl)-ethylester

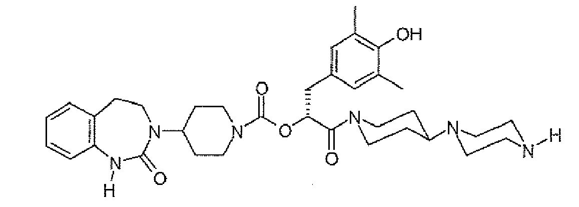

Eine Lösung von 32 mg (0.04 mmol) 4-(1-{(R)-3-(4-Hydroxy-3,5-dimethyl-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-*tert*-butylester (Beispiel 77) in 5 mL 2 M HCl wurde 20 h bei RT gerührt und und anschliessend lyophilisiert, wobei das Produkt als Bis-Hydrochlorid anfiel.

| | |
|---|---|
| Ausbeute: | quantitativ |
| ESI-MS: | (M+H)⁺ = 633 |
| Retentionszeit (HPLC-MS): | 5.0 min (Methode A) |

### Beispiel 81

### 4-(2-Oxo-1,2,4,5-tetrahydro-1,3-benzdiazepin-3-yl)-piperidin-1-carbonsäure-(R)-1-(4hydroxy-3,5-dimethyl-benzyl)-2-oxo-2-(4-piperidin-4-yl-piperazin-1-yl)-ethylester

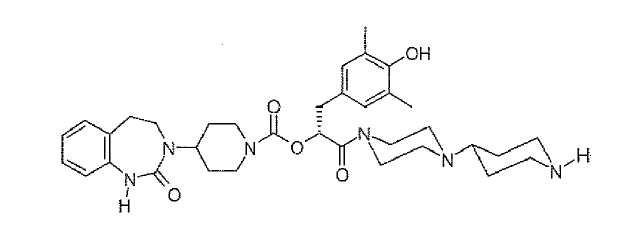

Eine Lösung von 54 mg (0.08 mmol) 4-(2-Oxo-1,2,4,5-tetrahydro-1,3-benzdiazepin-3-yl)-piperidin-1-carbonsäure-(R)-2-[4-(1-benzyl-piperidin-4-yl)-piperazin-1-yl]-1-(4-hydroxy-3,5-dimethyl-benzyl)-2-oxo-ethylester (Beispiel 78) in 5 mL MeOH wurden mit 20 mg 10% Pd/C versetzt und bei RT und 3 bar Wasserstoff bis zum Reaktionsstillstand geschüttelt. Der Katalysator wurde abgesaugt und das Lösungsmittel i.vac. eingeengt. Der Rückstand wurde mit DIPE verrieben, abgesaugt und im Hochvakuum getrocknet.

| | |
|---|---|
| Ausbeute: | 35.0 mg (74% der Theorie) |
| ESI-MS: | (M+H)⁺ = 633 |
| Retentionszeit (HPLC-MS): | 4.9 min (Methode A) |

### Beispiel 82

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-(4-hydroxy-4-methyl-[1,4']bipiperidinyl-1'-yl)-2-oxo-ethylester

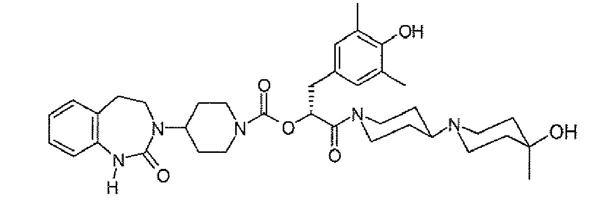

Eine Lösung von 50 mg (0.09 mmol) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-(4-oxo-piperidin-1-yl)-ethylester (Beispiel 79) in 1.5 mL DCM wurde mit 21 mg (0.18 mmol) 4-Mothyl-piperidin-4-ol und 10.3 µl (0.19 mmol) AcOH versetzt, auf 0°C abgekühlt und 2 h gerührt. Dann wurden 28 mg (0.19 mmol) Natrium-triacetoxyborhydrid zugegeben und über Nacht bei 0°C gerührt. Nach Entfernen des Lösungsmittels wurde der Rückstand mit 2 mL DMF versetzt und via HPLC gereinigt. Die das Produkt enthaltenden Fraktionen wurden vereinigt und lyophilisiert.

| | |
|---|---|
| Ausbeute: | 25 mg (42% der Theorie) |
| ESI-MS: | (M+H)⁺ = 662 |
| Retentionszeit (HPLC-MS): | 2.90 min (Methode E) |

### Beispiel 83

### 4-(2-Oxo-1,2,4,5-tetrahydro-1,3-benzdiazepin-3-yl)-piperidin-1-carbonsäure-(R)-2-(4,4-dimethyl-[1,4'] bipiperidinyl-1'-yl)-1-(4-hydroxy-3,5-dimethyl-benzyl)-2-oxo-ethylester

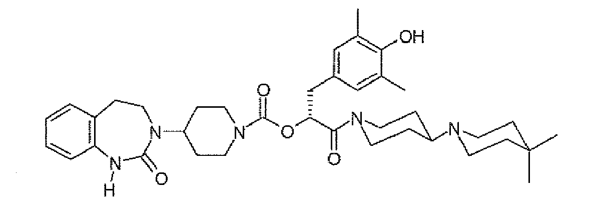

Analog Beispiel 82 konnte aus 50.0 mg (0.09 mmol) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-(4-oxo-piperidin-1-yl)-ethylester (Beispiel 79) und 31.0 mg (0.18 mmol) 4,4-Dimethylpiperidin das Produkt erhalten werden.

| | |
|---|---|
| Ausbeute: | 18.3 mg (31% der Theorie) |
| ESI-MS: | (M+H)⁺ = 660 |
| Retentionszeit (HPLC-MS): | 3.2 min (Methode E) |

### Beispiel 84

### 4-(2-Oxo-1,2,4,5-tetrahydro-1,3-benzdiazepin-3-yl)-piperidin-1-carbonsäure-(R)-2-(4-amino-4-methyl-[1,4']bipiperidinyl-1'-yl)-1-(4-hydroxy-3,5-dimethyl-benzyl)-2-oxo-ethylester

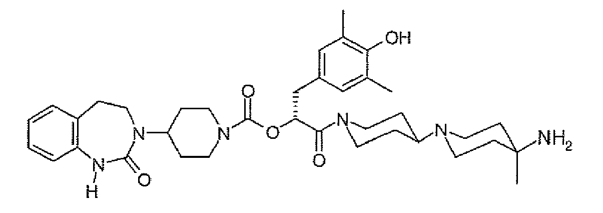

Eine Lösung von 150 mg (0.27 mmol) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-(4-oxo-piperidin-1-yl)-ethylester (Beispiel 79) in 4 mL DCM wurde mit 120 mg (0.53 mmol) (4-Methyl-piperidin-4-yl)-carbaminsäure-*tert*-butylester und 31 µL (0.56 mmol) AcOH versetzt, auf 0°C abgekühlt und 2 h gerührt. Dann wurden 85 mg (0.56 mmol) Natriumtriacetoxyborhydrid zugegeben und über Nacht bei 0°C gerührt. Dann wurde die Reaktionslösung mit 0.5 mL TFA versetzt und erneut über Nacht bei RT gerührt. Nach Entfernen der Lösemittel wurde der Rückstand in 2 mL DMF gelöst und via HPLC gereinigt. Die das Produkt enthaltenden Fraktionen wurden vereinigt und lyophilisiert, wobei das Produkt als TFA-Salz anfiel.

| | |
|---|---|
| Ausbeute: | 94 mg (46 % der Theorie) |
| ESI-MS: | (M+H)⁺ = 661 |
| Retentionszeit (HPLC-MS): | 2.50 min (Methode E) |

### Beispiel 85

### 4-(2-Oxo-1,2,4,5-tetrahydro-1,3-benzdiazepin-3-yl)-pipendin-1-carbonsäure-(R)-1-(4-amino-3,5-dimethyl-benzyl)-2-[4-(4-methyl-piperazin-1-yl)-piperidin-1-yl]-2-oxo-ethylester

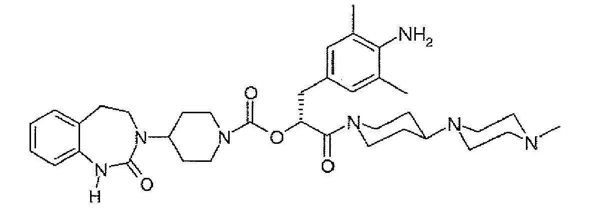

### 85a (Z,E)-2-Acetylamino-3-(4-amino-3,5-dimethyl-phenyl)-acrylsäuremethylester

Unter einer Stickstoffatmosphäre wurde zu einer Mischung aus 7.2 g (32.1 mmol) Pd(OAc)₂ und 10.1 g (32.1 mmol) Tri-*o*-tolyl-phosphan in 1.2 L Triethylamin und 600 mL Acetonitril zuerst eine Lösung von 90.0 g (441 mmol) 4-Brom-2,6-dimethylphenylamin in 200 mL Acetonitril gegeben und anschliessend eine Lösung von 65.0 g (445 mmol) 2-Acetylamino-acrylsäuremethylester in 200 mL Acetonitril zugetropft. Nach beendeter Zugabe wurde 18 h bei 80°C gerührt. Zur Vervollständigung der Reaktion wurde das Reaktionsgemisch erneut mit 4.0 g (17.8 mmol) Pd(OAc)₂ und 5.0 g (16.4 mmol) Tri-*o*-tolyl-phosphan versetzt und weitere 5 h bei 80°C gehalten. Man engt i.vac auf ca. 200 mL ein, versetzte den Rückstand mit 400 mL EtOAc, filtrierte den Rückstand (A) und trocknete die organische Phase über Na₂SO₄. Nach Entfernen des Trockenmittels durch Filtration über Aktivkohle wurde das Filtrat auf ca. 100 mL eingedampft, die ausgefallene Substanz abgesaugt, mit 30 mL EtOAc nach gewaschen und getrocknet.

Obiger Rückstand A wurde mit 1 L DCM, mit Na₂SO₄ und Aktivkohle versetzt und über Celite filtriert. Das Filtrat wurde eingeengt, der Rückstand mit 350 mL Diethylether versetzt, der entstandene Niederschlag abgesaugt, dieser mit 100 mL Diethylether gewaschen und getrocknet. Beide Produktfraktionen wurden vereinigt.

| | |
|---|---|
| Ausbeute: | 74.8 g (65% der Theorie) |
| ESI-MS: | (M+H)⁺ = 263 |
| R_{f} = | 0.51 (Kieselgel, EtOAc) |

### 85b 3-(4-Amino-3,5-dimethyl-phenyl)-2-oxo-propionsäure

Eine Suspension von 74.0 g (282 mmol) (Z,E)-2-Acetylamino-3-(4-amino-3,5-dimethyl-phenyl)-acrylsäuremethylester in 500 mL 1,4-Dioxan wurde auf 100°C erhitzt und mit 460 mL 4 M HCl versetzt, wobei sich eine Lösung bildete. Man erhitzte weitere 8 h bei 100°C und engte die abgekühlte Lösung i.vac. auf ca. 200 mL ein, wobei das Produkt kristallisierte. Man filtrierte, wusch den Rückstand mit 50 mL Wasser und trocknete das Produkt bei 50°C.

| | |
|---|---|
| Ausbeute: | 43.6 g (63% der Theorie) |
| ESI-MS: | (M+H)⁺ = 208 |
| R_{f} = | 0.68 (Kieselgel, PE/EtOAc 1:1) |

### 85c (R)-3-(4-Amino-3,5-dimethyl-phenyl)-2-hydroxy-propionsäuremethylester

Unter Stickstoffatmosphäre wurde eine Mischung aus 20.0 g (82.1 mmol) 3-(4-Amino-3,5-dimethyl-phenyl)-2-oxo-propionsäure und 25.7 mL (189 mmol) Triethylamin in 400 mL THF auf -35°C gekühlt. Dann wurde eine Lösung von 40.0 g (125 mmol) (1 R)-B-Chlordiisopinocampheylboran in 100 mL THF so zugetropft, dass die Reaktionstemperatur zwischen -35°C und -25°C blieb. Man hielt das Reaktionsgemisch 1 h bei dieser Temperatur, entfernte das Kältebad und ließ das Reaktionsgemisch über Nacht bei RT rühren. THF wurde i.vac. eingeengt, der Rückstand mit methanolischer HCl (1.25 M) versetzt und 2 h bei RT gerührt. Man engte i.vac. ein, nahm den Rückstand in 2 M HCl auf und extrahierte erschöpfend mit EtOAc. Die wässrige Phase wurde mit halbkonz. NaOH alkalisch gestellt und erschöpfend mit EtOAc extrahiert. Die vereinigten organischen Phasen wurden über Na₂SO₄ getrocknet, über Aktivkohle abgesaugt und eingeengt. Das Produkt konnte als braunes Öl erhalten werden.

| | |
|---|---|
| Ausbeute: | 8.3 g (45% der Theorie) |
| ESI-MS: | (M+H)⁺ = 224 |
| R_{f} = | 0.46 (Kieselgel, PE/EtOAc 1:1) |

### 85d 4-(2-Oxo-1,2,4,5-tetrahydro-1,3-benzdiazepin-3-yl)-piperidin-1-carbonsäure-(R)-2-(4-amino-3,5-dimethyl-phenyl)-1-methoxycarbonyl-ethylester

Analog Beispiel 31f konnte aus 4.0 g (17.9 mmol) (R)-3-(4-Amino-3,5-dimethylphenyl)-2-hydroxy-propionsäuremethylester und 4.8 g (19.6 mmol) 3-Piperidin-4-yl-1,3,4,5-tetrahydro-1,3-benzdiazepin-2-on das gewünschte Produkt erhalten werden.

| | |
|---|---|
| Ausbeute: | 3.2 g (36% der Theorie) |
| ESI-MS: | (M+H)⁺ = 495 |
| R_{f} = | 0.35 (Kieselgel, DCM/MeOH/NH₃ 90:10:1) |

### 85e 4-(2-Oxo-1,2,4,5-tetrahydro-1,3-benzdiazepin-3-yl)-piperidin-1-carbonsäure-(R)-2-(4-amino-3,5-dimethyl-phenyl)-1-carboxy-ethylester

Zu einer Lösung von 6.7 g (13.6 mmol) 4-(2-Oxo-1,2,4,5-tetrahydro-1,3-benzdiazepin-3-yl)-piperidin-1-carbonsäure-(R)-2-(4-amino-3,5-dimethyl-phenyl)-1-methoxycarbonyl-ethylester in 50 mL THF wurde eine Lösung von 500 mg (20.9 mmol) LiOH in 10 mL Wasser gegeben und das Reaktionsgemisch über Nacht bei RT gerührt. Zur Vervollständigung der Reaktion wurden weitere 300 mg (12.5 mmol) LiOH zugegeben und die Reaktionslösung 3 h bei 40°C gerührt. Man engte i.vac. ein, nahm den Rückstand in 15% K₂CO₃-Lösung auf und extrahierte erschöpfend mit DCM. Die wässrige Phase wurde mit 4 M HCl sauer gestellt, erschöpfend mit DCM extrahiert und die vereinigten organischen Phasen über Na₂SO₄ getrocknet. Nach Entfernen des Trocken- und Lösungsmittels wurde der Rückstand ohne Reinigung weiter umgesetzt.

| | |
|---|---|
| Ausbeute: | 4.2 g (65% der Theorie) |
| ESI-MS: | (M+H)⁺ = 481 |
| R_{f} = | 0.21 (Kieselgel, DCM/MeOH/Cyc/NH₃ 70:15:15:2) |

### 85f 4-(2-Oxo-1,2,4,5-tetrahydro-1,3-benzdiazepin-3-yl)-piperidin-1-carbonsäure-(R)-1-(4-amino-3,5-dimethyl-benzyl)-2-[4-(4-methyl-piperazin-1-yl)-piperidin-1-yl]-2-oxo-ethylester

Analog Beispiel 1h wurde aus 80 mg (0.17 mmol) 4-(2-Oxo-1,2,4,5-tetrahydro-1,3-benzdiazepin-3-yl)-piperidin-1-carbonsäure-(R)-2-(4-amino-3,5-dimethyl-phenyl)-1-carboxy-ethylester und 35 mg (0.19 mmol) 1-Methyl-4-piperidin-4-yl-piperazin das Produkt erhalten.

| | |
|---|---|
| Ausbeute: | 50 mg (47% der Theorie) |
| ESI-MS: | (M+H)⁺ = 646 |
| Retentionszeit (HPLC): | 4.9 min (Methode B) |

Analog wurden folgende Verbindungen aus jeweils 80 mg (Beispiele 86 bis 89) bzw. aus jeweils 100 mg (Beispiele 90 bis 92) 4-(2-Oxo-1,2,4,5-tetrahydro-1,3-benzdiazepin-3-yl)-piperidin-1-carbonsäure-(R)-2-(4-amino-3,5-dimethyl-phenyl)-1-carboxy-ethylester und der entsprechenden Menge an Amin hergestellt:

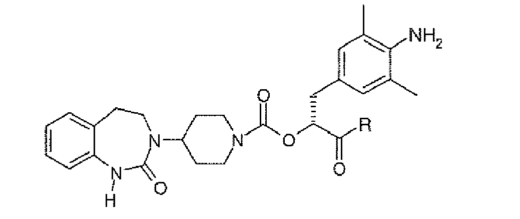

| **Beispiel** | **R** | **Ausbeute (%)** | **Massen-spektrum** | **Retentionszeit HPLC** **(Methode)** |
|---|---|---|---|---|
| **86** | 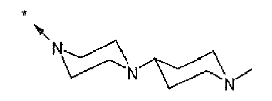 | 55 | 646 [M+H]⁺ | 3.6 min (B) |
| **87** | 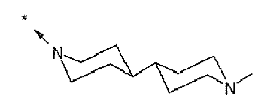 | 48 | 645 [M+H]⁺ | 4.5 min (B) |
| **88** | 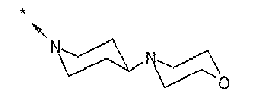 | 49 | 633 [M+H]⁺ | 4.3 min (B) |
| **89** | 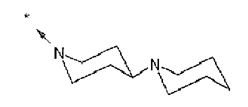 | 42 | 631 [M+H]⁺ | 4.6 min (B) |
| **90** | 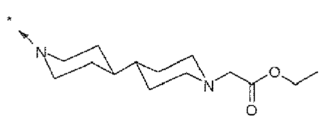 | 39 | 717 [M+H]⁺ | 4.7 min (B) |
| **91** | 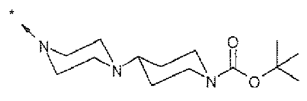 | 53 | 732 [M+H]⁺ | 5.2 min (B) |
| **92** | 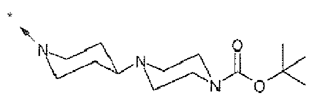 | 44 | 732 [M+H]⁺ | 5.0 min (B) |

### Beispiel 93

### 4-(2-Oxo-1,2,4,5-tetrahydro-1,3-benzdiazepin-3-yl)-piperidin-1-carbonsäure-(R)-1-(4-amino-3,5-dimethyl-benzyl)-2-oxo-2-(4-piperidin-4-yl-piperazin-1-yl)-ethylester

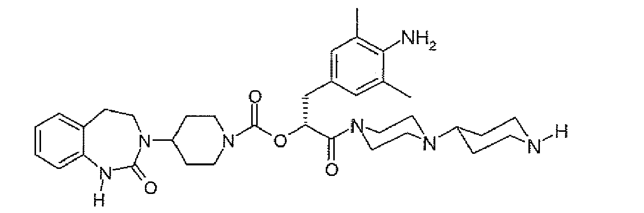

Eine Lösung von 80 mg (0.11 mmol) 4-(2-Oxo-1,2,4,5-tetrahydro-1,3-benzdiazepin-3-yl)-piperidin-1-carbonsäure-(R)-1-(4-amino-3,5-dimethyl-benzyl)-2-[4-(1-*tert*-butoxycarbonyl-piporidin-4-yl)-piperazin-1-yl]-2-oxo-ethylester (Beispiel 91) in 2 mL 4 M HCl wurde über Nacht bei RT gerührt. Das Reaktionsgemisch wurde mit festem K₂CO₃ alkalisch gestellt, erschöpfend mit DCM extrahiert und die vereinigten organischen Phasen i.vac. eingeengt. Weitere Reinigung erfolgte via HPLC. Die das Produkt enthaltenden Fraktionen wurden vereinigt und lyophilisiert.

| | |
|---|---|
| Ausbeute: | 4 mg (6% der Theorie) |
| ESI-MS: | (M+H)⁺ = 632 |
| Retentionszeit (HPLC): | 3.6 min (Methode A) |

### Beispiel 94

### 4-(2-Oxo-1,2,4,5-tetrahydro-1,3-benzdiazepin-3-yl)-piperidin-1-carbonsäure-(R)-1-(4-amino-3,5-dimethyl-benzyl)-2-oxo-2-(4-piperazin-1-yl-piperidin-1-yl)-ethylester

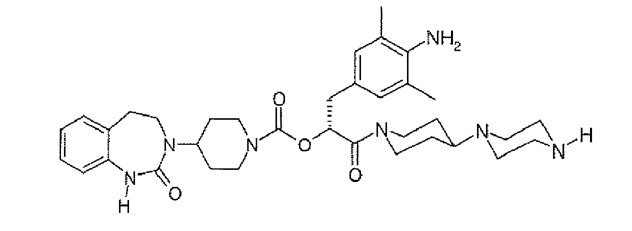

Analog Beispiel 93 wurde aus 65.0 mg (0.089 mmol) 4-(1-{(R)-3-(4-Amino-3,5-dimethyl-phenyl)-2-[4-(2-oxo-1,2,4,5-teirahydro-1,3-benzdiazepin-3-yl)-pipendin-1-carbonyloxy]-propionyl}-piperidin-4-yl)-piperazin-1-carbonsäure-*tert*-butylester (Beispiel 92) das Produkt erhalten.

| | |
|---|---|
| Ausbeute: | 7 mg (12% der Theorie) |
| ESI-MS: | (M+H)⁺ = 632 |
| Retentionszeit (HPLC): | 3.9 min (Methode A) |

### Beispiel 95

### 4-(2-Oxo-1,2,4,5-tetrahydro-1,3-benzdiazepin-3-yl)-piperidin-1-carbonsäure-(R)-1-(4-amino-3,5-dimethyl-benzyl)-2-(1'-carboxymethyl-4,4'-bipiperidinyl-1-yl)-2-oxo-ethylester

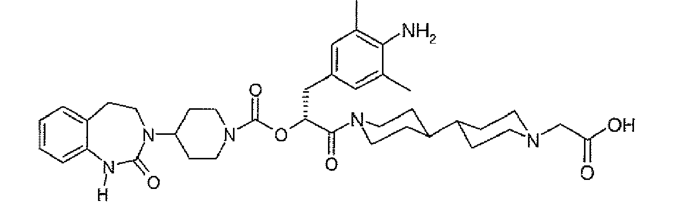

Zu einer Lösung von 55 mg (0.08 mmol) 4-(2-Oxo-1,2,4,5-tetrahydro-1,3-benzdiazepin-3-yl)-piperidin-1-carbonsäure-(R)-1-(4-amino-3,5-dimethyl-benzyl)-2-(1'-ethoxycarbonylmethyl-4,4'-bipiperidinyl-1-yl)-2-oxo-ethylester (Beispiel 90) in 5 mL THF wurde eine Lösung von 3.1 mg (0.13 mmol) LiOH in 1 mL Wasser gegeben und das Reaktionsgemisch über Nacht bei RT gerührt. Man engte i.vac. ein, nahm den Rückstand in 1 mL DMF auf und reinigte das Rohprodukt via HPLC. Die das Produkt enthaltenden Fraktionen wurden vereinigt und lyophilisiert.

| | |
|---|---|
| Ausbeute: | 22 mg (42% der Theorie) |
| ESI-MS: | (M+H)⁺ = 689 |
| Retentionszeit (HPLC): | 4.8 min (Methode A) |

### Beispiel 96

### 4-(2-Oxo-1,2,4,5-tetrahydro-1,3-benzdiazepin-3-yl)-piperidin-1-carbonsäure-(R)-1-(3-chlor-4-methyl-benzyl)-2-[4-(4-methyl-piperazin-1-yl)-piperidin-1-yl]-2-oxo-ethylester

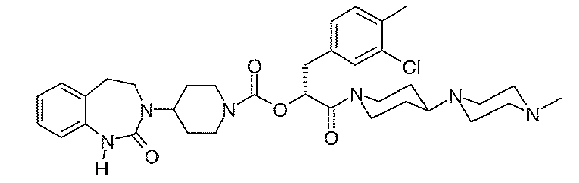

### 96a (Z,E)-2-Acetylamino-3-(3-chlor-4-methyl-phenyl)-acrylsäuremethylester

Unter Argonatmosphäre wurden zu einer Mischung aus 5.2 mL (39.0 mmol) 4-Brom-2-chlor-1-methyl-benzol und 10.0 g (69.9 mmol) 2-Acetylamino-acrylsäuremethylester in jeweils 100 mL Acetonitril und Triethylamin 0.65 g (2.9 mmol) Pd(OAc)₂ und 0.9 g (2.9 mmol) Tri-*o*-tolyl-phosphan zugegeben und das Reaktionsgemisch 20 h auf 90°C erhitzt. Nach dem Abkühlen wurde i.vac. eingeengt, der Rückstand mit DCM und Wasser versetzt, filtriert, die organische Phase abgetrennt und über Na₂SO₄ getrocknet. Nach Entfernen des Trocken- und Lösungsmittels wurde der Rückstand chromatographisch (Kieselgel, Gradient PE/EtOAc 1:1 zu EtOAc) gereinigt.

| | |
|---|---|
| Ausbeute: | 7.67 g (73% der Theorie) |
| ESI-MS: | (M+H)⁺ = 268/270 (Cl) |
| Schmelzpunkt: | 144-145°C |
| F_{f} = | 0.65 (Polygram, EtOAc) |

### 96b 3-(3-Chlor-4-methyl-phenyl)-2-oxo-propionsäure

Zu einer Lösung von 7.67 g (28.7 mmol) (Z,E)-2-Acetylamino-3-(3-chlor-4-methylphenyl)-acrylsäuremethylester in 150 mL EtOH wurden 100 mL 4 M HCl zugegeben und die Reaktionslösung 4 h unter Rückfluss erhitzt. Das EtOH wurde i.vac. entfernt, der Rückstand im Eisbad gekühlt, wobei der ölige Rückstand erstarrte. Der Feststoff wurde filtriert, mit Wasser gewaschen, mit PE verrührt, erneut abgesaugt, mit wenig PE nachgewaschen und getrocknet.
Die vereinigten Filtrate wurden eingeengt, in 50 mL MeOH gelöst, mit 50 mL 4 M NaOH versetzt und 2 h unter Rückfluss erhitzt. MeOH wurde i.vac. abgezogen, der wässrige Rückstand mit konz. HCl angesäuert und erschöpfend mit EtOAc extrahiert. Die vereinigten organischen Phasen wurden mit gesättigter NaCl-Lösung gewaschen und über Na₂SO₄ getrocknet. Nach Entfernen des Trocken- und Lösungsmittels wurde der Rückstand aus DCM umkristallisiert und mit der ersten Produktfraktion vereinigt.

| | |
|---|---|
| Ausbeute: | 2.02 g (33% der Theorie) |
| ESI-MS: | (M-H)⁻ = 211/213 (Cl) |

### 96c (R)-3-(3-Chlor-4-methyl-phenyl)-2-hydroxy-propionsäure

Unter Stickstoffatmosphäre wurde eine Mischung aus 2.6 g (12.23 mmol) 3-(3-Chlor-4-methyl-phenyl)-2-oxo-propionsäure und 2.1 mL (15.1 mmol) Triethylamin in 40 mL THF auf -35°C gekühlt. Dann wurde eine Lösung von 5.87 g (18.30 mmol) (1 R)-B-Chlordiisopinocampheylboran in 20 mL THF so zugetropft, dass die Reaktionstemperatur zwischen -35°C und -25°C blieb. Man entfernte das Kältebad und ließ das Reaktionsgemisch über Nacht bei RT rühren. Unter Eiskühlung wurden 30 mL 1 M NaOH und 60 mL Diethylether zugetropft und 15 min nachgerührt. Die wässrige Phase wurde abgetrennt, die organische Phase zweimal mit je 20 mL 1 M NaOH und einmal mit 20 mL Wasser extrahiert. Die vereinigten wässrigen Phasen wurden unter Eiskühlung mit halbkonz. HCl sauer gestellt, zweimal mit je 60 mL EtOAc extrahiert und die vereinigten organischen Phasen über Na₂SO₄ getrocknet. Nach Entfernen des Trocken- und Lösungsmittels wurde der Rückstand ohne Reinigung weiter umgesetzt.

| | |
|---|---|
| Ausbeute: | 2.8 g (85% der Theorie) |

### 96d (R)-3-(3-Chlor-4-methyl-phenyl)-2-hydroxy-propionsäuremethylester

Zu einer Lösung von 2.8 g (10.4 mmol) (R)-3-(3-Chlor-4-methyl-phenyl)-2-hydroxy-propionsäure in 100 mL MeOH wurden unter Eiskühlung 2.0 mL (27.4 mmol) SOCl₂ langsam zugetropft und die Reaktionslösung 1 h bei 0°C und 1 h bei RT nachgerührt. Das Reaktionsgemisch wurde i.vac. eingeengt, der Rückstand in EtOAc aufgenommen, die organische Phase mit gesättigter NaHCO₃-Lösung gewaschen und über Na₂SO₄ getrocknet. Nach Entfernen des Trocken- und Lösungsmittels wurde der Rückstand chromatographisch (Kieselgel, Gradient DCM zu DCM/MeOH 50:1) gereinigt.

| | |
|---|---|
| Ausbeute: | 2.12 g (89% der Theorie) |
| ESI-MS: | (M+H)⁺ = 229/231 (Cl) |
| R_{f} = | 0.34 (Polygram, DCM) |

### 96e 4-(2-Oxo-1,2,4,5-tetrahydro-1,3-benzdiazepin-3-yl)-pipendin-1-carbonsäure-(R)-2-(3-chlor-4-methyl-phenyl)-1-methoxycarbonyl-ethyleste

Analog Beispiel 31f konnte aus 2.1 g (9.18 mmol) (R)-3-(3-Chlor-4-methyl-phenyl)-2-hydroxy-propionsäuremethylester und 2.45 g (9.99 mmol) 3-Piperidin-4-yl-1,3,4,5-tetrahydro-1,3-benzdiazepin-2-on das gewünschte Produkt erhalten werden.

| | |
|---|---|
| Ausbeute: | 3.4 g (74% der Theorie) |
| ESI-MS: | (M+H)⁺ = 500/502 (Cl) |
| R_{f} = | 0.52 (Polygram, EtOAc) |

### 96f 4-(2-Oxo-1,2,4,5-tetrahydro-1,3-benzdiazepin-3-yl)-piperidin-1-carbonsäure-(R)-1-carboxy-2-(3-chlor-4-methyl-phenyl)-ethylester

Zu einer Lösung von 3.38 g (6.76 mmol) 4-(2-Oxo-1,2,4,5-tetrahydro-1,3-benzdiazepin-3-yl)-piperidin-1-carbonsäure-(R)-2-(3-chlor-4-methyl-phenyl)-1-methoxycarbonyl-ethylester in 30 mL THF wurde eine Lösung von 0.34 g (14.2 mmol) LiOH in 20 mL Wasser gegeben und das Reaktionsgemisch 7 h bei RT gerührt. Man engte i.vac. ein, verdünnte mit 80 mL Wasser, extrahierte die wässrige Phase zweimal mit je 50 mL Diethylether, säuerte die wässrige Phase mit 4 M HCl an und rührte 30 min nach. Das ausgefallene Produkt wurde abgesaugt, mit Wasser gewaschen und getrocknet.

| | |
|---|---|
| Ausbeute: | 3.2 g (97% der Theorie) |
| ESI-MS: | (M+H)⁺ = 486/488 (Cl) |

### 96g 4-(2-Oxo-1,2,4,5-tetrahydro-1,3-benzdiazepin-3-yl)-piperidin-1-carbonsäure-(R)-1-(3-chlor-4-methyl-benzyl)-2-[4-(4-methyl-piperazin-1-yl)-piperidin-1-yl]-2-oxo-ethylester

Eine Lösung von 100 mg (0.21 mmol) 4-(2-Oxo-1,2,4,5-tetrahydro-1,3-benzdiazepin-3-yl)-piperidin-1-carbonsäure-(R)-1-carboxy-2-(3-chlor-4-methyl-phenyl)-ethylester, 70.0 mg (0.22 mmol) TBTU und 35 µL (0.27 mmol) Triethylamin in 10 mL THF wurde 1 h bei RT gerührt. Dann erfolgte die Zugabe von 40 mg (0.22 mmol) 1-Methyl-4-piperidin-4-yl-piperazin und das Reaktionsgemisch wurde anschliessend über Nacht bei RT gerührt. Die Reaktionslösung wurde mit 20 mL halbgesättigter NaHCO₃-Lösung verrührt, zweimal mit je 20 mL EtOAc extrahiert und die vereinigten organischen Phasen über Na₂SO₄ getrocknet. Nach Entfernen des Trocken- und Lösungsmittels wurde der Rückstand chromatographisch (Alox, Aktivitätsstufe II-III DCM/MeOH 40:1) gereinigt.

| | |
|---|---|
| Ausbeute: | 123 mg (83% der Theorie) |
| ESI-MS: | (M+H)⁺ = 651/653 (Cl) |
| R_{f} = | 0.52 (Polygram-Alox, DCM/MeOH 25:1) |

Analog wurden folgende Verbindungen aus jeweils 100 mg 4-(2-Oxo-1,2,4,5-tetrahydro-1,3-benzdiazepin-3-yl)-piperidin-1-carbonsäure-(R)-1-carboxy-2-(3-chlor-4-methyl-phenyl)-ethylester und der entsprechenden Menge an Amin hergestellt:

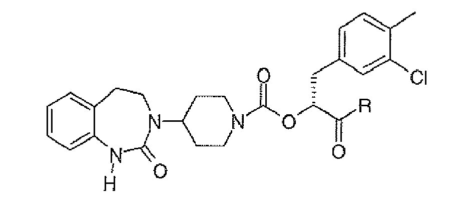

| **Beispiel** | **R** | **Ausbeute (%)** | **Massen-spektrum** | **R_{f} (Polygram-Alox, Laufmittel)** |
|---|---|---|---|---|
| **97** | 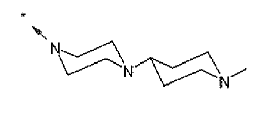 | 74 | 651/653 [M+H]⁺ | 0.48 DCM/MeOH 25:1 |
| **98** | 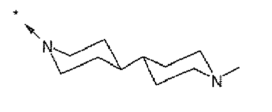 | 83 | 650/652 [M+H]⁺ | 0.55 (DCM/MeOH 25:1) |
| **99** | 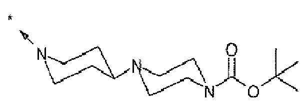 | 74 | 737/739 [M+H]⁺ | n.e |
| **100** | 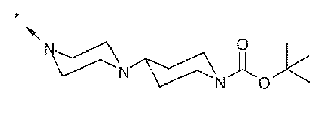 | 65 | 737/739 [M+H]⁺ | 0.33 (DCM/MeOH 50:1) |

### Beispiel 101

### 4-(2-Oxo-1,2,4,5-tetrahydro-1,3-benzdiazepin-3-yl)-piperidin-1-carbonsäure-(R)-1-(3-chlor-4-methyl-benzyl)-2-oxo-2-(4-piperazin-1-yl-piperidin-1-yl)-ethylester

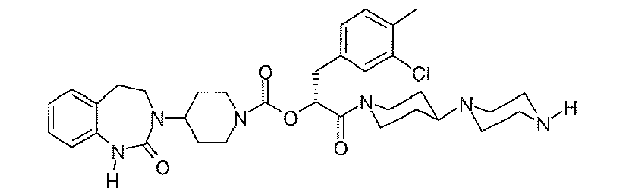

Zu einer Lösung von 130 mg (0.14 mmol) 4-(1-{(R)-3-(3-Chlor-4-methyl-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-*tert*-butylester (Beispiel 99) in 1 mL MeOH wurden 10 mL 1 M HCl zugegeben und das Reaktionsgemisch 24 h bei RT gerührt. Die Reaktionsmischung wurde ohne weitere Aufarbeitung direkt gefriergetrocknet. Das Produkt fiel als Bis-Hydrochlorid Salz an.

| | |
|---|---|
| Ausbeute: | 112 mg (95% der Theorie) |
| ESI-MS: | (M+H)⁺ = 637/639 (Cl) |

### Beispiel 102

### 4-(2-Oxo-1,2,4,5-tetrahydro-1,3-benzdiazepin-3-yl)-piperidin-1-carbonsäure-(R)-1-(3-chlor-4-methyl-benzyl)-2-oxo-2-(4-piperidin-4-yl-piperazin-1-yl)-ethylester

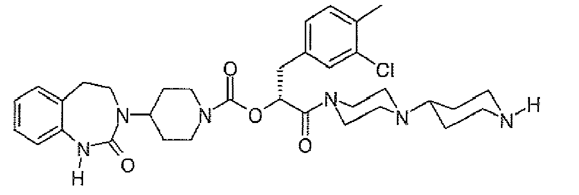

Analog Beispiel 101 konnte aus 110 mg (0.13 mmol) 4-(2-Oxo-1,2,4,5-tetrahydro-1,3-benzdiazepin-3-yl)-piperidin-1-carbonsäure-(R)-1-(3-chlor-4-methyl-benzyl)-2-[4-(1-*tert*-butoxy-carbonyl-piperidin-4-yl)-piperazin-1-yl]-2-oxo-ethylester (Beispiel 100) das Produkt erhalten werden, welches als Bis-Hydrochlorid Salz anfiel.

| | |
|---|---|
| Ausbeute: | 99 mg (93% der Theorie) |
| ESI-MS: | (M+H)⁺ = 637/639 (Cl) |

### Beispiel 103

### 4-(2-Oxo-1,2,4,5-tetrahydro-1,3-benzdiazepin-3-yl)-piperidin-1 -carbonsäure-(R)-1-(3-brom-4--methyl-benzyl)-2-[4-(4-methyl-piperazin-1-yl)-piperidin-1-yl]-2-oxo-ethylester

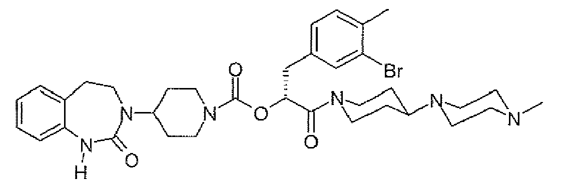

### 103a (Z,E)-2-Acetylamino-3-(3-brom-4-methyl-phenyl)-acrylsäure

Analog Beispiel 1a konnte aus 6.0 g (30.1 mmol) 3-Brom-4-methyl-benzaldehyd und 5.3 g (45.3 mol) N-Acetylglycin das Produkt erhalten werden.

| | |
|---|---|
| Ausbeute: | 4.7 g (52% der Theorie) |
| ESI-MS: | (M+H)⁺ = 298/300 (Br) |
| R_{f} = | 0.12 (Kieselgel, DCM/MeOH/Cyc/NH₃ 70:15:15:2) |

### 103b 3-(3-Brom-4-methyl-phenyl)-2-oxo-propionsäure

Zu einer auf 105°C erhitzten Lösung von 4.7 g (15.8 mmol) (Z,E)-2-Acetylamino-3-(3-brom-4-methyl-phenyl)-acrylsäure in 50 mL 1,4-Dioxan wurden 28 mL 4 M HCl gegeben und das Reaktionsgemisch weitere 5 h bei dieser Temperatur gehalten. Man entfernte das 1,4-Dioxan i.vac., versetzte den gekühlten Rückstand mit Wasser, filtrierte den entstandenen Niederschlag ab und trocknete diesen im Umlufttrockenschrank.

| | |
|---|---|
| Ausbeute: | 2.9 g (72% der Theorie) |
| ESI-MS: | (M-H)⁻ = 255/257 (Br) |
| R_{f} = | 0.18 (Kieselgel, DCM/MeOH/NH₃ 80:20:2) |

### 103c (R)-3-(3-Brom-4-methyl-phenyl)-2-hydroxy-propionsäure

Unter Stickstoffatmosphäre wurde eine Mischung aus 2.9 g (11.3 mmol) 3-(3-Brom-4-methyl-phenyl)-2-oxo-propionsäure und 2.0 mL (15.1 mmol) Triethylamin in 40 mL THF auf -35°C gekühlt. Dann wurde eine Lösung von 5.44 g (17.0 mmol) (1R)-B-Chlordiisopinocampheylboran in 20 mL THF so zugetropft, dass die Reaktionstemperatur zwischen -35°C und -25°C blieb; man hielt das Reaktionsgemisch 1 h bei dieser Temperatur, entfernte dann das Kältebad und ließ über Nacht bei RT rühren. Zur Vervollständigung der Reaktion wurden weitere von 3.0 g (9.4 mmol) (1R)-B-Chlordiisopinocampheylboran zugegeben und weitere 5 h gerührt. Unter Eiskühlung wurden 30 mL 1 M NaOH und 30 mL TBME zugetropft und 15 min nachgerührt. Die wässrige Phase wurde abgetrennt, die organische Phase mit 15 mL 1 M NaOH und mit 25 mL Wasser extrahiert. Die vereinigten wässrigen Phasen wurden unter Eiskühlung mit 2 M HCl sauer gestellt, dreimal mit je 40 mL TBME extrahiert und die vereinigten organischen Phasen über Na₂SO₄ getrocknet. Nach Entfernen des Trocken- und Lösungsmittels wurde der Rückstand ohne Reinigung weiter umgesetzt.

| | |
|---|---|
| Ausbeute: | 3.0 g (ca. 70% Produktanteil, 72% der Theorie) |
| ESI-MS: | (M-H)⁻ = 257/259 (Br) |
| R_{f} = | 0.12 (Kieselgel, PE/EtOAc 1:1) |

### 103d (R)-3-(3-Brom-4-methyl-phenyl)-2-hydroxy-propionsäuremethylester

Eine Lösung von 2.8 g (ca. 70%; 7.56 mmol) (R)-3-(3-Brom-4-methyl-phenyl)-2-hydroxy-propionsäure in methanolischer HCl (1.25 M) wurde 4 h bei RT gerührt. Man engte i.vac. ein und reinigte den Rückstand chromatographisch (Kieselgel, Gradient PE/EtOAc 9:1 zu PE/EtOAc 1:9).

| | |
|---|---|
| Ausbeute: | 1.6 g (77% der Theorie) |
| ESI-MS: | (M+H)⁺ = 273/275 (Br) |
| R_{f} = | 0.72 (Kieselgel, PE/EtOAc 1:1) |

### 103e 4-(2-Oxo-1,2,4,5-tetrahydro-1,3-benzdiazepin-3-yl)-piperidin-1-carbonsäure-(R)-2-(3-brom-4-methyl-phenyl)-1-methoxycarbonyl-ethylester

Analog Beispiel 1f konnte aus 1.5 g (5.49 mmol) (R)-3-(3-Brom-4-methyl-phenyl)-2-hydroxy-propionsäuremethylester und 1.7 g (5.52 mmol) 4-(2-Oxo-1,2,4,5-tetrahydro-1,3-benzdiazepin-3-yl)-pipendin-1-carbonylchlorid das Rohprodukt erhalten werden. Dieses wurde chromatographisch gereinigt (Kieselgel, Gradient DCM/MeOH/NH₃ 100:0:0 zu DCM/MeOH/NH₃ 0:95:5). Die Produktfraktionen wurden vereinigt, i.vac. eingeengt, mit DIPE verrieben, abgesaugt und getrocknet.

| | |
|---|---|
| Ausbeute: | 1.1 g (37% der Theorie) |
| ESI-MS: | (M+H)⁺ = 544/546 (Br) |
| R_{f} = | 0.70 (Kieselgel, DCM/MeOH/Cyc/NH₃ 70:15:15:2) |

### 103f 4-(2-Oxo-1,2,4,5-tetrahydro-1,3-benzdiazepin-3-yl)-piperidin-l-carbonsäure-(R)-2-(3-brom-4-methyl-phenyl)-1-carboxy-ethylester

Zu einer Lösung von 1.0 g (1.84 mmol) 4-(2-Oxo-1,2,4,5-tetrahydro-1,3-benzdiazepin-3-yl)-piperidin-1-carbonsäure-(R)-2-(3-brom-4-methyl-phenyl)-1-methoxycarbonyl-ethylester in 20 mL THF wurde eine Lösung von 70 mg (2.92 mmol) LiOH in 5 mL Wasser gegeben und das Reaktionsgemisch 5 h bei RT gerührt. Die Reaktionslösung wurde i.vac. eingeengt, der Rückstand mit DCM versetzt, die organische Phase mit 1 M KHSO₄-Lösung gewaschen und über Na₂SO₄ getrocknet. Nach Entfernen des Trocken- und Lösungsmittels wurde der Rückstand mit DIPE verrieben, abgesaugt und getrocknet.

| | |
|---|---|
| Ausbeute: | 0.95 g (98% der Theorie) |
| ESI-MS: | (M+H)⁺ = 530/532 (Br) |
| R_{f} = | 0.29 (Kieselgel, DCM/MeOH/Cyc/NH₃ 70:15:15:2) |

### 103g 4-(2-Oxo-1,2,4,5-tetrahydro-1,3-benzdiazepin-3-yl)-piperidin-1-carbonsäure-(R)-1-(3-brom-4-methyl-benzyl)-2-[4-(4-methyl-piperazin-1-yl)-piporidin-1-yl]-2-oxo-ethylester

Analog Beispiel 1h konnte aus 100 mg (0.19 mmol) 4-(2-Oxo-1,2,4,5-tetrahydro-1,3-benzdiazepin-3-yl)-piperidin-1-carbonsäure-(R)-2-(3-brom-4-methyl-phenyl)-1-carboxy-ethylester und 38 mg (0.21 mmol) 1-Methyl-4-piperidin-4-yl-piperazin das Produkt erhalten werden.

| | |
|---|---|
| Ausbeute: | 90 mg (69% der Theorie) |
| ESI-MS: | (M+H)⁺ = 695/697 (Br) |
| R_{f} = | 0.59 (Kieselgel, DCM/MeOH/Cyc/NH₃ 70:15:15:2) |

Analog wurden folgende Verbindungen aus jeweils 100 mg 4-(2-Oxo-1,2,4,5-tetrahydro-1,3-benzdiazepin-3-yl)-piperidin-1-carbonsäure-(R)-2-(3-brom-4-methylphenyl)-1-carboxy-ethylester und der entsprechenden Menge an Amin hergestellt:

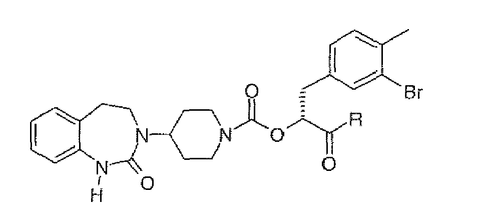

| **Beispiel** | **R** | **Ausbeute (%)** | **Massen-spektrum** | **R_{f}** **(Kieselgel, Laufmittel)** **oder** **Retentionszeit HPLC** **(Methode)** |
|---|---|---|---|---|
| **104** | 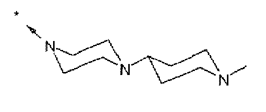 | 65 | 695/697 [M+H]⁺ | 0,58 (DCM/MeOH/ Cyc/NH₃ 70:15:15:2) |
| **105** | 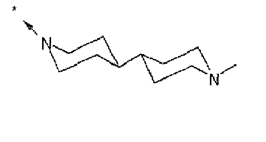 | 61 | 694/696 [M+H]⁺ | 0.57 (DCM/MeOH/ Cyc/NH₃ 70:15:15:2) |
| **105** | 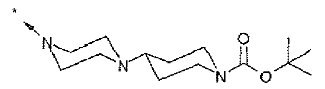 | 54 | 781/783 [M+H]⁺ | 7.1 min (A) |
| **106** | 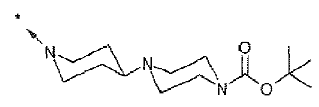 | 61 | 781/783 [M+H]⁺ | 6.9 min (A) |

### Beispiel 107

### 4-(2-Oxo-1,2,4,5-tetrahydro-1,3-benzdiazepin-3-yl)-piperidin-1-carbonsäure-(R)-1-(3-brom-4-methyl-benzyl)-2-oxo-2-(4-piperazin-1-yl-piperidin-1-yl)-ethylester

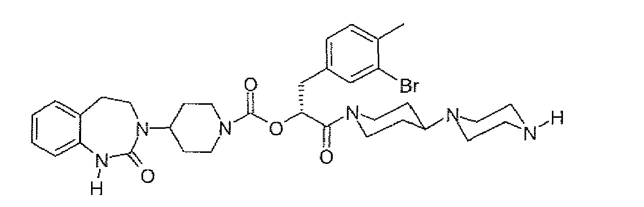

Analog Beispiel 93 konnte aus 90 mg (0.12 mmol) 4-(1-{(R)-3-(3-Brom-4-methylphenyl)-2-[4-(2-oxo-1,2,4,5-tetrahydro-1,3-benzdiazepin-3-yl)-piperidin-1-carbonyl-oxy]-propionyl}-piperidin-4-yl)-piporazin-1-carbonsäure-*tert*-butylester (Beispiel 106) das Produkt erhalten werden.

| | |
|---|---|
| Ausbeute: | 15 mg (19% der Theorie) |
| ESI-MS: | (M+H)⁺ = 681/683 (Br) |
| Retentionszeit (HPLC): | 5.7 min (Methode A) |

### Beispiel 108

### 4-(2-Oxo-1,2,4,5-tetrahydro-1,3-benzdiazepin-3-yl)-piperidin-1-carbonsäure-(R)-1-(3-brom-4-methyl-benzyl)-2-oxo-2-(4-piperidin-4-yl-piperazin-1-yl)-ethylester

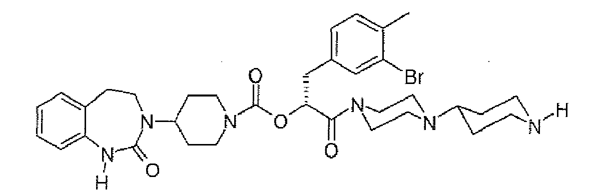

Analog Beispiel 93 konnte aus 80 mg (0.12 mmol) 4-(2-Oxo-1,2,4,5-tetrahydro-1,3-benzdiazepin-3-yl)-piperidin-1-carbonsäure-(R)-1-(3-brom-4-methyl-benzyl)-2-[4-(1-*tert*-butoxy-carbonyl-piperidin-4-yl)-piperazin-1-yl]-2-oxo-ethylester (Beispiel 105) das Produkt erhalten werden.

| | |
|---|---|
| Ausbeute: | 44 mg (63% der Theorie) |
| ESI-MS: | (M+H)⁺ = 681/683 (Br) |
| Retentionszeit (HPLC): | 5.5 min (Methode A) |

### Beispiel 109

### 4-(2-Oxo-1,2,4,5-tetrahydro-1,3-benzdiazepin-3-yl)-piperidin-1-carbonsäure-(R)-1-(6-amino-5-methyl-pyridin-3-ylmethyl)-2-[4-(4-methyl-piperazin-1-yl)-piperidin-1-yl]-2-oxo-ethylester

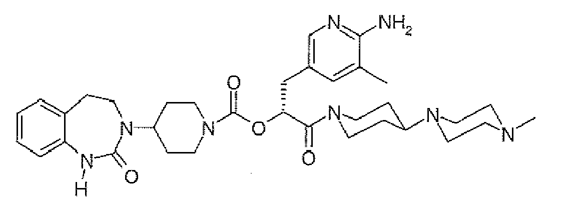

### 109a (Z,E)-2-Acetylamino-3-(6-amino-5-methyl-pyridin-3-yl)-acrylsäuremethylester

Unter Stickstoffatmosphäre wurde zu einer Mischung aus 33.6 g (180 mmol) 5-Brom-3-methyl-pyridin-2-ylamin, 28.9 g (198 mmol) 2-Acetylamino-acrylsäuremethylester, 4.42 g (14.4 mmol) Tri-*o*-tolyl-phosphan und 30.9 mL (180 mmol) Ethyldiisopropylamin in 500 mL Butyronitril 6.58 g (7.19 mmol) Tris-(dibenzylidenaceton)-Palladium gegeben und das Reaktionsgemisch 17 h auf 110°C erhitzt. Die Reaktionslösung wurde i.vac. eingeengt und der Rückstand mit ca. 500 mL Wasser verrührt. Der Niederschlag wurde filtriert, aus Acetonitril umkristallisiert und getrocknet.
Die wässrige Mutterlauge wurde eingeengt und der Rückstand chromatographisch (Kieselgel, EtOAc/MeOH/NH₃ 90:10:1) gereinigt. Die das Produkt enthaltenden Fraktionen wurden eingeengt, der Rückstand mit wenig Acetonitril verrieben, filtriert, getrocknet und mit obiger Produktfraktion vereinigt.

| | |
|---|---|
| Ausbeute: | 16.6 g (37% der Theorie) |
| ESI-NIS: | (M+H)⁺ = 250 |
| R_{f} = | 0.46 (Kieselgel, EtOAc/MeOH/NH₃ 90:10:1) |

### 109b 3-(6-Amino-5-methyl-pyridin-3-yl)-2-oxo-propionsäure

Zu einer Lösung von 15.57 g (62.46 mmol) (Z,E)-2-Acetylamino-3-(6-amino-5-methyl-pyridin-3-yl)-acrylsäuremethylester in 250 mL 1,4-Dioxan wurden 230 mL 4 M HCl gegeben, das Reaktionsgemisch für 1.5 h unter Rückfluss erhitzt und weitere 16 h bei RT gerührt. Man engte i.vac. ein, verrieb den Rückstand mit EtOAc/DIPE (1:1), filtrierte und trocknete im Umlufttrockenschrank. Das Produkt fiel als HydrochloridSalz an.

| | |
|---|---|
| Ausbeute: | 14.4 g (100% der Theorie) |
| ESI-MS: | (M+H)⁺ = 195 |
| Retentionszeit (HPLC): | 2.7 min (Methode A) |

### 109c (R)-3-(6-Amino-5-methyl-pyridin-3-yl)-2-hydroxy-propionsäuremethylester

Unter Argonatmosphäre wurde eine Mischung aus 13.8 g (59.9 mmol) 3-(6-Amino-5-methyl-pyridin-3-yl)-2-oxo-propionsäure und 17.5 mL (125.7 mmol) Triethylamin in 140 mL THF auf -35°C gekühlt. Dann wurde eine Lösung von 40.3 g (126 mmol) (1R)-B-Chlordiisopinocampheylboran in 210 mL THF so zugetropft, dass die Reaktionstemperatur zwischen -35°C und -25°C blieb; man hielt das Reaktionsgemisch 3 h bei dieser Temperatur ehe man bei 0-5°C mit 150 mL 1 M NaOH versetzte und das Reaktionsgemisch 2 h bei RT nachrührte. Man versetzte mit 200 mL TBME, trennte die organische Phase ab und säuerte diese mit 200 mL 2 M HCl an. Die wässrige Phase wurde abgetrennt, eingeengt, der Rückstand in THF/MeOH (1:1) aufgenommen, filtriert und das Filtrat anschliessend eingeengt. Das so erhaltene Rohprodukt (12.5 g) wurde in 300 mL MeOH gelöst, unter Eiskühlung tropfenweise mit 4.3 mL (59.3 mmol) SOCl₂ versetzt und weitere 2 h bei RT gerührt. Man engte i.vac ein und reinigte den Rückstand chromatographisch (Kieselgel, EtOAc/MeOH/NH₃ 90:10:1).

| | |
|---|---|
| Ausbeute: | 5.62 g (45% der Theorie) |
| ESI-MS: | (M+H)⁺ = 211 |
| Retentionszeit (HPLC): | 2.4 min (Methode A) |

### 109d 4-(2-Oxo-1,2,4,5-tetrahydro-1,3-benzdiazepin-3-yl)-piperidin-1-carbonsäure-(R)-2-(6-amino-5-methyl-pyridin-3-yl)-1-methoxycarbonyl-ethylester

Analog Beispiel 31f konnte aus 2.75 g (13.10 mmol) (R)-3-(6-Amino-5-methyl-pyridin-3-yl)-2-hydroxy-propionsäuremethylester und 3.21 g (13.10 mmol) 3-Piperidin-4-yl-1,3,4,5-tetrahydro-1,3-benzdiazepin-2-on das gewünschte Produkt erhalten werden.

| | |
|---|---|
| Ausbeute: | 1.38 g (22% der Theorie) |
| ESI-MS: | (M+H)⁺ = 482 |
| Retentionszeit (HPLC): | 4.9 min (Methode C) |

### 109e 4-(2-0xo-1,2,4,5-tetrahydro-1,3-benzdiazepin-3-yl)-piperidin-1-carbonsäure-(R)-2-(6-amino-5-methyl-pyridin-3-yl)-1-carboxy-ethylester

Zu einer Lösung von 1.27 g (2.64 mmol) 4-(2-Oxo-1,2,4,5-tetrahydro-1,3-benzdiazepin-3-yl)-piperidin-1-carbonsäure-(R)-2-(6-amino-5-methyl-pyridin-3-yl)-1-methoxycarbonyl-ethylester in 30 mL THF wurde eine Lösung von 100 mg (4.18 mmol) LiOH in 25 mL Wasser zugegeben und die Reaktionslösung für 1 h bei RT gerührt. Man engte i.vac. ein, nahm den Rückstand in Wasser auf, versetzte unter Rühren mit 2 M KHSO₄-Lösung, dekantierte die überstehende Lösung ab, trocknete den Rückstand, verrührte diesen mit THF und filtrierte das Produkt.

| | |
|---|---|
| Ausbeute: | 0.92 g (74% der Theorie) |
| ESI-MS: | (M+H)⁺ = 468 |
| Retentionszeit (HPLC): | 4.8 min (Methode A) |

### 109f 4-(2-Oxo-1,2,4,5-tetrahydro-1,3-benzdiazepin-3-yl)-piperidin-1-carbonsäure-(R)-1-(6-amino-5-methyl-pyridin-3-ylmethyl)-2-[4-(4-methyl-piperazin-1-yl)-piperidin-1-yl]-2-oxo-ethylester

Analog Beispiel 1h konnte aus 50 mg (0.11 mmol) 4-(2-Oxo-1,2,4,5-tetrahydro-1,3-benzdiazepin-3-yl)-piperidin-1-carbonsäure-(R)-2-(6-amino-5-methyl-pyridin-3-yl)-1-carboxy-ethylester und 20 mg (0.11 mmol) 1-Methyl-4-piperidin-4-yl-piperazin das Produkt erhalten werden.

| | |
|---|---|
| Ausbeute: | 6 mg (9% der Theorie) |
| ESI-MS: | (M+H)⁺ = 633 |
| Retentionszeit (HPLC): | 4.4 min (Methode A) |

Analog wurden folgende Verbindungen aus jeweils 50 mg 4-(2-Oxo-1,2,4,5-tetrahydro-1,3-benzdiazepin-3-yl)-piperidin-1-carbonsäure-(R)-2-(6-amino-5-methyl-pyridin-3-yl)-1-carboxy-ethylester und der entsprechenden Menge an Amin hergestellt:

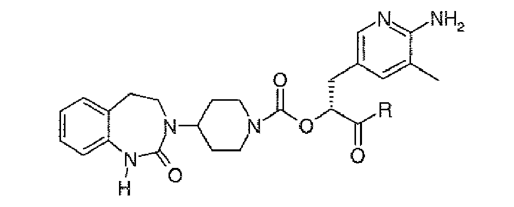

| **Beispiel** | **R** | **Ausbeute (%)** | **Massen-spektrum** | **Retentionszeit HPLC (Methode)** |
|---|---|---|---|---|
| **110** | 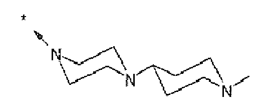 | 19 | 633 [M+H]⁺ | 4.4 min (D) |
| **111** | 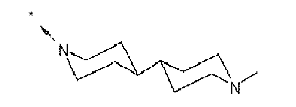 | 29 | 632 [M+H]⁺ | 5.6 min (D) |
| **112** | 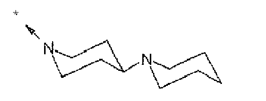 | 25 | 618 [M+H]⁺ | 5.4 min (D) |
| **113** | 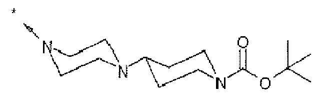 | 54 | 719 [M+H]⁺ | 6.8 min (A) |
| **114** | 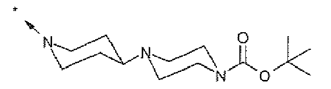 | 61 | 719 [M+H]⁺ | 6.5 min (D) |

### Beispiel 115

### 4-(2-Oxo-1,2,4,5-tetrahydro-1,3-benzdiazepin-3-yl)-piperidin-1-carbonsäure-(R)-1-(6-amino-5-methyl-pyridin-3-ylmethyl)-2-oxo-2-(4-piperazin-1-yl-piperidin-1-yl)-ethylester

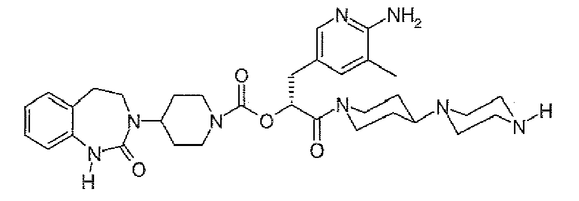

Eine Lösung von 75 mg (0.10 mmol) 4-(1-{(R)-3-(6-Amino-5-methyl-pyridin-3-yl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-*tert*-butylester (Beispiel 114) in 5 mL 2 M HCl wurde 20 h bei RT gerührt. Nach Lyophilisation des Reaktionsgemisches wurde der Rückstand in 1 mL DMF gelöst, mit 0.6 mL gesättigter K₂CO₃-Lösung alkalisch gestellt und via HPLC gereinigt. Die das Produkt enthaltenden Fraktionen wurden vereinigt und erneut lyophilisiert.

| | |
|---|---|
| Ausbeute: | 28 mg (44% der Theorie) |
| ESI-MS: | (M+H)⁺ = 619 |
| Retentionszeit (HPLC): | 3.8 min (Methode A) |

### Beispiel 116

### 4-(2-Oxo-1,2,4,5-tetrahydro-1,3-benzdiazepin-3-yl)-piperidin-1-carbonsäure-(R)-1-(6-amino-5-methyl-pyridin-3-ylmethyl)-2-oxo-2-(4-pjperidin-4-yl-piperazin-1-yl)-ethylester

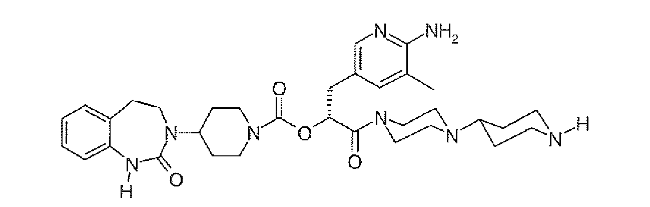

Analog Beispiel 115 konnte aus 66 mg (0.09 mmol) 4-(2-Oxo-1,2,4,5-tetrahydro-1,3-benzdiazepin-3-yl)-piperidin-1-carbonsäure-(R)-1-(6-amino-5-methyl-pyridin-3-ylmethyl)-2-[4-(1-*tert*-butoxy-carbonyl-piperidin-4-yl)-piperazin-1-yl]-2-oxo-ethylester (Beispiel 113) das Produkt erhalten werden.

| | |
|---|---|
| Ausbeute: | 29 mg (51 % der Theorie) |
| ESI-MS: | (M+H)⁺ = 619 |
| Retentionszeit (HPLC): | 3.5 min (Methode A) |

### Beispiel 117

### 4-(2-Oxo-1,2,4,5-tetrahydro-1,3-benzdiazepin-3-yl)-piperidin-1-carbonsäure-(R)-1-(4-hydroxy-benzyl)-2-[4-(4-methyl-piperazin-1-yl)-piperidin-1-yl]-2-oxo-ethylester

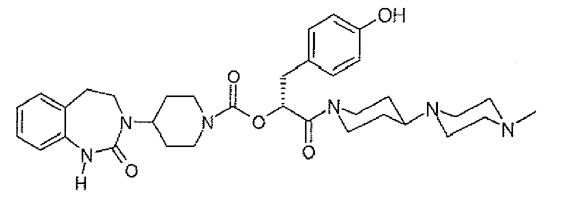

### 117a (Z,E)-2-Acetylamino-3-(4-benzyloxy-phenyl)-acrylsäure

Analog Beispiel 1a konnte aus 30.0 g (141 mmol) 4-Benzyloxy-benzaldehyd und 24.8 g (212 mmol) N-Acetyl-glycin das Produkt erhalten werden.

| | |
|---|---|
| Ausbeute: | 32.0 g (73% der Theorie) |
| ESI-MS: | (M+H)⁺ = 312 |
| R_{f} = | 0.35 (Kieselgel, DCM/MeOH/AcOH 90:10:1) |

### 117b 3-(4-Benzyloxy-phenyl)-2-oxo-propionsäure

Analog Beispiel 1b konnte aus 32.0 g (103 mmol) (Z,E)-2-Acetylamino-3-(4-benzyloxy-phenyl)-acrylsäure das Produkt erhalten werden.

| | |
|---|---|
| Ausbeute: | 12.4 g (45% der Theorie) |
| ESI-MS: | (M-H)⁻ = 269 |
| R_{f} = | 0.30 (Kieselgel, DCM/MeOH/AcOH 80:20:2) |

### 117c (R)-3-(4-Benzyloxy-phenyl)-2-hydroxy-propionsäure

Analog Beispiel 1c konnte aus 11.5 g (42.6 mmol) 3-(4-Benzyloxy-phenyl)-2-oxo-propionsäure und 16.7 g (52.1 mmol) (1R)-B-Chlordiisopinocamphenylboran das Produkt erhalten werden.

| | |
|---|---|
| Ausbeute: | 7.43 g (64% der Theorie) |
| ESI-MS: | (M+Na)⁺ = 294 |
| Retentionszeit (HPLC): | 13.3 min (Methode F) |

### 117d (R)-3-(4-Benzyloxy-phenyl)-2-hydroxy-propionsäuremethylester

Analog Beispiel 46d konnte aus 7.3 g (26.8 mmol) (R)-3-(4-Benzyloxy-phenyl)-2-hydroxy-propionsäure das Produkt erhalten werden.

| | |
|---|---|
| Ausbeute: | 7.6 g (99% der Theorie) |
| Retentionszeit (HPLC): | 16.7 min (Methode F) |

### 117e 4-(2-Oxo-1,2,4,5-tetrahydro-1,3-benzdiazepin-3-yl)-piperidin-1-carbonsäure-(R)-2-(4-benzyloxy-phenyl)-1-methoxycarbonyl-ethylester

Analog Beispiel 31f konnte aus 7.6 g (26.5 mmol) (R)-3-(4-Benzyloxy-phenyl)-2-hydroxy-propionsäuremethylester und 6.5 g (26.5 mmol) 3-Piperidin-4-yl-1,3,4,5-tetrahydro-1,3-benzdiazepin-2-on das Produkt erhalten werden.

| | |
|---|---|
| Ausbeute: | 4.1 g (28% der Theorie) |
| ESI-MS: | (M+H)⁺ = 558 |
| R_{f} = | 0.25 (Kieselgel, *n*-Hexan/EtOAc 3:7) |
| Retentionszeit (HPLC): | 22.0 min (Methode F) |

### 117f 4-(2-Oxo-1,2,4,5-tetrahydro-1,3-benzdiazepin-3-yl)-piperidin-1-carbonsäure-(R)-1-carboxy-2-(4-benzyloxy-phenyl)-ethylester

Analog Beispiel 58f konnte aus 4.1 g (7.4 mmol) (4-(2-Oxo-1,2,4,5-tetrahydro-1,3-benzdiazepin-3-yl)-piperidin-1-carbonsäure-(R)-2-(4-benzyloxy-phenyl)-1-methoxycarbonyl-ethylester und 264 mg (11.0 mmol) LiOH das Produkt erhalten werden.

| | |
|---|---|
| Ausbeute: | 2.7 g (68% der Theorie) |
| ESI-MS: | (M+H)⁺ = 544 |
| Retentionszeit (HPLC): | 18.8 min (Methode F) |

### 117g 4-(2-Oxo-1,2,4,5-tetrahydro-1,3-benzdiazepin-3-yl)-piperidin-1-carbonsäure-(R)-1-carboxy-2-(4-hydroxy-phenyl)-ethylester

Zu einer Lösung von 2.2 g (4.1 mmol) 4-(2-Oxo-1,2,4,5-tetrahydro-1,3-benzdiazepin-3-yl)-piperidin-1-carbonsäure-(R)-1-carboxy-2-(4-benzyloxy-phenyl)-ethylester und 450 mg (4.4 mmol) Triethylamin in 90 mL MeOH wurden 220 mg 10% Pd/C gegeben und das Reaktionsgemisch bei 3 bar H₂ 24 h hydriert. Der Katalysator wurde filtriert, zweimal mit MeOH gewaschen und das Filtrat i.vac. eingeengt. Der Rückstand wurde in 20 mL Wasser aufgenommen und mit 10% HCl auf pH 2-3 eingestellt. Der ausgefallene Niederschlag wurde filtriert, mit wenig Wasser gewaschen und bei 50°C getrocknet.

| | |
|---|---|
| Ausbeute: | 1.4 g (76% der Theorie) |
| ESI-MS: | (M+H)⁺ = 454 |
| R_{f} = | 0.65 (Kieselgel, DCM/MeOH/AcOH 80:20:2) |

### 117h 4-(2-Oxo-1,2,4,5-tetrahydro-1,3-benzdiazepin-3-yl)-piperidin-1-carbonsäure-(R)-1-(4-hydroxy-benzyl)-2-[4-(4-methyl-piperazin-1-yl)-piperidin-1-yl]-2-oxo-ethylester

Unter Stickstoffatmosphäre wurde zu einer Lösung von 140 mg (0.31 mmol) 4-(2-Oxo-1,2,4,5-tetrahydro-1,3-benzdiazepin-3-yl)-piperidin-1-carbonsäure-(R)-1-carboxy-2-(4-hydroxy-phenyl)-ethylester, 150 mg (0.39 mmol) HATU und 80 µL (0.47 mmol) Ethyldiisopropylamin in 5 mL DMF 74 mg (0.40 mmol) 1-Methyl-4-piperidin-4-yl-piperazin gegeben und das Reaktionsgemisch 6 h bei RT gerührt. Die Reaktionslösung wurde i.vac. eingeengt und der Rückstand chromatographisch (Kieselgel, DCM/MeOH/NH₃ 93:7:0.7) gereinigt.

| | |
|---|---|
| Ausbeute: | 100 mg (52% der Theorie) |
| ESI-MS: | (M+H)⁺ = 619 |
| R_{f} = | 0.6 (Kieselgel, DCM/MeOH/NH₃ 80:20:2) |

Analog wurden folgende Verbindungen aus jeweils 140 mg (Beispiele 118 und 119), 150 mg (Beispiele 120 und 121), 200 mg (Beispiele 122 und 123) oder 230 mg (Beispiel 124) 4-(2-Oxo-1,2,4,5-tetrahydro-1,3-benzdiazepin-3-yl)-piperidin-1-carbonsäure-(R)-1-carboxy-2-(4-hydroxy-phenyl)-ethylester und der entsprechenden Menge an Amin hergestellt:

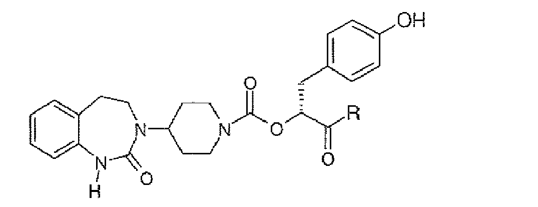

| **Beispiel** | **R** | **Ausbeute (%)** | **Massen-spektrum** | **R_{f} (Kieselgel, Laufmittel)** |
|---|---|---|---|---|
| **118** | 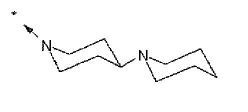 | 27 | 604 [M+H]⁺ | 0.70 (DCM/MeOH/ NH₃ 80:20:2) |
| **119** | 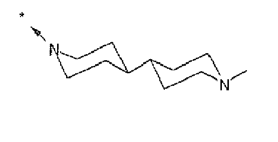 | 47 | 618 [M+H]⁺ | 0.65 (DCM/MeOH/ NH₃ 80:20:2) |
| **120** | 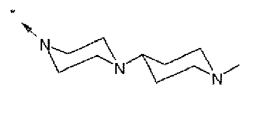 | 64 | 619 [M+H]⁺ | 0.35 (DCM/MeOH/ NH₃ 80:20:2) |
| **121** | 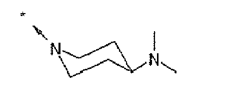 | 54 | 564 [M+H]⁺ | 0.40 (DCM/MeOH/ NH₃ 80:20:2) |
| **122** | 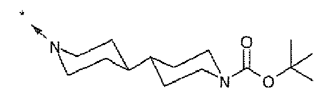 | 45 | 704 [M+H]⁺ | 0.65 (DCM/MeOH/ NH₃ 80:20:2) |
| **123** | 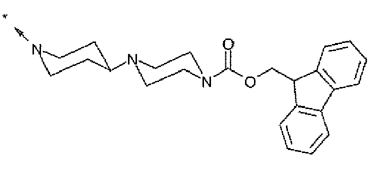 | 43 | 827 [M+H]⁺ | 0.40 (DCM/MeOH/ AcOH 90:10:1) |
| **124** | 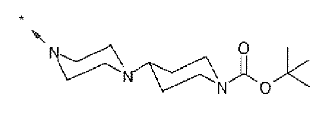 | 53 | 705 [M+H]⁺ | 0.65 (DCM/MeOH/ NH₃ 80:20:2) |

### Beispiel 125

### 4-(2-Oxo-1,2,4,5-tetrahydro-1,3-benzdiazepin-3-yl)-piperidin-1-carbonsäure-(R)-1-(4-hydroxy-benzyl)-2-oxo-2-(4-piperazin-1-yl-piperidin-1-yl)-ethylester

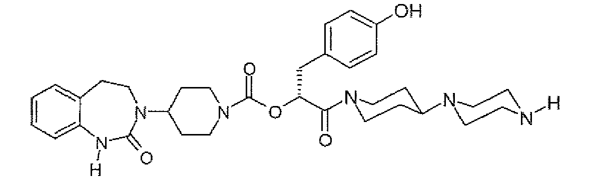

Eine Lösung von 250 mg (0.30 mmol) 4-(1-{(R)-3-(4-Hydroxy-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-9*H*-fluoren-9-ylmethylester (Beispiel 123) in 4 mL Piperidin wurde bei RT 30 min gerührt. Das Reaktionsgemisch wurde i.vac. eingeengt und der Rückstand chromatographisch (Kieselgel, DCM/MeOH/NH₃ 90:10:1) gereinigt.

| | |
|---|---|
| Ausbeute: | 40 mg (22% der Theorie) |
| ESI-MS: | (M+H)⁺ = 605 |
| Retentionszeit (HPLC): | 4.7 min (Methode F) |

### Beispiel 126

### 4-(2-Oxo-1,2,4,5-tetrahydro-1,3-benzdiazepin-3-yl)-piperidin-1-carbonsäure-(R)-1-(4-hydroxy-benzyl)-2-oxo-2-(4-piperidin-4-yl-piperazin-1-yl)-ethylester

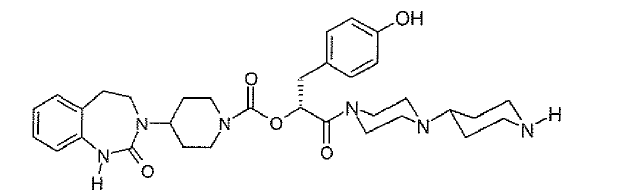

Zu einer Lösung von 210 mg (0.30 mmol) 4-(2-Oxo-1,2,4,5-tetrahydro-1,3-benzdiazepin-3-yl)-piperidin-1-carbonsäure-(R)-1-(4-hydroxy-benzyl)-2-[4-(1-*tert-*butoxy-carbonyl-piperidin-4-yl)-piperazin-1-yl]-2-oxo-ethylester (Beispiel 124) in 1.5 mL Ameisensäure wurde 3 h bei RT gerührt. Das Reaktionsgemisch wurde i.vac. eingeengt und der Rückstand chromatographisch (Kieselgel, DCM/MeOH/NH₃ 90:10:1) gereinigt.

| | |
|---|---|
| Ausbeute: | 40 mg (22% der Theorie) |
| ESI-MS: | (M+H)⁺ = 605 |
| R_{f} = | 0.45 (Kieselgel, DCM/MeOH/NH₃ 80:20:2) |
| Retentionszeit (HPLC): | 4.6 min (Methode F) |

### Beispiel 127

### 4-(2-Oxo-1,2,4,5-tetrahydro-1,3-benzdiazepin-3-yl)-piperidin-1-carbonsäure-(R)-2-4,4'-bipiperidinyl-1-yl-1-(4-hydroxy-benzyl)-2-oxo-ethylester

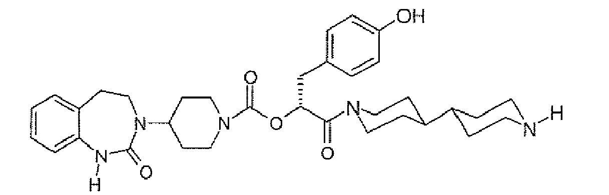

Analog Beispiel 126 konnte aus 130 mg (0.19 mmol) 1'-{(R)-3-(4-Hydroxy-phenyl)-2-[4-(2-oxo-1,2,4,5-tetrahydro-1,3-benzdiazepin-3-yl)-piperidin-1-carbonyloxy]-propionyl}-4,4'-bipiperidinyl-1-carbonsäure-*tert*-butylester (Beispiel 122) das Produkt erhalten werden.

| | |
|---|---|
| Ausbeute: | 70 mg (63% der Theorie) |
| ESI-MS: | (M+H)⁺ = 604 |
| R_{f} = | 0.20 (Kieselgel, DCM/MeOH/NH₃ 80:20:2) |
| Retentionszeit (HPLC): | 6.9 min (Methode F) |

### Beispiel 128

### 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-[4-(4-methyl-piperazin-1-yl)-piperidin-1-yl]-2-oxo-ethylester

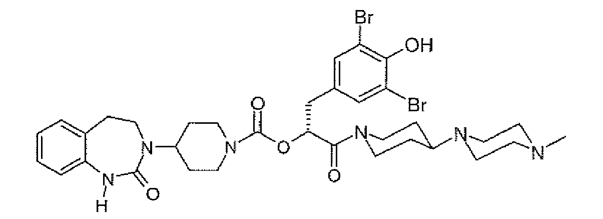

### 128a (Z,E)-3-(4-Acetoxy-3,5-dibrom-phenyl)-2-acetylamino-acrylsäure

Analog Beispiel 1a konnte aus 30 g (107 mmol) 3,5-Dibrom-4-hydroxy-benzaldehyd und 18.8 g (161 mmol) N-Acetyl-glycin das Produkt erhalten werden.

| | |
|---|---|
| Ausbeute: | 35.7 g (79% der Theorie) |
| ESI-MS: | (M+H)⁺ = 420/422/424 (2 Br) |
| R_{f} = | 0.20 (Kieselgel, DCM/MeOH/AcOH 90:10:1) |

### 128b 3-(3,5-Dibrom-4-hydroxy-phenyl)-2-oxo-propionsäure

Analog Beispiel 1b konnte aus 35.7 g (84.8 mmol) (Z,E)-3-(4-Acetoxy-3,5-dibromphenyl)-2-acetylamino-acrylsäure das Produkt erhalten werden.

| | |
|---|---|
| Ausbeute: | 20.5 g (72% der Theorie) |
| ESI-MS: | (M-H)⁻ = 335/337/339 (2 Br) |
| R_{f} = | 0.35 (Kieselgel, DCM/MeOH/AcOH 80:20:2) |

### 128c (R)-3-(3,5-Dibrom-4-hydroxy-phenyl)-2-hydroxy-propionsäure

Analog Beispiel 1c konnte aus 14.5 g (42.9 mmol) 3-(3,5-Dibrom-4-hydroxy-phenyl)-2-oxo-propionsäure und 30.9 g (96.33 mmol) (1R)-B-Chlordiisopinocampheylboran das Produkt erhalten werden.

| | |
|---|---|
| Ausbeute: | 12.7 g (87% der Theorie) |
| ESI-MS: | (M-H)⁻ = 337/339/341 (2 Br) |
| R_{f} = | 0.4 (Kieselgel, DCM/MeOH/AcOH 80:20:2) |
| Retentionszeit (HPLC): | 6.4 min (Methode F) |

### 128d (R)-3-(3,5-Dibrom-4-hydroxy-phenyl)-2-hydroxy-propionsäuremethylester

Analog Beispiel 46d konnte aus 14.0 g (34.8 mmol) 3(R)-3-(3,5-Dibrom-4-hydroxyphenyl)-2-hydroxy-propionsäure das Produkt erhalten werden.

| | |
|---|---|
| Ausbeute: | 7.0 g (57% der Theorie) |
| ESI-MS: | (M-H)⁻ = 351/353/355 (2 Br) |
| Retentionszeit (HPLC): | 9.8 min (Methode F) |

### 128e (R)-3-[3,5-Dibrom-4-(2-trimethylsilanyl-ethoxymethoxy)-phenyl]-2-hydroxypropionsäuremethylester

Unter Stickstoffatmosphäre wurden zu einer Lösung von 6.78 g (19.2 mmol) (R)-3-(3,5-Dibrom-4-hydroxy-phenyl)-2-hydroxy-propionsäuremethylester in 100 mL Acetonitril 11.1 g (76.6 mmol) 40% KF/Al₂O₃ gegeben und die resultierende Suspension für einige min bei RT gerührt. Anschliessend wurde eine Lösung von 4.07 mL (23.0 mmol) (2-Chlormethoxy-ethyl)-trimethyl-silan in 20 mL Acetonitril zugegeben und das Reaktionsgemisch 20 h bei RT gerührt. Man filtrierte über Celite, engte das Lösungsmittel i.vac. ein und reinigte Rückstand chromatographisch (Kieselgel, *n*-Hexan/EtOAc 7:3).

| | |
|---|---|
| Ausbeute: | 5.49 g (59% der Theorie) |
| R_{f} = | 0.45 (Kieselgel, *n*-Hexan/Et 1:1) |

### 128f 4-(2-Oxo-1,2,4,5-tetrahydro-1,3-benzdiazepin-3-yl)-piporidin-1-carbonsäure-(R)-2-[3,5-dibrom-4-(2-trimethylsilanyl-ethoxymethoxy)-phenyl]-1-methoxycarbonyl-ethylester

Analog Beispiel 31f konnte aus 4.63 g (9.56 mmol) (R)-3-[3,5-Dibrom-4-(2-trimethylsilanyl-ethoxymethoxy)-phenyl]-2-hydroxy-propionsäuremethylester und 2.35 g (9.56 mmol) 3-Piperidin-4-yl-1,3,4,5-tetrahydro-1,3-benzdiazepin-2-on das Produkt erhalten werden.

| | |
|---|---|
| Ausbeute: | 4.35 g (69% der Theorie) |
| ESI-MS: | (M+H)⁺ = 754/756/758 (2 Br) |
| Retentionszeit (HPLC): | 29.2 min (Methode F) |

### 128g 4-(2-Oxo-1,2,4,5-tetrahydro-1,3-benzdiazepin-3-yl)-piperidin-1-carbonsäure-(R)-2-(3,5-dibrom-4-hydroxy-phenyl)-1-methoxycarbonyl-ethylester

Unter Stickstoffatmosphäre wurden zu einer Lösung von 4.30 g (5.69 mmol) 4-(2-Oxo-1,2,4,5-tetrahydro-1,3-benzdiazepin-3-yl)-piporidin-1-carbonsäure-(R)-2-[3,5-dibrom-4-(2-trimethylsilanyl-ethoxymethoxy)-phenyl]-1-methoxy-carbonyl-ethylester in 40 mL THF und 40 mL MeOH 5.46 mL methanolische H₂SO₄ (0.5 M) gegeben und die Reaktionslösung 6 h bei RT gerührt. Das Reaktionsgemisch wurde i.vac eingeengt und der Rückstand ohne Reinigung weiter umgesetzt.

| | |
|---|---|
| Ausbeute: | quantitativ |
| ESI-MS: | (M+H)⁺ = 624/626/628 (2 Br) |
| Retentionszeit (HPLC): | 17.3 min (Methode F) |

### 128h 4-(2-Oxo-1,2,4,5-tetrahydro-1,3-benzdiazepin-3-yl)-piperidin-1-carbonsäure-(R)-1-carboxy-2-(3,5-dibrom-4-hydroxy-phenyl)-ethylester

Zu einer Lösung von 4-(2-Oxo-1,2,4,5-tetrahydro-1,3-benzdiazepin-3-yl)-piperidin-1-carbonsäure-(R)-2-(3,5-dibrom-4-hydroxy-phenyl)-1-methoxycarbonyl-ethylester (Rohprodukt aus Beispiel 128g) in 80 mL THF wurde eine Lösung von 0.51 g (21.3 mmol) LiOH gegeben und das Reaktionsgemisch 3 h bei RT gerührt. Man zog i.vac. das THF ab, wusch die wässrige Phase mit EtOAc, säuerte mit 10% HCl an und extrahierte die wässrige Phase erschöpfend mit EtOAc. Die vereinigten organischen Phasen wurden i.vac. eingeengt, in Diethylether suspendiert, filtriert, der Rückstand getrocknet und anschliessend chromatographisch (Kieselgel, DCM/MeOH/AcOH 90:10:1) gereinigt.

| | |
|---|---|
| Ausbeute: | 3.5 g (100% der Theorie) |
| ESI-MS: | (M+H)⁺ = 610/612/614 (2 Br) |
| Retentionszeit (HPLC): | 14.1 min (Methode F) |

### 128i 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-[4-(4-methyl-piperazin-1-yl)-piperidin-1-yl]-2-oxo-ethylester

Unter Stickstoffatmosphäre wurde zu einer Lösung von 151 mg (0.25 mmol) 4-(2-Oxo-1,2,4,5-tetrahydro-1,3-benzdiazepin-3-yl)-piperidin-1-carbonsäure-(R)-1-carboxy-2-(3,5-dibrom-4-hydroxy-phenyl)-ethylester, 104 mg (0.27 mmol) HATU und 47 µL (0.27 mmol) Ethyldiisopropylamin in 5 mL DMF 55 mg (0.30 mmol) 1-Methyl-4-piperidin-4-yl-piperazin gegeben und das Reaktionsgemisch 3 h bei RT gerührt. Die Reaktionslösung wurde i.vac. eingeengt und der Rückstand chromatographisch (Kieselgel, DCM/MeOH/NH₃ 80:20:2) gereinigt.

| | |
|---|---|
| Ausbeute: | 190 mg (99% der Theorie) |
| ESI-MS: | (M+H)⁺ = 775/777/779 (2 Br) |
| R_{f} = | 0.3 (Kieselgel, DCM/MeOH/AcOH 80:20:2) |
| Retentionszeit (HPLC): | 7.2 min (Methode F) |

Analog wurden folgende Verbindungen aus jeweils 151 mg 4-(2-Oxo-1,2,4,5-tetrahydro-1,3-benzdiazepin-3-yl)-piperidin-1-carbonsäure-(R)-1-carboxy-2-(3,5-dibrom-4-hydroxy-phenyl)-ethylester und der entsprechenden Menge an Amin hergestellt:

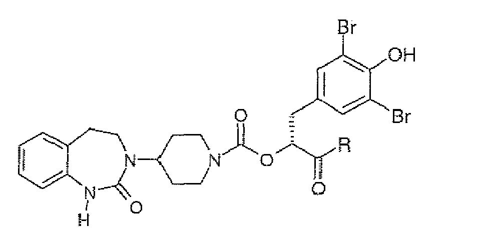

| **Beispiel** | **R** | **Ausbeute (%)** | **Massen-spektrum** | **Retentionszeit HPLC (Methode)** |
|---|---|---|---|---|
| **129** | 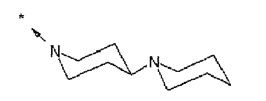 | 95 | 760/762/764 [M+H]⁺ | 9.5 min (F) |
| **130** | 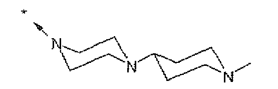 | 89 | 775/777/779 [M+H]⁺ | 6.7 min (F) |
| **131** | 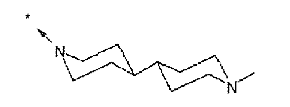 | 93 | 774/776/778 [M+H]⁺ | 9.6 min (F) |
| **132** | 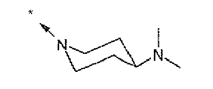 | 82 | 720/722/724 [M+H]⁺ | 8.7 min (F) |
| **133** | 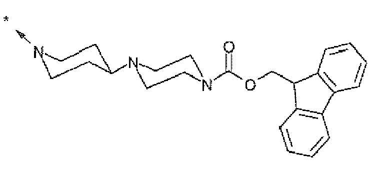 | 91 | 761/763/765 [M+H-Fmoc]⁺ | 15.0 min (F) |

### Beispiel 134

### 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-oxo-2-(4-piperidin-4-yl-piperazin-1-yl)-ethylester

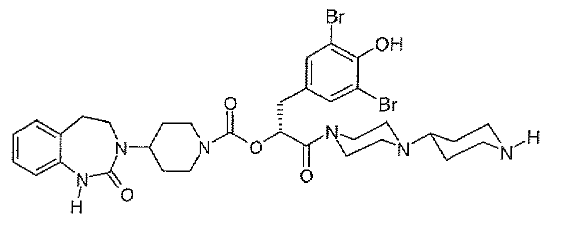

Analog Beispiel 128i wurden aus 200 mg (0.33 mmol) 4-(2-Oxo-1,2,4,5-tetrahydro-1,3-benzdiazepin-3-yl)-piperidin-1-carbonsäure-(R)-1-carboxy-2-(3,5-dibrom-4-hydroxy-phenyl)-ethylester, 140 µL (0.82 mmol) Ethyldiisopropyamin und 135 mg (0.44 mmol) 4-Piperazin-1-yl-piperidin-1-carbonsäure-*tert*-butylester (eingesetzt als Hydrochlorid Salz) das Rohprodukt erhalten. Dieses wurde in 2 mL Ameisensäure gelöst und 2 h bei RT gerührt. Das Reaktionsgemisch wurde i.vac. eingeengt und der Rückstand chromatographisch (Kieselgel, Eluation zuerst mit DCM/MeOH/NH₃ 80:20:2 dann mit DCM/MeOH/NH₃ 50:50:5) gereinigt.

| | |
|---|---|
| Ausbeute: | 20 mg (8% der Theorie) |
| ESI-MS: | (M+H)⁺ = 761/763/765 (2 Br) |
| R_{f} = | 0.35 (Kieselgel, DCM/MeOH/NH₃ 80:20:2) |
| Retentionszeit (HPLC): | 6.5 min (Methode F) |

### Beispiel 135

### 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-oxo-2-(4-piperazin-1-yl-piperidin-1-yl)-ethylester

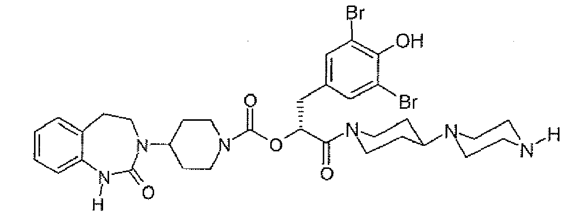

Eine Lösung von 200 mg (0.20 mmol) 4-(1-{(R)-3-(3,5-Dibrom-4-hydroxy-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-9*H*-fluoren-9-ylmethylester (Beispiel 133) in 4 mL Piperidin wurde 30 min bei RT gerührt. Das Reaktionsgemisch wurde i.vac. eingeengt und der Rückstand chromatographisch (Kieselgel, DCM/MeOH/NH₃ 80:20:2) gereinigt.

| | |
|---|---|
| Ausbeute: | 20 mg (8% der Theorie) |
| ESI-MS: | (M+H)⁺ = 761/763/765 (2 Br) |
| Retentionszeit (HPLC): | 6.8 min (Methode F) |

### Beispiel 136

### 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-(1'-methansulfonyl-4,4'-bipiperidinyl-1-yl)-2-oxo-ethylester

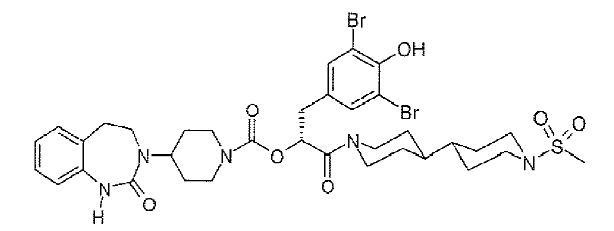

Eine Lösung von 130 mg (0.21 mmol) 4-(2-Oxo-1,2,4,5-tetrahydro-1,3-benzdiazepin-3-yl)-piperidin-1-carbonsäure-(R)-1-carboxy-2-(3,5-dibrom-4-hydroxy-phenyl)-ethylester, 80 mg (0.25 mmol) TBTU und 50 µL (0.27 mmol) Ethyldiisopropylamin in 10 mL THF wurde 50 min bei RT gerührt. Dann erfolgte die Zugabe von 60 mg (0.24 mmol) 1-Methansulfonyl-[4,4']bipiperidinyl und das Reaktionsgemisch wurde über Nacht bei RT gerührt. Die Reaktionslösung wurde mit 50 mL EtOAc verdünnt, zweimal mit je 30 mL 15% K₂CO₃-Lösung extrahiert, die organische Phase abgetrennt und über MgSO₄ getrocknet. Nach Entfernen des Trocken- und Lösungsmittels wurde der Rückstand mit Wasser verrieben und filtriert. Der Feststoff wurde mit 5 mL 1 M HCl versetzt und über Nacht gerührt. Weitere Reinigung des Rohproduktes erfolgte chromatographisch (Kieselgel, Gradient DCM zu DCM/MeOH/NH₃ 50:45:5). Die das Produkt enthaltenden Fraktionen wurden vereinigt, i.vac. eingeengt, mit DIPE verrieben, abgesaugt und bei 40°C getrocknet.

| | |
|---|---|
| Ausbeute: | 80 mg (45% der Theorie) |
| ESI-MS: | (M+H)⁺ = 838/840/842 (2 Br) |
| R_{f} = | 0.42 (Kieselgel, DCM/MeOH/Cyc/NH₃ 70:15:15:2) |

### Beispiel 137

### 4-(2-Oxo-1,2,4,5-tetrahydro-1,3-benzdiazepin-3-yl)-piperidin-1-carbonsäure-(R)-2-(4-amino-4-methyl-1,4'-bipiperidinyl-1'-yl)-1-(3,5-dibrom-4-hydroxy-benzyl)-2-oxo-ethyl-ester

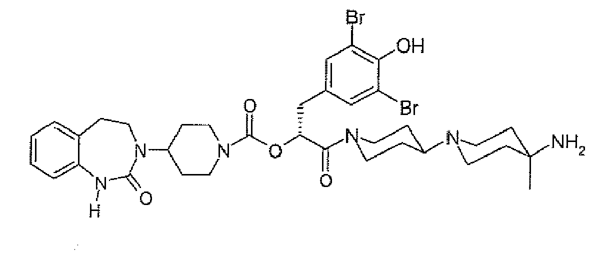

Analog Beispiel 1h konnte aus 80 mg (0.13 mmol) 4-(2-Oxo-1,2,4,5-tetrahydro-1,3-benzdiazepin-3-yl)-piperidin-1-carbonsäure-(R)-1-carboxy-2-(3,5-dibrom-4-hydroxyphenyl)-ethylester und 36 mg (0.14 mmol) 4-Methyl-[1,4']bipiperidinyl-4-ylamin (eingesetzt als Bis-Hydrochlorid Salz) das Produkt erhalten werden, welches als Formiat-Salz anfiel.

| | |
|---|---|
| Ausbeute: | 6 mg (6% der Theorie) |
| ESI-MS: | (M+H)⁺ = 789/791/793 (2 Br) |
| Retentionszeit (HPLC): | 4.9 min (Methode A) |

### Beispiel 138

### 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-(1'-ethoxycarbonylmethyl-4,4'-bipiperidinyl-1-yl)-2-oxo-ethylester

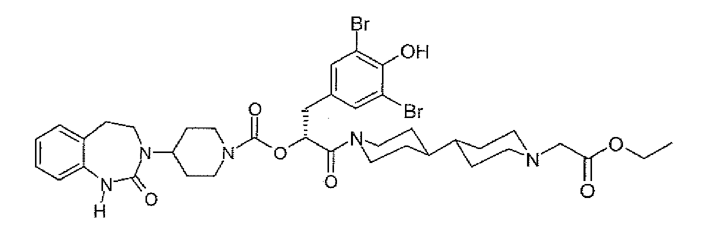

Zu einer Lösung von 500 mg (0.82 mmol) 4-(2-Oxo-1,2,4,5-tetrahydro-1,3-benzdiazepin-3-yl)-piperidin-1-carbonsäure-(R)-1-carboxy-2-(3,5-dibrom-4-hydroxyphenyl)-ethylester, 315 mg (0.98 mmol) TBTU und 150 µL (1.06 mmol) Triethylamin in 5 mL DMF wurden 208 mg (0.82 mmol) [4,4']Bipiperidinyl-1-yl-essigsäureethylester gegeben und das Reaktionsgemisch über Nacht bei RT gerührt. Zur Vervollständigung der Reaktion wurden weitere 315 mg (0.98 mmol) TBTU, 150 µL (1.06 mmol) Triethylamin und 208 mg (0.82 mmol) [4,4']Bipiperidinyl-1-yl-essigsäureethylester zugegeben und erneut über Nacht bei RT gerührt. Das Reaktionsgemisch wurde ohne weitere Aufarbeitung direkt via HPLC gereinigt. Die das Produkt enthaltenden Fraktionen wurden vereinigt, i.vac. eingeengt, mit gesättigter NaHCO₃-Lösung neutralisiert, die wässrige Phase zweimal mit je 100 mL DCM extrahiert und die vereinigten organischen Phasen über Na₂SO₄ getrocknet. Nach Entfernen des Trocken- und Lösungsmittels wurde der Rückstand mit DIPE verrieben, abgesaugt und an der Luft getrocknet.

| | |
|---|---|
| Ausbeute: | 204 mg (29% der Theorie) |
| ESI-MS: | (M+H)⁺ = 846/848/850 (2 Br) |
| Retentionszeit (HPLC): | 6.5 min (Methode A) |

### Beispiel 139

### 4-(2-Oxo-1,2,4,5-tetrahydro-1,3-benzdiazepin-3-yl)-piperidin-1-carbonsäure-(R)-2-(1' carboxymethyl-4,4'-bipiperidinyl-1-yl)-1-(3,5-dibrom-4-hydroxy-benzyl)-2-oxo-ethyl-ester

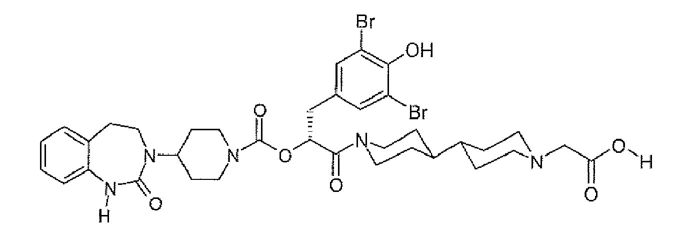

Zu einer Lösung von 30 mg (0.04 mmol) 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-(1'-ethoxycarbonylmethyl-4,4'-bipiporidinyl-1-yl)-2-oxo-ethylester (Beispiel 138) in 3 mL THF wurde eine Lösung von 1.4 mg (0.06 mmol) LiOH in 1 mL Wasser gegeben und die Reaktionslösung über Nacht bei RT gerührt. Man entfernte das Lösungsmittel im Stickstoffstrom, nahm den Rückstand in 1 mL Wasser auf, säuerte mit Ameisensäure an, saugte den entstandenen Niederschlag ab und trocknete diesen im Vakuum.

| | |
|---|---|
| Ausbeute: | 20 mg (69% der Theorie) |
| ESI-MS: | (M+H)⁺ = 818/820/822 (2 Br) |
| Retentionszeit (HPLC): | 6.5 min (Methode A) |

### Beispiel 140

### 4-(2-Oxo-1,2,4,5-tetrahydro-1,3-benzdiazepin-3-yl)-piperidin-1-carbonsäure-(R)-2-(4-amino-4-methyl-1,4'-bipiperidinyl-1'-yl)-1-(3,5-bis-trifluormethyl-benzyl)-2-oxo-ethyl-ester

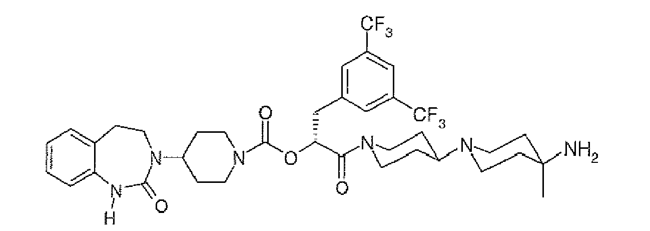

Analog Beispiel 1h konnte aus 80 mg (0.14 mmol) 4-(2-Oxo-1,2,4,5-tetrahydro-1,3-benzdiazepin-3-yl)-piperidin-1-carbonsäure-(R)-2-(3,5-bis-trifluormethyl-phenyl)-1-carboxy-ethylester (Beispiel 46f) und 38 mg (0.14 mmol) 4-Methyl-[1,4']bipiperidinyl-4-ylamin (eingesetzt als Bis-Hydrochlorid Salz) das Produkt erhalten werden, welches als Formiat-Salz anfiel.

| | |
|---|---|
| Ausbeute: | 47 mg (42% der Theorie) |
| ESI-MS: | (M+H)⁺ = 753 |
| Retentionszeit (HPLC): | 5.4 min (Methode A) |

### Beispiel 141

### 4-(2-Oxo-1,2,4,5-tetrahydro-1,3-benzdiazepin-3-yl)-piperidin-1-carbonsäure-(R)-2-(1'-ethoxycarbonylmethyl-4,4'-bipiperidinyl-1-yl)-1-(4-hydroxy-3,5-dimethyl-benzyl)-2-oxo-ethylester

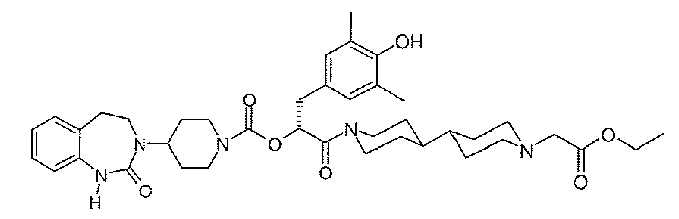

Analog Beispiel 46g konnte aus 200 mg (0.42 mmol) 4-(2-Oxo-1,2,4,5-tetrahydro-1,3-benzdiazepin-3-yl)-piperidin-1-carbonsäure-(R)-1-carboxy-2-(4-hydroxy-3,5-dimethylphenyl)-ethylester (Beispiel 69h) und 117 mg (0.46 mmol) [4,4']Bipiperidinyl-1-yl-essigsäureethylester das Produkt erhalten werden.

| | |
|---|---|
| Ausbeute: | 222 mg (74% der Theorie) |
| ESI-MS: | (M+H)⁺ = 718 |
| Retentionszeit (HPLC): | 3.1 min (Methode E) |

### Beispiel 142

### 4-(2-Oxo-1,2,4,5-tetrahydro-1,3-benzdiazepin-3-yl)-piperidin-1-carbonsäure-(R)-2-(1'-carboxymethyl-4,4'-bipiperidinyl-1-yl)-1-(4-hydroxy-3,5-dimethyl-benzyl)-2-oxo-ethylester

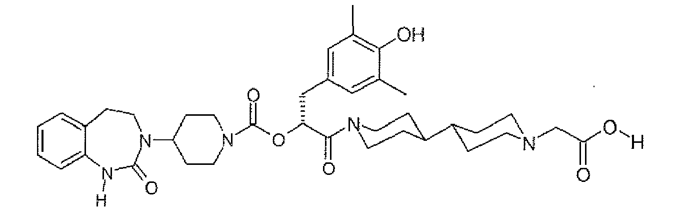

Analog Beispiel 139 konnte aus 100 mg (0.14 mmol) 4-(2-Oxo-1,2,4,5-tetrahydro-1,3-benzdiazepin-3-yl)-piperidin-1-carbonsäure-(R)-2-(1'-ethoxycarbonylmethyl-4,4'-bipiperidinyl-1-yl)-1-(4-hydroxy-3,5-dimethyl-benzyl)-2-oxo-ethylester (Beispiel 141) und 3.8 mg (0.16 mmol) LiOH das Produkt erhalten werden.

| | |
|---|---|
| Ausbeute: | 56 mg (58% der Theorie) |
| ESI-MS: | (M-H)⁻ = 688 |
| Retentionszeit (HPLC): | 3.0 min (Methode E) |

### Beispiel 143

### 4-(2-Oxo-1,2,4,5-tetrahydro-1,3-benzdiazepin-3-yl)-piperidin-1-carbonsäure-(R)-2-(4-cyclohexyl-piperazin-1-yl)-1-(4-hydroxy-3,5-dimethyl-benzyl)-2-oxo-ethylester

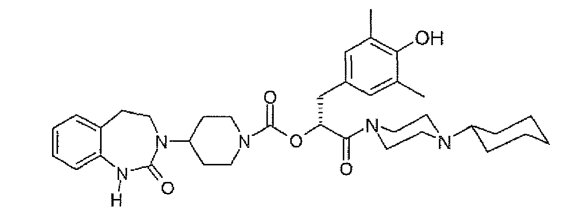

Analog Beispiel 1h konnte aus 69 mg (0.14 mmol) 4-(2-Oxo-1,2,4,5-tetrahydro-1,3-benzdiazepin-3-yl)-piperidin-1-carbonsäure-(R)-1-carboxy-2-(4-hydroxy-3,5-dimethylphenyl)-ethylester (Beispiel 69h) und 24 mg (0.14 mmol) 1-Cyclohexyl-piperazin das Produkt erhalten werden.

| | |
|---|---|
| Ausbeute: | 51 mg (91% der Theorie) |
| ESI-MS: | (M+H)⁺ = 632 |
| Retentionszeit (HPLC): | 3.1 min (Methode E) |

### Beispiel 144

### 4-(2-Oxo-1,2,4,5-tetrahydro-1,3-benzdiazepin-3-yl)-piperidin-1-carbonsäure-(R)-2-[4-(4-methyl-piperazin-1-yl)-piperidin-1-yl]-2-oxo-1-(3-trifluormethyl-benzyl)-ethylester

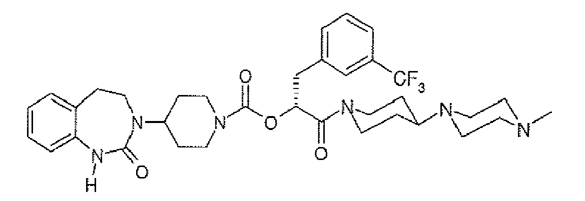

### 144a (Z,E)-2-Acetylamino-3-(3-trifluormethyl-phenyl)-acrylsäure

Analog Beispiel 1a konnte aus 15.8 g (115 mmol) 3-Trifluormethyl-benzaldehyd und 21.3 g (182 mmol) N-Acetyl-glycin das Produkt erhalten werden.

| | |
|---|---|
| Ausbeute: | 16.7 g (53% der Theorie) |
| ESI-MS: | (M+H)⁺ = 274 |
| R_{f} = | 0.4 (Kieselgel, DCM/MeOH/AcOH 90:10:1) |

### 144b 2-Oxo-3-(3-trifluormethyl-phenyl)-propionsäure

Analog Beispiel 46b konnte aus 16.6 g (60.8 mmol) (Z,E)-2-Acotylamino-3-(3-trifluormethyl-phenyl)-acrylsäure das Produkt erhalten werden.

| | |
|---|---|
| Ausbeute: | 5.7 g (40% der Theorie) |
| ESI-MS: | (M-H)⁻ = 231 |
| R_{f} = | 0.19 (Kieselgel, DCM/MeOH/Cyc/NH₃ 70:15:15:2) |

### 144c (R)-2-Hydroxy-3-(3-trifluormethyl-phenyl)-propionsäure

Analog Beispiel 1c konnte aus 5.70 g (24.6 mmol) 2-Oxo-3-(3-trifluormethyl-phenyl)-propionsäure und 11.8 g (36.8 mmol) (1R)-B-Chlordiisopinocampheylboran das Produkt erhalten werden.

| | |
|---|---|
| Ausbeute: | 4.25 g (74% der Theorie) |
| ESI-MS: | (M-H)⁻ = 233 |
| R_{f} = | 0.35 (Kieselgel, DCM/MeOH/Cyc/NH₃ 70:15:15:2) |

### 144d (R)-2-Hydroxy-3-(3-trifluormethyl-phenyl)-propionsäuremethylester

Analog Beispiel 46d konnte aus 4.20 g (17.9 mmol) (R)-2-Hydroxy-3-(3-trifluormethylphenyl)-propionsäure das Produkt erhalten werden.

| | |
|---|---|
| Ausbeute: | 2.47 g (55% der Theorie) |
| ESI-MS: | (M+H)⁺ = 249 |
| R_{f} = | 0.73 (Kieselgel, DCM/MeOH/Cyc/NH₃ 70:15:15:2) |

### 144e 4-(2-Oxo-1,2,4,5-tetrahydro-1,3-benzdiazepin-3-yl)-piperidin-1-carbonsäure-(R)-1-methoxycarbonyl-2-(3-trifluormethyl-phenyl)-ethylester

Analog Beispiel 31f konnte aus 2.47 g (9.95 mmol) (R)-2-Hydroxy-3-(3-trifluormethylphenyl)-propionsäuremethylester und 4.10 g (65% Reinheit, 10.9 mmol) 3-Piperidin-4-yl-1,3,4,5-tetrahydro-1,3-benzdiazepin-2-on das Produkt erhalten werden.

| | |
|---|---|
| Ausbeute: | 3.16 g (61 % der Theorie) |
| ESI-MS: | (M+H)⁺ = 520 |
| R_{f} = | 0.93 (Kieselgel, EtOAc/MeOH/NH₃ 80:20:2) |

### 144f 4-(2-Oxo-1,2,4,5-tetrahydro-1,3-benzdiazepin-3-yl)-piperidin-1-carbonsäure-(R)-1-carboxy-2-(3-trifluormethyl-phenyl)-ethylester

Analog Beispiel 46f konnte aus 3.10 g (5.97 mmoi) 4-(2-Oxo-1,2,4,5-tetrahydro-1,3-benzdiazepin-3-yl)-piperidin-1-carbonsäure-(R)-1-methoxycarbonyl-2-(3-trifluormethyl-phenyl)-ethylester und 0.22 g (9.00 mmol) LiOH das Produkt erhalten werden.

| | |
|---|---|
| Ausbeute: | 2.80 g (93% der Theorie) |
| ESI-MS: | (M+H)⁺ = 506 |
| R_{f} = | 0.58 (Kieselgel, EtOAc/MeOH/NH₃ 70:30:3) |

### 144g 4-(2-Oxo-1,2,4,5-tetrahydro-1,3-benzdiazepin-3-yl)-piperidin-1-carbonsäure-(R)-2-[4-(4-methyl-piperazin-1-yl)-piperidin-1-yl]-2-oxo-1-(3-trifluormethyl-benzyl)-ethylester

Analog Beispiel 1h konnte aus 80.0 mg (0.16 mmol) 4-(2-Oxo-1,2,4,5-tetrahydro-1,3-benzdiazepin-3-yl)-piperidin-1-carbonsäure-(R)-1-carboxy-2-(3-trifluormethyl-phenyl)-ethylester und 29.6 mg (0.16 mmol) 1-Methyl-4-piperidin-4-yl-piperazin das Produkt erhalten werden.

| | |
|---|---|
| Ausbeute: | 67 mg (63% der Theorie) |
| ESI-MS: | (M+H)⁺ = 671 |
| R_{f} = | 0.4 (Kieselgel, DCM/MeOH/Cyc/NH₃ 70:15:15:2) |

Analog wurden folgende Verbindungen aus jeweils 80 mg (Beispiele 145 bis 148) bzw. 140 mg (Beispiele 149 und 150) 4-(2-Oxo-1,2,4,5-tetrahydro-1,3-benzdiazepin-3-yl)-piperidin-1-carbonsäure-(R)-1-carboxy-2-(3-trifluor-methyl-phenyl)-ethylester und der entsprechenden Menge an Amin hergestellt:

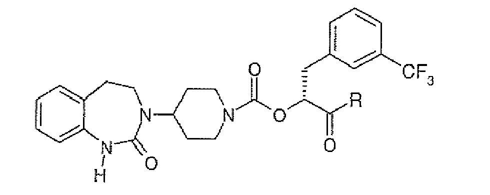

| **Beispiel** | **R** | **Ausbeute (%)** | **Massen-spektrum** | **R_{f}** **(Kieselgel, DCM/MeOH/ Cyc/NH₃ 70:15:15:2)** **oder** **Retentionszeit HPLC** **(Methode)** |
|---|---|---|---|---|
| **145** | 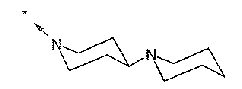 | 93 | 656 [M+H]⁺ | 0.68 |
| **146** | 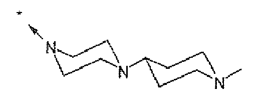 | 64 | 671 [M+H]⁺ | 0.35 |
| **147** | 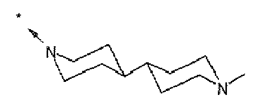 | 74 | 670 [M+H]⁺ | 0.53 |
| **148** | 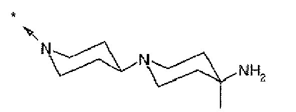 | 48 | 685 [M+H]⁺ | 5.1 min (A) |
| **149** | 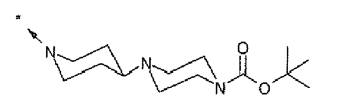 | 62 | 757 [M+H]⁺ | 0.51 |
| **150** | 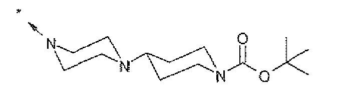 | 71 | 757 [M+H]⁺ | 0.50 |

### Beispiel 151

### 4-(2-Oxo-1,2,4,5-tetrahydro-1.3-benzdiazepin-3-yl)-piperidin-1-carbonsäure-(R)-1-(3-methyl-benzyl)-2-oxo-2-(4-piperazin-1-yl-piperidin-1-yl)-ethylester

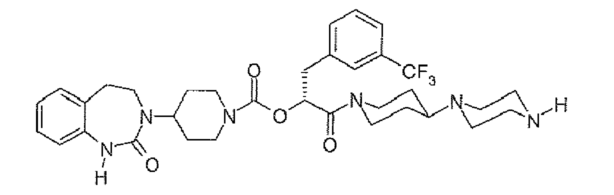

Eine Lösung von 131 mg (0.17 mmol) 4-{1-[(R)-2-[4-(2-Oxo-1,2,4,5-tetrahydro-1,3-benzdiazepin-3-yl)-piperidin-1-carbonyloxy]-3-(3-trifluormethyl-phenyl)-propionyl]-piperidin-4-yl}-piperazin-1-carbonsäure-*tert*-butylester (Beispiel 149) in 1.5 mL 4 M HCl wurde über Nacht bei RT gerührt. Die Reaktionslösung wurde ohne weitere Aufarbeitung via HPLC gereinigt. Die das Produkt enthaltenden Fraktionen wurden vereinigt und lyophilisiert.

| | |
|---|---|
| Ausbeute: | 75 mg (67% der Theorie) |
| ESI-MS: | (M+H)⁺ = 657 |
| R_{f} = | 0.38 (Kieselgel, DCM/MeOH/Cyc/NH₃ 70:15:15:2) |

### Beispiel 152

### 4-(2-Oxo-1,2,4,5-tetrahydro-1,3-benzdiazepin-3-yl)-piperidin-1-carbonsäure-(R)-2-oxo-2-(4-piperidin-4-yl-piperazin-1-yl)-1-(3-trifluormethyl-benzyl)-ethylester

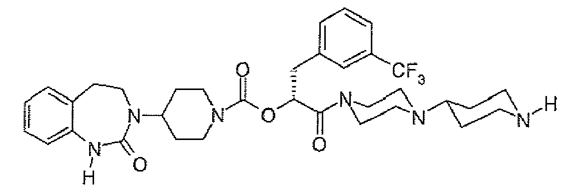

Analog Beispiel 151 wurde aus 149 mg (0.20 mmol) 4-(2-Oxo-1,2,4,5-tetrahydro-1,3-benzdiazepin-3-yl)-piperidin-1-carbonsäure-(R)-1-(3-trifluormethyl-benzyl)-2-[4-(1-*tert*-butoxy-carbonyl-piperidin-4-yl)-piperazin-1-yl]-2-oxo-ethylester (Beispiel 150) das Produkt erhalten.

| | |
|---|---|
| Ausbeute: | 66 mg (51 % der Theorie) |
| ESI-MS: | (M+H)⁺ = 657 |
| R_{f} = | 0.18 (Kieselgel, DCM/MeOH/Cyc/NH₃ 70:15:15:2) |

### Beispiel 153

### 4-(2-Oxo-1,2,4,5-tetrahydro-1,3-benzdiazepin-3-yl)-piperidin-1-carbonsäure-(R)-2-[4-(4-methyl-piperazin-1-yl)-piperidin-1-yl]-2-oxo-1-(3-methyl-benzyl)-ethylester

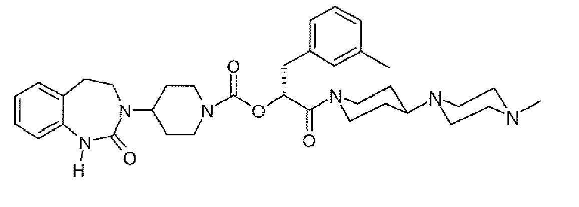

### 153a (Z,E)-2-Acetylamino-3-m-tolyl-acrylsäure

Analog Beispiel 1a konnte aus 25.0 g (212 mmol) 3-Methyl-benzaldehyd und 24.9 g (212 mmol) N-Acetyl-glycin das Produkt erhalten werden.

| | |
|---|---|
| Ausbeute: | 26.0 g (56% der Theorie) |
| ESI-MS: | (M+H)⁺ = 220 |
| Retentionszeit (HPLG): | 5.4 min (Methode A) |

### 153b 2-Oxo-3-m-tolyl-propionsäure

Analog Beispiel 46b konnte aus 13.0 g (59.3 mmol) (Z,E)-2-Acetylamino-3-m-tolyl-acrylsäure das Produkt erhalten werden.

| | |
|---|---|
| Ausbeute: | 9.2 g (88% der Theorie) |
| ESI-MS: | (M-H)⁻ = 177 |
| Retentionszeit (HPLC): | 7.3 min (Methode A) |

### 153c (R)-2-Hydroxy-3-m-tolyl-propionsäure

Analog Beispiel 1c konnte aus 9.24 g (51.9 mmol) 2-Oxo-3-*m*-tolyl-propionsäure und 24.0 g (74.8 mmol) (1R)-B-Chlordiisopinocampheylboran das Produkt erhalten werden.

| | |
|---|---|
| Ausbeute: | 8.4 g (90% der Theorie) |
| ESI-MS: | (M-H)⁻ = 179 |
| Retentionszeit (HPLC): | 7.2 min (Methode A) |

### 153d (R)-2-Hydroxy-3-m-tolyl-propionsäuremethylester

Analog Beispiel 31e konnte aus 8.40 g (46.6 mmol) (R)-2-Hydroxy-3-*m*-tolyl-propionsäure und 3.74 mL (51.28 mmol) SOCl₂ das Produkt erhalten werden.

| | |
|---|---|
| Ausbeute: | 6.28 g (69% der Theorie) |
| ESI-MS: | (M+H)⁺ = 195 |
| Retentionszeit (HPLC): | 6.9 min (Methode A) |

### 153e 4-(2-Oxo-1,2,4,5-tetrahydro-1,3-benzdiazepin-3-yl)-piperidin-1-carbonsäure-(R)-1-methoxycarbonyl-2-m-tolyl-ethylester

Analog Beispiel 31f konnte aus 1.12 g (5.76 mmol) (R)-2-Hydroxy-3-*m*-tolyl-propionsäuremethylester und 1.41 g (5.76 mmol) 3-Piperidin-4-yl-1,3,4,5-tetrahydro-1,3-benzdiazepin-2-on das Produkt erhalten werden.

| | |
|---|---|
| Ausbeute: | 2.07 g (77% der Theorie) |
| ESI-MS: | (M+H)⁺ = 466 |
| Retentionszeit (HPLC): | 9.0 min (Methode A) |

### 153f 4-(2-Oxo-1,2,4,5-tetrahydro-1,3-benzdiazepin-3-yl)-piperidin-1-carbonsäure-(R)-1-carboxy-2-m-tolyl-ethylester

Analog Beispiel 46f konnte aus 2.07 g (4.45 mmol) 4-(2-Oxo-1,2,4,5-tetrahydro-1,3-benzdiazepin-3-yl)-piperidin-1-carbonsäure-(R)-1-methoxycarbonyl-2-*m*-tolyl-ethylester und 0.16 g (6.72 mmol) LiOH das Produkt erhalten werden.

| | |
|---|---|
| Ausbeute: | 1.86 g (93% der Theorie) |
| ESI-MS: | (M+H)⁺ = 452 |
| Retentionszeit (HPLC): | 8.0 min (Methode A) |

### 153g 4-(2-Oxo-1,2,4,5-tetrahydro-1,3-benzdiazepin-3-yl)-piperidin-1-carbonsäure-(R)-2-[4-(4-methyl-piperazin-1-yl)-piperidin-1-yl]-2-oxo-1-(3-methyl-benzyl)-ethylester

Analog Beispiel 1h konnte aus 80.0 mg (0.18 mmol) 4-(2-Oxo-1,2,4,5-tetrahydro-1,3-benzdiazepin-3-yl)-piperidin-1-carbonsäure-(R)-1-carboxy-2-*m*-tolyl-ethylester und 33.1 mg (0.18 mmol) 1-Methyl-4-piperidin-4-yl-piperazin das Produkt erhalten werden.

| | |
|---|---|
| Ausbeute: | 46.7 mg (43% der Theorie) |
| ESI-MS: | (M+H)⁺ = 617 |
| Retentionszeit (HPLC): | 5.5 min (Methode A) |

Analog wurden folgende Verbindungen aus jeweils 80 mg 4-(2-Oxo-1,2,4,5-tetrahydro-1,3-benzdiazepin-3-yl)-piperidin-1-carbonsäure-(R)-1-carboxy-2-*m*-tolyl-ethylester und der entsprechenden Menge an Amin hergestellt:

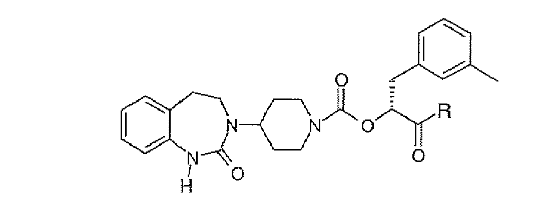

| **Beispiel** | **R** | **Ausbeute (%)** | **Massen-spektrum** | **Retentionszeit HPLC** **(Methode)** |
|---|---|---|---|---|
| **154** | 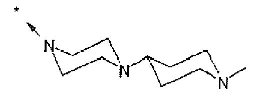 | 80 | 617 [M+H]⁺ | 5.0 min (A) |
| **155** | 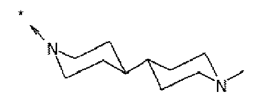 | 75 | 616 [M+H]⁺ | 6.2 min (A) |

Die nachfolgenden Beispiele beschreiben die Herstellung pharmazeutischer Anwendungsformen, die als Wirkstoff eine beliebige Verbindung der allgemeinen Formel (I) enthalten:

### Beispiel I

| Kapseln zur Pulverinhalation mit 1 mg Wirkstoff | |
|---|---|
| Zusammensetzung: | |
| 1 Kapsel zur Pulverinhalation enthält: | |
| Wirkstoff | 1.0 mg |
| Milchzucker | 20.0 mg |
| Hartgelatinekapseln | 50.0 mg |
| | 71.0 mg |

### Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel II

| Inhalationslösung für Respimat^{®} mit 1 mg Wirkstoff | | |
|---|---|---|
| Zusammensetzung: | | |
| 1 Hub enthält: | | |
| Wirkstoff | 1.0 | mg |
| Benzalkoniumchlorid | 0.002 | mg |
| Dinatriumedetat | 0.0075 | mg |
| Wasser gereinigt ad | 15.0 | µl |

### Herstellungsverfahren:

Der Wirkstoff und Benzalkoniumchlorid werden in Wasser gelöst und in Respimat^{®}-Kartuschen abgefüllt.

### Beispiel III

| Inhalationslösung für Vernebler mit 1 mg Wirkstoff | | |
|---|---|---|
| Zusammensetzung: | | |
| 1 Fläschchen enthält: | | |
| Wirkstoff | 0.1 | g |
| Natriumchlorid | 0.18 | g |
| Benzalkoniumchlorid | 0.002 | g |
| Wasser gereinigt ad | 20.0 | ml |

### Herstellungsverfahren:

Wirkstoff, Natriumchlorid und Benzalkoniumchlorid werden in Wasser gelöst.

### Beispiel IV

| Treibgas-Dosieraerosol mit 1 mg Wirkstoff | | |
|---|---|---|
| Zusammensetzung: | | |
| 1 Hub enthält: | | |
| Wirkstoff | 1.0 | mg |
| Lecithin | 0.1 | % |
| Treibgas ad | 50.0 | µl |

### Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

### Beispiel V

| Nasalspray mit 1 mg Wirkstoff | | |
|---|---|---|
| Zusammensetzung: | | |
| Wirkstoff | 1.0 | mg |
| Natriumchlorid | 0.9 | mg |
| Benzalkoniumchlorid | 0.025 | mg |
| Dinatriumedetat | 0.05 | mg |
| Wasser gereinigt ad | 0.1 | ml |

### Herstellungsverfahren:

Der Wirkstoff und die Hilfsstoffe werden in Wasser gelöst und in ein entsprechendes Behältnis abgefüllt.

### Beispiel VI

| Injektionslösung mit 5 mg Wirksubstanz pro 5 ml | |
|---|---|
| Zusammensetzung: | |
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel VII

| | |
|---|---|
| Injektionslösung mit 100 mg Wirksubstanz pro 20 ml | |
| Zusammensetzung: | |
| Wirksubstanz | 100 mg |
| Monokaliumdihydrogenphosphat = KH₂PO₄ | 12 mg |
| Dinatriumhydrogenphosphat = Na₂HPO₄·2H₂O | 2 mg |
| Natriumchlorid | 180 mg |
| Human-Serum-Albumin | 50 mg |
| Polysorbat 80 | 20 mg |
| Wasser für Injektionszwecke ad | 10 ml |

### Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

### Beispiel VIII

| Lyophilisat mit 10 mg Wirksubstanz | |
|---|---|
| Zusammensetzung: | |
| Wirksubstanz | 10 mg |
| Mannit | 300 mg |
| Human-Serum-Albumin | 20 mg |
| Wasser für Injektionszwecke ad | 2 ml |

### Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

| | |
|---|---|
| Lösungsmittel für Lyophilisat: | |
| Polysorbat 80 = Tween 80 | 20 mg |
| Mannit | 200 mg |
| Wasser für Injektionszwecke ad | 10 ml |

### Herstellung:

Polysorbat 80 und Mannit in Wasser für Injektionszwecke (Wfl) auflösen; in Ampullen abfüllen.

### Beispiel IX

| Tabletten mit 20 mg Wirksubstanz | |
|---|---|
| Zusammensetzung: | |
| Wirksubstanz | 20 mg |
| Lactose | 120 mg |
| Maisstärke | 40 mg |
| Magnesiumstearat | 2 mg |
| Povidort K 25 | 18 mg |

### Herstellung:

Wirksubstanz, Lactose und Maisstärke homogen mischen; mit einer wässerigen Lösung von Povidon granulieren; mit Magnesiumstearat mischen; auf einer Tablettenpresse abpressen; Tablettengewicht 200 mg.

### Beispiel X

| Kapseln mit 20 mg Wirksubstanz | | |
|---|---|---|
| Zusammensetzung: | | |
| Wirksubstanz | 20 | mg |
| Maisstärke | 80 | mg |
| Kieselsäure, hochdispers | 5 | mg |
| Magnesiumstearat | 2.5 | mg |

### Herstellung:

Wirksubstanz, Maisstärke und Kieselsäure homogen mischen; mit Magnesiumstearat mischen; Mischung auf einer Kapselfüllmaschine in Hartgelatine-Kapseln Größe 3 abfüllen.

### Beispiel XI

| Zäpfchen mit 50 mg Wirksubstanz | |
|---|---|
| Zusammensetzung: | |
| Wirksubstanz | 50 mg |
| Hartfett (Adeps solidus) q.s. ad | 1700 mg |

### Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgießen.

### Beispiel XII

| Injektionslösung mit 10 mg Wirksubstanz pro 1 mL | |
|---|---|
| Zusammensetzung: | |
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

## Patentansprüche

1. CGRP-Antagonisten der allgemeinen Formel 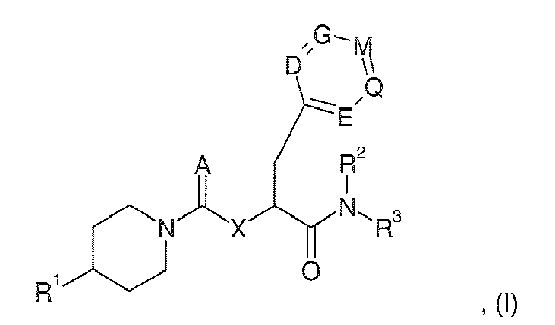 in der
A ein Sauerstoff- oder Schwefelatom,
X ein Sauerstoff- oder Schwefelatom,
(a) D, E unabhängig voneinander jeweils eine Methingruppe oder das Stickstoffatom und
G eine durch die Gruppe R^{a} substituierte Methingruppe,
M eine durch die Gruppe R^{b} substituierte Methingruppe,
Q eine durch die Gruppe R^{c} substituierte Methingruppe,
wobei eine oder zwei der Gruppen G, M und Q jeweils auch ein Stickstoffatom bedeuten können,
oder
(b) D und E jeweils eine Methingruppe, wobei eine der Gruppen D und E auch ein Stickstoffatom bedeuten kann, und
G, M und Q jeweils ein Stickstoffatom,
wobei R^{a}, R^{b} und R^{c} unabhängig voneinander jeweils ein Wasserstoff_ oder Halogenatom, eine C₁₋₆-Alkyl-, C₂₋₆-Alkenyl-, C₂₋₆-Alkinyl-, Cyclo-C₃₋₇-alkyl-, Cyclo-C₃₋₇-alkenyl-, Cyano-, Hydroxy-, Hydroxy-C₁₋₆-alkyl-, Hydroxy-C₃₋₆-alkenyl-, Hydroxy-C₃₋₆-alkinyl-, C₁₋₆-Alkoxy-, C₁₋₆-Alkoxy-C₁₋₆-alkyl-, C₁₋₆-Alkoxy-C₃₋₆-alkenyl-, C₁₋₆-Alkoxy-C₃₋₆-alkinyl-, C₃₋₆-Alkenoxy-C₁₋₆-alkyl-, C₃₋₆-Alkenoxy-C₃₋₆-alkenyl-, C₃₋₆-Alkenoxy-C₃₋₆-alkinyl-, C₃₋₆-Alkinoxy-C₁₋₆-alkyl-, C₃₋₆-Alkinoxy-C₃₋₆-alkenyl-, C₃₋₆-Alkinoxy-C₃₋₆-alkinyl-, Thiohydroxy-, C₁₋₆-Alkylthio-, C₃₋₆-Alkenylthio-, C₃₋₆-Alkinylthio-, Amin-, C₁₋₆-Alkyl-amino-, C₃₋₆-Alkenyl-amino-, C₃₋₆-Alkinyl-amino-, Di-(C₁₋₆-alkyl)-amino-, Di-(C₃₋₆-alkenyl)-amino-, Di-(C₃₋₆-alkinyl)-amino-, Amino-C₁₋₆-alkyl-, C₁₋₃-Alkyl-amino-C₁₋₆-alkyl-, Di(C₁₋₃-Alkyl)-amino-C₁₋₆-alkyl-, Amino-C₃₋₆-alkenyl-, C₁₋₃-Alkyl-amino-C₃₋₆-alkonyl-, Di-(C₁₋₃-alkyl)-amino-C₃₋₆-alkenyl-, Amino-C₃₋₆-alkinyl-, C₁₋₃-Alkyl-amino-C₃₋₆-alkinyl-, Di-(C₁₋₃-alkyl)-amino-C₃₋₆-alkinyl-, Hydroxycarbonyl-, Phenylcarbonyl-, Pyridylcarbonyl-, C₁₋₆-Alkyl-carbonyl-, C₂₋₆-Alkenyl-carbonyl-, C₂₋₆-Alkinyl-carbonyl-, Formyl-, C₁₋₆-Alkoxy-carbonyl-, C₃₋₆-Alkenoxy-carbonyl-, C₃₋₆-Alkinoxy-carbonyl-, Aminocarbonyl-, C₁₋₆-Alkyl-aminocarbonyl-, C₃₋₆-Alkenyl-aminocarbonyl-, C₃₋₆-Alkinyl-aminocarbonyl-, Di-(C₁₋₆-alkyl)-aminocarbonyl-, Di-(C₃₋₆-alkenyl)-aminocarbonyl-, Di-(C₃₋₆-alkinyl)-aminocarbonyl-, Formylamino-, C₁₋₆-Alkyl-carbonylamino-, C₂₋₆-Alkenyl-carbonylamino-, C₂₋₆-Alkinyl-carbonylamino-, Formyl-C₁₋₆-alkyl-amino-, Formyl-C₃₋₆-alkenyl-amino-, Formyl-C₃₋₆-alkinyl-amino-, C₁₋₆-Alkyl-carbonyl-C₁₋₆-alkyl-amino-, C₂₋₆-Alkenyl-carbonyl-C₁₋₆-alkyl-amino-, C₂₋₆-Alkinyl-carbonyl-C₁₋₆-alkyl-amino-, C₁₋₆-Alkyl-carbonyl-C₃₋₆-alkenyl-amino-, C₂₋₆-Alkenyl-carbonyl-C₃₋₆-alkenyl-amino, C₂₋₆-Alkinyl-carbonyl-C₃₋₆-alkenyl-amino-, C₁₋₆-Alkyl-carbonyl-C₃₋₆-alkinyl-amino-, C₂₋₆-Alkenyl-carbonyl-C₃₋₆-alkinyl-amino-, C₂₋₆-Alkinyl-carbonyl-C₃₋₆-alkinyl-amino-, C₁₋₆-Alkyl-sulfonyl-, C₂₋₆-Alkenyl-sulfonyl-, C₂₋₆-Alkinyl-sulfonyl-, C₁₋₆-Alkyl-sulfinyl, C₂₋₅-Alkenyl-sulfinyl-, C₂₋₆-Alkinyl-sulfinyl-, C₁₋₆-Alkyl-sulfonylamino-, C₂₋₆-Alkenyl-sulfonylamino-, C₂₋₆-Alkinyl-sulfonylamino-, C₁₋₆-Alkyl-sulfonyl-C₁₋₆-alkylamino-, C₁₋₆-Alkyl-sulfonyl-C₃₋₆-alkenylamino-, C₁₋₆-Alkyl-sulfonyl-C₃₋₆-alkinylamino-, C₂₋₆-Alkenyl-sulfonyl-C₁₋₆-alkylamino-, C₂₋₆-Alkenyl-sulfonyl-C₃₋₆-alkenylamino-, C₂₋₆-Alkenyl-sulfonyl-C₃₋₆-alkinylamino-, C₂₋₆-Alkinyl-sulfonyl-C₁₋₆-alkylamino-, C₂₋₆-Alkinyl-sulfonyl-C₃₋₆-alkenylamino-, C₂₋₆-Alkinyl-suifonyl-C₃₋₆-alkinylamino-, Aminosulfonyl-, C₁₋₆-Alkylaminosulfonyl-, Di-(C₁₋₆-alkyl)-aminosulfonyl-, C₃₋₆-Alkenyl-aminosulfonyl-, Di-(C₃₋₆-alkenyl)-aminosulfonyl-, C₃₋₆-Alkinylaminosulfonyl- oder Di-(C₃₋₆-alkinyl)-aminosulfonylgruppe bedeuten
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₆-Alkylgruppe darstellen,
(ii) R^{c} kein Wasserstoffatom bedeutet, wenn R^{a} sowie R^{b} jeweils eine C₁₋₆-Alkylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine C₁₋₆-Alkyl-, C₂₋₆-Aikenyl- oder C₂₋₆-Alkinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine C₁₋₆-Alkyl-, C₂₋₆-Alkenyl- oder C₂₋₆-Alkinylgruppe und R^{b} ein Chlor- oder Bromatom, eine Amino-, Methylamino- oder Hydroxygruppe darstellen,
R¹ einen gesättigten, einfach oder zweifach ungesättigten 5- bis 7-gliedrigen Aza-, Diaza-, Triaza-, Oxaza-, Thiaza-, Thiadiaza- oder *S,S*-Dioxido-thiadiaza-Heterocyclus,
wobei die vorstehend erwähnten Heterocyclen über ein Kohlenstoff- oder Stickstoffatom mit dem Piperidinring in Formel I verknüpft sind oder
über zwei Kohlenstoffatome, über ein Kohlenstoff- und ein Stickstoffatom, über ein Kohlenstoff- und ein Sauerstoffatom oder über ein Kohlenstoff- und ein Schwefelatom mit dem Piperidinring in Formel I spirocyclisch verknüpft sind,
eine oder zwei Carbonyl- oder Thiocarbonylgruppen benachbart zu einem Stickstoffatom enthalten,
an einem der Stickstoffatome durch eine C₁₋₆-Alkyl-, C₃₋₆-Alkonyl- oder C₃₋₆-Alkenylgruppe substituiert sein können,
an einem oder an zwei Kohlenstoffatomen durch eine C₁₋₆-Alkyl-, C₂₋₆-Alkenyl-oder C₂₋₆-Alkinylgruppe, durch eine Phenyl-, Phenylmethyl-, Naphthyl-, Biphenylyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-(C₁₋₃-Alkyl)-pyrazolyl-, Imidazolyl- oder 1-(C₁₋₃-Alkyl)-imidazolylgruppe substituiert sein können, wobei die Substituenten gleich oder verschieden sein können, und
eine olefinische Doppelbindung eines der vorstehend erwähnten ungesättigten Heterocyclen mit einem Phenyl-, Naphthyl-, Pyridin-, Diazin-, 1,3-Oxazol-, Thienyl-, Furan-, Thiazol-, Pyrrol-, *N*-C₁₋₃-Alkyl-pyrrol- oder Chinolin-Ring, mit einem am Stickstoffatom gegebenenfalls durch eine C₁₋₆-Alkyl-, C₃₋₆-Alkenyl-oder C₃₋₆-Alkinylgruppe substituierten 1*H*-Chinolin-2-on-Ring oder mit einem Imidazol- oder *N*-C₁₋₃-Alkyl-imidazol-Ring kondensiert sein kann oder auch zwei olefinische Doppelbindungen eines der vorstehend erwähnten ungesättigten Heterocyclen jeweils mit einem Phenyl- oder Pyridinring kondensiert sein können,
wobei die in R¹ enthaltenen Phenyl-, Pyridinyl-, Diazinyl-, Furyl-, Thienyl-, Pyrrolyl-, 1,3-Oxazolyl-, 1,3-Thiazolyl-, Isoxazolyl-, Pyrazolyl-, 1-C₁₋₃-Alkyl-pyrazolyl-, Imidazolyl- oder 1-C₁₋₃-Alkyl-imidazolylgruppen sowie benzo-, thieno-, pyrido- und diazinokondensierten Heterocyclen im Kohlenstoffgerüst zusätzlich durch Halogenatome, C₁₋₆-Alkyl-, C₂₋₆-Alkenyl-, C₂₋₆-Alkinyl-, Cyclo-C₃₋₇-alkyl-, Cyclo-C₃₋₇-alkenyl-, Cyano-, Hydroxy-, Hydroxy-C₁₋₆-alkyl-, Hydroxy-C₃₋₆-alkenyl-, Hydroxy-C₃₋₆-alkinyl-, C₁₋₆-Alkoxy-, C₁₋₆-Alkoxy-C₁₋₆-alkyl-, C₁₋₆-Alkoxy-C₃₋₆-alkenyl-, C₁₋₆-Alkoxy-C₃₋₆-alkinyl-, C₃₋₆-Alkenoxy-C₁₋₆-alkyl-, C₃₋₆-Alkenoxy-C₃₋₆-alkenyl-, C₃₋₆-Alkenoxy-C₃₋₆-alkinyl-, C₃₋₆-Alkinoxy-C₁₋₆-alkyl-, C₃₋₆-Alkinoxy-C₃₋₆-alkenyl-, C₃₋₆-Alkinoxy-C₃₋₆-alkinyl-, Thiohydroxy-, C₁₋₆-Alkylthio-, C₃₋₆-Alkenylthio-, C₃₋₆-Alkinylthio-, Amino-, C₁₋₆-Alkyl-amino-, C₃₋₆-Alkenyl-amino-, C₃₋₆-Alkinyl-amino-, Di-(C₁₋₆-alkyl)-amino-, Di-(C₃₋₆-alkenyl)-amino-, Di-(C₃₋₆-alkinyl)-amino-, Amino-C₁₋₆-alkyl-, C₁₋₃-Alkyl-amino-C₁₋₆-alkyl-, Di-(C₁₋₃-alkyl)-amino-C₁₋₆-alkyl-, Amino-C₃₋₆-alkenyl-, C₁₋₃-Alkyl-amino-C₃₋₆-alkenyl-, Di-(C₁₋₃-alkyl)-amino-C₃₋₆-alkenyl-, Amino-C₃₋₆-alkinyl-, C₁₋₃-Alkyl-amino-C₃₋₆-alkinyl-, Di-(C₁₋₃-alkyl)-amino-C₃₋₆-alkinyl-, Hydroxycarbonyl-, Phenylcarbonyl-, Pyridylcarbonyl-, C₁₋₆-Alkyl-carbonyl-, C₂₋₆-Alkenyl-carbonyl-, C₂₋₆-Alkinyl-carbonyl-, Formyl-, C₁₋₆-Alkoxy-carbonyl-, C₃₋₆-Alkenoxy-carbonyl-, C₃₋₆-Alkinoxy-carbonyl-, Aminocarbonyl-, C₁₋₆-Alkyl-aminocarbonyl-, C₃₋₆-Alkenyl-aminocarbonyl-, C₃₋₆-Alkinyl-aminocarbonyl-, Di-(C₁₋₆-alkyl)-aminocarbonyl-, Di-(C₃₋₆-alkenyl)-aminocarbonyl-, Di-(C₃₋₆-alkinyl)-aminocarbonyl-, Formylamino-, C₁₋₆-Alkyl-carbonylamino-, C₂₋₆-Alkonyl-carbonylamino-, C₂₋₆-Alkinyl-carbonylamino-, Formyl-C₁₋₆-alkyl-amino-, Formyl-C₃₋₆-alkenyl-amino-, Formyl-C₃₋₆-alkinyl-amino-, C₁₋₆-Alkyl-carbonyl-C₁₋₆-alkyl-amino-, C₂₋₆-Alkonyl-carbonyl-C₁₋₆-alkyl-amino-, C₂₋₆-Alkinyl-carbonyl-C₁₋₆-alkyl-amino-, C₁₋₆-Alkyl-carbonyl-C₃₋₆-alkenyl-amino-, C₂₋₆-Alkenyl-carbonyl-C₃₋₆-alkenyl-amino-, C₂₋₆-Alkinyl-carbonyl-C₃₋₆-alkenyl-amino-, C₁₋₆-Alkyl-carbonyl-C₃₋₆-alkinyl-amino-, C₂₋₆-Alkenyl-carbonyl-C₃₋₆-alkinyl-amino-, C₂₋₆-Alkinyl-carbonyl-C₃₋₆-alkinyl-amino-, C₁₋₆-Alkylsulfonyl-, C₂₋₆-Alkenyl-sulfonyl-, C₂₋₆-Alkinyl-sulfonyl-, C₁₋₆-Alkyl-sulfinyl-, C₂₋₆-Alkenyl-sulfinyl-, C₂₋₆-Alkinyl-sulfinyl-, C₁₋₆-Alkyl-sulfonylamino-, C₂₋₆-Alkonyl-sulfonylamino-, C₂₋₆-Alkinyl-sulfonylamino-, C₁₋₆-Alkyl-sulfonyl-C₁₋₆-alkylamino-, C₁₋₆-Alkyl-sulfonyl-C₃₋₆-alkenylamino-, C₁₋₆-Alkyl-sulfonyl-C₃₋₆-alkinylamino-, C₂₋₆-Alkenyl-sulfonyl-C₁₋₆-alkylamino-, C₂₋₆-Alkenyl-sulfonyl-C₃₋₆-alkenylamino-, C₂₋₆-Alkenyl-sulfonyl-C₃₋₆-alkinylamino-, C₂₋₆-Alkinyl-sulfonyl-C₁₋₆-alkylamino-, C₂₋₆-Alkinyl-sulfonyl-C₃₋₆-alkenylamino-, C₂₋₆-Alkinyl-sulfonyl-C₃₋₆-alkinylamino-, Aminosulfonyl-, C₁₋₆-Aikylaminosulfonyl-, Di-(C₁₋₆-alkyl)-aminosulfonyl-, C₃₋₆-Alkenylaminosulfonyl-, Di-(C₃₋₆-alkenyl)-aminosulfonyl-, C₃₋₆-Alkinylaminosulfonyl-, Di-(C₃₋₆-alkinyl)-aminosulfonylgruppen mono-, di- oder trisubstituiert sein können, wobei die Substituenten gleich oder verschieden sein können und
wobei die Doppel- und Dreifachbindungen der in den für R^{a}, R^{b}, R^{c} und R¹ vorstehend definierten Gruppen enthaltenen C₃₋₆-Alkenyl- oder C₃₋₆-Alkinylgruppen von in diesen Gruppen gegebenenfalls ebenfalls enthaltenen Heteroatomen isoliert sind,
R² das Wasserstoffatom,
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Diazinyl-, Hydroxy-, Amino-, C₁₋₆-Alkylamino-, Di-(C₁₋₆-alkyl)-amino-, C₃₋₆-Alkenylamino-, Di-(C₃₋₆-alkenyl)amino-, C₃₋₆-Alkinylamino-, Di-(C₃₋₆-alkinyl)amino-, Hydroxycarbonyl-, C₁₋₆-Alkoxycarbonyl-, Aminocarbonyl-, Aminocarbonylamino-, C₁₋₆-Alkylcarbonylamino-, C₂₋₆-Alkenylcarbonylamino-, C₂₋₆-Alkinylcarbonylamino-, 4-Morpholinyl-, [Bis-(2-hydroxyethyl)]amino-, 4-(C₁₋₆-Alkyl)-1-piperazinyl- oder 4-(ω-Hydroxy-C₂₋₇-alkyl)-1-piperazinylgruppe substituiert sein kann,
eine Phenyl- oder Pyridinylgruppe,
wobei die in den für R² vorstehend definierten Gruppen genannten oder als Substituenten enthaltenen Phenyl-, Pyridinyl- und Diazinylgruppen zusätzlich im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₃-Alkylamino-, Di-(C₁₋₃-alkyl)-amino-, Amino-C₁₋₃-alkyl-, C₁₋₃-Alkyl-amino-C₁₋₃-alkyl-, Di-(C₁₋₃-alkyl)-amino-C₁₋₃-alkyl-, C₁₋₃-Alkylcarbonylamino-, C₁₋₃-Alkylcarbonylamino-C₁₋₃-alkyl-, Aminocarbonyl-, C₁₋₃-Alkyl-aminocarbonyl-, Di-(C₁₋₃-alkyl)-aminocarbonyl-, Cyano-, Aminosulfonyl-, C₁₋₃-Alkyl-aminosulfonyl-, Di-(C₁₋₃-alkyl)-aminosulfonyl-, C₁₋₃-Alkyl-thio-, C₁₋₃-Alkyl-sulfinyl-, oder C₁₋₃-Alkyl-sulfonyl- mono-, di- oder trisubstituiert und die Substituenten gleich oder verschieden sein können,
R³ das Wasserstoffatom oder eine durch eine Phenyl- oder Pyridinylgruppe, substituierte C₁₋₃-Alkylgruppe,
wobei die C₁₋₃-Alkylgruppe mit einer in R² enthaltenen Alkylgruppe oder einem in R² enthaltenen Phenyl- oder Pyridylring unter Einschluss des Stickstoffatoms, an weiches R² und R³ gebunden sind, unter Ausbildung eines 4- 7-gliedrigen Ringes verbunden sein kann,
oder
R² und R³ zusammen mit dem eingeschlossenen Stickstoffatom einen Rest der allgemeinen Formel 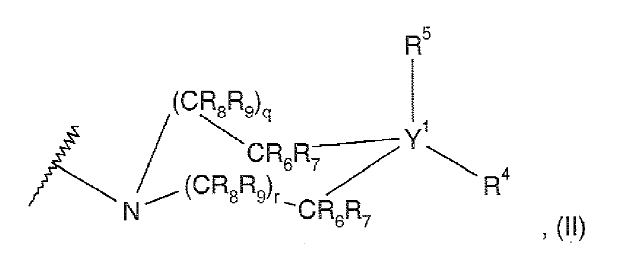 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, oder
q und r, wenn Y¹ das Stickstoffatom darstellt, die Zahlen 1 oder 2,
R⁴ das Wasserstoffatom, eine Amino-, C₁₋₄-Alkylamino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl- oder Cyclo-C₃₋₇-alkenylgruppe, eine Amino-C₂₋₇-alkyl-, C₁₋₄-Alkyl-amino-C₂₋₇-alkyl-, Di-(C₁₋₄-alkyl-amino)-C₂₋₇-alkyl-, Aminoiminomethyl-, Aminocarbonylamino-, C₁₋₄-Alkyl-aminocarbonylamino-, Di-(C₁₋₄-alkyl)-aminocarbonylamino-, C₁₋₄-Alkyl-aminocarbonyl-C₁₋₄-alkyl-amino-, Di-(C₁₋₄-alkyl)-aminocarbonyl-C₁₋₄-alkyl-amino-, Phenylaminocarbonylamino-, Aminocarbonyl-, C₁₋₄-Alkyl-aminocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl-, Di-(C₁₋₄-alkyl)-aminocarbonyl-C₁₋₃-alkyl-, Aminocarbonylamlno-C₁₋₃-alkyl-, C₁₋₆-Alkoxycarbonyl-, C₃₋₆-Alkenoxycarbonyl-, C₃₋₆-Alkinoxycarbonyl-, C₁₋₆-Alkoxycarbonyl-C₁₋₃-alkyl-, C₁₋₆-Alkenoxycarbonyl-C₁₋₃-alkyl-, C₁₋₆-Alkinoxycarbonyl-C₁₋₃-alkyl- oder Hydroxycarbonyl-C₁₋₃-alkylgruppe,
eine Phenyl-, Pyridinyl-, Diazinyl-, 1-Naphthyl-, 2-Naphthyl-, Pyridinylcarbonyl-oder Phenylcarbonylgruppe, die jeweils im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkyl-amino-C₁₋₃-alkyl-, Di-(C₁₋₄-alkyl)-amino-C₁₋₃-alkyl-, C₁₋₄-Alkylcarbonylamino-, C₁₋₄-Alkylcarbonylamino-C₁₋₃-alkyl-, Aminocarbonyl-, C₁₋₄-Alkyl-aminocarbonyl-, Di-(C₁₋₄-alkyl)-aminocarbonyl-, Cyano-, Aminosulfonyl-, C₁₋₄-Alkyl-aminosulfonyl-, Di-(C₁₋₄-alkyl)-aminosulfonyl-, C₁₋₄-Alkyl-thio-, C₁₋₄-Alkyl-sulfinyl-, oder C₁₋₄-Alkylsulfonyl- mono-, di- oder trisubstituiert und die Substituenten gleich oder verschieden sein können,
ein Heterocyclus ausgewählt aus einer 4- bis 10-gliedrigen Azacycloalkylgruppe, einer 6- bis 10-gliedrige Oxaza-, Thiaza-, *S,S*-Dioxothiaza- und Diazacycloalkylgruppe sowie einer 6- bis 10-gliedrigen Azabicycloalkylgruppe,
eine 1-Alkyl-4-piperidinylcarbonyl- oder 4-Alkyl-1-piperazinylcarbanylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methingruppe durch ein Fluoratom sowie eine nicht direkt mit einem Stickstoff-, Sauerstoff- oder Schwefelatom verknüpfte Methylengruppe durch ein oder zwei Fluoratome substituiert sein kann,
die vorstehend genannten mono- und bicyclischen Heterocyclen sowie die 1-(C₁₋₆-Alkyl)-4-piperidinylcarbonyl- und 4-(C₁₋₆-Alkyl)-1-piperazinylcarbonylgruppe im Ring ein- bis vierfach durch Hydroxy-, C₁₋₆-Alkyl- oder Hydroxy-C₁₋₃-alkylgruppen, oder, gegebenenfalls zusätzlich, einfach durch eine Cyclo-C₃₋₇-alkyl-, Hydroxy-C₃₋₇-cycloalkyl-, Cyclo-C₃₋₇-alkenyl-, Cyclo-C₃₋₇-alkyl-C₁₋₃-alkyl-, Phenyl-C₁₋₃-alkyl-, Pyridyl-C₁₋₃-alkyl-, C₁₋₆-Alkylcarbonyl-, C₁₋₆-Alkylcarbonyl-C₁₋₃-alkyl-, Hydroxy-, C₁₋₆-Alkoxy-, Amin-, C₁₋₄-Alkyiamino-, Di-(C₁₋₄-alkyl)amino-, Phenylcarbonyl-, Pyridinylcarbonyl-, C₁₋₆-Alkoxycarbonyl-, Hydroxycarbonyl-carbonyl-, C₁₋₆-Alkoxycarbonyl-carbonyl-, Hydroxycarbonyl-C₁₋₃-alkyl-, C₁₋₆-Alkoxycarbonyl-C₁₋₃-alkyl, Hydroxycarbonyl-C₁₋₃-alkylcarbonyl-, C₁₋₆-Alkoxycarbonyl-C₁₋₃-alkylcarbonyl-, Aminocarbonyl-, C₁₋₄-Alkylaminocarbonyl-, Di-(C₁₋₄-alkyl)aminocarbonyl-, Aminosulfonyl-, C₁₋₄-Alkylaminosulfonyl-, Di-(C₁₋₄-alkyl)aminosulfonyl-, C₁₋₃-Alkylsulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)aminocarbonyl-C₁₋₃-alkyl-, Hydroxyaminocarbonyl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppe, durch eine im Ring gegebenenfalls C₁₋₃-alkylsubstituierte Cyclo-C₃₋₇-alkylcarbonyl-, Azacyclo-C₄₋₇-alkylcarbonyl-, Diazacydo-C₅₋₇-alkylcarbonyl- oder Oxazacyclo-C₅₋₇-alkylcarbonylgruppe substituiert sein können, wobei die Substituenten gleich oder verschieden und an ein Ring-Kohlenstoff- oder Ring-Stickstoffatom gebunden sein können,
wobei die in den für R⁴ vorstehend definierten Resten enthaltenen Phenyl- und Pyridinyl-Reste ihrerseits durch Halogenatome, durch C₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkyl-amino-C₁₋₃-alkyl-, Di-(C₁₋₄-alkyl)-amino-C₁₋₃-alkyl-, C₁₋₄-Alkylcarbonyl-amino-, C₁₋₄-Alkyloarbonylamino-C₁₋₃-alkyl-, Aminocarbonyl-, C₁₋₃-Alkylaminocarbonyl-, Di-C₁₋₄-alkyl-aminocarbonyl-, Cyano-, Aminosulfonyl-, C₁₋₄-Alkyl-aminosulfonyl-, Di-(C₁₋₄-alkyl)-aminosulfonyl-, C₁₋₃-Alkylthio-, C₁₋₃-Alkyl-sulfinyl-, oder C₁₋₃-Alkyl-sulfonyl- mono-, di- oder trisubstituiert sein können, wobei die Substituenten gleich oder verschieden sein können,
oder auch, wenn Y¹ das Kohlenstoftatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom oder eine Hydroxygruppe,
einen C₁₋₄-Alkylrest, wobei ein unverzweigter Alkylrest in ω-Stellung durch eine Phenyl-, Pyridinyl-, Diazinyl-, Amin-, C₁₋₄-Alkylamino-, Di-(C₁₋₄-alkyl)-amino-, 4 C₁₋₄-Alkyl-1-piperazinyl- oder 4-Morpholinylgruppe substituiert sein kann,
eine C₁₋₆-Alkoxycarbonyl-, Cyano- oder Aminocarbonylgruppe oder auch, wenn Y¹ ein Stickstoffatom darstellt, ein freies Elektronenpaar,
oder, wenn Y¹ das Kohlenstoffatom darstellt, auch das Fluoratom, oder
R⁴ zusammen mit R⁵ und Y¹ einen 4- bis 7-gliedrigen cycloaliphatischen Ring, in dem eine Methylengruppe durch eine Gruppe -NH-, -N(C₁₋₄-Alkyl)-, -N(C₃₋₄-Alkenyl)-, -N(C₃₋₄-A!kiny!)-, -N(Cyclo-C₃₋₇-alkyl)-, -N(C₃₋₇-Cycloalkyl-C₁₋₃-alkyl)-, -N(Hydroxycarbonyl-C₁₋₃-alkyl)- oder -N(C₁₋₆-Alkoxycarbonyl-C₁₋₃-alkyl)- ersetzt sein kann,
wobei ein an ein Stickstoffatom in einer der für R⁴ vorstehend definierten Gruppen gebundenes Wasserstoffatom durch einen Schutzrest ersetzt sein kann,
R⁶ und R⁷, die gleich oder verschieden sein können, jeweils ein Wasserstoffatom, eine C₁₋₄-Alkylgruppe oder auch, wenn Y¹ ein Kohlenstoffatom darstellt, das Fluoratom, eine Amino-, C₁₋₄-Alkylamino- oder Di-(C₁₋₄-alkyl)-aminogruppe, wobei die beiden C₁₋₄-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen, bedeuten,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyan- oder Hydroxygruppen mono-, di- oder trisubstituiert und die Substituenten gleich oder verschieden sein können und
unter den in den vor- und nachstehenden Definitionen genannten Schutzresten die aus der Peptidchemie geläufigen Schutzgruppen zur verstehen sind, insbesondere
eine im Phenylkern gegebenenfalls durch ein Halogenatom, durch eine Vitro- oder Phenylgruppe, durch eine oder zwei Methoxygruppe substituierte Phenylalkoxycarbonylgruppe mit 1 bis 3 Kohlenstoffatomen im Alkoxyteil,
beispielsweise die Benzyloxycarbonyl-, 2-Nitrobenzyloxycarbonyl-, 4-Nitro-benzyloxycarbonyl-, 4-Methoxy-benzyloxycarbonyl-, 2-Chlor-benzyloxycarbonyl-, 3-Chlor-benzyloxycarbonyl-, 4-Chlor-benzyloxycarbonyl-, 4-Biphenylyl-α,α-dimethyl-benzyloxycarbonyl- oder 3,5-Dimethoxy-α,α-dimethyl-benzyloxycarbonylgruppe,
eine Alkoxycarbonylgruppe mit insgesamt 1 bis 5 Kohlenstoffatomen im Alkylteil, beispielsweise die Methoxycarbonyl-, Ethoxycarbonyl-, *n*-Propoxycarbonyl-, Isopropoxycarbonyl-, *n*-Butoxycarbonyl-, 1-Methylpropoxycarbonyl-, 2-Methylpropoxy-carbonyl- oder *tert*.Butyloxycarbonylgruppe,
die Allyloxycarbonyl-, 2,2,2-Trichlor-(1,1-dimethylethoxy)carbonyl- oder 9-Fluorenylmethoxycarbonylgruppe oder
die Formyl-, Acetyl- oder Trifluoracetylgruppe,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

2. CGRP-Antagonisten der allgemeinen Formel (I) gemäß Anspruch 1, in denen
A, X, D, E, G, M, Q und R¹ wie in Anspruch 1 erwähnt definiert sind und
R² das Wasserstoffatom,
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Diazinyl-, Hydroxy-, Amin-, C₁₋₆-Alkylamino-, Di-(C₁₋₆-alkyl)-amino-, C₃₋₆-Alkenylamino-, Di-(C₃₋₆-alkenyl)amino-, C₃₋₆-Alkinylamino-, Di-(C₃₋₆-alkinyl)amino-, Hydroxycarbonyl-, C₁₋₆Alkoxycarbonyl-, Aminocarbonyl-, Aminocarbonylamino-, C₁₋₆-ALkylcarbonylamino-, C₂₋₆-Alkenyl-carbonylamino-, C₂₋₆-Alkinylcarbonylamino-, 4-Morpholinyl-, [Bis-(2-hydroxyethyl)]amino-, 4-(C₁₋₆-Alkyl)-1-piperazinyl- oder 4-(ω-Hydroxy-C₂₋₇-alkyl)-1-piperazinylgruppe substituiert sein kann,
eine Phenyl- oder Pyridinylgruppe,
wobei die in den für R² vorstehend definierten Gruppen genannten oder als Substituenten enthaltenen Phenyl-, Pyridinyl- und Diazinylgruppen zusätzlich im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₃-Alkylamino-, Di-(C₁₋₃-alkyl)-amino-, Amino-C₁₋₃-alkyl-, C₁₋₃-Alkyl-amino-C₁₋₃-alkyl-, Di-(C₁₋₃-alkyl)-amino-C₁₋₃-alkyl-, C₁₋₃-Alkylcarbonylamino-, C₁₋₃-Alkylcarbonylamino-C₁₋₃-alkyl., Aminocarbooyl-, C₁₋₃-Alkyl-aminocarbonyl-, Di-(C₁₋₃-alkyl)-aminocarbonyl-, Cyano-, Aminosulfonyl-, C₁₋₃-Alkyl-aminosulfonyl-, Di-(C₁₋₃-alkyl)-aminosulfonyl-, C₁₋₃-Alkyl-thio-, C₁₋₃-Alkyl-sulfinyl-, oder C₁₋₃-Alkyl-sulfonyl- mono-, di- oder trisubstituiert und die Substituenten gleich oder verschieden sein können,
R³ das Wasserstoffatom oder eine durch eine Phenyl- oder Pyridinylgruppe substituierte C₁₋₃-Alkylgruppe,
wobei die C₁₋₃-Alkkylgruppe mit einer in R² enthaltenen Alkylgruppe oder einem in R² enthaltenen Phenyl- oder Pyridylring unter Einschluss des Stickstoffiatoms, an welches R² und R³ gebunden sind, unter Ausbildung eines 4- 7-gliedrigen Ringes verbunden sein kann,
oder
R² und R³ zusammen mit dem eingeschossene Stickstoffatom einen Rest der allgemeinen Formel 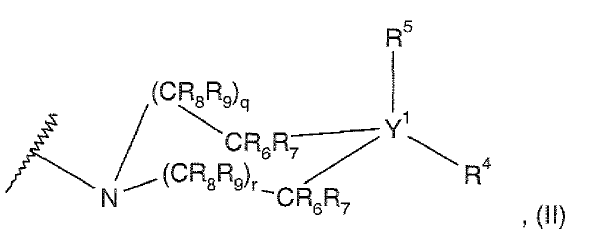 in der
Y¹ das Kohlenstoffatom oder, wenn R⁵ ein freies Elektrodenpaar darstellt, auch das Stickstoffatom,
q und r, wenn Y¹ das Kohlenstoffatom darstellt, die Zahlen 0, 1 oder 2, oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, eine Cyclo-C₃₋₇-alkyl-, Cyclo-C₃₋₇-alkenyl-, Amino-C₂₋₇-alkyl-, C₁₋₄-Alkyl-amino-C₂₋₇-alkyl-, Di-(C₁₋₄-alkyl-amino)-C₂₋₇-alkyl-, Aminciminomethyl-, Aminocarbonylamino-, C₁₋₄-Alkyl-aminocarbonylamino-, Di-(C₁₋₄-alkyl)-aminocarbonylamino-, C₁₋₄-Alkyl-aminocarbonyl-C₁₋₄-alkyl-amino-, Di-(C₁₋₄-alkyl)-aminocarbonyl-C₁₋₄-alkyl-amino-, Phenylaminocarbonylamino-, Aminocarbonyl-, C₁₋₄-Alkyl-aminocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl-, Di-(C₁₋₄-alkyl)-aminocarbonyl-C₁₋₃-alkyl-, Aminocarbonylamino-C₁₋₃-alkyl-, C₁₋₆-Alkoxycarbonyl-, C₃₋₆-Alkenoxycarbonyl-, C₃₋₆-Alkinoxycarbonyl-, C₁₋₆-Alkoxycarbonyl-C₁₋₃-alkyl-, C₁₋₆-Alkenoxycarbonyl-C₁₋₃-alkyl-, C₁₋₆-Alkinoxycarbonyl-C₁₋₃-alkyl- oder Hydroxycarbonyl-C₁₋₃-alkylgrcuppe,
eine Phenyl-, Pyridinyl-, Diazinyl-, 1-Naphthyl-, 2-Naphthyl-, Pyridinylcarbonyl-oder Phenylcarbonylgruppe, die jeweils im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkyl-amino-C₁₋₃-alkyl-, Di-(C₁₋₄-alkyl)-amino-C₁₋₃-alkyl-, C₁₋₄-Alkylcarbonylamino-, C₁₋₄-Alkylcarbonylamino-C₁₋₃-alkyl-, Aminocarbonyl-, C₁₋₄-Alkyl-aminocarbonyl-, Di-(C₁₋₄-alkyl)-aminocarbonyl-, Cyano-, Aminosulfonyl-, C₁₋₄-Alkyl-aminosulfonyl-, Di-(C₁₋₄-alkyl)-aminosultonyl-, C₁₋₄-Alkyl-thio-, C₁₋₄-Alkyl-sulfinyl-, oder C₁₋₄-Alkylsulfonyl- mono-, di- oder trisubstituiert und die Substituenten gleich oder verschieden sein können,
ein Heterocyclus ausgewählt aus einer 4- bis 10-gliedrigen Azacycloalkylgruppe, einer 6- bis 10-gliedrige Oxaza-, Thiaza- und Diazacycloalkylgruppe sowie einer 6- bis 10-gliedrigen Azabicycloalkylgruppe,
eine 1-Alkyl-4-piperidinylcarbonyl- oder 4-Alkyl-1-piperazinylcarbonylgruppe,
wobei die vorstehend genannten mono- und bicyclische Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methingruppe durch ein Fluoratom sowie eine nicht direkt mit einem Stickstoff-, Sauerstoff- oder Schwefelatom verknüpfte Methylengruppe durch ein oder zwei Fluoratome substituiert sein kann,
die vorstehend genannten mono- und bicyclischen Heterocyclen sowie die 1-(C₁₋₆-Alkyl)-4-piperidinylcarbonyl- und 4-(C₁₋₆-Alkyl)-1-piperazinylcarbonylgruppe im Ring ein- bis vierfach durch C₁₋₆-Alkylgruppen, oder, gegebenenfalls zusätzlich, einfach durch eine Cyclo-C₃₋₇-alkyl-, Cyclo-C₃₋₇-alkenyl-, Cyclo-C₃₋₇-alkyl-C₁₋₃-alkyl-, Phenyl-C₁₋₃-alkyl-, Pyridyl-C₁₋₃-alkyl-, C₁₋₆-Alkylcarbonyl-, Hydroxy-, C₁₋₆-Alkoxy-, Amino-, C₁₋₄-Alkylamino-, Di-(C₁₋₄-alkyl)amino-, Phenylcarbonyl-, Pyridinylcarbonyl-, Hydroxycarbonyl-, Hydroxycarbonyl-C₁₋₃-alkyl-, C₁₋₆-Alkoxycarbonyl-, C₁₋₆-Alkoxycarbonyl-C₁-₃-alkyl, Aminocarbonyl-, C₁₋₄-Alkylaminocarbonyl-, Di-(C₁₋₄-alkyl)aminocarbonyl-, C₁₋₃-Alkylsulfonylgruppe, durch eine im Ring gegebenenfalls C₁₋₃-alkylsubstituierte Cyclo-C₃₋₇-alkylcarbonyl-, Azacyclo-C₄₋₇-alkylcarbonyl-, Diazacyclo-C₅₋₇-alkylcarbonyl- oder Oxazacyclo-C₅₋₇-alkylcarbonylgruppe substituiert sein können, wobei die Substituenten gleich oder verschieden und an ein Ring-Kohlenstoff- oder Ring-Stickstoffatom gebunden sein können,
wobei die in den für R⁴ vorstehend definierten Resten enthaltenen Phenyl- und Pyridinyl-Reste ihrerseits durch Halogenatome, durch C₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkyl-amino-C₁₋₃-allkyl-, Di-(C₁₋₄-alkyl)-amino-C₁₋₃-alkyl-, C₁₋₄-Alkylcarbonyl-amino-, C₁₋₄-Alkylcarbonylamino-C₁₋₃-alkyl-, Aminocarbonyl-, C₁₋₃-Alkyl-aminocarbonyl-, Di-C₁₋₄-alkyl-aminocarbonyl-, Cyano-, Aminosulfonyl-, G₁₋₄-Alkyl-aminosulfonyl-, Di-(C₁₋₄-alkyl)-aminosulfonyl-, C₁₋₃-Alkylthio-, C₁₋₃-Alkyl-sulfinyl-, oder C₁₋₃-Alkyl-sulfonyl- mono-, di- oder trisubstituiert sein können, wobei die Substituenten gleich oder verschieden sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom,
einen C₁₋₄-Alkylrest, wobei ein unverzweigter Alkylrest in ω-Stellung durch eine Phenyl, Pyridinyl-, Diazinyl-, Amino-, C₁₋₄-Alkylamino-, Di-(C₁₋₄-alkyl)-amino-, 4 C₁₋₄-Alkyl-1-piperazinyl- oder 4-Morpholinylgruppe substituiert sein kann,
eine C₁₋₆-Alkoxycarbonyl-, Cyano- oder Aminocarbonylgruppe oder auch, wenn Y¹ ein Stickstoffatom darstellt, ein freies Elektronenpaar,
oder, wenn Y¹ das Kohlenstoffatom darstellt, auch das Fluoratom, oder
R⁴ zusammen mit R⁵ und Y¹ einen 4- bis 7-gliedrigen cycloaliphatischen Ring, in dem eine Methylengruppe durch eine Gruppe -NH-, -N(C₁₋₄-Alkyl)-, -N(C₃₋₄-Alkenyl)-, -N(C₃₋₄-Alkinyl)-, -N(Cyclo-C₃₋₇-alkyl)- oder -N(C₃₋₇-Cycloalkyl-C₁₋₃-alkyl)- ersetzt sein kann,
wobei ein an ein Stickstoffatom in einer der für R⁴ vorstehend definierten Gruppen gebundenes Wasserstoffatom durch einen Schutzrest ersetzt sein kann,
R⁶ und R⁷, die gleich oder verschieden sein können, jeweils ein Wasserstoffatom, eine C₁₋₄-Alkylgruppe oder auch, wenn Y¹ ein Kohlenstoffatom darstellt, das Fluoratom, eine C₁₋₄-Alkylamino- oder Di-(C₁₋₄-alkyl)-aminogruppe,
wobei die beiden C₁₋₄-Alkylgruppen unter Ausbildung eines Ringes miteinander verbunden sein können und
R⁸ und F⁹, die gleich oder verschieden sein können, jeweils ein Wasserstoffatom oder eine C₁₋₃-Alkylgruppe darstellen, bedeuten,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

3. CGRP-Antagonisten der allgemeinen Formel (I) gemäß Anspruch 1, in denen
A, X, D, E, G, M, Q, R² und R³ wie in Anspruch 1 oder 2 erwähnt definiert sind und
R¹ einen einfach oder zweifach ungesättigten 5- bis 7-gliedrigen Aza-, Diaza-, Triaza-oder Thiaza-Heterocyclus bedeutet,
wobei die vorstehend erwähnten Heterocyclen über ein Kohlenstoff- oder Stickstoffatom verknüpft sind oder
über ein Kohlenstoff- und ein Stickstoffatom, über ein Kohlenstoff- und ein Sauerstoffatom oder über ein Kohlenstoff- und ein Schwefelatom spirocyclisch verknüpft sind,
eine oder zwei Carbonylgruppen benachbart zu einem Stickstoffatom enthalten,
an einem Kohlenstoffatom durch eine Phenyl-, Pyridinyl-, Diazinyl-, Thienyl-, Pyrrolyl-, 1,3-Thiazolyl-, Isoxazolyl-, Pyrazolyl- oder 1-(C₁₋₄-Alkyl)-pyrazolylgruppe substituiert sein können und
wobei eine olefinische Doppelbindung eines der vorstehend erwähnten ungesättigten Heterocyclen mit einem Phenyl-, Naphthyl-, Pyridin-, Diazin-, Thienyl- oder Chinolin-Ring oder mit einem gegebenenfalls am Stickstoffatom durch eine Methylgruppe substituierten 1*H*-Chinolin-2-on-Ring kondensiert sein kann,
wobei die in R¹ enthaltenen Phenyl-, Pyridinyl-, Diazinyl-, Thienyl-, Pyrrolyl-, 1,3-Thiazolyl-, Isoxazolyl-, Pyrazolyl- oder 1-(C₁₋₄-Alkyl)-pyrazolylgruppen sowie die benzo-, pyrido- und diazinokondensierten Heterocyclen im Kohlenstoffgerüst zusätzlich durch Halogen, durch C₁₋₆-Alkyl-, Cyclo-C₃₋₇-alkyl-, Cyclo-C₃₋₇alkenyl-, C₁₋₆-Alkoxy-, Hydroxycarbonyl-, C₁₋₆-Alkoxycarbonyl-, Cyano-, Hydroxy-, Amino-, C₁₋₄-Alkylamino-, Di-C₁₋₄-alkylamino-, C₁₋₄-Alkylcarbonylamino- oder C₁₋₄-Alkylcarbonylgruppen mono-, di- oder trisubstituiert sein können, wobei die Substituenten gleich oder verschieden sein können, bedeuten,
wobei, soweit nichts anderes erwähnt wurde, alle in den vorstehend unter R¹ definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können, und
alle in den vorstehend unter R¹ definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

4. CGRP-Antagonisten der allgemeinen Formel (I) gemäß Anspruch 1, in denen
A, X, D, E, G, M, Q, R² und R³ wie in Anspruch 1 oder 2 erwähnt definiert sind und
R¹ einen einfach ungesättigten 5- bis 7-gliedrigen Diaza- oder Triaza-Helerocyclus bedeutet,
wobei die vorstehend erwähnten Heterocyclen über ein Stickstoffatom verknüpft sind oder
über ein Kohlenstoff- und ein Stickstoffatom oder über ein Kohlenstoff- und ein Sauerstoffatom spirocyclisch verknüpft sind,
eine Carbonylgruppe benachbart zu einem Stickstoffatom enthalten,
zusätzlich an einem Kohlenstoffatom durch eine Phenylgruppe substituiert sein können und
wobei eine olefinische Doppelbindung eines der vorstehend erwähnten ungesättigten Heterocyclen mit einem Phenyl-, Thienyl- oder Chinolin-Ring kondensiert sein kann,
wobei die in R¹ enthaltenen Phenylgruppen sowie benzokondensierten Heterocyclen im Kohlenstoffgerüst zusätzlich durch Halogen, durch Methyl-, Methoxy-, Difluormethyl-, Trifluormethyl-, Hydroxy-, Amino-, C₁₋₄-Alkylamlno-, Di-(C₁-₄-alkyl)-amino-, Acetylamino-, Acetyl-, Hydroxycarbonyl-, C₁₋₃-Alkoxycarbonyl-, Cyano- Difluormethoxy- oder Trifluormethoxygruppen mono-, di- oder trisubstituiert sein können, wobei die Substituenten gleich oder verschieden sein können, vorzugsweise jedoch unsubstituiert oder durch ein Halogenatom, durch eine Methyl- oder Methoxygruppe monosubstituiert sind, bedeuten,
wobei, soweit nichts anderes erwähnt wurde, alle in den vorstehend unter R¹ definierten Resten erwähnten oder enthaltenen Alkylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

5. CGRP-Antagonisten der allgemeine Formel (I) gemäß Anspruch 1, in denen
A, X, D, E, G, M, Q, R² und R³ wie in Anspruch 1 oder 2 erwähnt definiert sind und
R¹ eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-*c*]chinolin-2-on-3-yl-, 1,3-Dihydro-naphth[1,2-*d*]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H*-thieno-[3,2-*d*]pyrimidin-2-on-3-yl- oder 3,4-Dihydro-1*H*-thieno[3,4-*d*]pyrimidin-2-on-3-ylgruppe bedeutet,
wobei die vorstehend unter R¹ erwähnten Heterocyclen im Kohlenstoffgerüst zusätzlich durch eine Methoxygruppe monosubstituiert sein können,
wobei alle in den vorstehend unter R¹ definierten Resten erwähnten oder enthaltenen aromatischen und heteroaromatischen Reste zusätzlich durch Halogenatome, durch Cyano- oder Hydroxygruppen mono-, di- oder trisubstituiert und die Substituenten gleich oder verschieden sein können,
deren Tautomere, deren lsomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

6. CGRP-Antagonisten der allgemeinen Formel (I) gemäß Anspruch 1, in denen
A, X, R¹, R² und R³ wie in einem der Ansprüche 1 bis 5 erwähnt definiert sind und
(a) D, E unabhängig voneinander jeweils eine Methingruppe oder das Stickstoffatom und
G eine durch die Gruppe R^{a} substituierte Methingruppe,
M eine durch die Gruppe R^{b} substituierte Methingruppe,
Q eine durch die Gruppe R^{c} substituierte Methingruppe,
wobei eine oder zwei der Gruppen G, M und Q jeweils auch ein Stickstoffatom bedeuten kann,
oder
(b) D und E jeweils eine Methingruppe, wobei eine der Gruppen D und E auch ein Stickstoffatom bedeuten kann, und
G, M und Q jeweils ein Stickstoffatom bedeuten,
wobei R^{a}, R^{b} und R^{c} unabhängig voneinander jeweils ein Wasserstoff- oder - Halogenatom, eine C₁₋₄-Alkyl-, C₂₋₄-Alkonyl-, C₂₋₄-Alkinyl-, Cyclo-C₃₋₆-alkyl-, Cyclo-C₃₋₆-alkenyl-, Cyano-, Hydroxy-, Hydroxy-C₁₋₄-alkyl-, Hydroxy-C₃₋₄-alkenyl-, Hydroxy-C₃₋₄-alkinyl-, C₁₋₄-Alkoxy-, C₁₋₄-Alkoxy-C₁₋₄-alkyl-, C₁₋₄-Alkoxy-C₃₋₄-alkenyl-, C₁₋₄-Alkoxy-C₃₋₄-alkinyl-, Thiohydroxy-, C₁₋₄-Alkylthio-, Amino-, G₁₋₄-Alkyl-amino-, C₃₋₄-Alkonyl-amino-, G₃₋₄-Alkinyl-amino-, Di-(C₁₋₄-alkyl)-amino-, Di-(C₃₋₄-alkenyl)-amino-, Di-(C₃₋₄-alkinyl)-amino-, Amino-C₁₋₄-alkyl-, C₁₋₃-Alkyl-amino-C₁₋₄-alkyl-, Di-(C₁₋₃-alkyl)-amino-C₁₋₄-alkyl-, Amino-C₃₋₄-alkenyl-, C₁₋₃-Alkyl-amino-C₃₋₄-alkenyl-, Di-(C₁₋₃-alkyl)-amino-C₃₋₄-alkenyl-, Amino-C₃₋₄-alkinyl-, C₁₋₃-Alkyl-amino-C₃₋₄-alkinyl-, Di-(C₁₋₃-alkyl)-amino-C₃₋₄-alkinyl-, Hydroxycarbonyl-, Phenylcarbonyl-, Pyridylcarbonyl-, C₁₋₄-Alkyl-carbonyl-, Formyl-, C₁₋₄-Alkoxy-carbonyl-, C₃₋₄-Alkenoxy-carbonyl-, C₃₋₄-Alkinoxy-carbonyl-, Aminocarbonyl-, C₁₋₄-Alkyl-aminocarbonyl-, C₃₋₄-Alkenyl-aminocarbonyl-, C₃₋₄-Alkinyl-aminocarbonyl-, Di-(C₁₋₄-alkyl)-aminocarbonyl-, Di-(C₃₋₄-alkenyl)-aminocarbonyl-, Di-C₃₋₄-(alkinyl)-aminocarbonyl-, Formylamirio-, C₁₋₄-Alkyl-carbonylamino-, Formyl-C₁₋₄-alkyl-amino-, Formyl-C₃₋₄-alkenyl-amino-, Formyl-C₃₋₄-alkinyl-amino-, C₁₋₄-Alkyl-carbonyl-C₁₋₄-alkyl-amino-, C₁₋₄-Alkyl-carbonyl-C₃₋₄-alkenyl-amino-, C₁₋₄-Alkyl-carbonyl-C₃₋₄-alkinyl-amino-, C₁₋₄-Alkyl-sulfonyl-, C₂₋₄-Alkenyl-sulfonyl-, C₂₋₄-Alkinyl-sulfonyl-, C₁₋₄-Alkyl-sulfinyl-, C₂₋₄-Alkenyl-sulfinyl-C₂₋₄-Alkinyl-sulfinyl-, C₁₋₄-Alkyl-sulfonylamino-, C₁₋₄-Alkyl-sulfonyl-C₁₋₄-alkylamino-, C₁₋₄-Alkyl-sulfonyl-C₃₋₄-aikenylamino-, C₁₋₄-Alkyl-sulfonyl-C₃₋₄-alkinylamino-, Aminsulfonyl-, C₁₋₄-Alkylaminosulfonyl-, Di-(C₁₋₄-alkyl)-aminosulfonyl-, C₃₋₄-Alkenylamino-sulfonyl-, Di-(C₃₋₄-alkenyl)-aminosulfonyl-, C₃₋₄-Alkinylaminosulfonyl- oder Di-(C₃₋₄-alkinyl)-aminosulfonylgruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₄-Alkylgruppe darstellen,
(ii) R^{c} kein Wasserstoffatom bedeutet, wenn R^{a} sowie R^{b} jeweils eine C₁₋₄-Alkylgruppe darstellen,
(iii) R^{a} nicht die Bedeutungen eines Wasserstoff-, Fluor-, Chlor-, Brom- oder Iodatoms oder einer Difluor- oder Trifluormethylgruppe annimmt, wenn R^{c} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine C₁₋₄-Alkyl-, C₂₋₄-Alkenyl- oder C₂₋₄-Alkinylgruppe und R^{b} ein Chlor- oder Bromatom, eine Amino-, Methylamin- oder Hydroxygruppe darstellen,
wobei, soweit nichts anderes erwähnt wurde, alle in den vorstehend für R^{a}, R^{b} und R^{c} definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können,
die Doppel- und Dreifachbindungen der in den für R^{a}, R^{b} und R^{c} vorstehend definierten Gruppen enthaltenen C₃₋₄-Alkenyl- oder C₃₋₄-Alkinylgruppen von in diesen Gruppen gegebenenfalls ebenfalls enthaltenen Heteroatomen isoliert sind,
und alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

7. CGRP-Antagonisten der allgemeinen Formel (I) gemäß Anspruch 1, in denen
A, X, R¹, R² und R³ wie in einem der Ansprüche 1 bis 5 erwähnt definiert sind und
(a) D, E unabhängig voneinander jeweils eine Methingruppe oder das Stickstoffatom und
G eine durch die Gruppe R^{a} substituierte Methingruppe,
M eine durch die Gruppe R^{b} substituierte Methingruppe,
Q eine durch die Gruppe R^{c} substituierte Methingruppe,
wobei eine oder zwei der Gruppen G, Mund Q jeweils auch ein Stickstoffatom bedeuten kann,
oder
(b) D und E jeweils eine Methingruppe, wobei eine der Gruppen D und E auch ein Stickstoffatom bedeuten kann, und
G, M und Q jeweils ein Stickstoffatom bedeuten,
wobei R^{a}, R^{b} und R^{c} unabhängig voneinander jeweils ein Wasserstoff- oder Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₂-alkyl-, Hydroxy-C₃-alkenyl-, Hydroxy-C₃-alkinyl-, C₁₋₄-Alkoxy-, C₁₋₄-Alkoxy-C₁₋₂-alkyl-, Amino-, C₁₋₄-Alkyl-amino-, C₃₋₄-Alkenyl-amino-, C₃₋₄-Alkinyl-amino-, Di-(C₁₋₄-alkyl)-amino-, Di-(C₃₋₄-alkenyl)-amino-, Di-(C₃₋₄-alkinyl)-amino-, Amino-C₁₋₂-alkyl-, C₁₋₃-Alkyl-amino-G₁₋₂-alkyl-, Di-(C₁₋₃-alkyl)-amino-C₁₋₂-alkyl-, Amino-C₃-alkenyl-, C₁₋₃-Alkyl-amino-C₃-alkenyl-, Di-(C₁₋₃-alkyl)-amino-C₃-alkenyl-, Amino-C₃-alkinyl-, C₁₋₃-Alkyl-amino-C₃-alkinyl-, Di-(C₁₋₃-alkyl)-amino-C₃-alkinyl-, Hydroxycarbonyl-, C₁₋₄-Alkyl-carbonyl-, Formyl-, C₁₋₄-Alkoxy-carbonyl-, Aminocarbonyl-, C₁₋₄-Alkyl-aminocarbonyl-, Di-(C₁₋₄-alkyl)-aminocarbonyl-, Formylamino-, C₁₋₄-Alkyl-carbonylamino-, Formyl-C₁₋₄-alkyl-amino-, C₁₋₄-Alkyl-carbonyl-C₁₋₄-alkyl-ammo-, C₁₋₄-Alkyl-sulfonyl-, C₁₋₄-Alkyl-sulfinyl-, C₁₋₄-Alkyl-sulfonylamino-, C₁₋₄-Alkyl-sulfony-C₁₋₄-alkylamino-, Aminosulfonyl-, C₁₋₄-Alkylaminosulfonyl- oder Di-(C₁₋₄-alkyl)-aminosulfonylgruppe darstellen,
mit den Maßgaben, dass, sofern keine der Gruppen D, E, G, M und Q ein Stickstoffatom, darstellt,
(i) R^{a} kein Wasserstoffatom bedeutet, wenn R^{b} sowie R^{c} jeweils eine C₁₋₄-Alkylgruppe darstellen,
(ii) R^{c} kein Wasserstoffatom bedeutet, wenn R^{a} sowie R^{b} jeweils eine C₁₋₄-Alkylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine C₁₋₄-Alkyl-, C₂₋₄-Alkenyl- oder C₂₋₄-Alkinylgruppe und R^{b} ein Chlor- oder Bromatom, eine Amino-, Methylamin- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine C₁₋₄-Alkyl-, C₂₋₄-Alkenyl- oder C₂₋₄-Alkinylgruppe und R^{b} ein Chlor- oder Bromatom, eine Amino-, Methylamin- oder Hydroxygruppe darstellen,
wobei, soweit nichts anderes erwähnt wurde, alle in den vorstehend für R^{a}, R^{b} und R^{c} definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können, und
die Doppel- und Dreifachbindungen der in den für R^{a}, R^{b} und R^{c} vorstehend definierten Gruppen enthaltenen C₃₋₄-Alkenyl- oder C₃₋₄-Alkinylgruppen von in diesen Gruppen gegebenenfalls ebenfalls enthaltenen Heteroatomen isoliert sind,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

8. CGRP-Antagonisten der allgemeinen Formel (I) gemäß Anspruch 1, in denen
A, X, R¹, R² und R³ wie in einem der Ansprüche 1 bis 5 erwähnt definiert sind und
(a) D, E unabhängig voneinander jeweils eine Methingruppe oder das Stickstoffatom und
G eine durch die Gruppe R^{a} substituierte Methingruppe,
M eine durch die Gruppe R^{b} substituierte Methingruppe,
Q eine durch die Gruppe R^{c} substituierte Methingruppe,
wobei eine oder zwei der Gruppen G, M und Q jeweils auch ein Stickstoffatom bedeuten kann,
oder
(b) D und E jeweils eine Methingruppe, wobei eine der Gruppen D und E auch ein Stickstoffatom bedeuten kann, und
G, M und Q jeweils ein Stickstoffatom bedeuten,
wobei R^{a}, R^{b} und R^{c} unabhängig voneinander jeweils ein Wasserstoff- oder Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₂-alkyl-, C₁₋₄-Alkoxy-, Amino-, G₁₋₄-Alkyl-amino-, Di-(C₁₋₄-alkyl)-amino-, Amino-C₁₋₂-alkyl-, C₁₋₃-Alkyl-amino-C₁₋₂-alkyl-, Di-(C₁₋₃-alkyl)-amlno-C₁₋₂-alkyl-, Hydroxycarbonyl-, C₁₋₄-Alkyl-carbonyl-, Formyl-, C₁₋₄-Alkoxy-carbonyl-, Aminocarbonyl-, C₁₋₄-Alkyl-aminocarbonyl-, Di-(C₁₋₄-alkyl)-aminocarbonyl-, Formylamino-, C₁₋₄-Alkyl-carbonylamino-, Formyl-C₁₋₄-alkyl-amino-oder C₁₋₄-Alkyl-carbonyl-C₁₋₄-alkyl-aminogruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₄-Alkylgruppe darstellen,
(ii) R^{c} kein Wasserstoffatom bedeutet, wenn R^{a} sowie R^{b} jeweils eine C₁₋₄-Alkylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine C₁₋₄-Alkyl-, C₂₋₄-Alkenyl- oder C₂₋₄-Alkinylgruppe und R^{b} ein Chlor- oder Bromatom, eine Amino-, Methylamin- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine C₁₋₄-Alkyl-, C₂₋₄-Alkenyl- oder C₂₋₄-Alkinylgruppe und F^{b} ein Chlor- oder Bromatom, eine Amino-, Methylamin- oder Hydroxygruppe darstellen,
wobei, soweit nichts anderes erwähnt wurde, alle in den vorstehend für R^{a}, F^{b} und R^{c} definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom, gebundene Alkyl- und Alkenylgruppen unter Ausbildung eines 4- bis 7-gliedrigen, gesättigten oder ungesättigten heterocyclischen Ringes miteinander verbunden sein können,
deren Tautomere, deren lsomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

9. CGRP-Antagonisten der allgemeinen Formel (I) gemäß Anspruch 1, in denen
A, X, R¹, R² und R³ wie in einem der Ansprüche 1 bis 5 erwähnt definiert sind und
(a) D, E unabhängig voneinander jeweils eine Methingruppe oder das Stickstoffatom und
G eine durch die Gruppe R^{a} substituierte Methingruppe,
M eine durch die Gruppe R^{b} substituierte Methingruppe,
Q eine durch die Gruppe R^{c} substituierte Methingruppe,
wobei eine oder zwei der Gruppen G, M und Q jeweils auch ein Stickstoffatom bedeuten kann,
oder
(b) D und E jeweils eine Methingruppe, wobei eine der Gruppen D und E auch ein Stickstoffatom bedeuten kann, und
G, M und Q jeweils ein Stickstoffatom bedeuten,
wobei R^{a}, R^{b} und R^{c} unabhängig voneinander jeweils ein Wasserstoff- oder Halogenatom, eine Methyl-, Difluormethyl-, Trifluormethyl-, Ethyl-, Vinyl-, Ethinyl-, Cyano-, Hydroxy-, Methoxy-, Difluormethoxy-, Trifluormethoxy-, Amin-, Methylamino- oder Dimethylaminogruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine Methyl-_ oder Ethylgruppe darstellen,
(ii) R^{c} kein Wasserstoffatom bedeutet, wenn R^{a} sowie R^{b} jeweils eine Methyl-_ oder Ethylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine Methyl-, Ethyl-, Vinyl- oder Ethinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder Iodatoms oder einer Difluor- oder Trifluormethylgruppe annimmt, wenn R^{a} eine Methyl-, Ethyl-, Vinyl- oder Ethinylgruppe und R^{b} ein Chlor- oder Bromatom, eine Amino-, Methylamino- oder Hydroxygruppe darstellen,
deren Tautomere, deren lsomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

10. CGRP-Antagonisten der allgemeinen Formel (I) gemäß Anspruch 1, in denen
A, X, D, E, G, M, Q und R¹ wie in einem der Ansprüche 1 bis 9 erwähnt definiert sind und
R² das Wasserstoffatom oder
eine Phenylmethylgruppe oder eine C₂₋₇-Alkylgruppe, die in ω-Stellung durch eine Cyclo-C₃-₇-alkyl-, Cyclo-C₃₋₇-alkenyl-, Phenyl-, Pyridinyl-, Hydroxy-, Amino-, C₁₋₆-Akkylamino-, Di-(C₁₋₆-alkyl)-amino-, Hydroxycarbonyl-, C₁₋₆-Alkoxycarbonyl-, Aminocarbonyl-, Aminocarbonylamino-, C₁₋₆-Alkylamino-, 4-Morpholinylgruppe substituiert sein kann,
wobei die in den für R² vorstehend definierten Gruppen genannten oder als Substituenten enthaltenen Phenyl- und Pyridinylreste zusätzlich im Kohlenstoffgerüst durch Halogen, durch C₁₋₃-Alkyl-, C₁₋₃-Alkoxy-, Hydroxy-, Amino-, C₁₋₃-Alkylamino-, Di-(C₁₋₃-alkyl)-amino-, Amino-C₁₋₃-alkyl-, C₁₋₃-Alkyl-amino-C₁₋₃-alkyl-, Di-(C₁₋₃-alkyl)-amino-C₁₋₃-alkyl-, C₁₋₃-Alkylcarbonylamino-, C₁₋₃-Alkylcarbonyl-C₁₋₃-alkylamino-, Aminocarbonyl-, C₁₋₃-Alkylaminocarbonyl-oder Di-(C₁₋₃-alkyl)-aminocarbonylgruppen mono-, di- oder trisubstituiert und die Substituenten gleich oder verschieden sein können,
R³ das Wasserstoffatom oder eine C₁₋₃-Alkylgruppe darstellen oder
R² und R³ zusammen mit dem eingeschlossenen Stickstoffatom einen Rest der allgemeinen Formel 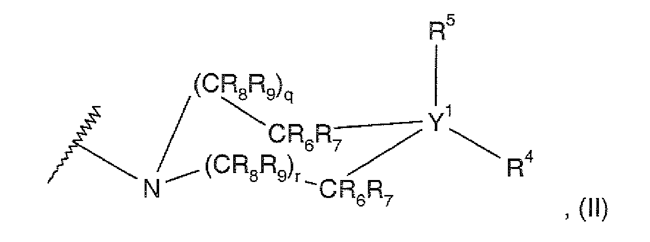 in der
Y¹ das Kohlenstoffatom oder, wenn R⁵ ein freies Elektrodenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl- oder Cyclo-C₃₋₇-alkenylgruppe, eine Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₄-Alkyl-aminocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl-, Di-(C₁₋₄-alkyl)-aminocarbonyl-C₁₋₃-alkyl-, Aminocarbonylamino-C₁₋₃-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl-, Pyridinyl- oder Diazinylgruppe, die jeweils durch ein Halogen, durch eine C₁₋₃-Alkyl-, C₁₋₃-Alkoxy-, Hydroxy-, Amino-, C₁₋₄-Alkyl-amino-, Di-(C₁₋₄-alkyl)-amino- Amino-C₁₋₃-alkyl-, C₁₋₄-Alkyl-amino-C₁₋₃-alkyl-, Di-(C₁₋₄-alkyl)-amino-C₁₋₃-alkylgruppe substituiert sein können,
ein Heterocyclus ausgewählt aus einer 4- bis 7-gliedrigen Azacycloalkylgruppe, einer 6- bis 7-gliedrigen Oxaza-, *S*,*S*-Dioxothiaza- und Diazacycloalkylgruppe und einer 7- bis 9-gliedrigen Azabicycloalkylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methylengruppe durch ein oder zwei Fluoratome substituiert sein kann und die vorstehend genannten mono- und bicyclische Heterocyclen ein- oder zweifach durch Hydroxy-, C₁₋₃-Alkyl- oder Hydroxy-C₁₋₃-alkylgruppen oder einfach durch eine Benzyl-, Cyclo-C₃₋₆-alkyl-, Hydroxycyclo-C₃₋₆-alkyl-, Cyclo-C₃₋₆-alkyl-C₁₋₃-alkyl, C₁₋₄-Alkylcarbonyl-, C₁₋₄-Alkylcarbonyl-C₁₋₃-alkyl-, Hydroxy-, C₁₋₄-Alkoxy-, Amino-, C₁₋₄-Alkylamino-, Di-(C₁₋₄-alkyl)-amino-, C₁₋₃-Alkoxycarbonyl-, Hydroxycarbonyl-carbonyl-, C₁₋₃-Alkoxycarbonyl-carbonyl-, Hydroxycarbonyl-C₁₋₃-alkyl-, C₁₋₃-Alkoxycarbonyl-C₁₋₃-alkyl-, Hydroxycarbonyl-C₁₋₃-alkylcarbonyl-, C₁₋₃-Alkoxycarbonyl-C₁₋₃-alkylcarbonyl-, Aminosulfonyl-, C₁₋₄-Alkylaminosulfonyl-, Di-(C₁₋₄-alkyl)-aminosulfonyl-, C₁₋₃-Alkylsulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)-aminocarbonyl-C₁₋₃-alkyl-, Hydroxyaminocarbonyl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppen substituiert sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom, einen C₁₋₃-Alkylrest oder, wenn Y¹ ein Stickstoffatom darstellt, auch ein freies Elektronenpaar,
R⁶ und R⁷, die gleich oder verschieden sein können, jeweils ein Wasserstoffatom, eine C₁₋₃-Alkylgruppe oder auch, wenn Y¹ ein Kohlenstoffatom darstellt, eine Amino-, C₁₋₃-Alkylamino- oder Di-(C₁₋₃-alkyl)-aminogruppe, wobei die beiden C₁₋₃-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
bedeuten,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

11. CGRP-Antagonisten der allgemeinen Formel (I) gemäß Anspruch 1, in denen
A, X, D, E, G, M, Q und R¹ wie in einem der Ansprüche 1 bis 9 erwähnt definiert sind und
R² das Wasserstoffatom oder
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Hydroxy-, Amino-, C₁₋₆-Alkylamino-, Di-(C₁₋₆-alkyl)-amino-, Hydroxycarbonyl-, C₁₋₆-Alkoxycarbonyl-, Aminocarbonyl-, Aminocarbonylamino-, C₁₋₆-Alkylamino-, 4-Morpholinylgruppe substituiert sein kann,
wobei die in den für R² vorstehend definierten Gruppen genannten oder als Substituenten enthaltenen Phenyl- und Pyridinylreste zusätzlich im Kohlenstoffgerüst durch Halogen, durch C₁₋₃-Alkyl-, C₁₋₃-Alkoxy-, Hydroxy-, Amino-, C₁₋₃-Alkylamino-, Di-(C₁₋₃-alkyl)-amino-, Amino-C₁₋₃-alkyl-, C₁₋₃-Alkyl-amino-C₁₋₃-alkyl-, Di-(C₁₋₃-alkyl)-amino-C₁₋₃-alkyl-, C₁₋₃-Alkylcarbonylamino-, C₁₋₃-Alkylcarbonyl-C₁₋₃-alkylamino-, Aminocarbonyl-, C₁₋₃-Alkylaminocarbonyl-oder Di-(C₁₋₃-alkyl)-aminocarbonylgruppen mono-, di- oder trisubstituiert und die Substituenten gleich oder verschieden sein können,
R³ das Wasserstoffatom oder eine C₁₋₃-Alkylgruppe darstellen oder
R² und R³ zusammen mit dem eingeschlossenen Stickstoffatom einen Rest der allgemeinen Formel 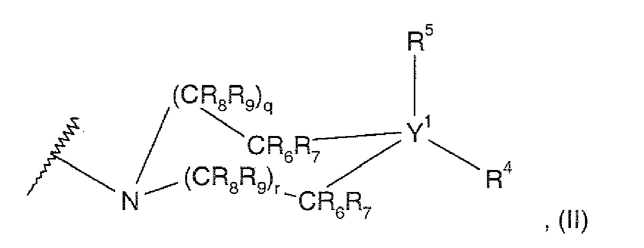 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, Cyclo-C₃₋₇-alkyl-, Cyclo-C₃₋₇-alkenyl-, Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₄-Alkyl-aminocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl-, Di-(C₁₋₄-alkyl)-aminocarbonyl-C₁₋₃-alkyl-, Aminocarbonylamino-C₁₋₃-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl-, Pyridinyl- oder Diazinylgruppe, die jeweils durch ein Halogen, durch eine C₁₋₃-Alkyl-, C₁₋₃-Alkoxy-, Hydroxy-, Amino-, C₁₋₄-Alkyl-amino-, Di-(C₁₋₄-alkyl)-amino- Amino-C₁₋₃-alkyl-, C₁₋₄-Alkyl-amino-C₁₋₃-alkyl-, Di-(C₁₋₄-alkyl)-amino-C₁₋₃-alkylgruppe substituiert sein können,
ein Heterocyclus ausgewählt aus einer 4- bis 7-gliedrigen Azacycloalkylgruppe, einer 6- bis 7-gliedrigen Oxaza- und Diazacycloalkylgruppe und einer 7- bis 9-gliedrigen Azabicycloalkylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methylengruppe durch ein oder zwei Fluoratome substituiert sein kann und
die vorstehend genannten mono- und bicyclischen Heterocyclen ein- oder mehrfach, beispielsweise ein- bis dreifach, durch C₁₋₃-Alkylgruppen oder einfach durch eine Benzyl-, Cyclo-C₃₋₆-alkyl-, Cyclo-C₃₋₆-alkyl-C₁₋₃-alkyl, C₁₋₄-Alkylcarbonyl-, Hydroxy-, C₁₋₄-Alkoxy-, Amino-, C₁₋₄-Alkylamino-, Di-(C₁₋₄-alkyl)-amino-, Hydroxycarbonyl-, C₁₋₃-Alkoxycarbonyl-, Hydroxycarbonyl-carbonyl-, C₁₋₃-Alkoxycarbonyl-carbonyl-, Hydroxycarbonyl-C₁₋₃-alkyl-, C₁₋₃-Alkoxycarbonyl-C₁₋₃-alkyl- oder C₁₋₃-Alkylsulfonylgruppen substituiert sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom, einen C₁₋₃-Alkylrest oder, wenn Y¹ ein Stickstoffatom darstellt, auch ein freies Elektronenpaar,
R⁶ und R⁷, die gleich oder verschieden sein können, jeweils ein Wasserstoffatom, eine C₁₋₃-Alkylgruppe oder auch, wenn Y¹ ein Kohlenstoffatom darstellt, eine C₁₋₃-Alkylamino- oder Di-(C₁₋₃-alkyl)-aminogruppe, wobei die beiden C₁₋₃-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
bedeuten,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

12. CGRP-Antagonisten der allgemeinen Formel (I) gemäß Anspruch 1, in denen
A, X, D, E, G, M, Q und R¹ wie in einem der Ansprüche 1 bis 9 erwähnt definiert sind und
R² das Wasserstoffatom oder
eine Phenylmethylgruppe oder eine C₂₋₇-Alkylgruppe, die in ω-Stellung durch eine Phenyl-, Amino-, C₁₋₆-Alkylamino-, Di-(C₁₋₆-alkyl)-aminogruppe substituiert sein kann,
wobei die vorstehend erwähnte Phenyl- und Phenylmethylgruppe zusätzlich an einem aromatischen Kohlenstoffatom durch Halogen, durch C₁₋₃-Alkyl-, C₁₋₃-Alkoxy-, Amino-C₁₋₃-alkyl-, C₁₋₃-Alkylamino-C₁₋₃-alkyl- oder Di-(C₁₋₃-alkyl)-amino-C₁₋₃-alkylgruppen mono- oder disubstituiert und die Substituenten gleich oder verschieden sein können,
R³ das Wasserstoffatom oder eine C₁₋₃-Alkylgruppe darstellen oder
R² und R³ zusammen mit dem eingeschlossenen Stickstoffatom einen Rest der allgemeinen Formel 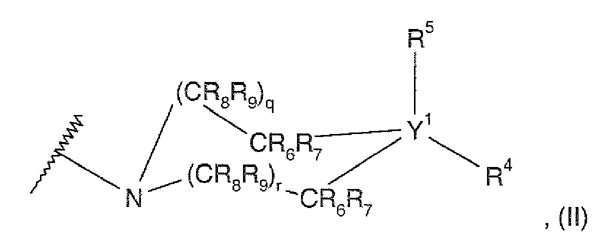 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl- oder Cyclo-C₃₋₇₋alkenylgruppe, eine Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl- oder Pyridylgruppe, die jeweils durch ein Halogen, durch eine C₁₋₃-Alkyl-, C₁₋₃-Alkoxy-, Amino-, C₁₋₄-Alkyl-amino-, Di-(C₁₋₄-alkyl)-aminogruppe substituiert sein kann,
ein Heterocyclus ausgewählt aus einer 6- bis 7-gliedrige Azacycloalkylgruppe, einer 6- bis 7-gliedrige *S*,*S*-Dioxothiaza- und Diazacycloalkylgruppe und einer 7-bis 9-gliedrige Azabicycloalkylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methylengruppe durch ein oder zwei Fluoratome substituiert sein kann und
die vorstehend genannten mono- und bicyclischen Heterocyclen ein- oder zweifach durch eine Hydroxy-, C₁₋₃-Alkyl- oder Hydroxy-C₁₋₃-alkylgruppe, durch eine Benzyl-, Cyclo-C₃₋₆-alkyl-, Hydroxy-C₃₋₆-cycloalkyl-, Cyclo-C₃₋₆-alkyl-C₁₋₃-alkyl, C₁₋₃-Alkylcarbonyl-C₁₋₃-alkyl-, Amino-, C₁₋₄-Alkylamino-oder Di-(C₁₋₄-alkyl)-amino-, Hydroxycarbonyl-carbonyl-, C₁₋₆-Alkoxycarbonyl-carbonyl-, Hydroxycarbonyl-C₁₋₃-alkylcarbonyl-, C₁₋₃-Alkoxycarbonyl-C₁₋₃-alkylcarbonyl-, Aminosulfonyl-, C₁₋₄-Alkylaminosulfonyl-, Di-(C₁₋₄-alkyl)-aminosulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)-aminocarbonyl-C₁₋₃-alkyl-, Hydroxyaminocarbonyl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppen substituiert sein können,
oder auch, wenn Y¹ das Kohlenstoffatom darstellt die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom oder, wenn Y¹ ein Stickstoffatom darstellt, auch ein freies Elektrodenpaar,
R⁶ und R⁷, die gleich oder verschieden sein können, jeweils ein Wasserstoffatom, eine C₁₋₃-Alkylgruppe oder auch, wenn Y¹ ein Kohlenstoffatom darstellt, eine C₁₋₃-Alkylamino- oder Di-(C₁₋₃-alkyl)-aminogruppe, wobei die beiden C₁₋₃-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
bedeuten, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

13. CGRP-Antagonisten der allgemeinen Formel (I) gemäß Anspruch 1, in denen
A, X, D, E, G, M, Q und R¹ wie in einem der Ansprüche 1 bis 9 erwähnt definiert sind und
R² eine Phenylmethylgruppe oder eine C₂₋₇-Alkylgruppe, die in ω-Stellung durch eine Phenyl-, Amino-, C₁₋₆-Alkylamino-, Di-(C₁₋₆-alkyl)-aminogruppe substituiert sein kann,
wobei die vorstehend genannte Phenyl- und Phenylmethylgruppe an einem aromatischen Kohlenstoffatom durch eine Amino-C₁₋₃-alkyl-, C₁₋₃-Alkylamino-C₁₋₃-alkyl- oder Di-(C₁₋₃-alkyl)-amino-C₁₋₃-alkylgruppe substituiert sein kann,
R³ das Wasserstoffatom oder eine C₁₋₃-Alkylgruppe oder
R² und R³ zusammen mit dem eingeschlossenen Stickstoffatom einen Rest der allgemeinen Formel 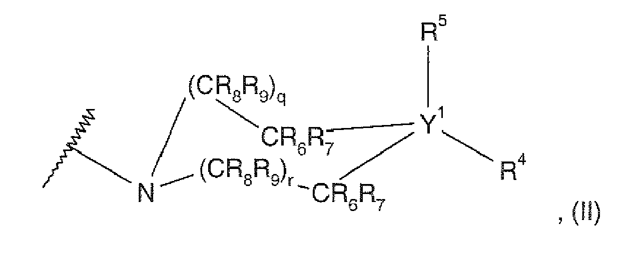 in der
R⁶ und R⁷ jeweils ein Wasserstoffatom oder eine Dimethylaminogruppe,
R⁸ und R⁹ jeweils das Wasserstoffatom und
(a) Y¹ das Kohlenstoffatom,
q und r die Zahlen 0 oder 1,
R⁴ das Wasserstoffatom,
eine Phenyl-, Pyridinyl- oder Pyrimidinylgruppe, die jeweils durch ein Halogen, durch eine Amin-, Methylamino-, Dimethylamino-, Methyl-oder Methoxygruppe substituiert sein kann,
eine Hydroxy-, 2-Diethylamino-ethyl-, Amino-, Methylamino-, Dimethylamino-, Diethylamino-, Pyrrolidin-1-yl-, 3-Hydroxy-pyrrolidin-1-yl-, 2-Hydroxycarbonyl-pyrrolidin-1-yl-, 2-Methoxycarbonyl-pyrrolidin-1-yl-, Piperidin-1-yl-, 4,4-Dimethylpiperidin-1-yl-, 4-Amino-4-mothyl-piperidin-1-yl-, 2-Hydroxycarbonyl-piperidin-1-yl-, 2-Methoxycarbonyl-piperidin-1-yl-, 4-Hydroxymethyl-piperidin-1-yl-, 4-(1-Hydroxycyclopropyl)-piperidin-1-yl-, 4-Amino-piperidin-1-yl-, 4-Methylamino-piperidin-1-yl-, 4-Dimethylamino-piperidin-1-yl-, 4-Hydroxy-4-methyl-piperidin-1-yl-, 4-Hydroxy-4-ethyl-piperidin-1-yl-, 4-Hydroxy-4-trifluormethyl-piperidin-1-yl-, 4-Hydroxy-4-hydroxymethyl-piperidin-1-yl-, 3-Amino-piperidin-1-yl-, 3-Methylamino-piperidin-1-yl-, 3-Dimethylamino-piperidin-1-yl-, 3-Hydroxy-piperidin-1-yl-, 4-Hydroxypiperidin-1-yl-, 4-Hydroxycarbonylmethyl-piperidin-1-yl-, 4-Ethoxycarbonylmethyl-piperidin-1-yl-, Perhydro-azepin-1-yl-, Perhydro-1,4-diazepin-1-yl-, 4-Methyl-perhydro-1,4-diazepin-1-yl-, 1-Methyl-piperidin-4-yl-, Piperidin-4-yl-, 1-Ethylpiperidin-4-yl-, 1-(2-Hydroxyethyl)-piperidin-4-yl-, 1-Cyclopropyl-piperidin-4-yl-, 1-Cyclopropylmethyl-piperidin-4-yl-, 1-Methylsulfonyl-piperidin-4-yl-, 1-Ethylsulfonyl-piperidin-4-yl-, 1-Isopropylsulfonyl-piperidin-4-yl-, 1-Cyclopropylsulfonyl-piperidin-4-yl-, 4-Hydroxy-1-methylsulfonyl-piperidin-4-yl-, 1-Aminosulfonyl-piperidin-4-yl-, 1-(Methylaminosulfonyl)-piperidin-4-yl-, 1-(Dimethylaminosulfonyl)-piperidin-4-yl-, 1-Hydroxycarbonylmethyl-piperidin-4-yl-, 1-Ethoxycarbonylmethyl-piperidin-4-yl-, 1-(2-Hydroxycarbonylethyl)-piperidin-4-yl-, 1-(2-Ethoxycarbonylethyl)-piperidin-4-yl-, 1-(3-Hydroxycarbonyl-propionyl)-piperidin-4-yl-, 1-(3-Ethoxycarbonyl-propionyl)-piperidin-4-yl-, 1-(Hydroxycarbamoyl-methyl)-piperidin-4-yl-, 1-(Hydroxy-methylcarbamoyl-methyl)-piperidin-4-yl-, 1-(Methoxycarbamoyl-methyl)-piperidin-4-yl-, 1-Oxalyl-piperidin-4-yl-, 1-Ethoxyoxalyl-piperidin-4-yl-, Piperazin-1-yl-, 4-Methyl-piperazin-1-yl-, 4-Cyclopropylmethyl-piperazin-1-yl-, 4-Ethyl-piperazin-1-yl-, 4-(2-Hydroxyethyl)-piperazin-1-yl-, 4-Cyclopropyl-piperazin-1-yl-, 4-Methylsulfonyl-piperazin-1-yl-, 4-Aminosulfonyl-piperazin-1-yl-, 4-(Methylaminosulfonyl)-piperazin-1-yl-, 4-(Dimethylaminosulfonyl)-piperazin-1-yl-, 4-Hydroxycarbonylmethyl-piperazin-1-yl-, 4-Ethoxycarbonylmethyl-piperazin-1-yl-, 4-(2-Hydroxycarbonylethyl)-piperazin-1-yl-, 4-(2-Ethoxycarbonylethyl)-piperazin-1-yl-, 4-(3-Hydroxycarbonyl-propionyl)-piperazin-1-yl-, 4-(3-Ethoxycarbonyl-propionyl)-piperazin-1-yl-, 4-(Hydroxycarbamoyl)-methyl-piperazin-1-yl-, 4-(Hydroxy-methyl-carbamoyl)-methyl-piperazin-1-yl-, 4-(Methoxycarbamoyl)-methyl-piperazin-1-yl-, 1,2-Dimethyl-piperazin-1-yl-, 3-Methyl-piperazin-1-yl-, 3,4,5-Trimethyl-piperazin-1-yl-, 3,5-Dimethyl-piperazin-1-yl-, 3,3,4-Trimethyl-piperazin-1-yl-, 3,3-Dimethyl-piperazin-1-yl-, 3,3,4,5,5-Pentamethyl-piperazin-1-yl-, 3,3,5,5-Tetramethyl-piperazin-1-yl-, 3,3,3-Trifluor-2-oxo-propyl)-piperazin-1-yl-, Morpholin-4-yl-, 1,1-Dioxo-1λ⁶-thiomorpholin-4-yl-, Tetrahydropyran-4-yl-, 4,4-Difluor-piperidin-1-yl-, 8-Methyl-8-aza-bicyclo[3.2.1]oct-3-yl-, 8-Aza-bicyclo[3.2.1]oct-3-yl-, Azetidin-1-yl-, 1-(Methoxycarbonylmethyl)-piperidin-4-yl-, 1-(Ethoxycarbonylmethyl)-piperidin-4-yl-, 4-(Ethoxycarbonylmethyl)-piperazin-1-yl-, 1-Hydroxycarbonylmethyl-piperidin-4-yl- oder 4-Hydroxycarbonylmethyl-piperazin-1-ylgruppe, und
R⁵ ein Wasserstoffatom, oder
(b) Y¹ ein Stickstoffatom,
q und r die Zahlen 1 oder 2,
R⁴ das Wasserstoffatom,
eine Phenyl-, Pyridinyl- oder Pyrimidinylgruppe, die jeweils durch ein Halogen, durch eine Amino-, Methylamino-, Dimethylamino-, Methyl-oder Methoxygruppe substituiert sein kann,
eine Methyl-, Ethyl-, Isopropyl-, Cyclopropyl-, Cyclopentyl-, Cyclohexyl-, Cycloheptyl-, 4-Hydroxycarbonylmethyl-cylohexyl-, 4-Ethoxycarbonylmethyl-cyclohexyl-, Cyclopropylmethyl-, 2-Diethylamino-propyl-, 1-Chinuclidin-3-yl-, Tetrahydropyran-4-yl-, 1-Piperidin-4-yl-, 1-Methyl-piperidin-4-yl-, 1-Ethyl-piperidin-4-yl-, 1-(2-Hydroxyethyl)-piperidin-4-yl-, 1-Methylsulfonyl-piperidin-4-yl-, 1-Aminosulfonyl-piperidin-4-yl-, 1-(Methylaminosulfonyl)-piperidin-4-yl-, 1-(Dimethylaminosulfonyl)-piperidin-4-yl-, 1-Hydroxycarbonylmethyl-piperidin-4-yl-, 1-Ethoxycarbonylmethyl-piperidin-4-yl-, 1-(2-Hydroxycarbonylethyl)-piperidin-4-yl-, 1-(2-Ethoxycarbonylethyl)-piperidin-4-yl-, 1-(3-Hydroxycarbonyl-propionyl)-piperidin-4-yl-, 1-(3-Ethoxycarbonyl-propionyl)-piperidin-4-yl-, 1-(Hydroxycarbamoyl-methyl)-piperidin-4-yl-, 1 -(Hydroxy-methyl-carbamoyl-methyl)-piperidin-4-yl-, 1-(Methoxycarbamoyl-methyl)-piperidin-4-yl-, 1-Cyclopropyl-piperidin-4-yl-, 1-Cyclopropylmethyl-piperidin-4-yl-, 1-Hydroxycarbonylmethyl-piperidin-4-yl- oder 1-Ethoxycarbonylmethyl-piperidin-4-ylgruppe und
R⁵ ein freies Elektronenpaar darstellen,
bedeuten, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

14. CGRP-Antagonisten der allgemeinen Formel (I) gemäß Anspruch 1, in denen
A, X, D, E, G, M, Q und R¹ wie in einem der Ansprüche 1 bis 9 erwähnt definiert sind und
R² eine Phenylmethylgruppe oder eine C₂₋₇-Alkylgruppe, die in ω-Stellung durch eine Phenyl-, Amino-, C₁₋₆-Alkylamino-, Di-(C₁₋₆-alkyl)-aminogruppe substituiert sein kann,
wobei die vorstehend genannte Phenyl- und Phenylmethylgruppe an einem aromatischen Kohlenstoffatom durch eine Amino-C₁₋₃-alkyl-, C₁₋₃-Alkylamino-C₁₋₃-alkyl- oder Di-(C₁₋₃-alkyl)-amino-C₁₋₃-alkylgruppe substituiert sein kann,
R³ das Wasserstoffatom oder eine C₁₋₃-Alkylgruppe oder
R² und R³ zusammen mit dem eingeschlossenen Stickstoffatom einen Rest der allgemeinen Formel 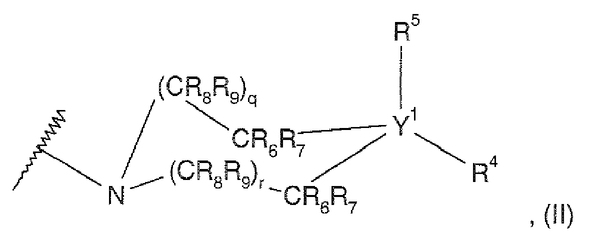 in der
R⁶ und R⁷ jeweils ein Wasserstoffatom oder eine Dimethylaminogruppe,
R⁸ und R⁹ jeweils das Wasserstoffatom und
(a) Y¹ das Kohlenstoffatom,
q und r die Zahlen 0 oder 1,
R⁴ das Wasserstoffatom,
eine Phenyl-, Pyridinyl- oder Pyrimidinylgruppe, die jeweils durch ein Halogen, durch eine Amino-, Methylamino-, Dimethylamino-, Methyl-oder Methoxygruppe substituiert sein kann,
eine Hydroxy-, 2-Diethylamino-ethyl-, Amino-, Methylamino-, Dimethylamino-, Diethylamino-, Pyrrolidin-1-yl-, Piperidin-1-yl-, 4-Amino-piperidin-1-yl-, 4-Methylamino-piperidin-1-yl-, 4-Dimethylamino-piperidin-1-yl-, 3-Amino-piperidin-1-yl-, 3-Methylamino-piperidin-1-yl-, 3-Dimethylamino-piperidin-1-yl-, Perhydro-azepin-1-yl-, Perhydro-1,4-diazepin-1-yl-, 4-Methyl-perhydro-1,4-diazepin-1-yl-, 1-Methyl-piperidin-4-yl-, Piperidin-4-yl-, 1-Ethylpiperidin-4-yl-, 1-Cyclopropyl-piperidin-4-yl-, 1-Cyclopropylmethyl-piperidin-4-yl-, Piperazin-1-yl-, 4-Methyl-piperazin-1-yl-, 4-Cyclopropylmethyl-piperazin-1-yl-, 4-Ethyl-piperazin-1-yl-, 4-Cyclopropyl-piperazin-1-yl-, 1,2-Dimethyl-piperazin-1-yl-, 3-Methylpiperazin-1-yl-, 3,4,5-Trimethyl-piperazin-1-yl-, 3,5-Dimethyl-piperazin-1-yl-, 3,3,4-Trimethyl-piperazin-1-yl-, 3,3-Dimethyl-piperazin-1-yl-, 3,3,4,5,5-Pentamethyl-piperazin-1-yl-, 3,3,5,5-Tetramethyl-piperazin-1-yl-, Morpholin-4-yl-, 4,4-Difluor-piperidin-1-yl-, 8-Methyl-8-aza-bicyclo[3.2.1]oct-3-yl-, 8-Aza-bicyclo[3.2.1]oct-3-yl-, Azetidin-1-yl-, 1-(Methoxycarbonylmethyl)-piperidin-4-yl-, 1-(Ethoxycarbonylmethyl)-piperidin-4-yl-, 4-(Ethoxycarbonylmethyl)-piperazin-1-yl-, 1-Hydroxycarbonylmethyl-piperidin-4-yl- oder 4-Hydroxycarbonylmethyl-piperazin-1-ylgruppe, und
R⁵ ein Wasserstoffatom, oder
(b) Y¹ ein Stickstoffatom,
q und r die Zahlen 1 oder 2,
R⁴ das Wasserstoffatom,
eine Phenyl-, Pyridinyl- oder Pyrimidinylgruppe, die jeweils durch ein Halogen, durch eine Amino-, Methylamino-, Dimethylamino-, Methyl-oder Methoxygruppe substituiert sein kann,
eine Methyl-, Ethyl-, Isopropyl-, Cyclopropyl-, Cyclopropylmethyl-, 2-Diethylamino-propyl-, 1-Chinuclidin-3-yl-, 1-Piperidin-4-yl-, 1-Methylpiperidin-4-yl-, 1-Ethyl-piperidin-4-yl-, 1-Cyclopropyl-piperidin-4-yl-, 1-Cyclopropylmethyl-piperidin-4-yl-, 1 -Hydroxycarbonylmethyl-piperidin-4-yl- oder 1-Ethoxycarbonylmethyl-piperidin-4-ylgruppe und
R⁵ ein freies Elektronenpaar darstellen,
bedeuten, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

15. CGRP-Antagonisten der allgemeinen Formel (I) gemäß Anspruch 1, in denen
D, E, G, M, Q, R¹, R² und R³ wie in einem der Ansprüche 1 bis 13 erwähnt definiert sind und
A und X jeweils ein Sauerstoffatom darstellen,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

16. CGRP-Antagonisten der allgemeinen Formel (I) gemäß Anspruch 1, in denen
A und X jeweils ein Sauerstoffatom darstellen,
R¹ eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-*c*]-chinolin-2-on-3-yl-, 1,3-Dihydro-naphth[1,2-*d*]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H*-thieno-[3,2-*d*]pyrimidin-2-on-3-yl- oder 3,4-Dihydro-1*H*-thieno[3,4-*d*]pyrimidin-2-on-3-ylgruppe bedeutet,
und R² und R³ wie in Anspruch 1 oder 2 erwähnt definiert sind,
wobei die vorstehend unter R¹ erwähnten Heterocyclen im Kohlenstoffgerüst zusätzlich durch eine Methoxygruppe monosubstituiert sein können,
und wobei alle in den unter R¹ definierten Resten erwähnten oder enthaltenen aromatischen und heteroaromatischen Reste und Molekülteile zusätzlich durch Halogenatome, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

17. CGRP-Antagonisten der allgemeinen Formel (I) gemäß Anspruch 1, in denen
A und X jeweils ein Sauerstoffatom bedeuten,
R¹ wie in Anspruch 5 definiert ist,
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe und
R^{a}, R^{b} und R^{c} unabhängig voneinander jeweils ein Wasserstoff- oder Halogenatom, eine Methyl-, Difluormethyl-, Trifluormethyl-, Ethyl-, Vinyl-, Ethinyl-, Cyano-, Hydroxy-, Methoxy-, Difluormethoxy-, Trifluormethoxy-, Amino-, Methylamino- oder Dimethylaminogruppe darstellen,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

18. CGRP-Antagonisten der allgemeinen Formel (I) gemäß Anspruch 1, in denen
A und X jeweils ein Sauerstoffatom,
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe,
R^{a}, R^{b} und R^{c} unabhängig voneinander jeweils ein Wasserstoff- oder Halogenatom, eine C₁₋₃-Alkyl-, Trifluormethyl-, Cyano-, Hydroxy-, Methoxy-, Trifluormethoxy-, Amino-, Methylamino- oder Dimethylaminogruppe,
R¹ einen einfach ungesättigten, über ein Stickstoffatom mit dem Piperidinring in Formel (I) verknüpften 5- bis 7-gliedrigen Diaza-Hoterocyclus,
wobei der vorstehend erwähnte Heterocyclus eine Carbonylgruppe benachbart zu einem Stickstoffatom enthält und die Carbonylgruppe vorzugsweise mit zwei Stickstoffatomen verknüpft ist sowie
die olefinische Doppelbindung des Heterocyclus mit einem Phenyl- oder Thienyl-Ring kondensiert ist und der Phenyl- und Thienyl-Ring durch Halogenatome, durch Methyl-, Methoxy-, Difluormethyl-, Trifluormethyl-, Hydroxy-, Amino-, C₁₋₃-Alkylamino-, Di-(C₁₋₃-alkyl)-amino-, Acetylamino-, Acetyl-, Cyano-Difluormethoxy- oder Trifluormethoxygruppen mono-, di- oder trisubstituiert sein kann, wobei die Substituenten gleich oder verschieden sein können, vorzugsweise jedoch unsubstituiert oder durch ein Halogenatom, durch eine Methyl- oder Methoxygruppe monosubstituiert ist,
und für R¹ beispielsweise eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-c]chinolin-2-on-3-yl-, 1,3-Dihydro-naphth[1,2-*d*]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H*-thieno[3,2-*d*]pyrimidin-2-on-3-yl- oder 3,4-Dihydro-1*H*-thieno[3,4-*d*]pyrimidin-2-on-3-ylgruppe genannt seien, die an einem ungesättigten Kohlenstoffatom des aromatischen oder heteroaromatischen Teils durch Halogenatome, durch Cyano- oder Hydroxygruppen mono-, di- oder trisubstituiert und die Substituenten gleich oder verschieden sein können, vorzugsweise jedoch unsubstituiert sind,
R² und R³ zusammen mit dem eingeschlossenen Stickstoffatom einen Rest der allgemeinen Formel 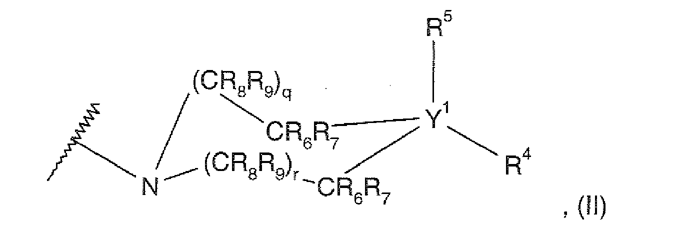 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, wobei die Summe aus q und r 1, 2 oder 3 beträgt,
q und r, wenn Y¹ das Stickstoffatom darstellt, die Zahlen 1 oder 2, wobei die Summe aus q und r 2 oder 3 beträgt,
R⁴ das Wasserstoffatom, eine Amino-, C₁₋₄-Alkyl-amino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl-, Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl- oder Pyridylgruppe, die jeweils durch ein Halogenatom, durch eine C₁₋₃-Alkyl-, C₁₋₃-Alkoxy-, Amino-, C₁₋₄-Alkyl-amino-, oder Di-(C₁₋₄-alkyl)-aminogruppe substituiert sein kann,
ein Heterocyclus ausgewählt aus einer 5- bis 7-gliedrigen Azacycloalkyl- oder *S*,*S*-Dioxothiazagruppe und einer 6- bis 7-gliedrigen Diazacycloalkylgruppe,
wobei die vorstehend genannten Heterocyclen über ein Stickstoff- oder ein Kohlenstoffatom an Y¹ in Formel (II) gebunden sind und
durch eine oder zwei Hydroxy-, C₁₋₃-Alkyl- oder Hydroxy-C₁₋₃-alkylgruppen oder durch eine Cyclo-C₃₋₆-alkyl-, Hydroxy-C₃₋₆-cycloalkyl-, Cyclo-C₃₋₆-alkyl-C₁₋₃-alkyl, C₁₋₃-Alkylcarbonyl-C₁₋₃-alkyl-, Amino-, C₁₋₄-Alkylamino-, Di-(C₁₋₄-alkyl)-amino-, Hydroxycarbonyl-carbonyl-, C₁₋₃-Alkoxycarbonyl-carbonyl-, Hydroxycarbonyl-C₁₋₃-alkylcarbonyl-, C₁₋₃-Alkoxycarbonyl-C₁₋₃-alkylcarbonyl-, Aminosulfonyl-, C₁₋₄-Alkylaminosulfonyl-, Di-(C₁₋₄-alkyl)-aminosulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)-aminocarbonyl-C₁₋₃-alkyl-, Hydroxyaminocarbonyl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppen substituiert sein können,
R⁵ ein Wasserstoffatom oder, wenn Y¹ ein Stickstoffatom darstellt, auch ein freies Elektronenpaar und
R⁶, R⁷, R⁸ und R⁹, die gleich oder verschieden sein können, jeweils ein Wasserstoffatom oder eine C₁₋₃-Alkylgruppe darstellen,
bedeuten, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

19. CGRP-Antagonisten der allgemeinen Formel (I) gemäß Anspruch 1, in denen
A und X jeweils ein Sauerstoffatom,
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe,
R^{a}, R^{b} und R^{c} unabhängig voneinander jeweils ein Wasserstoff- oder Halogenatom, eine C₁₋₃-Alkyl-, Trifluormethyl-, Cyano-, Hydroxy-, Methoxy-, Trifluormethoxy-, Amino-, Methylamino- oder Dimethylaminogruppe,
R¹ einen einfach ungesättigten, über ein Stickstoffatom mit dem Piperidinring in Formel (I) verknüpften 5- bis 7-gliedrigen Diaza-Heterocyclus,
wobei der vorstehend erwähnte Heterocyclus eine Carbonylgruppe benachbart zu einem Stickstoffatom enthält und die Carbonylgruppe vorzugsweise mit zwei Stickstoffatomen verknüpft ist sowie
die olefinische Doppelbindung des Heterocyclus mit einem Phenyl- oder Thienyl-Ring kondensiert ist und der Phenyl- und Thienyl-Ring durch Halogenatome, durch Methyl-, Methoxy-, Difluormethyl-, Trifluormethyl-, Hydroxy-, Amino-, C₁₋₃-Alkylamino-, Di-(C₁₋₃-alkyl)-amino-, Acetylamino-, Acetyl-, Cyano-Difluormethoxy- oder Trifluormethoxygruppen mono-, di- oder trisubstituiert sein kann, wobei die Substituenten gleich oder verschieden sein können, vorzugsweise jedoch unsubstituiert oder durch ein Halogenatom, durch eine Methyl- oder Methoxygruppe monosubstituiert ist,
und für R¹ beispielsweise eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-*c*]chinolin-2-on-3-yl-, 1,3-Dihydro-naphth[1,2-*d*]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H*-thieno[3,2-*d*]pyrimidin-2-on-3-yl- oder 3,4-Dihydro-1*H*-thieno[3,4-*d*]pyrimidin-2-on-3-ylgruppe genannt seien, die an einem ungesättigten Kohlenstoffatom des aromatischen oder heteroaromatischen Teils durch Halogenatome, durch Cyano- oder Hydroxygruppen mono-, di- oder trisubstituiert und die Substituenten gleich oder verschieden sein können, vorzugsweise jedoch unsubstituiert sind,
R² und R³ zusammen mit dem eingeschlossenen Stickstoffatom einen Rest der allgemeinen Formel 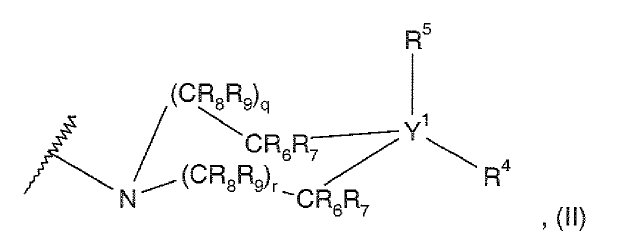 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, wobei die Summe aus q und r 1, 2 oder 3 beträgt,
q und r, wenn Y¹ das Stickstoffatom darstellt, die Zahlen 1 oder 2, wobei die Summe aus q und r 2 oder 3 beträgt,
R⁴ das Wasserstoffatom, eine Amino-, C₁₋₄-Alkyl-amino-, Di-(C₁₋₄-alkyl)-alkylamin-, C₁₋₆-Alkyl-, Cyclo-C₃₋₇-alkyl-, Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl- oder Pyridylgruppe, die jeweils durch ein Halogenatom, durch eine C₁₋₃-Alkyl-, C₁₋₃-Alkoxy-, Amin-, C₁₋₄-Alkyl-amino-, oder Di-(C₁₋₄-alkyl)-aminogruppe substituiert sein kann,
ein Heterocyclus ausgewählt aus einer 5- bis 7-gliedrigen Azacycloalkylgruppe und einer 6- bis 7-gliedrigen Diazacycloalkylgruppe,
wobei die vorstehend genannten Heterocyclen über ein Stickstoff- oder ein Kohlenstoffatom an Y¹ in Formel (II) gebunden sind und
durch eine C₁₋₃-Alkyl-, Cyclo-C₃₋₆-alkyl-, Cyclo-C₃₋₆-alkyl-C₁₋₃-alkyl, Amino-, C₁₋₄-Alkylamino- oder Di-(C₁₋₄-alkyl)-aminogruppen substituiert sein können,
R⁵ ein Wasserstoffatom oder, wenn Y¹ ein Stickstoffatom darstellt, auch ein freies Elektronenpaar und
R⁶, R⁷, R⁸ und R⁹, die gleich oder verschieden sein können, jeweils ein Wasserstoffatom oder eine C₁₋₃-Alkylgruppe darstellen,
bedeuten, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

20. Physiologisch verträgliche Salze der Verbindungen nach einem der Ansprüche 1 bis 19 mit anorganischen oder organischen Säuren oder Basen.

21. Arzneimittel, enthaltend eine Verbindung nach einem der Ansprüche 1 bis 19 oder ein physiologisch verträgliches Salz gemäß Anspruch 20 neben gegebenenfalls einem oder mehreren inerten Trägerstoffen und/oder Verdünnungsmitteln.

22. Verwendung einer Verbindung nach einem der Ansprüche 1 bis 20 zur Herstellung eines Arzneimittels zur akuten und prophylaktischen Behandlung von Kopfschmerzen, insbesondere Migräne- bzw. Cluster-Kopfschmerz, zur Behandlung von nicht-insulinabhängigem Diabetes mellitus ("NIDDM"), des complex regional pain syndrome (CRPS1), cardiovaskulärer Erkrankungen, Morphintoleranz, Clostritiumtoxin-bedingten Durchfallerkrankungen, Erkrankungen der Haut, insbesondere thermische und strahlenbedingte Hautschäden inklusive Sonnenbrand, entzündlicher Erkrankungen, z.B. entzündlicher Gelenkerkrankungen (Arthritis), neurogenen Entzündungen der oralen Mucosa, entzündlichen Lungenerkrankungen, allergischer Rhinitis, Asthma, Erkrankungen, die mit einer überschießenden Gefäßerweiterung und **dadurch** bedingter verringerter Gewebedurchblutung einhergehen, z.B. Schock und Sepsis, zur Linderung von Schmerzzuständen, oder zur präventiven oder akut-therapeutisch Behandlung der Symptomatik menopausaler, durch Gefäßerweiterung und erhöhten Blutfluss verursachter Hitzewallungen östrogendefizienter Frauen sowie hormonbehandelter Prostatakarzinompatienten.

23. Verfahren zur Herstellung eines Arzneimittels gemäß Anspruch 21, **dadurch gekennzeichnet, dass** auf nichtchemischem Weg eine Verbindung nach einem der Ansprüche 1 bis 20 in einen oder mehrere inerte Trägerstoffe und/oder Verdünnungsmittel eingearbeitet wird.

24. Verfahren zur Herstellung der Verbindungen der allgemeinen Formel (I) nach einem der Ansprüche 1 bis 20, **dadurch gekennzeichnet, dass**
(a) ein Piperidin der allgemeinen Formel 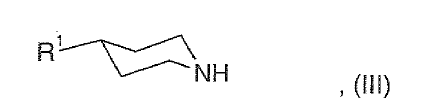 in der R¹ wie im Anspruch 1 definiert ist,
mit einem Kohlensäurederivat der allgemeinen Formel 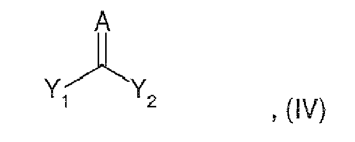 in der A wie im Anspruch 1 definiert ist und Y₁ und Y₂ nucleofuge Gruppen bedeuten,
sowie mit einer Verbindung der allgemeinen Formel 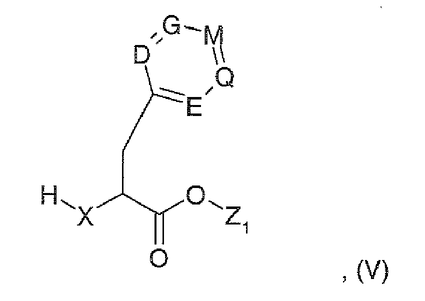 in der X, D, E, G, M und Q wie im Anspruch 1 definiert sind und Z₁ eine Schutzgruppe für eine Carboxygruppe darstellt, umgesetzt wird, wobei vor Durchführung der Reaktion im Rest R¹ einer Verbindung der Formel (III) und/oder in einer Verbindung der Formel (V) gegebenenfalls vorhandene Carbonsäurefunktionen, primäre oder sekundäre Aminofunktionen oder Hydroxyfunktionen, falls erforderlich, durch Schutzreste geschützt und gegebenenfalls verwendete Schutzreste nach Durchführung der Reaktion wieder abgespalten werden, oder
(b) eine Carbonsäure der allgemeinen Formel 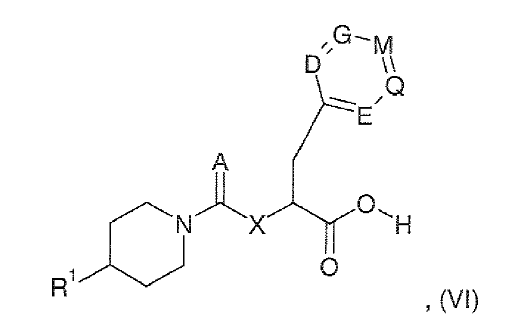 in der alle Reste wie im Anspruch 1 definiert sind, mit einem Amin der allgemeinen Formel HNR²R³, in der R² und R³ wie wie im Anspruch 1 definiert sind, gekuppelt wird, wobei vor Durchführung der Reaktion in einer Verbindung der Formel (VI) und/oder in den Resten R² und R³ des Amins der Formel HNR²R³ gegebenenfalls vorhandene Carbonsäurefunktionen, primäre oder sekundäre Aminofunktionen oder Hydroxyfunktionen, falls erforderlich, durch Schutzreste geschützt und gegebenenfalls verwendete Schutzreste nach Durchführung der Reaktion wieder abgespalten werden, oder
(c) eine Verbindung der allgemeinen Formel 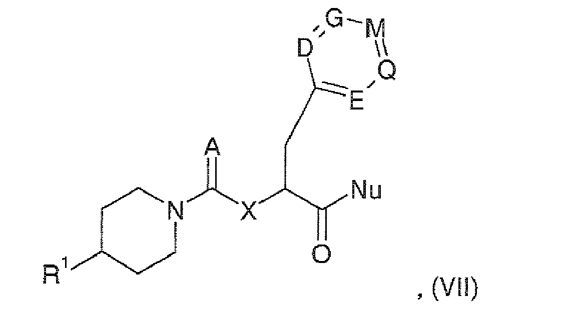 mit einem Amin der allgemeinen Formel HNR²R³, in denen alle Reste wie im Anspruch 1 definiert sind und Nu eine Austrittsgruppe, bedeutet, gekuppelt wird,
wobei vor Durchführung der Reaktion in einer Verbindung der Formel (VII) und/oder in den Resten R² und R³ des Amins der Formel HNR²R³ gegebenenfalls vorhandene Carbonsäurefunktionen, primäre oder sekundäre Aminofunktionen oder Hydroxyfunktionen, falls erforderlich, durch Schutzreste geschützt und gegebenenfalls verwendete Schutzreste nach Durchführung der Reaktion wieder abgespalten werden,
gewünschtenfalls eine so erhaltene Verbindung der allgemeinen Formel (I) in ihre Stereoisomere aufgetrennt wird und/oder
eine so erhaltene Verbindung der allgemeinen Formel (I) in ihre Salze, insbesondere für die pharmazeutische Anwendung in ihre physiologisch verträglichen Salze übergeführt wird.
